# FASCICULE DE BREVET EUROPEEN

(11) **EP 3 853 384 B1**
(45) Date de publication et mention de la délivrance du brevet: **26.11.2025**
(21) Numéro de dépôt: 19766293.5
(22) Date de dépôt: 17.09.2019
(51) Int. Cl.: C12Q 1/6895

(54) **UTILISATION DE SONDES POUR LA DETECTION D'ALGUES TOXIQUES, PROCEDE DE DETECTION ET KITS CORRESPONDANTS**
VERWENDUNG VON SONDEN ZUM NACHWEIS TOXISCHER ALGEN, NACHWEISVERFAHREN UND ENTSPRECHENDE KITS
USE OF PROBES TO DETECT TOXIC ALGAE, DETECTION METHOD AND CORRESPONDING KITS

(30) Priorité: 17.09.2018 FR 1858362
(43) Date de publication de la demande: 28.07.2021
(73) Titulaire: Microbia Environnement, 66300 Saint Jean Lasseille (FR)
(72) Inventeur: GUILLEBAULT, Delphine, 66300 SAINT-JEAN-LASEILLE (FR); VILLA, Elisa, 66660 PORT VENDRES (FR); MEDLIN CRAWFORD, Linda, Karen, 33890 PESSAC SUR DORDOGNE (FR)
(74) Mandataire: Grosset-Fournier, Chantal Catherine
(86) Numéro de dépôt international: PCT/EP2019/074910
(87) Numéro de publication internationale: WO 2020/058291

(56) Documents cités:
- EP-A2- 1 516 067
- EP-A2- 1 516 067
- WO-A1-2015/008011
- DE-A1- 10 228 785
- DE-A1- 10 228 785
- JP-A- 2004 298 103
- US-B2- 10 294 533
- US-B2- 10 294 533
- YANG JI-FEI ET AL: "Molecular evidence for piroplasms in wild Reeves' muntjac (Muntiacus reevesi) in China", PARASITOLOGY INTERNATIONAL, vol. 63, no. 5, 1 October 2014 (2014-10-01), AMSTERDAM, NL, pages 713 - 716, XP055917797, ISSN: 1383-5769, DOI: 10.1016/j.parint.2014.06.002
- MEDLIN LINDA K. ET AL: "Advances in the Detection of Toxic Algae Using Electrochemical Biosensors", BIOSENSORS, vol. 10, no. 12, 16 December 2020 (2020-12-16), pages 207, XP055918637, DOI: 10.3390/bios10120207
- MEDLIN LINDA K. ET AL: "Advances in the Detection of Toxic Algae Using Electrochemical Biosensors", BIOSENSORS, vol. 10, no. 12, 16 December 2020 (2020-12-16), pages 207, XP055918637, DOI: 10.3390/bios10120207
- NOYER CHARLOTTE ET AL: "Phytochip: Development of a DNA-microarray for rapid and accurate identification ofPseudo-nitzschiaspp and other harmful algal species", JOURNAL OF MICROBIOLOGICAL METHODS, vol. 112, 10 March 2015 (2015-03-10), pages 55 - 66, XP029178366, ISSN: 0167-7012, DOI: 10.1016/J.MIMET.2015.03.002
- S. AHN ET AL: "Fiber-Optic Microarray for Simultaneous Detection of Multiple Harmful Algal Bloom Species", APPLIED AND ENVIRONMENTAL MICROBIOLOGY, vol. 72, no. 9, 1 September 2006 (2006-09-01), US, pages 5742 - 5749, XP055609519, ISSN: 0099-2240, DOI: 10.1128/AEM.00332-06
- SOOHYOUN AHN ET AL: "Detection of Salmonella spp. Using Microsphere-Based, Fiber-Optic DNA Microarrays", ANALYTICAL CHEMISTRY, vol. 77, no. 15, 1 August 2005 (2005-08-01), US, pages 5041 - 5047, XP055609608, ISSN: 0003-2700, DOI: 10.1021/ac0505270
- OROZCO JAHIR ET AL: "Electrochemical RNA genosensors for toxic algal species: enhancing selectivity and sensitivity", TALANTA, ELSEVIER, AMSTERDAM, NL, vol. 161, 30 August 2016 (2016-08-30), pages 560 - 566, XP029772052, ISSN: 0039-9140, DOI: 10.1016/J.TALANTA.2016.08.073

## Description

La présente invention a pour objet l'utilisation de sondes pour la détection de cellules vivantes actives d'algues toxiques, un procédé de détection de cellules vivantes actives d'algues toxiques et des kits correspondants.

Les eaux récréatives et les sites de production aquacoles sont fréquemment et de plus en plus touchés par des efflorescences d'algues toxiques. Ces dernières sont une réelle menace pour la santé humaine, les activités économiques et l'environnement car elles produisent des phycotoxines très nocives qui contaminent la chaîne alimentaire rendant les produits de la mer impropre à la consommation. La présence de ces phycotoxines menace également les écosystèmes et les activités économiques et touristiques.

Dans ce contexte, l'anticipation des risques est déterminante pour une croissance bleue durable, c'est à dire pour une économie aquacole, maritime et touristique pérenne. Une identification rapide, fiable et sensible des algues toxiques incriminées est essentielle pour une surveillance efficace et une anticipation des efflorescences dans les secteurs d'activités concernés.

Seulement, la composition de ces eaux environnementales d'intérêt dépend de facteurs biotiques et abiotiques (organismes présents, matières organiques diverses, pollution chimique ou biologique, *etc*.). Si bien que l'hétérogénéité en termes
- de diversité, *i.e.* variabilité en contenu d'organismes et de matières organique et inorganique) ; et
- d'état physiologique, e.g. une population d'un genre de micro-algues peut regrouper plusieurs espèces différentes et peut contenir tous les stades de vie d'une cellule,
va directement impacter et ce, de manière délétère la détection des algues toxiques incriminées.

Les méthodes actuelles de détection des algues toxiques sont essentiellement basées sur l'analyse par microscopie ou sur la technique d'hybridation sandwich. Toutefois, ces méthodes sont longues et fastidieuses. Leurs limites de détection vont de 1 000 à 3 000 cellules/L pour les algues *Chattonella, Fibrocapsa, Heterosigma, Olithodiscus, Nannochloropsis* (Compositions and methods for detecting raphidophytes, JV Tyrrell, PR Bergquist, PL Bergquist, CA Scholin, US Patent 6,787,648), jusqu'à 12 500 cellules/L *d'Alexandrium* (Colorimetric detection of the toxic Dinoflagellate Alexandrium minutum using sandwich hybridization in a microtiter plate assay, Sonja Diercks, Linda K Medlin, Katja Metfies - Harmful Algae, Vol. 7, Issue 2, Feb 2008, Pages 137-145) mais encore sont de 70 ng/µL d'ARN pour les algues *Gymnodinium, Prorocentrum, Lingulodinium, Prymnesium, Chrysochromulina et Pseudo-nitzschia,* (Molecular probe sets for the detection of toxic algae for use in sandwich hybridization formats, Sonja Diercks, Katja Metfies, Linda K. Medlin, Journal of Plankton Research, Vol. 30, Issue 4, 1 April 2008, Pages 439-448).

S. AHN et al enseigne un procédé de détection d'algues toxiques du genre Alexandrium par une technique d'hybridation sandwich utilisant un couple de sondes ("Fiber-Optic Microarray for Simultaneous Detection of Multiple Harmful Algal Bloom Species", APPLIED AND ENVIRONMENTAL MICROBIOLOGY, 2006-09-01, pages 5742-5749).

La technique d'hybridation sandwich (SHA) est connue et consiste en la détection d'un acide nucléique d'une espèce à détecter grâce, à la fois, à l'utilisation d'une sonde capture immobilisée sur un support solide et d'une sonde signal, toutes deux étant spécifiques de l'acide nucléique de l'espèce à détecter. La présence de l'acide nucléique à détecter entraîne la formation d'un complexe constitué de la sonde capture, l'acide nucléique de l'espèce à détecter et la sonde signal. Divers moyens de détection peuvent ensuite être utilisés afin de révéler la présence du complexe.

Il existe donc un besoin de techniques, rapides sensibles et fiables permettant de détecter la présence d'algues toxiques dans divers milieux aquatiques.

L'un des buts de l'invention est donc de fournir des outils fiables, sensibles et rapides pour la détection de cellules vivantes actives d'algues toxiques, lesquels surmontent et outrepassent efficacement la variabilité des eaux environnementales d'intérêt.

Un premier aspect de l'invention concerne l'utilisation de sondes nucléotidiques pour la mise en œuvre d'un procédé de détection de cellules vivantes actives d'algues toxiques, comme définie dans les revendications.

Un second aspect de l'invention concerne des couples de sondes pour la détection de cellules vivantes actives d'algues toxiques, comme définis dans les revendications.

Un troisième aspect ne faisant pas partie de l'invention concerne des sondes pour la détection de cellules vivantes actives d'algues toxiques.

Un quatrième aspect de l'invention concerne un procédé de détection de cellules vivantes actives d'algues toxiques, comme défini dans les revendications.

Un cinquième aspect de l'invention concerne des kits pour la détection de cellules vivantes actives d'algues toxiques, comme définis dans les revendications.

Un sixième aspect ne faisant pas partie de l'invention concerne des dispositifs pour la détection de cellules vivantes actives d'algues toxiques.

La présente invention est basée sur l'utilisation de sondes particulières et la mise en œuvre de la technique d'hybridation sandwich afin de détecter et quantifier spécifiquement les acides nucléiques de cellules vivantes actives d'algues toxiques éventuellement présents dans un milieu marin, saumâtre ou industriel à des seuils de détection et de quantification très bas et en un temps inférieur à 1 heure. Ainsi, la présente invention fourni un moyen d'alerte précoce permettant d'anticiper les efflorescences d'algues toxiques.

La nouveauté et le caractère inventif de l'invention réside dans la mise au point de sondes capables de reconnaitre et d'hybrider de façon très sensible les acides nucléiques ribosomiques (ARNr ou ADNr) de la grande ou des petites sous-unités des algues toxiques actives ciblées et ce, en moins d'une heure. Le fait de cibler les acides nucléiques ribosomiques permet de ne détecter que les cellules vivantes actives d'algues toxiques, c'est-à-dire les algues capables de croître et proliférer, représentant un risque potentiel toxique majeur. Ainsi, la présente invention permet de détecter les cellules vivantes actives d'algues toxiques. Grâce à une ou plusieurs courbe(s) de calibration, il est ensuite possible d'estimer le nombre de cellules vivantes actives d'algues toxiques présentes dans un échantillon en tenant compte de la phase de croissance et du type de cellule recherché (Taylor et al., Harmful Algae 37 (2014) 17-27; Yuji Tanaka and Makoto Tsuneoka (2018), Control of Ribosomal RNA Transcription by Nutrients).

On entend par « *algues toxiques* », les algues produisant des phycotoxines et provoquant des intoxications alimentaires, des paralysies, des amnésies, des irritations cutanées ou encore de la fièvre. Les algues toxiques peuvent être de différents genres. Les sondes utilisées dans la présente invention permettent de détecter les algues toxiques du genre *Alexandrium.*

On entend par « *cellules vivantes actives* », des cellules qui sont capables de croître et de se diviser quelles que soient les conditions environnementales et qui pourront se multiplier et proliférer rapidement dès que ces conditions environnementales seront favorables, à l'opposé des cellules en dormance qui ont une activité cellulaire minimale pour se protéger de conditions environnementales défavorables à leur développement, ou encore des cellules sénéscentes qui ont entamé un processus de mort cellulaire et qui voient leurs matériels cellulaire et génétique se dégrader.

Selon la présente invention, les termes « *algues toxiques* »et « *micro-algues toxiques* »sont utilisés indifféremment l'un de l'autre.

On entend par « *seuil de détection* »ou « *limite de détection* (LOD) », la plus petite quantité d'ARN d'algue toxique pouvant être détectée en mettant en œuvre la présente invention. Celle-ci est déterminée à partir d'une mesure d'absorbance à 450 nm ou 630 nm faite sur un blanc analytique dont est calculé l'écart type du signal, et correspond à la concentration d'ARN d'algue toxique qui produit un signal dont l'intensité est égale à 3 fois celle de l'écart type du blanc analytique. En d'autres termes, il s'agit de la valeur en-dessous de laquelle l'ARN d'algue toxique est considéré comme non détecté. (ACS (1980) Guidelines for Data Acquisition and Data Quality Evaluation in Environmental Chemistry, Analytical chemistry, 52, 14, 2242-2249).

On entend par « *seuil de quantification* »ou « *limite de quantification* (LOQ) », la plus petite quantité d'ARN d'algue toxique pouvant être quantifiée en mettant en œuvre la présente invention. Celle-ci est calculée en prenant comme valeur du signal, 10 fois la valeur de l'écart type du blanc analytique. En d'autres termes, il s'agit de la valeur en-dessous de laquelle il n'est pas possible de déterminer la quantité d'ARN d'algues toxiques. (ACS (1980) Guidelines for Data Acquisition and Data Quality Evaluation in Environmental Chemistry, Analytical chemistry, 52, 14, 2242-2249).

Ainsi dans un premier aspect, la présente invention concerne l'utilisation d'au moins un couple de sondes spécifiques d'algues toxiques pour la mise en œuvre d'un procédé de détection d'au moins une algue toxique du genre Alexandrium, comme définie dans les revendications.

Selon la présente invention, la limite de détection des algues listées ci-dessus est inférieure ou égale à 0,10 ng d'ARN par litre d'échantillon et en particulier est de 0,01 ng d'ARN par litre d'échantillon, ce qui correspond, selon le type d'algue à une limite de détection de 100 à 500 cellules vivantes actives par litre d'échantillon (cellules/L) et en particulier inférieure à 200 cellules vivantes actives par litre d'échantillon (cellules/L). Par ailleurs, selon la présente invention, la limite de quantification des algues listées ci-dessus est de 0,04 à 0,12 ng d'ARN par litre d'échantillon selon le type d'algue.

On entend par « *limite de détection [...] inférieure ou égale à 0,10 ng d'ARN par litre d'échantillon* », la concentration correspondante à la quantité de matériel minimale mesurée selon l'invention (*i.e.* '0,10 ng d'ARN') rapporté un volume d'un litre d'échantillon naturel brut prélevé *in situ.* Par conséquent, il ne s'agit pas de la concentration « chimique »mesurée dans un volume réactionnel suite à la mise en œuvre du procédé de l'invention pour mesurer la quantité d'ARN cible dans l'échantillon.

De la même façon, on entend par « *limite de détection [*...*] de 100 à 500 cellules vivantes actives par litre d'échantillon (cellules*/*L)* », la concentration cellulaire minimale **estimée** grâce à des courbes étalon qui peut être mesurée dans un volume d'un litre d'échantillon naturel brut prélevé *in situ.* Par conséquent, il ne s'agit pas de la concentration cellulaire mesurée dans un volume réactionnel suite à la mise en œuvre du procédé de l'invention, lequel implique un traitement, dont une lyse cellulaire, dudit échantillon brut prélevé *in situ.*

La présente invention utilisant une mesure d'absobance lue à 450 nm ou 630 nm à l'aide d'un spectrophotomètre, il convient de noter que des courbes de calibration peuvent être réalisées et utilisées pour passer d'une unité à l'autre (*e.g.* ng d'ARN par litre d'échantillon) à un équivalent cellulaire exprimé en cellules vivantes actives par litre d'échantillon (cellules/L).

Par ailleurs, il convient de noter qu'aux fins de l'invention, il est également possible d'utiliser des ARN synthétiques comme contrôle interne et outil de normalisation, lesdits ARN synthétiques (ou standard synthétique) étant des séquences oligonucléotidiques obtenues par synthèse chimique. Il est par ailleurs également entendu que les cultures de micro-algues utilisées ne sont pas axéniques et l'extraction des ARN totaux de la culture englobent à la fois les ARN totaux de l'algue cible mais aussi les ARN totaux des contaminants (*e.g*. bactéries) de la culture.

L'invention est telle que définie dans les revendications.

### Mode de réalisation A

Un mode de réalisation ne faisant pas partie de l'invention concerne l'utilisation d'au moins un couple de sondes spécifiques d'algues toxiques du genre ***Alexandrium*** pour la mise en œuvre d'un procédé de détection de cellules vivantes actives d'algues toxiques dans un échantillon susceptible de contenir au moins une algue toxique du genre ***Alexandrium,*** les séquences desdites sondes étant choisies parmi x éléments de l'un des ensembles suivants :
- (SEQ ID NO: 1, SEQ ID NO : 2 ou SEQ ID NO : 3)
- (SEQ ID NO : 4 et SEQ ID NO : 5)
- (SEQ ID NO : 6 et SEQ ID NO : 7)
- (SEQ ID NO : 8, SEQ ID NO : 9 ou SEQ ID NO : 10)
- (SEQ ID NO : 11, SEQ ID NO : 12 ou SEQ ID NO : 13)
- (SEQ ID NO : 14, SEQ ID NO : 15 ou SEQ ID NO : 16)
- (SEQ ID NO : 17, SEQ ID NO : 18 ou SEQ ID NO : 19)
- (SEQ ID NO : 20, SEQ ID NO : 21 ou SEQ ID NO : 22)
- (SEQ ID NO : 23, SEQ ID NO : 24 ou SEQ ID NO : 25)
- (SEQ ID NO : 26, SEQ ID NO : 27 ou SEQ ID NO : 28)

x étant 2 ou 3,
ou les séquences desdites sondes ayant au moins 92% d'identité avec les susdites séquences SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 25, SEQ ID NO : 26, SEQ ID NO : 27, SEQ ID NO : 28,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'acide nucléique ribosomique d'une algue toxique du genre ***Alexandrium*** éventuellement présent dans ledit échantillon afin de former un complexe,
le seuil de détection minimal de l'algue toxique du genre ***Alexandrium*** étant
   - de 100 à 500 cellules vivantes actives par litre d'échantillon (cellules/L) et en particulier inférieur à 200 cellules vivantes actives par litre d'échantillon (cellules/L), ou
   - inférieur ou égal à 0,10 ng d'ARN par litre d'échantillon et en particulier compris de 0,01 à 0,09 ng d'ARN par litre d'échantillon.

Comme indiqué précédemment, un seuil de détection minimal de l'algue toxique du genre ***Alexandrium*** inférieur à 120 cellules vivantes actives par litre d'échantillon correspond également à un seuil de détection minimal compris de 0,01 ng à 0,09 ng d'ARN par litre d'échantillon.

Par exemple, on entend par « *un couple de sondes, les séquences desdites sondes étant choisies parmi x éléments de l'un des ensembles suivants : (SEQ ID NO : 1, SEQ ID NO : 2 ou SEQ ID NO : 3)* », les couples de sondes suivant :
- SEQ ID NO : 1 et SEQ ID NO : 2
- SEQ ID NO : 1 et SEQ ID NO : 3
- SEQ ID NO : 2 et SEQ ID NO : 3.

De la même façon, on entend par « *un couple de sondes, les séquences desdites sondes étant choisies parmi x éléments de l'un des ensembles suivants : (SEQ ID NO : 4 et SEQ ID NO : 5)* », le couple de sondes suivant :
- SEQ ID NO : 4 et SEQ ID NO : 5.

De plus, on entend par « *inférieur à 200 cellules vivantes actives par litre d'échantillon (cellules*/*L)* », un seuil de détection minimal inférieur à 195, 190, 185, 180, 175, 170, 165, 160, 155, 150, 145, 140, 135, 130, 125, 120, 115, 110, 100, 95, 90, 85, 80, 75, 70, 65, 60, 55, 50, 45, 40, 35, 30, 25, 20, 15, 10, 5, 4, 3, 2 ou égal à 1 cellule(s) vivante(s) active(s) par litre d'échantillon (cellules/L). On entend également de cette expression un seuil de détection minimal compris de 1 à 200, de 1 à 50, de 50 à 100, de 100 à 150, de 150 à 200, de 50 à 200, de 100 à 200 ou de 150 à 200 cellule(s) vivante(s) active(s) par litre d'échantillon (cellules/L), ledit seuil de détection minimal pouvant alors être égal à 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 168, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199 ou 200 cellule(s) vivante(s) active(s) par litre d'échantillon (cellules/L).

De la même façon, on entend par « *inférieur ou égal à 0,10 ng d'ARN par litre d'échantillon (d'eau, de culture)* », un seuil de détection minimal inférieur à 0,09, 0,08, 0,07, 0,06, 0,05, 0,04, 0,03, 0,02 ou 0,01 ng d'ARN par litre d'échantillon. On entend également de cette expression un seuil de détection minimal compris de 0,01 à 0,10, de 0,01 à 0,09, de 0,05 à 0,10, 0,05 à 0,09 ou de 0,01 à 0,05 ng d'ARN par litre d'échantillon, ledit seuil de détection minimal pouvant alors égal à 0,01, 0,02, 0,03, 0,04, 0,05, 0,06, 0,07, 0,08, 0,09 ou 0,10 ng d'ARN par litre d'échantillon.

Le même raisonnement peut être appliqué à l'ensemble des aspects et modes de réalisation décrits.

Dans ce mode de réalisation, est également décrite l'utilisation d'au moins un couple de sondes spécifiques d'algues toxiques du genre ***Alexandrium*** pour la mise en œuvre d'un procédé de détection de cellules vivantes actives d'algues toxiques dans un échantillon susceptible de contenir au moins une algue toxique du genre ***Alexandrium,*** les séquences desdites sondes étant choisies parmi x éléments de l'un des ensembles suivants :
- (SEQ ID NO : 1, SEQ ID NO : 2 ou SEQ ID NO : 3)
- (SEQ ID NO : 4 et SEQ ID NO : 5)
- (SEQ ID NO : 6 et SEQ ID NO : 7)
- (SEQ ID NO : 8, SEQ ID NO : 9 ou SEQ ID NO : 10)
- (SEQ ID NO : 11, SEQ ID NO : 12 ou SEQ ID NO : 13)
- (SEQ ID NO : 14, SEQ ID NO : 15 ou SEQ ID NO : 16)
- (SEQ ID NO : 17, SEQ ID NO : 18 ou SEQ ID NO : 19)
- (SEQ ID NO : 20, SEQ ID NO : 21 ou SEQ ID NO : 22),
- (SEQ ID NO : 23, SEQ ID NO : 24 ou SEQ ID NO : 25),
- (SEQ ID NO : 26, SEQ ID NO : 27 ou SEQ ID NO : 28)

x étant 2 ou 3,
ou les séquences desdites sondes ayant au moins 92% d'identité avec les susdites séquences SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 25, SEQ ID NO : 26, SEQ ID NO : 27, SEQ ID NO : 28,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'acide nucléique ribosomique d'une algue toxique du genre ***Alexandrium*** éventuellement présent dans ledit échantillon afin de former un complexe,
le seuil de détection minimal de l'algue toxique du genre ***Alexandrium*** étant
   - de 100 à 500 cellules vivantes actives par litre d'échantillon (cellules/L) et en particulier inférieur à 200 cellules vivantes actives par litre d'échantillon (cellules/L), ou
   - inférieur ou égal à 0,10 ng d'ARN par litre d'échantillon et en particulier compris de 0,01 à 0,09 ng d'ARN par litre d'échantillon,
la durée de la mise en œuvre dudit procédé de détection étant inférieure à une heure.

Dans ce mode de réalisation, est aussi décrite l'utilisation d'au moins un couple de sondes spécifiques d'algues toxiques du genre ***Alexandrium*** tel que décrit précédemment dans laquelle le seuil de détection minimal de l'algue toxique du genre ***Alexandrium*** est inférieur à 120 cellules vivantes actives par litre d'échantillon (d'eau, de culture) (cellules/L) ou inférieur ou égal à 0,10 ng d'ARN par litre d'échantillon (d'eau, de culture) µLet la durée de la mise en œuvre dudit procédé de détection est inférieure à une heure.

Selon ce mode de réalisation, est également décrite l'utilisation d'au moins un couple de sondes spécifiques d'algues toxiques du genre ***Alexandrium*** telle que décrite précédemment pour la mise en œuvre d'un procédé de détection de cellules vivantes actives d'algues toxiques dans un échantillon susceptible de contenir au moins une algue toxique du genre ***Alexandrium,*** dans laquelle les séquences des sondes desdits couples sont les suivants :
- (SEQ ID NO: 1 et SEQ ID NO: 2), (SEQ ID NO : 1 et SEQ ID NO : 3), (SEQ ID NO : 2 et SEQ ID NO : 3)
- (SEQ ID NO : 4 et SEQ ID NO : 5)
- (SEQ ID NO : 6 et SEQ ID NO : 7)
- (SEQ ID NO : 8 et SEQ ID NO : 9), (SEQ ID NO : 8 et SEQ ID NO : 10), (SEQ ID NO : 9 et SEQ ID NO : 10)
- (SEQ ID NO : 11 et SEQ ID NO : 12), (SEQ ID NO : 11 et SEQ ID NO : 13), (SEQ ID NO : 12 et SEQ ID NO : 13)
- (SEQ ID NO : 14 et SEQ ID NO : 15), (SEQ ID NO : 14 et SEQ ID NO : 16), (SEQ ID NO : 15 et SEQ ID NO : 16)
- (SEQ ID NO : 17 et SEQ ID NO : 18), (SEQ ID NO : 17 et 19), (SEQ ID NO : 18 et SEQ ID NO : 19)
- (SEQ ID NO : 20 et SEQ ID NO : 21), (SEQ ID NO : 20 et SEQ ID NO : 22), (SEQ ID NO : 21 et SEQ ID NO : 22), ou
- (SEQ ID NO : 23 et SEQ ID NO : 24), (SEQ ID NO : 23 et SEQ ID NO : 25), (SEQ ID NO : 24 et SEQ ID NO : 25)
- (SEQ ID NO : 26 et SEQ ID NO : 27), (SEQ ID NO : 26 et SEQ ID NO : 28), (SEQ ID NO : 27 et SEQ ID NO : 28).

Selon un autre mode de réalisation, l'invention a pour objet l'utilisation d'au moins un couple de sondes spécifiques d'algues toxiques du genre *Alexandrium* pour la mise en œuvre d'un procédé de détection de cellules vivantes actives d'algues toxiques dans un échantillon naturel susceptible de contenir au moins une algue toxique du genre *Alexandrium,* ledit procédé étant réalisé à partir des ARN extraits totaux dudit échantillon naturel susceptible de contenir au moins une algue toxique du genre *Alexandrium* et dans lequel les séquences des sondes desdits couples étant les suivantes :
- (SEQ ID NO : 1 et SEQ ID NO : 2),
- (SEQ ID NO : 4 et SEQ ID NO : 5),
- (SEQ ID NO : 11 et SEQ ID NO : 12), ou
- (SEQ ID NO : 17 et SEQ ID NO : 18),

une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'ARN ribosomique d'une algue toxique du genre *Alexandrium* éventuellement présent dans ledit échantillon afin de former un complexe.

En plus de la détection d'algues toxiques du genre *Alexandrium,* la présente invention concerne en plus de l'utilisation d'au moins un couple de sondes spécifiques d'algues toxiques du genre *Alexandrium,* l'utilisation d'au moins un couple de sondes spécifiques d'algues toxiques choisis parmi le groupe consistant en *Dinophysis, Pseudo-nitzschia, Prorocentrum, Chattonella, Gymnodinium, Karenia, Lingulodinium* et *Heterosigma.*

### Mode de réalisation B

Ainsi, l'invention concerne également l'utilisation telle que décrite dans la revendication 1 pour la détection de cellules vivantes actives d'algues toxiques du genre *Alexandrium* comprenant **en plus** l'utilisation d'au moins un couple de sondes spécifiques d'algues toxiques du genre ***Dinophysis*** pour la mise en œuvre d'un procédé de détection de cellules vivantes actives d'algues toxiques dans un échantillon susceptible de contenir au moins une algue toxique du genre ***Alexandrium*** et/ou ***Dinophysis,*** les séquences desdites sondes étant choisies parmi x éléments de l'un des ensembles suivants :
- (SEQ ID NO : 29, SEQ ID NO : 30 ou SEQ ID NO : 31)
- (SEQ ID NO : 32 et SEQ ID NO : 33)
- (SEQ ID NO : 34 et SEQ ID NO : 35)
- (SEQ ID NO : 36 et SEQ ID NO : 37)
- (SEQ ID NO : 38 et SEQ ID NO : 39)
- (SEQ ID NO : 40, SEQ ID NO : 41 ou SEQ ID NO : 42)
- (SEQ ID NO : 43, SEQ ID NO : 44 ou SEQ ID NO : 45), ou
- (SEQ ID NO : 46, SEQ ID NO : 47 ou SEQ ID NO : 48)

x étant 2 ou 3,
ou les séquences desdites sondes ayant au moins 92% d'identité avec les susdites séquences SEQ ID NO : 29, SEQ ID NO : 30, SEQ ID NO : 31, SEQ ID NO : 32, SEQ ID NO : 33, SEQ ID NO : 34, SEQ ID NO : 35, SEQ ID NO : 36, SEQ ID NO : 37, SEQ ID NO : 38, SEQ ID NO : 39, SEQ ID NO : 40, SEQ ID NO : 41, SEQ ID NO : 42, SEQ ID NO : 43, SEQ ID NO : 44, SEQ ID NO : 45, SEQ ID NO : 46, SEQ ID NO : 47 ou SEQ ID NO : 48,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'acide nucléique ribosomique d'une algue toxique du genre ***Dinophysis*** éventuellement présent dans ledit échantillon afin de former un complexe.

Un autre mode de réalisation décrit mais ne faisant pas partie de l'invention concerne l'utilisation d'au moins un couple de sondes spécifiques d'algues toxiques du genre ***Dinophysis*** telle que décrite précédemment pour la mise en œuvre d'un procédé de détection de cellules vivantes actives d'algues toxiques dans un échantillon susceptible de contenir au moins une algue toxique du genre ***Dinophysis,*** dans laquelle les séquences des sondes desdits couples sont les suivants :
- (SEQ ID NO : 29 et SEQ ID NO : 30), (SEQ ID NO : 29 et SEQ ID NO : 31), (SEQ ID NO : 30 et SEQ ID NO : 31)
- (SEQ ID NO : 32 et SEQ ID NO : 33)
- (SEQ ID NO : 34 et SEQ ID NO : 35)
- (SEQ ID NO : 36 et SEQ ID NO : 37)
- (SEQ ID NO : 38 et SEQ ID NO : 39)
- (SEQ ID NO : 40 et SEQ ID NO : 41), (SEQ ID NO : 40 et SEQ ID NO : 42), (SEQ ID NO : 41 et SEQ ID NO : 42)
- (SEQ ID NO : 43 et SEQ ID NO : 44), (SEQ ID NO : 43 et SEQ ID NO : 45), (SEQ ID NO : 44 et SEQ ID NO : 45)
- (SEQ ID NO : 46 et SEQ ID NO : 47), (SEQ ID NO : 46 et SEQ ID NO : 48), (SEQ ID NO : 47 et SEQ ID NO : 48.

Comme précédemment pour la détection d'*Alexandrium* selon le mode de réalisation A, et dans un mode de réalisation particulier, un seuil de détection minimal de l'algue toxique du genre ***Dinophysis*** inférieur à 200 cellules vivantes actives par litre d'échantillon (cellules/L), correspond également à un seuil de détection minimal compris de 0,01 à 0,09 ng d'ARN par litre d'échantillon.

De la même façon, dans un mode de réalisation particulier, la durée de la mise en œuvre dudit procédé de détection est inférieure à une heure.

Ainsi, le mode de réalisation B permet la détection de *Alexandrium* et *Dinophysis* grâce à l'utilisation de sondes spécifiques d'algues toxiques du genre *Alexandrium* et *Dinophysis.*

### Mode de réalisation C

De la même façon, est décrite l'une des utilisations telles que décrites précédemment selon le mode de réalisation A ou selon le mode de réalisation B, comprenant **en plus** l'utilisation d'au moins un couple de sondes spécifiques d'algues toxiques du genre ***Pseudo-nitzschia*** pour la mise en œuvre d'un procédé de détection de cellules vivantes actives d'algues toxiques dans un échantillon susceptible de contenir au moins une algue toxique du genre ***Alexandrium*** et/ou *Pseudo-nitzschia,* les séquences desdites sondes étant choisies parmi x éléments de l'un des ensembles suivants :
- (SEQ ID NO : 49 et SEQ ID NO : 50)
- (SEQ ID NO : 51 et SEQ ID NO : 52)
- (SEQ ID NO : 53, SEQ ID NO : 54 ou SEQ ID NO : 55)
- (SEQ ID NO : 56, SEQ ID NO : 57, SEQ ID NO : 58)
- (SEQ ID NO : 59, SEQ ID NO : 60, SEQ ID NO : 61)

x étant 2 ou 3,
ou les séquences desdites sondes ayant au moins 92% d'identité avec les susdites séquences SEQ ID NO : 49, SEQ ID NO : 50, SEQ ID NO : 51, SEQ ID NO : 52, SEQ ID NO : 53, SEQ ID NO : 54, SEQ ID NO : 55, SEQ ID NO : 56, SEQ ID NO : 57, SEQ ID NO : 58, SEQ ID NO : 59, SEQ ID NO : 60, SEQ ID NO : 61,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'acide nucléique ribosomique d'une algue toxique du genre ***Pseudo-nitzschia*** éventuellement présent dans ledit échantillon afin de former un complexe,
le seuil de détection minimal de l'algue toxique du genre ***Pseudo-nitzschia*** étant
   - de 100 à 500 cellules vivantes actives par litre d'échantillon (cellules/L) et en particulier inférieur à 200 cellules vivantes actives par litre d'échantillon (cellules/L), ou
   - inférieur ou égal à 0,10 ng d'ARN par litre d'échantillon et en particulier compris de 0,01 à 0,09 ng d'ARN par litre d'échantillon.

Dans un mode de réalisation particulier, est décrite l'utilisation d'au moins un couple de sondes spécifiques d'algues toxiques du genre ***Pseudo-nitzschia*** telle que décrite précédemment pour la mise en œuvre d'un procédé de détection de cellules vivantes actives d'algues toxiques dans un échantillon susceptible de contenir au moins une algue toxique du genre ***Pseudo-nitzschia*** dans laquelle les séquences des sondes desdits couples sont les suivants :
- (SEQ ID NO : 49 et SEQ ID NO : 50)
- (SEQ ID NO : 51 et SEQ ID NO : 52)
- (SEQ ID NO : 53 et SEQ ID NO : 54), (SEQ ID NO : 53 et SEQ ID NO : 55), (SEQ ID NO : 54 et SEQ ID NO : 55)
- (SEQ ID NO : 56 et SEQ ID NO : 57), (SEQ ID NO : 56 et SEQ ID NO : 58), (SEQ ID NO : 57 et SEQ ID NO : 58)
- (SEQ ID NO : 59 et SEQ ID NO : 60), (SEQ ID NO : 59 et SEQ ID NO : 61), (SEQ ID NO : 60 et SEQ ID NO : 61).

Comme précédemment pour les modes de réalisation A et B, et dans un mode de réalisation particulier, un seuil de détection minimal de l'algue toxique du genre ***Pseudo-nitzschia*** inférieur à 200 cellules vivantes actives par litre d'échantillon (cellules/L), correspond également à un seuil de détection minimal compris de 0,01 à 0,09 ng d'ARN par litre d'échantillon et en particulier de 0,01 ng d'ARN par litre d'échantillon.

De la même façon, dans un mode de réalisation particulier, la durée de la mise en œuvre dudit procédé de détection est inférieure à une heure.

Ainsi, le mode de réalisation C permet la détection :
- C1 : d'*Alexandrium* et *Pseudo-nitzschia* (combinaison avec le mode A)
- C2 : d'*Alexandrium, Dinophysis* et *Pseudo-nitzschia* (combinaison avec le mode B) grâce à l'utilisation de sondes spécifiques d'algues toxiques du genre *Alexandrium, Dinophysis* et *Pseudo-nitzschia.*

### Mode de réalisation D

De la même façon, est décrite l'une des utilisations telles que décrites précédemment selon les modes de réalisation A, B ou C, comprenant **en plus** l'utilisation d'au moins un couple de sondes spécifiques d'algues toxiques du genre ***Prorocentrum*** pour la mise en œuvre d'un procédé de détection de cellules vivantes actives d'algues toxiques dans un échantillon susceptible de contenir au moins une algue toxique du genre ***Alexandrium*** et/ou ***Prorocentrum,*** les séquences desdites sondes étant choisies parmi x éléments de l'un des ensembles suivants :
- (SEQ ID NO : 62, SEQ ID NO : 63 ou SEQ ID NO : 64)
- (SEQ ID NO : 65, SEQ ID NO : 66 ou SEQ ID NO : 67)
- (SEQ ID NO : 68, SEQ ID NO : 69 ou SEQ ID NO : 70)
- (SEQ ID NO : 71, SEQ ID NO : 72 ou SEQ ID NO : 73)

x étant 3,
ou les séquences desdites sondes ayant au moins 92% d'identité avec les susdites séquences SEQ ID NO : 62, SEQ ID NO : 63, SEQ ID NO : 64, SEQ ID NO : 65, SEQ ID NO : 66, SEQ ID NO : 67, SEQ ID NO : 68, SEQ ID NO : 69, SEQ ID NO : 70, SEQ ID NO : 71, SEQ ID NO : 72 ou SEQ ID NO : 73,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'acide nucléique ribosomique d'une algue toxique du genre ***Prorocentrum*** éventuellement présent dans ledit échantillon afin de former un complexe,
le seuil de détection minimal de l'algue toxique du genre ***Prorocentrum*** étant
   - de 100 à 500 cellules vivantes actives par litre d'échantillon (cellules/L) et en particulier inférieur à 200 cellules vivantes actives par litre d'échantillon (cellules/L), ou
   - inférieur ou égal à 0,10 ng d'ARN par litre d'échantillon et en particulier compris de 0,01 à 0,09 ng d'ARN par litre d'échantillon.

Dans un mode de réalisation particulier, est décrite l'utilisation d'au moins un couple de sondes spécifiques d'algues toxiques du genre ***Prorocentrum*** telle que décrite précédemment pour la mise en œuvre d'un procédé de détection de cellules vivantes actives d'algues toxiques dans un échantillon susceptible de contenir au moins une algue toxique du genre ***Prorocentrum*** dans laquelle les séquences des sondes desdits couples sont les suivants :
- (SEQ ID NO : 62 et SEQ ID NO : 63), (SEQ ID NO : 62 et SEQ ID NO : 64), (SEQ ID NO : 63 et SEQ ID NO : 64)
- (SEQ ID NO : 65 et SEQ ID NO : 66), (SEQ ID NO : 65 et SEQ ID NO : 67), (SEQ ID NO : 66 et SEQ ID NO : 67)
- (SEQ ID NO : 68 et SEQ ID NO : 69), (SEQ ID NO : 68 et SEQ ID NO : 70), (SEQ ID NO : 69 et SEQ ID NO : 70)
- (SEQ ID NO : 71 et SEQ ID NO : 72), (SEQ ID NO : 71 et SEQ ID NO : 73), (SEQ ID NO : 72 et SEQ ID NO : 73)

Comme précédemment pour les modes de réalisation A, B et C, et dans un mode de réalisation particulier, un seuil de détection minimal de l'algue toxique du genre ***Prorocentrum*** inférieur à 200 cellules vivantes actives par litre d'échantillon (cellules/L) correspond également à un seuil de détection minimal compris de 0,01 ng à 0,09 ng d'ARN par litre d'échantillon.

De la même façon, dans un mode de réalisation particulier, la durée de la mise en œuvre dudit procédé de détection est inférieure à une heure.

Ainsi, le mode de réalisation D permet la détection :
- D1 : d'*Alexandrium* et *Prorocentrum* (combinaison avec le mode A)
- D2 : d'*Alexandrium, Dinophysis* et *Prorocentrum* (combinaison avec le mode B)
- D3 : d'*Alexandrium, Pseudo-nitzschia et Prorocentrum* (combinaison avec le mode C1)
- D4 : d'*Alexandrium, Dinophysis, Pseudo-nitzschia* et *Prorocentrum* (combinaison avec le mode C2)
grâce à l'utilisation de sondes spécifiques d'algues toxiques du genre *Alexandrium, Dinophysis, Pseudo-nitzschia* et *Prorocentrum.*

### Mode de réalisation E

De la même façon, est décrite l'une des utilisations telles que décrites précédemment selon les modes de réalisation A, B, C ou D, comprenant **en plus** l'utilisation d'au moins un couple de sondes spécifiques d'algues toxiques du genre ***Chattonella*** pour la mise en œuvre d'un procédé de détection de cellules vivantes actives d'algues toxiques dans un échantillon susceptible de contenir au moins une algue toxique du genre ***Alexandrium*** et/ou ***Chattonella,*** les séquences desdites sondes étant choisies parmi x éléments de l'un des ensembles suivants :
- (SEQ ID NO : 74, SEQ ID NO : 75 ou SEQ ID NO : 76)
- (SEQ ID NO : 77, SEQ ID NO : 78 ou SEQ ID NO : 79)
- (SEQ ID NO : 80, SEQ ID NO : 81 ou SEQ ID NO : 82)

x étant 3,
ou les séquences desdites sondes ayant au moins 92% d'identité avec les susdites séquences SEQ ID NO : 74, SEQ ID NO : 75, SEQ ID NO : 76, SEQ ID NO : 77, SEQ ID NO : 78, SEQ ID NO : 79, SEQ ID NO : 80, SEQ ID NO : 81 ou SEQ ID NO : 82,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'acide nucléique ribosomique d'une algue toxique du genre ***Chattonella*** éventuellement présent dans ledit échantillon afin de former un complexe,
le seuil de détection minimal de l'algue toxique du genre ***Chattonella*** étant
   - de 100 à 500 cellules vivantes actives par litre d'échantillon (cellules/L) et en particulier inférieur à 200 cellules vivantes actives par litre d'échantillon (cellules/L), ou
   - inférieur ou égal à 0,10 ng d'ARN par litre d'échantillon et en particulier compris de 0,01 à 0,09 ng d'ARN par litre d'échantillon.

Dans un mode de réalisation particulier, est décrite l'utilisation d'au moins un couple de sondes spécifiques d'algues toxiques du genre ***Chattonella*** telle que décrite précédemment pour la mise en œuvre d'un procédé de détection de cellules vivantes actives d'algues dans un échantillon susceptible de contenir au moins une algue toxique du genre ***Chattonella*** dans laquelle les séquences des sondes desdits couples sont les suivants :
- (SEQ ID NO : 74 et SEQ ID NO : 75), (SEQ ID NO : 74 et SEQ ID NO : 76), (SEQ ID NO : 75 et SEQ ID NO : 76)
- (SEQ ID NO : 77 et SEQ ID NO : 78), (SEQ ID NO : 77 et SEQ ID NO : 79), (SEQ ID NO : 78 et SEQ ID NO : 79)
- (SEQ ID NO : 80 et SEQ ID NO : 81), (SEQ ID NO : 80 et SEQ ID NO : 82), (SEQ ID NO : 81 et SEQ ID NO : 82)

Comme précédemment pour les modes de réalisation A, B, C et D, et dans un mode de réalisation particulier, un seuil de détection minimal de l'algue toxique du genre ***Chattonella*** inférieur à 200 cellules vivantes actives par litre d'échantillon (cellules/L) correspond également à un seuil de détection minimal compris de 0,01 ng à 0,09 ng d'ARN par litre d'échantillon.

De la même façon, dans un mode de réalisation particulier, la durée de la mise en œuvre dudit procédé de détection est inférieure à une heure.

Ainsi, le mode de réalisation E permet la détection :
- E1 : d'*Alexandrium* et *Chattonella* (combinaison avec le mode A)
- E2 : d'*Alexandrium, Dinophysis* et *Chattonella* (combinaison avec le mode B)
- E3 : d'*Alexandrium, Pseudo-nitzschia* et *Chattonella* (combinaison avec le mode C1)
- E4 : d'*Alexandrium, Dinophysis, Pseudo-nitzschia* et *Chattonella* (combinaison avec le mode C2)
- E5 : d'*Alexandrium, Prorocentrum* et *Chattonella* (combinaison avec le mode D1)
- E6 : d'*Alexandrium, Dinophysis, Prorocentrum* et *Chattonella* (combinaison avec le mode D2)
- E7 : d'*Alexandrium, Pseudo-nitzschia, Prorocentrum* et *Chattonella* (combinaison avec le mode D3)
- E8 : *d'Alexandrium, Dinophysis, Pseudo-nitzschia, Prorocentrum* et *Chattonella* (combinaison avec le mode D4)
grâce à l'utilisation de sondes spécifiques d'algues toxiques du genre *Alexandrium, Dinophysis, Pseudo-nitzschia, Prorocentrum* et *Chattonella.*

### Mode de réalisation F

De la même façon, est décrite l'une des utilisations telles que décrites précédemment selon le mode de réalisation A, B, C, D ou E, comprenant **en plus** l'utilisation d'au moins un couple de sondes spécifiques d'algues toxiques du genre ***Gymnodinium*** pour la mise en œuvre d'un procédé de détection de cellules vivantes actives d'algues toxiques dans un échantillon susceptible de contenir au moins une algue toxique du genre ***Alexandrium*** et/ou ***Gymnodinium*** les séquences desdites sondes étant choisies parmi x éléments de l'un des ensembles suivants :
- (SEQ ID NO : 83, SEQ ID NO : 84 ou SEQ ID NO : 85)
- (SEQ ID NO : 86, SEQ ID NO : 87 ou SEQ ID NO : 88)
- (SEQ ID NO : 89, SEQ ID NO : 90 ou SEQ ID NO : 91)
- (SEQ ID NO : 92, SEQ ID NO : 93 ou SEQ ID NO : 94)

x étant 3,
ou les séquences desdites sondes ayant au moins 92% d'identité avec les susdites séquences SEQ ID NO : 83, SEQ ID NO : 84, SEQ ID NO : 85, SEQ ID NO : 6, SEQ ID NO : 87, SEQ ID NO : 88, SEQ ID NO : 89, SEQ ID NO : 90, SEQ ID NO : 91, SEQ ID NO : 92, SEQ ID NO : 93 ou SEQ ID NO : 94,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'acide nucléique ribosomique d'une algue toxique du genre ***Gymnodinium*** éventuellement présent dans ledit échantillon afin de former un complexe,
le seuil de détection minimal de l'algue toxique du genre ***Gymnodinium*** étant
   - de 100 à 500 cellules vivantes actives par litre d'échantillon (cellules/L) et en particulier inférieur à 200 cellules vivantes actives par litre d'échantillon (cellules/L), ou
   - inférieur ou égal à 0,10 ng d'ARN par litre d'échantillon et en particulier compris de 0,01 à 0,09 ng d'ARN par litre d'échantillon.

Dans un mode de réalisation particulier, est décrite l'utilisation d'au moins un couple de sondes spécifiques d'algues toxiques du genre ***Gymnodinium*** telle que décrite précédemment pour la mise en œuvre d'un procédé de détection de cellules vivantes actives d'algues toxiques dans un échantillon susceptible de contenir au moins une algue toxique du genre ***Gymnodinium*** dans laquelle les séquences des sondes desdits couples sont les suivants :
- (SEQ ID NO : 83 et SEQ ID NO : 84), (SEQ ID NO : 83 et SEQ ID NO : 85), (SEQ ID NO : 84 et SEQ ID NO : 85)
- (SEQ ID NO : 86 et SEQ ID NO : 87), (SEQ ID NO : 86 et SEQ ID NO : 88), (SEQ ID NO : 87 et SEQ ID NO : 88)
- (SEQ ID NO : 89 et SEQ ID NO : 90), (SEQ ID NO : 89 et SEQ ID NO : 91), (SEQ ID NO : 90 et SEQ ID NO : 91)
- (SEQ ID NO : 92 et SEQ ID NO : 93), (SEQ ID NO : 92 et SEQ ID NO : 94), (SEQ ID NO : 93 et SEQ ID NO : 94).

Comme précédemment pour les modes de réalisation A, B, C, D et E, et dans un mode de réalisation particulier, un seuil de détection minimal de l'algue toxique du genre ***Gymnodinium*** inférieur à 200 cellules vivantes actives par litre d'échantillon (cellules/L) correspond également à un seuil de détection minimal compris de 0,01 ng à 0,09 ng d'ARN par litre d'échantillon.

De la même façon, dans un mode de réalisation particulier, la durée de la mise en œuvre dudit procédé de détection est inférieure à une heure.

Ainsi, le mode de réalisation F permet la détection :
- F1 : d'*Alexandrium* et *Gymnodinium* (combinaison avec le mode A)
- F2 : d'*Alexandrium, Dinophysis* et *Gymnodinium* (combinaison avec le mode B)
- F3 : d'*Alexandrium, Pseudo-nitzschia* et *Gymnodinium* (combinaison avec le mode C1)
- F4 : d'*Alexandrium, Dinophysis, Pseudo-nitzschia* et *Gymnodinium* (combinaison avec le mode C2)
- F5 : d'*Alexandrium, Prorocentrum* et *Gymnodinium* (combinaison avec le mode D1)
- F6 : d'*Alexandrium, Dinophysis, Prorocentrum* et *Gymnodinium* (combinaison avec le mode D2)
- F7 : d'*Alexandrium, Pseudo-nitzschia, Prorocentrum* et *Gymnodinium* (combinaison avec le mode D3)
- F8 : *d'Alexandrium, Dinophysis, Pseudo-nitzschia, Prorocentrum* et *Gymnodinium* (combinaison avec le mode D4)
- F9 : d'*Alexandrium, Chattonella* et *Gymnodinium* (combinaison avec le mode E1)
- F10 : d'*Alexandrium, Dinophysis, Chattonella* et *Gymnodinium* (combinaison avec le mode E2)
- F11 : d'*Alexandrium, Pseudo-nitzschia, Chattonella* et *Gymnodinium* (combinaison avec le mode E3)
- F12 : *d'Alexandrium, Dinophysis, Pseudo-nitzschia, Chattonella* et *Gymnodinium* (combinaison avec le mode E4)
- F13 *:* d'*Alexandrium, Prorocentrum, Chattonella* et *Gymnodinium* (combinaison avec le mode E5)
- F14 : d'*Alexandrium, Dinophysis, Prorocentrum, Chattonella* et *Gymnodinium* (combinaison avec le mode E6)
- F15 : *d'Alexandrium, Pseudo-nitzschia, Prorocentrum, Chattonella* et *Gymnodinium* (combinaison avec le mode E7)
- F16 : *d'Alexandrium, Dinophysis, Pseudo-nitzschia, Prorocentrum, Chattonella* et *Gymnodinium* (combinaison avec le mode E8)
grâce à l'utilisation de sondes spécifiques d'algues toxiques du genre *Alexandrium, Dinophysis, Pseudo-nitzschia, Prorocentrum, Chattonella* et *Gymnodinium.*

### Mode de réalisation G

De la même façon, est décrite l'une des utilisations telles que décrites précédemment selon les modes A, B, C, D, E ou F, comprenant **en plus** l'utilisation d'au moins un couple de sondes spécifiques d'algues toxiques du genre ***Karenia*** pour la mise en œuvre d'un procédé de détection de cellules vivantes actives d'algues toxiques dans un échantillon susceptible de contenir au moins une algue toxique du genre ***Alexandrium*** et/ou ***Karenia*** les séquences desdites sondes étant choisies parmi x éléments de l'un des ensembles suivants :
- (SEQ ID NO : 95, SEQ ID NO : 96 ou SEQ ID NO : 97)
- (SEQ ID NO : 98, SEQ ID NO : 99 ou SEQ ID NO : 100)
- (SEQ ID NO : 101, SEQ ID NO : 102 ou SEQ ID NO : 103)
- (SEQ ID NO: 104, SEQ ID NO : 105 ou SEQ ID NO : 106)
- (SEQ ID NO: 107, SEQ ID NO : 108 ou SEQ ID NO : 109)
- (SEQ ID NO : 110, SEQ ID NO : 111 ou SEQ ID NO : 112)
- (SEQ ID NO: 113, SEQ ID NO : 114 ou SEQ ID NO : 115)
- (SEQ ID NO : 116, SEQ ID NO : 117 ou SEQ ID NO : 118)

x étant 3,
ou les séquences desdites sondes ayant au moins 92% d'identité avec les susdites séquences SEQ ID NO : 95, SEQ ID NO : 96, SEQ ID NO : 97, SEQ ID NO : 98, SEQ ID NO : 99, SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO : 116, SEQ ID NO : 117 ou SEQ ID NO : 118,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'acide nucléique ribosomique d'une algue toxique du genre ***Karenia*** éventuellement présent dans ledit échantillon afin de former un complexe,
le seuil de détection minimal de l'algue toxique du genre ***Karenia*** étant
   - de 100 à 500 cellules vivantes actives par litre d'échantillon (cellules/L) et en particulier inférieur à 200 cellules vivantes actives par litre d'échantillon (cellules/L), ou
   - inférieur ou égal à 0,10 ng d'ARN par litre d'échantillon et en particulier compris de 0,01 à 0,09 ng d'ARN par litre d'échantillon.

Dans un mode de réalisation particulier, est décrite l'utilisation d'au moins un couple de sondes spécifiques d'algues toxiques du genre ***Karenia*** telle que décrite précédemment pour la mise en œuvre d'un procédé de détection de cellules vivantes actives d'algues toxiques dans un échantillon susceptible de contenir au moins une algue toxique du genre ***Karenia*** dans laquelle les séquences des sondes desdits couples sont les suivants :
- (SEQ ID NO : 95 et SEQ ID NO : 96), (SEQ ID NO : 95 et SEQ ID NO : 97), (SEQ ID NO : 96 et SEQ ID NO : 97)
- (SEQ ID NO : 98 et SEQ ID NO : 99), (SEQ ID NO : 98 et SEQ ID NO : 100), (SEQ ID NO : 99 et SEQ ID NO : 100)
- (SEQ ID NO : 101 et SEQ ID NO : 102), (SEQ ID NO : 101 et SEQ ID NO : 103), (SEQ ID NO: 102 et SEQ ID NO : 103)
- (SEQ ID NO : 104 et SEQ ID NO : 105), (SEQ ID NO : 104 et SEQ ID NO : 106), (SEQ ID NO : 105 et SEQ ID NO : 106)
- (SEQ ID NO : 107 et SEQ ID NO : 108), (SEQ ID NO : 107 et SEQ ID NO : 109), (SEQ ID NO : 108 et SEQ ID NO : 109)
- (SEQ ID NO : 110 et SEQ ID NO : 111), (SEQ ID NO : 110 et SEQ ID NO : 112), (SEQ ID NO : 111 et SEQ ID NO : 112)
- (SEQ ID NO : 113 et SEQ ID NO : 114), (SEQ ID NO : 113 et SEQ ID NO : 115), (SEQ ID NO : 114 et SEQ ID NO : 115)
- (SEQ ID NO : 116 et SEQ ID NO : 117), (SEQ ID NO : 116 et SEQ ID NO : 118), (SEQ ID NO : 117 et SEQ ID NO : 118).

Comme précédemment pour les modes de réalisation A, B, C, D, E et F, et dans un mode de réalisation particulier, un seuil de détection minimal de l'algue toxique du genre ***Karenia*** inférieur à 200 cellules vivantes actives par litre d'échantillon (cellules/L) correspond également à un seuil de détection minimal compris de 0,01 ng à 0,09 ng d'ARN par litre d'échantillon.

De la même façon, dans un mode de réalisation particulier, la durée de la mise en œuvre dudit procédé de détection est inférieure à une heure.

Ainsi, le mode de réalisation G permet la détection :
- G1 : d'*Alexandrium* et *Karenia* (combinaison avec le mode A)
- G2 : d'*Alexandrium, Dinophysis* et *Karenia* (combinaison avec le mode B)
- G3 : d'*Alexandrium, Pseudo-nitzschia et Karenia* (combinaison avec le mode C1)
- G4 : d'*Alexandrium, Dinophysis, Pseudo-nitzschia* et *Karenia* (combinaison avec le mode C2)
- G5 : d'*Alexandrium, Prorocentrum et Karenia* (combinaison avec le mode D1)
- G6 : *d'Alexandrium, Dinophysis, Prorocentrum* et *Karenia* (combinaison avec le mode D2)
- G7 : d'*Alexandrium, Pseudo-nitzschia, Prorocentrum* et *Karenia* (combinaison avec le mode D3)
- G8 : *d'Alexandrium, Dinophysis, Pseudo-nitzschia, Prorocentrum* et *Karenia* (combinaison avec le mode D4)
- G9 : d'*Alexandrium, Chattonella* et *Karenia* (combinaison avec le mode E1)
- G10 : d'*Alexandrium, Dinophysis, Chattonella* et *Karenia* (combinaison avec le mode E2)
- G11 : d'*Alexandrium, Pseudo-nitzschia, Chattonella* et *Karenia* (combinaison avec le mode E3)
- G12 : *d'Alexandrium, Dinophysis, Pseudo-nitzschia, Chattonella* et *Karenia* (combinaison avec le mode E4)
- G13 : d'*Alexandrium, Prorocentrum, Chattonella* et *Karenia* (combinaison avec le mode E5)
- G14 : d'*Alexandrium, Dinophysis, Prorocentrum, Chattonella et Karenia* (combinaison avec le mode E6)
- G15 : *d'Alexandrium, Pseudo-nitzschia, Prorocentrum, Chattonella* et *Karenia* (combinaison avec le mode E7)
- G16 : *d'Alexandrium, Dinophysis, Pseudo-nitzschia, Prorocentrum, Chattonella* et *Karenia* (combinaison avec le mode E8)
- G17 : d'*Alexandrium, Gymnodinium* et *Karenia* (combinaison avec le mode F1)
- G18 : d'*Alexandrium, Dinophysis, Gymnodinium* et *Karenia* (combinaison avec le mode F2)
- G19 : d'*Alexandrium, Pseudo-nitzschia, Gymnodinium* et *Karenia* (combinaison avec le mode F3)
- G20 : d'*Alexandrium, Dinophysis, Pseudo-nitzschia, Gymnodinium* et *Karenia* (combinaison avec le mode F4)
- G21 : d'*Alexandrium, Prorocentrum, Gymnodinium* et *Karenia* (combinaison avec le mode F5)
- G22 : d'*Alexandrium, Dinophysis, Prorocentrum, Gymnodinium* et *Karenia* (combinaison avec le mode F6)
- G23 : d'*Alexandrium, Pseudo-nitzschia, Prorocentrum, Gymnodinium* et *Karenia* (combinaison avec le mode F7)
- G24 : *d'Alexandrium, Dinophysis, Pseudo-nitzschia, Prorocentrum, Gymnodinium* et *Karenia* (combinaison avec le mode F8)
- G25 : d'*Alexandrium, Chattonella, Gymnodinium* et *Karenia* (combinaison avec le mode F9)
- G26 : d'*Alexandrium, Dinophysis, Chattonella, Gymnodinium et Karenia* (combinaison avec le mode F10)
- G27 : d'*Alexandrium, Pseudo-nitzschia, Chattonella, Gymnodinium* et *Karenia* (combinaison avec le mode F11)
- G28 : *d'Alexandrium, Dinophysis, Pseudo-nitzschia, Chattonella, Gymnodinium* et *Karenia* (combinaison avec le mode F12)
- G29 : d'*Alexandrium, Prorocentrum, Chattonella, Gymnodinium* et *Karenia* (combinaison avec le mode F13)
- G30 : *d'Alexandrium, Dinophysis, Prorocentrum, Chattonella, Gymnodinium* et *Karenia* (combinaison avec le mode F14)
- G31 : d'*Alexandrium, Pseudo-nitzschia, Prorocentrum, Chattonella, Gymnodinium* et *Karenia* (combinaison avec le mode F15)
- G32 : *d'Alexandrium, Dinophysis, Pseudo-nitzschia, Prorocentrum, Chattonella, Gymnodinium* et *Karenia* (combinaison avec le mode F16)
grâce à l'utilisation de sondes spécifiques d'algues toxiques du genre *Alexandrium, Dinophysis, Pseudo-nitzschia, Prorocentrum, Chattonella, Gymnodinium* et *Karenia.*

### Mode de réalisation H

De la même façon, est décrite l'une des utilisations telles que décrites précédemment selon les modes de réalisation A, B, C, D, E, F ou G, comprenant **en plus** l'utilisation d'au moins un couple de sondes spécifiques d'algues toxiques du genre ***Lingulodinium*** pour la mise en œuvre d'un procédé de détection de cellules vivantes actives d'algues toxiques dans un échantillon susceptible de contenir au moins une algue toxique du genre ***Alexandrium*** et/ou ***Lingulodinium,*** les séquences desdites sondes étant choisies parmi x éléments de l'un des ensembles suivants :
- (SEQ ID NO : 119, SEQ ID NO : 120 ou SEQ ID NO : 121)
- (SEQ ID NO : 122, SEQ ID NO : 123 ou SEQ ID NO : 124)
- (SEQ ID NO : 125, SEQ ID NO : 126 ou SEQ ID NO : 127)

x étant 3,
ou les séquences desdites sondes ayant au moins 92% d'identité avec les susdites séquences SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126 ou SEQ ID NO : 127,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'acide nucléique ribosomique d'une algue toxique du genre ***Lingulodinium*** éventuellement présent dans ledit échantillon afin de former un complexe,
le seuil de détection minimal de l'algue toxique du genre ***Lingulodinium*** étant
   - de 100 à 500 cellules vivantes actives par litre d'échantillon (cellules/L) et en particulier inférieur à 200 cellules vivantes actives par litre d'échantillon (cellules/L), ou
   - inférieur ou égal à 0,10 ng d'ARN par litre d'échantillon et en particulier compris de 0,01 à 0,09 ng d'ARN par litre d'échantillon.

Dans un mode de réalisation particulier, est décrite l'utilisation d'au moins un couple de sondes spécifiques d'algues toxiques du genre ***Lingulodinium*** telle que décrite précédemment pour la mise en œuvre d'un procédé de détection de cellules vivantes actives d'algues toxiques dans un échantillon susceptible de contenir au moins une algue toxique du genre ***Lingulodinium*** dans laquelle les séquences des sondes desdits couples sont les suivants :
- (SEQ ID NO : 119 et SEQ ID NO : 120), (SEQ ID NO : 119 et SEQ ID NO : 121), (SEQ ID NO : 120 et SEQ ID NO : 121)
- (SEQ ID NO : 122 et SEQ ID NO : 123), (SEQ ID NO : 122 et SEQ ID NO : 124), (SEQ ID NO : 123 et SEQ ID NO : 124)
- (SEQ ID NO : 125 et SEQ ID NO : 126), (SEQ ID NO : 125 et SEQ ID NO : 127), (SEQ ID NO : 126 et SEQ ID NO : 127).

Comme précédemment pour les modes de réalisation A, B, C, D, E, F et G, et dans un mode de réalisation particulier, un seuil de détection minimal de l'algue toxique du genre ***Lingulodinium*** inférieure à 200 cellules vivantes actives par litre d'échantillon (d'eau, de culture) (cellules/L) correspond également à un seuil de détection minimal compris de 0,01 ng à 0,09 ng d'ARN par litre d'échantillon (d'eau, de culture).

De la même façon, dans un mode de réalisation particulier, la durée de la mise en œuvre dudit procédé de détection est inférieure à une heure.

Ainsi, le mode de réalisation H permet la détection :
- H1 : d'*Alexandrium* et *Lingulodinium* (combinaison avec le mode A)
- H2 : d'*Alexandrium, Dinophysis* et *Lingulodinium* (combinaison avec le mode B)
- H3 : d'*Alexandrium, Pseudo-nitzschia* et *Lingulodinium* (combinaison avec le mode C1)
- H4 : d'*Alexandrium, Dinophysis, Pseudo-nitzschia et Lingulodinium* (combinaison avec le mode C2)
- H5 : d'*Alexandrium, Prorocentrum et Lingulodinium* (combinaison avec le mode D1)
- H6 : d'*Alexandrium, Dinophysis, Prorocentrum et Lingulodinium* (combinaison avec le mode D2)
- H7 : d'*Alexandrium, Pseudo-nitzschia, Prorocentrum* et *Lingulodinium* (combinaison avec le mode D3)
- H8 : *d'Alexandrium, Dinophysis, Pseudo-nitzschia, Prorocentrum* et *Lingulodinium* (combinaison avec le mode D4)
- H9 : d'*Alexandrium, Chattonella* et *Lingulodinium* (combinaison avec le mode E1)
- H10 : d'*Alexandrium, Dinophysis, Chattonella* et *Lingulodinium* (combinaison avec le mode E2)
- H11 : d'*Alexandrium, Pseudo-nitzschia, Chattonella* et *Lingulodinium* (combinaison avec le mode E3)
- H12 : *d'Alexandrium, Dinophysis, Pseudo-nitzschia, Chattonella* et *Lingulodinium* (combinaison avec le mode E4)
- H13 : d'*Alexandrium, Prorocentrum, Chattonella* et *Lingulodinium* (combinaison avec le mode E5)
- H14 : d'*Alexandrium, Dinophysis, Prorocentrum, Chattonella* et *Lingulodinium* (combinaison avec le mode E6)
- H15 : *d'Alexandrium, Pseudo-nitzschia, Prorocentrum, Chattonella* et *Lingulodinium* (combinaison avec le mode E7)
- H16 : *d'Alexandrium, Dinophysis, Pseudo-nitzschia, Prorocentrum, Chattonella* et *Lingulodinium* (combinaison avec le mode E8)
- H17 : d'*Alexandrium, Gymnodinium* et *Lingulodinium* (combinaison avec le mode F1)
- H18 : d'*Alexandrium, Dinophysis, Gymnodinium* et *Lingulodinium* (combinaison avec le mode F2)
- H19 : d'*Alexandrium, Pseudo-nitzschia, Gymnodinium* et *Lingulodinium* (combinaison avec le mode F3)
- H20 : d'*Alexandrium, Dinophysis, Pseudo-nitzschia, Gymnodinium* et *Lingulodinium* (combinaison avec le mode F4)
- H21 : d'*Alexandrium, Prorocentrum, Gymnodinium* et *Lingulodinium* (combinaison avec le mode F5)
- H22 : d'*Alexandrium, Dinophysis, P*r*orocentrum, Gymnodinium* et *Lingulodinium* (combinaison avec le mode F6)
- H23 : d'*Alexandrium, Pseudo-nitzschia, Prorocentrum, Gymnodinium* et *Lingulodinium* (combinaison avec le mode F7)
- H24 : *d'Alexandrium, Dinophysis, Pseudo-nitzschia, Prorocentrum, Gymnodinium* et *Lingulodinium* (combinaison avec le mode F8)
- H25 : d'*Alexandrium, Chattonella, Gymnodinium* et *Lingulodinium* (combinaison avec le mode F9)
- H26 : d'*Alexandrium, Dinophysis, Chattonella, Gymnodinium* et *Lingulodinium* (combinaison avec le mode F10)
- H27 : d'*Alexandrium, Pseudo-nitzschia, Chattonella, Gymnodinium* et *Lingulodinium* (combinaison avec le mode F11)
- H28 : *d'Alexandrium, Dinophysis, Pseudo-nitzschia, Chattonella, Gymnodinium* et *Lingulodinium* (combinaison avec le mode F12)
- H29 : d'*Alexandrium, Prorocentrum, Chattonella, Gymnodinium* et *Lingulodinium* (combinaison avec le mode F13)
- H30 : *d'Alexandrium, Dinophysis, Prorocentrum, Chattonella, Gymnodinium* et *Lingulodinium* (combinaison avec le mode F14)
- H31 : d'*Alexandrium, Pseudo-nitzschia, Prorocentrum, Chattonella, Gymnodinium* et *Lingulodinium* (combinaison avec le mode F15)
- H32 : *d'Alexandrium, Dinophysis, Pseudo-nitzschia, Prorocentrum, Chattonella, Gymnodinium* et *Lingulodinium* (combinaison avec le mode F16)
- H33 : d'*Alexandrium, Karenia* et *Lingulodinium* (combinaison avec le mode G1)
- H34 : d'*Alexandrium, Dinophysis, Karenia* et *Lingulodinium* (combinaison avec le mode G2)
- H35 : d'*Alexandrium, Pseudo-nitzschia, Karenia* et *Lingulodinium* (combinaison avec le mode G3)
- H36 : d'*Alexandrium, Dinophysis, Pseudo-nitzschia, Karenia* et *Lingulodinium* (combinaison avec le mode G4)
- H37 : d'*Alexandrium, Prorocentrum, Karenia* et *Lingulodinium* (combinaison avec le mode G5)
- H38 : d'*Alexandrium, Dinophysis, Prorocentrum, Karenia* et *Lingulodinium* (combinaison avec le mode G6)
- H39 : d'*Alexandrium, Pseudo-nitzschia, Prorocentrum, Karenia* et *Lingulodinium* (combinaison avec le mode G7)
- H40 : d'*Alexandrium, Dinophysis, Pseudo-nitzschia, Prorocentrum, Karenia* et *Lingulodinium* (combinaison avec le mode G8)
- H41 : d'*Alexandrium, Chattonella, Karenia* et *Lingulodinium* (combinaison avec le mode G9)
- H42 : d'*Alexandrium, Dinophysis, Chattonella, Karenia* et *Lingulodinium* (combinaison avec le mode G10)
- H43 : d'*Alexandrium, Pseudo-nitzschia, Chattonella, Karenia* et *Lingulodinium* (combinaison avec le mode G11)
- H44 : d'*Alexandrium, Dinophysis, Pseudo-nitzschia, Chattonella, Karenia* et *Lingulodinium* (combinaison avec le mode G12)
- H45 : d'*Alexandrium, Prorocentrum, Chattonella, Karenia* et *Lingulodinium* (combinaison avec le mode G13)
- H46 : d'*Alexandrium, Dinophysis, Prorocentrum, Chattonella, Karenia* et *Lingulodinium* (combinaison avec le mode G14)
- H47 : d'*Alexandrium, Pseudo-nitzschia, Prorocentrum, Chattonella, Karenia* et *Lingulodinium* (combinaison avec le mode G15)
- H48 : *d'Alexandrium, Dinophysis, Pseudo-nitzschia, Prorocentrum, Chattonella, Karenia* et *Lingulodinium* (combinaison avec le mode G16)
- H49 : d'*Alexandrium, Gymnodinium, Karenia* et *Lingulodinium* (combinaison avec le mode G17)
- H50 : d'*Alexandrium, Dinophysis, Gymnodinium, Karenia* et *Lingulodinium* (combinaison avec le mode G18)
- H51 : d'*Alexandrium, Pseudo-nitzschia, Gymnodinium, Karenia* et *Lingulodinium* (combinaison avec le mode G19)
- H52 : d'*Alexandrium, Dinophysis, Pseudo-nitzschia, Gymnodinium, Karenia* et *Lingulodinium* (combinaison avec le mode G20)
- H53 : d'*Alexandrium, Prorocentrum, Gymnodinium, Karenia* et *Lingulodinium* (combinaison avec le mode G21)
- H54 : d'*Alexandrium, Dinophysis, Prorocentrum, Gymnodinium, Karenia* et *Lingulodinium* (combinaison avec le mode G22)
- H55 : d'*Alexandrium, Pseudo-nitzschia, Prorocentrum, Gymnodinium, Karenia* et *Lingulodinium* (combinaison avec le mode G23)
- H56 : *d'Alexandrium, Dinophysis, Pseudo-nitzschia, Prorocentrum, Gymnodinium, Karenia* et *Lingulodinium* (combinaison avec le mode G24)
- H57: d'*Alexandrium, Chattonella, Gymnodinium, Karenia* et *Lingulodinium* (combinaison avec le mode G25)
- H58 : d'*Alexandrium, Dinophysis, Chattonella, Gymnodinium, Karenia* et *Lingulodinium* (combinaison avec le mode G26)
- H59: d'*Alexandrium, Pseudo-nitzschia, Chattonella, Gymnodinium, Karenia* et *Lingulodinium* (combinaison avec le mode G27)
- H60 : *d'Alexandrium, Dinophysis, Pseudo-nitzschia, Chattonella, Gymnodinium, Karenia* et *Lingulodinium* (combinaison avec le mode G28)
- H61 : d'*Alexandrium, Prorocentrum, Chattonella, Gymnodinium, Karenia* et *Lingulodinium* (combinaison avec le mode G29)
- H62 : d'*Alexandrium, Dinophysis, Prorocentrum, Chattonella, Gymnodinium, Karenia* et *Lingulodinium* (combinaison avec le mode G30)
- H63 : *d'Alexandrium, Pseudo-nitzschia, Prorocentrum, Chattonella, Gymnodinium, Karenia* et *Lingulodinium* (combinaison avec le mode G31)
- H64 : *d'Alexandrium, Dinophysis, Pseudo-nitzschia, Prorocentrum, Chattonella, Gymnodinium, Karenia* et *Lingulodinium* (combinaison avec le mode G32)
grâce à l'utilisation de sondes spécifiques d'algues toxiques du genre *Alexandrium, Dinophysis, Pseudo-nitzschia, Prorocentrum, Chattonella, Gymnodinium, Karenia* et *Lingulodinium.*

### Mode de réalisation I

De la même façon, est décrite l'une des utilisations telles que décrites précédemment selon les modes de réalisation A, B, C, D, E, F, G ou H comprenant **en plus** l'utilisation d'au moins un couple de sondes spécifiques d'algues toxiques du genre ***Heterosigma*** pour la mise en œuvre d'un procédé de détection de cellules vivantes actives d'algues toxiques dans un échantillon susceptible de contenir au moins une algue toxique du genre ***Alexandrium*** et/ou ***Heterosigma*** les séquences desdites sondes étant choisies parmi x éléments de l'un des ensembles suivants :
- (SEQ ID NO : 128 et SEQ ID NO : 129)

x étant 2,
ou les séquences desdites sondes ayant au moins 92% d'identité avec les susdites séquences SEQ ID NO : 128 ou SEQ ID NO : 129,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'acide nucléique ribosomique d'une algue toxique du genre ***Heterosigma*** éventuellement présent dans ledit échantillon afin de former un complexe,
le seuil de détection minimal de l'algue toxique du genre ***Heterosigma*** étant
   - de 100 à 500 cellules vivantes actives par litre d'échantillon (cellules/L) et en particulier inférieur à 200 cellules vivantes actives par litre d'échantillon (cellules/L), ou
   - inférieur ou égal à 0,10 ng d'ARN par litre d'échantillon et en particulier compris de 0,01 à 0,09 ng d'ARN par litre d'échantillon.

Comme précédemment pour les modes de réalisation A, B, C, D, E, F, G et H, et dans un mode de réalisation particulier, un seuil de détection minimal de l'algue toxique du genre ***Heterosigma*** inférieur à 200 cellules vivantes actives par litre d'échantillon (cellules/L) correspond également à un seuil de détection minimal compris de 0,01 ng à 0,09 ng d'ARN par litre d'échantillon.

De la même façon, dans un mode de réalisation particulier, la durée de la mise en œuvre dudit procédé de détection est inférieure à une heure.

Ainsi, le mode de réalisation I permet la détection :
- I1 : d'*Alexandrium* et *Heterosigma* (combinaison avec le mode A)
- I2 : d'*Alexandrium, Dinophysis* et *Heterosigma* (combinaison avec le mode B)
- I3 : d'*Alexandrium, Pseudo-nitzschia* et *Heterosigma* (combinaison avec le mode C1)
- I4 : d'*Alexandrium, Dinophysis, Pseudo-nitzschia et Heterosigma* (combinaison avec le mode C2)
- I5 : d'*Alexandrium, Prorocentrum et Heterosigma* (combinaison avec le mode D1)
- I6 : *d'Alexandrium, Dinophysis, Prorocentrum* et *Heterosigma* (combinaison avec le mode D2)
- I7 : d'*Alexandrium, Pseudo-nitzschia, Prorocentrum* et *Heterosigma* (combinaison avec le mode D3)
- I8 : *d'Alexandrium, Dinophysis, Pseudo-nitzschia, Prorocentrum* et *Heterosigma* (combinaison avec le mode D4)
- I9 : d'*Alexandrium, Chattonella* et *Heterosigma* (combinaison avec le mode E1)
- I10 : d'*Alexandrium, Dinophysis, Chattonella* et *Heterosigma* (combinaison avec le mode E2)
- I11 : d'*Alexandrium, Pseudo-nitzschia, Chattonella* et *Heterosigma* (combinaison avec le mode E3)
- I12 : *d'Alexandrium, Dinophysis, Pseudo-nitzschia, Chattonella* et *Heterosigma* (combinaison avec le mode E4)
- I13 : d'*Alexandrium, Prorocentrum, Chattonella* et *Heterosigma* (combinaison avec le mode E5)
- I14 : d'*Alexandrium, Dinophysis, Prorocentrum, Chattonella* et *Heterosigma* (combinaison avec le mode E6)
- I15 : *d'Alexandrium, Pseudo-nitzschia, Prorocentrum, Chattonella* et *Heterosigma* (combinaison avec le mode E7)
- I16 : *d'Alexandrium, Dinophysis, Pseudo-nitzschia, Prorocentrum, Chattonella* et *Heterosigma* (combinaison avec le mode E8)
- I17 : d'*Alexandrium, Gymnodinium* et *Heterosigma* (combinaison avec le mode F1)
- I18 : d'*Alexandrium, Dinophysis, Gymnodinium* et *Heterosigma* (combinaison avec le mode F2)
- 119 : d'*Alexandrium, Pseudo-nitzschia, Gymnodinium* et *Heterosigma* (combinaison avec le mode F3)
- I20 : d'*Alexandrium, Dinophysis, Pseudo-nitzschia, Gymnodinium* et *Heterosigma* (combinaison avec le mode F4)
- I21 : d'*Alexandrium, Prorocentrum, Gymnodinium* et *Heterosigma* (combinaison avec le mode F5)
- I22 : d'*Alexandrium, Dinophysis, Prorocentrum, Gymnodinium* et *Heterosigma* (combinaison avec le mode F6)
- I23 *:* d'*Alexandrium, Pseudo-nitzschia, Prorocentrum, Gymnodinium* et *Heterosigma* (combinaison avec le mode F7)
- I24 : *d'Alexandrium, Dinophysis, Pseudo-nitzschia, Prorocentrum, Gymnodinium* et *Heterosigma* (combinaison avec le mode F8)
- I25 : d'*Alexandrium, Chattonella, Gymnodinium* et *Heterosigma* (combinaison avec le mode F9)
- I26 : d'*Alexandrium, Dinophysis, Chattonella, Gymnodinium* et *Heterosigma* (combinaison avec le mode F10)
- I27 : d'*Alexandrium, Pseudo-nitzschia, Chattonella, Gymnodinium* et *Heterosigma* (combinaison avec le mode F11)
- I28 : *d'Alexandrium, Dinophysis, Pseudo-nitzschia, Chattonella, Gymnodinium* et *Heterosigma* (combinaison avec le mode F12)
- I29 : d'*Alexandrium, Prorocentrum, Chattonella, Gymnodinium* et *Heterosigma* (combinaison avec le mode F13)
- I30 : *d'Alexandrium, Dinophysis, Prorocentrum, Chattonella, Gymnodinium* et *Heterosigma* (combinaison avec le mode F14)
- I31 : *d'Alexandrium, Pseudo-nitzschia, Prorocentrum, Chattonella, Gymnodinium* et *Heterosigma* (combinaison avec le mode F15)
- I32 : *d'Alexandrium, Dinophysis, Pseudo-nitzschia, Prorocentrum, Chattonella, Gymnodinium* et *Heterosigma* (combinaison avec le mode F16)
- I33 : d'*Alexandrium, Karenia* et *Heterosigma* (combinaison avec le mode G1)
- I34 : d'*Alexandrium, Dinophysis, Karenia* et *Heterosigma* (combinaison avec le mode G2)
- I35 : d'*Alexandrium, Pseudo-nitzschia, Karenia* et *Heterosigma* (combinaison avec le mode G3)
- I36 : d'*Alexandrium, Dinophysis, Pseudo-nitzschia, Karenia* et *Heterosigma* (combinaison avec le mode G4)
- I37 : d'*Alexandrium, Prorocentrum, Karenia* et *Heterosigma* (combinaison avec le mode G5)
- I38 : d'*Alexandrium, Dinophysis, Prorocentrum, Karenia et Heterosigma* (combinaison avec le mode G6)
- I39 : d'*Alexandrium, Pseudo-nitzschia, Prorocentrum, Karenia* et *Heterosigma* (combinaison avec le mode G7)
- I40 : d'*Alexandrium, Dinophysis, Pseudo-nitzschia, Prorocentrum, Karenia* et *Heterosigma* (combinaison avec le mode G8)
- I41 : d'*Alexandrium, Chattonella, Karenia* et *Heterosigma* (combinaison avec le mode G9)
- I42 : d'*Alexandrium, Dinophysis, Chattonella, Karenia* et *Heterosigma* (combinaison avec le mode G10)
- I43 : d'*Alexandrium, Pseudo-nitzschia, Chattonella, Karenia* et *Heterosigma* (combinaison avec le mode G11)
- I44 : d'*Alexandrium, Dinophysis, Pseudo-nitzschia, Chattonella, Karenia* et *Heterosigma* (combinaison avec le mode G12)
- I45 : d'*Alexandrium, Prorocentrum, Chattonella, Karenia* et *Heterosigma* (combinaison avec le mode G13)
- I46 : d'*Alexandrium, Dinophysis, Prorocentrum, Chattonella, Karenia* et *Heterosigma* (combinaison avec le mode G14)
- I47 : d'*Alexandrium, Pseudo-nitzschia, Prorocentrum, Chattonella, Karenia* et *Heterosigma* (combinaison avec le mode G15)
- I48 : *d'Alexandrium, Dinophysis, Pseudo-nitzschia, Prorocentrum, Chattonella, Karenia* et *Heterosigma* (combinaison avec le mode G16)
- I49 : d'*Alexandrium, Gymnodinium, Karenia* et *Heterosigma* (combinaison avec le mode G17)
- I50 : d'*Alexandrium, Dinophysis, Gymnodinium, Karenia et Heterosigma* (combinaison avec le mode G18)
- I51 : d'*Alexandrium, Pseudo-nitzschia, Gymnodinium, Karenia* et *Heterosigma* (combinaison avec le mode G19)
- I52 : d'*Alexandrium, Dinophysis, Pseudo-nitzschia, Gymnodinium, Karenia* et *Heterosigma* (combinaison avec le mode G20)
- I53 : d'*Alexandrium, Prorocentrum, Gymnodinium, Karenia* et *Heterosigma* (combinaison avec le mode G21)
- I54 : d'*Alexandrium, Dinophysis, Prorocentrum, Gymnodinium, Karenia* et *Heterosigma* (combinaison avec le mode G22)
- I55 : d'*Alexandrium, Pseudo-nitzschia, Prorocentrum, Gymnodinium, Karenia* et *Heterosigma* (combinaison avec le mode G23)
- I56 : *d'Alexandrium, Dinophysis, Pseudo-nitzschia, Prorocentrum, Gymnodinium, Karenia* et *Heterosigma* (combinaison avec le mode G24)
- I57 : d'*Alexandrium, Chattonella, Gymnodinium, Karenia et Heterosigma* (combinaison avec le mode G25)
- I58 : d'*Alexandrium, Dinophysis, Chattonella, Gymnodinium, Karenia* et *Heterosigma* (combinaison avec le mode G26)
- I59 : d'*Alexandrium, Pseudo-nitzschia, Chattonella, Gymnodinium, Karenia* et *Heterosigma* (combinaison avec le mode G27)
- I60 : *d'Alexandrium, Dinophysis, Pseudo-nitzschia, Chattonella, Gymnodinium, Karenia* et *Heterosigma* (combinaison avec le mode G28)
- I61 : d'*Alexandrium, Prorocentrum, Chattonella, Gymnodinium, Karenia* et *Heterosigma* (combinaison avec le mode G29)
- I62 : d'*Alexandrium, Dinophysis, Prorocentrum, Chattonella, Gymnodinium, Karenia* et *Heterosigma* (combinaison avec le mode G30)
- I63 : *d'Alexandrium, Pseudo-nitzschia, Prorocentrum, Chattonella, Gymnodinium, Karenia* et *Heterosigma* (combinaison avec le mode G31)
- I64 : *d'Alexandrium, Dinophysis, Pseudo-nitzschia, Prorocentrum, Chattonella, Gymnodinium, Karenia* et *Heterosigma* (combinaison avec le mode G32)
- I65 : d'*Alexandrium, Lingulodinium* et *Heterosigma* (combinaison avec le mode H1)
- I66 : d'*Alexandrium, Dinophysis, Lingulodinium* et *Heterosigma* (combinaison avec le mode H2)
- I67 : d*'Alexandrium, Pseudo-nitzschia, Lingulodinium* et *Heterosigma* (combinaison avec le mode H3)
- I68 : d'*Alexandrium, Dinophysis, Pseudo-nitzschia, Lingulodinium* et *Heterosigma* (combinaison avec le mode H4)
- I69 : d'*Alexandrium, Prorocentrum, Lingulodinium* et *Heterosigma* (combinaison avec le mode H5)
- I70 : d'*Alexandrium, Dinophysis, Prorocentrum, Lingulodinium* et *Heterosigma* (combinaison avec le mode H6)
- I71 : d'*Alexandrium, Pseudo-nitzschia, Prorocentrum, Lingulodinium* et *Heterosigma* (combinaison avec le mode H7)
- I72 : d'*Alexandrium, Dinophysis, Pseudo-nitzschia, Prorocentrum, Lingulodinium* et *Heterosigma* (combinaison avec le mode H8)
- I73 : d'*Alexandrium, Chattonella* et *Lingulodinium* (combinaison avec le mode H9)
- I74 : d'*Alexandrium, Dinophysis, Chattonella, Lingulodinium* et *Heterosigma* (combinaison avec le mode H10)
- I75 : d'*Alexandrium, Pseudo-nitzschia, Chattonella, Lingulodinium* et *Heterosigma* (combinaison avec le mode H11)
- I76 : d'*Alexandrium, Dinophysis, Pseudo-nitzschia, Chattonella, Lingulodinium* et *Heterosigma* (combinaison avec le mode H12)
- I76 : d'*Alexandrium, Prorocentrum, Chattonella, Lingulodinium* et *Heterosigma* (combinaison avec le mode H13)
- I78 : d'*Alexandrium, Dinophysis, Prorocentrum, Chattonella, Lingulodinium* et *Heterosigma* (combinaison avec le mode H14)
- I79 : d'*Alexandrium, Pseudo-nitzschia, Prorocentrum, Chattonella, Lingulodinium* et *Heterosigma* (combinaison avec le mode H15)
- I80 : *d'Alexandrium, Dinophysis, Pseudo-nitzschia, Prorocentrum, Chattonella, Lingulodinium* et *Heterosigma* (combinaison avec le mode H16)
- I81 : d'*Alexandrium, Gymnodinium, Lingulodinium, et Heterosigma* (combinaison avec le mode H17)
- I82 : d'*Alexandrium, Dinophysis, Gymnodinium, Lingulodinium* et *Heterosigma* (combinaison avec le mode H18)
- I83 : d'*Alexandrium, Pseudo-nitzschia, Gymnodinium, Lingulodinium* et *Heterosigma* (combinaison avec le mode H19)
- 184 : d'*Alexandrium, Dinophysis, Pseudo-nitzschia, Gymnodinium, Lingulodinium* et *Heterosigma* (combinaison avec le mode H20)
- I85 : d'*Alexandrium, Prorocentrum, Gymnodinium, Lingulodinium* et *Heterosigma* (combinaison avec le mode H21)
- I86 : d'*Alexandrium, Dinophysis, Prorocentrum, Gymnodinium, Lingulodinium* et *Heterosigma* (combinaison avec le mode H22)
- 187 : d'*Alexandrium, Pseudo-nitzschia, Prorocentrum, Gymnodinium, Lingulodinium* et *Heterosigma* (combinaison avec le mode H23)
- I88 : *d'Alexandrium, Dinophysis, Pseudo-nitzschia, Prorocentrum, Gymnodinium, Lingulodinium* et *Heterosigma* (combinaison avec le mode H24)
- 189 : d'*Alexandrium, Chattonella, Gymnodinium, Lingulodinium* et *Heterosigma* (combinaison avec le mode H25)
- I90 : d'*Alexandrium, Dinophysis, Chattonella, Gymnodinium, Lingulodinium* et *Heterosigma* (combinaison avec le mode H26)
- I91 : d'*Alexandrium, Pseudo-nitzschia, Chattonella, Gymnodinium, Lingulodinium* et *Heterosigma* (combinaison avec le mode H27)
- I92 : *d'Alexandrium, Dinophysis, Pseudo-nitzschia, Chattonella, Gymnodinium, Lingulodinium* et *Heterosigma* (combinaison avec le mode H28)
- I93 : d'*Alexandrium, Prorocentrum, Chattonella, Gymnodinium, Lingulodinium* et *Heterosigma* (combinaison avec le mode H29)
- I94 : *d'Alexandrium, Dinophysis, Prorocentrum, Chattonella, Gymnodinium, Lingulodinium* et *Heterosigma* (combinaison avec le mode H30)
- 195 : *d'Alexandrium, Pseudo-nitzschia, Prorocentrum, Chattonella, Gymnodinium, Lingulodinium* et *Heterosigma* (combinaison avec le mode H31)
- I96 : *d'Alexandrium, Dinophysis, Pseudo-nitzschia, Prorocentrum, Chattonella, Gymnodinium, Lingulodinium* et *Heterosigma* (combinaison avec le mode H32)
- 197 : d'*Alexandrium, Karenia, Lingulodinium* et *Heterosigma* (combinaison avec le mode H33)
- I98 : d'*Alexandrium, Dinophysis, Karenia, Lingulodinium* et *Heterosigma* (combinaison avec le mode H34)
- I99 : d'*Alexandrium, Pseudo-nitzschia, Karenia, Lingulodinium* et *Heterosigma* (combinaison avec le mode H35)
- I100 : d'*Alexandrium, Dinophysis, Pseudo-nitzschia, Karenia, Lingulodinium* et *Heterosigma* (combinaison avec le mode H36)
- I101 : d'*Alexandrium, Prorocentrum, Karenia, Lingulodinium* et *Heterosigma* (combinaison avec le mode H37)
- I102 : d'*Alexandrium, Dinophysis, Prorocentrum, Karenia, Lingulodinium* et *Heterosigma* (combinaison avec le mode H38)
- I103 : d'*Alexandrium, Pseudo-nitzschia, Prorocentrum, Karenia, Lingulodinium* et *Heterosigma* (combinaison avec le mode H39)
- I104 : d'*Alexandrium, Dinophysis, Pseudo-nitzschia, Prorocentrum, Karenia, Lingulodinium* et *Heterosigma* (combinaison avec le mode H40)
- I105 : d'*Alexandrium, Chattonella, Karenia, Lingulodinium* et *Heterosigma* (combinaison avec le mode H41)
- I106 : d'*Alexandrium, Dinophysis, Chattonella, Karenia, Lingulodinium* et *Heterosigma* (combinaison avec le mode H42)
- I107 : d'*Alexandrium, Pseudo-nitzschia, Chattonella, Karenia, Lingulodinium* et *Heterosigma* (combinaison avec le mode H43)
- I108 : d'*Alexandrium, Dinophysis, Pseudo-nitzschia, Chattonella, Karenia, Lingulodinium* et *Heterosigma* (combinaison avec le mode H44)
- I109 : d'*Alexandrium, Prorocentrum, Chattonella, Karenia, Lingulodinium* et *Heterosigma* (combinaison avec le mode H45)
- I110 : d'*Alexandrium, Dinophysis, Prorocentrum, Chattonella, Karenia, Lingulodinium* et *Heterosigma* (combinaison avec le mode H46)
- I111 : d'*Alexandrium, Pseudo-nitzschia, Prorocentrum, Chattonella, Karenia, Lingulodinium* et *Heterosigma* (combinaison avec le mode H47)
- I112 : *d'Alexandrium, Dinophysis, Pseudo-nitzschia, Prorocentrum, Chattonella, Karenia, Lingulodinium* et *Heterosigma* (combinaison avec le mode H48)
- I113 : d'*Alexandrium, Gymnodinium, Karenia, Lingulodinium* et *Heterosigma* (combinaison avec le mode H49)
- I114 : d'*Alexandrium, Dinophysis, Gymnodinium, Karenia, Lingulodinium* et *Heterosigma* (combinaison avec le mode H50)
- I115 : d'*Alexandrium, Pseudo-nitzschia, Gymnodinium, Karenia, Lingulodinium* et *Heterosigma* (combinaison avec le mode H51)
- I116 : d'*Alexandrium, Dinophysis, Pseudo-nitzschia, Gymnodinium, Karenia, Lingulodinium* et *Heterosigma* (combinaison avec le mode H52)
- I117 : d'*Alexandrium, Prorocentrum, Gymnodinium, Karenia, Lingulodinium* et *Heterosigma* (combinaison avec le mode H53)
- I118 : d'*Alexandrium, Dinophysis, Prorocentrum, Gymnodinium, Karenia, Lingulodinium* et *Heterosigma* (combinaison avec le mode H54)
- I119 : d'*Alexandrium, Pseudo-nitzschia, Prorocentrum, Gymnodinium, Karenia, Lingulodinium* et *Heterosigma* (combinaison avec le mode H55)
- 1120 : *d'Alexandrium, Dinophysis, Pseudo-nitzschia, Prorocentrum, Gymnodinium, Karenia, Lingulodinium* et *Heterosigma* (combinaison avec le mode H56)
- I121 : d*'Alexandrium, Chattonella, Gymnodinium, Karenia, Lingulodinium* et *Heterosigma* (combinaison avec le mode H57)
- 1122 : d'*Alexandrium, Dinophysis, Chattonella, Gymnodinium, Karenia, Lingulodinium* et *Heterosigma* (combinaison avec le mode H58)
- I123 : d'*Alexandrium, Pseudo-nitzschia, Chattonella, Gymnodinium, Karenia, Lingulodinium* et *Heterosigma* (combinaison avec le mode H59)
- I124 : *d'Alexandrium, Dinophysis, Pseudo-nitzschia, Chattonella, Gymnodinium, Karenia, Lingulodinium* et *Heterosigma* (combinaison avec le mode H60)
- I125 : d'*Alexandrium, Prorocentrum, Chattonella, Gymnodinium, Karenia, Lingulodinium* et *Heterosigma* (combinaison avec le mode H61)
- 1126 : d'*Alexandrium, Dinophysis, Prorocentrum, Chattonella, Gymnodinium, Karenia Lingulodinium* et *Heterosigma* (combinaison avec le mode H62)
- 1127 : *d'Alexandrium, Pseudo-nitzschia, Prorocentrum, Chattonella, Gymnodinium, Karenia, Lingulodinium* et *Heterosigma* (combinaison avec le mode H63)
- I128 : *d'Alexandrium, Dinophysis, Pseudo-nitzschia, Prorocentrum, Chattonella, Gymnodinium, Karenia, Lingulodinium* et *Heterosigma* (combinaison avec le mode H64)
grâce à l'utilisation de sondes spécifiques d'algues toxiques du genre *Alexandrium, Dinophysis, Pseudo-nitzschia, Prorocentrum, Chattonella, Gymnodinium, Karenia, Lingulodinium* et *Heterosigma.*

Par « *pourcentage d'identité* »par rapport à une séquence donnée, on désigne le pourcentage des acides aminés identiques à ceux d'une séquence de référence et qui se retrouvent aux mêmes positions.

On entend *par* « *au moins 92% d'identité* » les plages de valeurs d'au moins 92%, d'au moins 93%, d'au moins 94%, d'au moins 95%, d'au moins 96%, d'au moins 97%, d'au moins 98%, d'au moins 99% et de 100% d'identité.

Dans l'ensemble des modes de réalisation de ce premier aspect, et selon un mode de réalisation particulier, ladite sonde capture est liée à au moins une molécule d'attache positionnée en 5' de sa séquence et ladite sonde signal est liée à au moins une molécule de marquage positionnée en 5' de sa séquence.

Dans l'ensemble des modes de réalisation de ce premier aspect, et selon un mode de réalisation particulier, ladite sonde capture est liée à au moins une molécule d'attache positionnée en 5' de sa séquence et ladite sonde signal est liée à au moins une molécule de marquage positionnée en 3' de sa séquence.

Dans l'ensemble des modes de réalisation de ce premier aspect, et selon un mode de réalisation particulier, ladite sonde capture est liée à au moins une molécule d'attache positionnée en 3' de sa séquence et ladite sonde signal est liée à au moins une molécule de marquage positionnée en 5' de sa séquence.

Dans l'ensemble des modes de réalisation de ce premier aspect, et selon un mode de réalisation particulier, ladite sonde capture est liée à au moins une molécule d'attache positionnée en 3' de sa séquence et ladite sonde signal est liée à au moins une molécule de marquage positionnée en 3' de sa séquence.

Dans l'ensemble des modes de réalisation de ce premier aspect, la « au moins une molécule d'attache » peut être choisie parmi une molécule de biotine, avidine, streptavidine, un groupe thiol, un groupe amine et un carbone.

Dans l'ensemble des modes de réalisation de ce premier aspect, et dans un mode de réalisation particulièrement préféré, la « *au moins une molécule d'attache* » est une molécule de biotine.

Dans l'ensemble des modes de réalisation de ce premier aspect, la « *au moins une molécule de marquage* » peut être choisie parmi un fluorochrome, une biotine, une molécule liée à une biotine, la digoxigénine, une enzyme utilisant un substrat chimioluminescent, une enzyme utilisant un substrat chromogène ou une enzyme utilisant un substrat à oxydation électrochimique.

Dans l'ensemble des modes de réalisation de ce premier aspect, et dans un mode de réalisation particulièrement préféré, la « *au moins une molécule de marquage* » est la digoxigénine.

Ainsi, un mode de réalisation particulier concerne l'utilisation telle que décrite ci-dessus dans laquelle,
ladite sonde capture est liée à au moins une molécule d'attache positionnée en 5' de sa séquence et ladite sonde signal est liée à au moins une molécule de marquage positionnée en 5' de sa séquence, ou
ladite sonde capture est liée à au moins une molécule d'attache positionnée en 5' de sa séquence et ladite sonde signal est liée à au moins une molécule de marquage positionnée en 3' de sa séquence, ou
ladite sonde capture est liée à au moins une molécule d'attache positionnée en 3' de sa séquence et ladite sonde signal est liée à au moins une molécule de marquage positionnée en 5' de sa séquence, ou
ladite sonde capture est liée à au moins une molécule d'attache positionnée en 3' de sa séquence et ladite sonde signal est liée à au moins une molécule de marquage positionnée en 3' de sa séquence,
ladite « *au moins une molécule d'attache* »étant notamment choisie parmi une molécule de biotine, avidine, streptavidine, un groupe thiol, un groupe amine et un carbone, de préférence une molécule de biotine,
laquelle ladite « *au moins une molécule de marquage* » étant notamment choisie parmi un fluorochrome, une biotine, une molécule liée à une biotine, la digoxigénine, une enzyme utilisant un substrat chimioluminescent ou une enzyme utilisant un substrat chromogène ou une enzyme utilisant un substrat à oxydation électrochimique, de préférence la digoxigénine.

Dans l'ensemble des modes de réalisation de ce premier aspect, ledit fluorochrome peut être choisi parmi le groupe consistant en : Alexa fluor, en particulier Alexa fluor 350, 405, 430, 488, 500, 514, 532, 546, 555, 568, 594, 610, 633, 647, 660, 680, 700, 750 ou 790, Isothiocyanate de fluorescéine (FITC), Rhodamine, Allophycocyanine (APC) et Phycoérythrine (PE).

Dans l'ensemble des modes de réalisation de ce premier aspect, ladite enzyme utilisant un substrat chimioluminescent peut être la péroxydase de raifort (HRP) et ledit substrat chimioluminescent peut être le luminol, ou encore, ladite enzyme utilisant un substrat chimioluminescent peut être la luciférase et ledit substrat chimioluminescent peut être la luciférine. Dans ce cas, la réaction de l'enzyme et de son substrat génère de la lumière pouvant être mesurée ou par un lecteur de luminescence.

Dans l'ensemble des modes de réalisation de ce premier aspect, ladite enzyme utilisant un substrat chromogène peut être la phosphatase alcaline et ledit substrat chromogène peut être Nitrobleu de tetrazolium (NBT) ou le bromochlorylindolophosphate (BCIP), ou encore, ladite enzyme utilisant un substrat chromogène peut être la péroxydase de raifort (HRP) et ledit substrat chromogène peut être choisi parmi le 3,3'-Diaminobenzidine (DAB), le 3,3',5,5'-Tetramethylbenzidine (TMB), ou le 2,2'-azino-bis(acide 3-ethylbenzothiazoline-6-sulphonique) (ABTS). Dans ce cas, l'enzyme oxide un substrat, qui, une fois réduit, produit un précipité coloré, pouvant être mesuré par lecteur d'absorbance.

Dans l'ensemble des modes de réalisation de ce premier aspect, ladite enzyme utilisant un substrat à oxydation électrochimique peut être la péroxydase de raifort (HRP) et ledit substrat à oxydation électrochimique peut être le 3,3',5,5'-Tetramethylbenzidine (TMB). Dans ce cas, l'enzyme (par exemple la Peroxidase de raifort), réagit en présence de H₂O₂, et oxide un substrat (par exemple le TMB) qui, une fois réduit, produit une différence de potentiel électrique. Cette différence de potentiel électrique peut être mesurée par une électrode.

Dans l'ensemble des modes de réalisation de ce premier aspect, l'échantillon peut être un échantillon d'eau de mer, d'eau saumâtre, de milieux de cultures ou de culture de microalgues produites à des fins commerciales.

Le terme « *échantillon d'eau de mer* »se réfère à un volume d'eau provenant de mer et d'océans qui contient des organismes vivants tels que phytoplancton et zooplancton.

Le terme « *échantillon d'eau saumâtre* »se réfère à un volume d'eau provenant de la rencontre de masses d'eau douce et salée, comme un estuaire d'un fleuve, une lagune, un bassin.

Le terme « *milieu de culture* »se réfère à un support qui permet la culture de micro-organismes telle que micro-algues, bactéries, levures.

Le terme « *cultures de micro -algues* »se réfère à la gestion d'un écosystème aquatique en vue d'y favoriser la production d'une ou de plusieurs espèces d'intérêt commercial telles que les algues microscopiques unicellulaires ou coloniales.

Dans un second aspect, sont décrits des couples de sondes pour la détection de cellules vivantes actives d'algues toxiques.

Ainsi, un aspect décrit mais ne faisant pas partie de l'invention concerne au moins un couple de sondes pour la détection de cellules vivantes actives d'algues toxiques du genre ***Alexandrium*** dont les séquences sont choisies parmi x éléments de l'un des ensembles suivants :
- (SEQ ID NO : 1, SEQ ID NO : 2 ou SEQ ID NO : 3)
- (SEQ ID NO : 4 et SEQ ID NO : 5)
- (SEQ ID NO : 6 et SEQ ID NO : 7)
- (SEQ ID NO : 8, SEQ ID NO : 9 ou SEQ ID NO : 10)
- (SEQ ID NO : 11, SEQ ID NO : 12 ou SEQ ID NO : 13)
- (SEQ ID NO : 14, SEQ ID NO : 15 ou SEQ ID NO : 16)
- (SEQ ID NO : 17, SEQ ID NO : 18 ou SEQ ID NO : 19)
- (SEQ ID NO : 20, SEQ ID NO : 21 ou SEQ ID NO : 22),
- (SEQ ID NO : 23, SEQ ID NO : 24 ou SEQ ID NO : 25),
- (SEQ ID NO : 26, SEQ ID NO : 27 ou SEQ ID NO : 28)

x étant 2 ou 3,
ou dont les séquences ont au moins 92% d'identité avec les susdites séquences SEQ ID NO : 1 à SEQ ID NO : 28.

Dans un mode de réalisation particulier, est décrite au moins un couple de sondes pour la détection de cellules vivantes actives d'algues toxiques du genre ***Alexandrium,*** les séquences des sondes desdits couples sont les suivantes :
- (SEQ ID NO: 1 et SEQ ID NO: 2), (SEQ ID NO : 1 et SEQ ID NO : 3), (SEQ ID NO : 2 et SEQ ID NO : 3)
- (SEQ ID NO : 4 et SEQ ID NO : 5)
- (SEQ ID NO : 6 et SEQ ID NO : 7)
- (SEQ ID NO : 8 et SEQ ID NO : 9), (SEQ ID NO : 8 et SEQ ID NO : 10), (SEQ ID NO : 9 et SEQ ID NO : 10)
- (SEQ ID NO : 11 et SEQ ID NO : 12), (SEQ ID NO : 11 et SEQ ID NO : 13), (SEQ ID NO : 12 et SEQ ID NO : 13)
- (SEQ ID NO : 14 et SEQ ID NO : 15), (SEQ ID NO : 14 et SEQ ID NO : 16), (SEQ ID NO : 15 et SEQ ID NO : 16)
- (SEQ ID NO : 17 et SEQ ID NO : 18), (SEQ ID NO : 17 et 19), (SEQ ID NO : 18 et SEQ ID NO : 19)
- (SEQ ID NO : 20 et SEQ ID NO : 21), (SEQ ID NO : 20 et SEQ ID NO : 22), (SEQ ID NO : 21 et SEQ ID NO : 22), ou
- (SEQ ID NO : 23 et SEQ ID NO : 24), (SEQ ID NO : 23 et SEQ ID NO : 25), (SEQ ID NO : 24 et SEQ ID NO : 25)
- (SEQ ID NO : 26 et SEQ ID NO : 27), (SEQ ID NO : 26 et SEQ ID NO : 28), (SEQ ID NO : 27 et SEQ ID NO : 28).

Dans un mode de réalisation particulier, l'invention a pour objet un couple de sondes pour la détection de cellules vivantes actives d'algues toxiques du genre *Alexandrium* dont les séquences des sondes desdits couples sont les suivantes :
- (SEQ ID NO : 1 et SEQ ID NO : 2),
- (SEQ ID NO : 4 et SEQ ID NO : 5),
- (SEQ ID NO : 11 et SEQ ID NO : 12), ou
- (SEQ ID NO : 17 et SEQ ID NO : 18).

Dans ce second aspect, est également décrit au moins un couple de sondes pour la détection de cellules vivantes actives d'algues toxiques du genre ***Dinophysis*** dont les séquences sont choisies parmi x éléments de l'un des ensembles suivants :
- (SEQ ID NO : 29, SEQ ID NO : 30 ou SEQ ID NO : 31)
- (SEQ ID NO : 32 et SEQ ID NO : 33)
- (SEQ ID NO : 34 et SEQ ID NO : 35)
- (SEQ ID NO : 36 et SEQ ID NO : 37)
- (SEQ ID NO : 38 et SEQ ID NO : 39)
- (SEQ ID NO : 40, SEQ ID NO : 41 ou SEQ ID NO : 42)
- (SEQ ID NO : 43, SEQ ID NO : 44 ou SEQ ID NO : 45), ou
- (SEQ ID NO : 46, SEQ ID NO : 47 ou SEQ ID NO : 48)

x étant 2 ou 3,
ou dont les séquences ont au moins 92% d'identité avec les susdites séquences SEQ ID NO : 29 à SEQ ID NO : 48.

Dans un mode de réalisation particulier, est décrit au moins un couple de sondes pour la détection de cellules vivantes actives d'algues toxiques du genre ***Dinophysis,*** les séquences des sondes desdits couples sont les suivantes :
- (SEQ ID NO : 29 et SEQ ID NO : 30), (SEQ ID NO : 29 et SEQ ID NO : 31), (SEQ ID NO : 30 et SEQ ID NO : 31)
- (SEQ ID NO : 32 et SEQ ID NO : 33)
- (SEQ ID NO : 34 et SEQ ID NO : 35)
- (SEQ ID NO : 36 et SEQ ID NO : 37)
- (SEQ ID NO : 38 et SEQ ID NO : 39)
- (SEQ ID NO : 40 et SEQ ID NO : 41), (SEQ ID NO : 40 et SEQ ID NO : 42), (SEQ ID NO : 41 et SEQ ID NO : 42)
- (SEQ ID NO : 43 et SEQ ID NO : 44), (SEQ ID NO : 43 et SEQ ID NO : 45), (SEQ ID NO : 44 et SEQ ID NO : 45)
- (SEQ ID NO : 46 et SEQ ID NO : 47), (SEQ ID NO : 46 et SEQ ID NO : 48), (SEQ ID NO : 47 et SEQ ID NO : 48).

Dans ce second aspect, est décrit au moins un couple de sondes pour la détection de cellules vivantes actives d'algues toxiques du genre ***Pseudo-nitzschia*** dont les séquences sont choisies parmi x éléments de l'un des ensembles suivants :
- (SEQ ID NO : 49 et SEQ ID NO : 50)
- (SEQ ID NO : 51 et SEQ ID NO : 52)
- (SEQ ID NO : 53, SEQ ID NO : 54 ou SEQ ID NO : 55)
- (SEQ ID NO : 56, SEQ ID NO : 57 ou SEQ ID NO : 58)
- (SEQ ID NO : 59, SEQ ID NO : 60 ou SEQ ID NO : 61)

x étant 2 ou 3,
ou dont les séquences ont au moins 92% d'identité avec les susdites séquences SEQ ID NO : 49 à SEQ ID NO : 61.

Dans un mode de réalisation particulier, est décrit au moins un couple de sondes pour la détection de ***Pseudo-nitzschia,*** les séquences des sondes desdits couples sont les suivantes :
- (SEQ ID NO : 49 et SEQ ID NO : 50)
- (SEQ ID NO : 51 et SEQ ID NO : 52)
- (SEQ ID NO : 53 et SEQ ID NO : 54), (SEQ ID NO : 53 et SEQ ID NO : 55), (SEQ ID NO : 54 et SEQ ID NO : 55)
- (SEQ ID NO : 56 et SEQ ID NO : 57), (SEQ ID NO : 56 et SEQ ID NO : 58), (SEQ ID NO : 57 et SEQ ID NO : 58)
- (SEQ ID NO : 59 et SEQ ID NO : 60), (SEQ ID NO : 59 et SEQ ID NO : 61), (SEQ ID NO : 60 et SEQ ID NO : 61).

Dans ce second aspect, est décritau moins un couple de sondes pour la détection de cellules vivantes actives d'algues toxiques du genre ***Prorocentrum*** dont les séquences sont choisies parmi x éléments de l'un des ensembles suivants :
- (SEQ ID NO : 62, SEQ ID NO : 63 ou SEQ ID NO : 64)
- (SEQ ID NO : 65, SEQ ID NO : 66 ou SEQ ID NO : 67)
- (SEQ ID NO : 68, SEQ ID NO : 69 ou SEQ ID NO : 70)
- (SEQ ID NO : 71, SEQ ID NO : 72 ou SEQ ID NO : 73)

x étant 3,
ou dont les séquences ont au moins 92% d'identité avec les susdites séquences SEQ ID NO : 62 à SEQ ID NO : 73.

Dans un mode de réalisation particulier, est décrit au moins un couple de sondes pour la détection de cellules vivantes actives d'algues toxiques du genre ***Prorocentrum,*** les séquences des sondes desdits couples sont les suivantes :
- (SEQ ID NO : 62 et SEQ ID NO : 63), (SEQ ID NO : 62 et SEQ ID NO : 64), (SEQ ID NO : 63 et SEQ ID NO : 64)
- (SEQ ID NO : 65 et SEQ ID NO : 66), (SEQ ID NO : 65 et SEQ ID NO : 67), (SEQ ID NO : 66 et SEQ ID NO : 67)
- (SEQ ID NO : 68 et SEQ ID NO : 69), (SEQ ID NO : 68 et SEQ ID NO : 70), (SEQ ID NO : 69 et SEQ ID NO : 70)
- (SEQ ID NO : 71 et SEQ ID NO : 72), (SEQ ID NO : 71 et SEQ ID NO : 73), (SEQ ID NO : 72 et SEQ ID NO : 73).

Dans ce second aspect, est décrit au moins un couple de sondes pour la détection de cellules vivantes actives d'algues toxiques du genre ***Chattonella*** dont les séquences sont choisies parmi x éléments de l'un des ensembles suivants :
- (SEQ ID NO : 74, SEQ ID NO : 75 ou SEQ ID NO : 76)
- (SEQ ID NO : 77, SEQ ID NO : 78 ou SEQ ID NO : 79)
- (SEQ ID NO : 80, SEQ ID NO : 81 ou SEQ ID NO : 82)

x étant 3,
ou dont les séquences ont au moins 92% d'identité avec les susdites séquences SEQ ID NO : 74 à SEQ ID NO : 82.

Dans un mode de réalisation particulier, est décrit au moins un couple de sondes pour la détection de cellules vivantes actives d'algues toxiques du genre ***Chattonella,*** les séquences des sondes desdits couples sont les suivantes :
- (SEQ ID NO : 74 et SEQ ID NO : 75), (SEQ ID NO : 74 et SEQ ID NO : 76), (SEQ ID NO : 75 et SEQ ID NO : 76)
- (SEQ ID NO : 77 et SEQ ID NO : 78), (SEQ ID NO : 77 et SEQ ID NO : 79), (SEQ ID NO : 78 et SEQ ID NO : 79)
- (SEQ ID NO : 80 et SEQ ID NO : 81), (SEQ ID NO : 80 et SEQ ID NO : 82), (SEQ ID NO : 81 et SEQ ID NO : 82).

Dans ce second aspect, est décrit au moins un couple de sondes pour la détection de cellules vivantes actives d'algues toxiques du genre ***Gymnodinium*** dont les séquences sont choisies parmi x éléments de l'un des ensembles suivants :
- (SEQ ID NO : 83, SEQ ID NO : 84 ou SEQ ID NO : 85)
- (SEQ ID NO : 86, SEQ ID NO : 87 ou SEQ ID NO : 88)
- (SEQ ID NO : 89, SEQ ID NO : 90 ou SEQ ID NO : 91)
- (SEQ ID NO : 92, SEQ ID NO : 93 ou SEQ ID NO : 94)

x étant 3,
ou dont les séquences ont au moins 92% d'identité avec les susdites séquences SEQ ID NO : 83 à SEQ ID NO : 94.

Dans un mode de réalisation particulier, est décrit au moins un couple de sondes pour la détection de cellules vivantes actives d'algues toxiques du genre ***Gymnodinium,*** les séquences des sondes desdits couples sont les suivantes :
- (SEQ ID NO : 83 et SEQ ID NO : 84), (SEQ ID NO : 83 et SEQ ID NO : 85), (SEQ ID NO : 84 et SEQ ID NO : 85)
- (SEQ ID NO : 86 et SEQ ID NO : 87), (SEQ ID NO : 86 et SEQ ID NO : 88), (SEQ ID NO : 87 et SEQ ID NO : 88)
- (SEQ ID NO : 89 et SEQ ID NO : 90), (SEQ ID NO : 89 et SEQ ID NO : 91), (SEQ ID NO : 90 et SEQ ID NO : 91)
- (SEQ ID NO : 92 et SEQ ID NO : 93), (SEQ ID NO : 92 et SEQ ID NO : 94), (SEQ ID NO : 93 et SEQ ID NO : 94).

Dans ce second aspect, est décrit au moins un couple de sondes pour la détection de cellules vivantes actives d'algues toxiques du genre ***Karenia*** dont les séquences sont choisies parmi x éléments de l'un des ensembles suivants :
- (SEQ ID NO : 95, SEQ ID NO : 96 ou SEQ ID NO : 97)
- (SEQ ID NO : 98, SEQ ID NO : 99 ou SEQ ID NO : 100)
- (SEQ ID NO : 101, SEQ ID NO : 102 ou SEQ ID NO : 103)
- (SEQ ID NO : 104, SEQ ID NO : 105 ou SEQ ID NO : 106)
- (SEQ ID NO : 107, SEQ ID NO : 108 ou SEQ ID NO : 109)
- (SEQ ID NO : 110, SEQ ID NO : 111 ou SEQ ID NO : 112)
- (SEQ ID NO : 113, SEQ ID NO : 114 ou SEQ ID NO : 115)
- (SEQ ID NO : 116, SEQ ID NO : 117 ou SEQ ID NO : 118)

x étant 3,
ou dont les séquences ont au moins 92% d'identité avec les susdites séquences SEQ ID NO : 95 à SEQ ID NO : 118.

Dans un mode de réalisation particulier, est décrit au moins un couple de sondes pour la détection de cellules vivantes actives d'algues toxiques du genre ***Karenia,*** les séquences des sondes desdits couples sont les suivantes :
- (SEQ ID NO : 95 et SEQ ID NO : 96), (SEQ ID NO : 95 et SEQ ID NO : 97), (SEQ ID NO : 96 et SEQ ID NO : 97)
- (SEQ ID NO : 98 et SEQ ID NO : 99), (SEQ ID NO : 98 et SEQ ID NO : 100), (SEQ ID NO : 99 et SEQ ID NO : 100)
- (SEQ ID NO : 101 et SEQ ID NO : 102), (SEQ ID NO : 101 et SEQ ID NO : 103), (SEQ ID NO : 102 et SEQ ID NO : 103)
- (SEQ ID NO : 104 et SEQ ID NO : 105), (SEQ ID NO : 104 et SEQ ID NO : 106), (SEQ ID NO : 105 et SEQ ID NO : 106)
- (SEQ ID NO : 107 et SEQ ID NO : 108), (SEQ ID NO : 107 et SEQ ID NO : 109), (SEQ ID NO : 108 et SEQ ID NO : 109)
- (SEQ ID NO : 110 et SEQ ID NO : 111), (SEQ ID NO : 110 et SEQ ID NO : 112), (SEQ ID NO : 111 et SEQ ID NO : 112)
- (SEQ ID NO : 113 et SEQ ID NO : 114), (SEQ ID NO : 113 et SEQ ID NO : 115), (SEQ ID NO : 114 et SEQ ID NO : 115)
- (SEQ ID NO : 116 et SEQ ID NO : 117), (SEQ ID NO : 116 et SEQ ID NO : 118), (SEQ ID NO : 117 et SEQ ID NO : 118).

Dans ce second aspect, est décrit au moins un couple de sondes pour la détection de cellules vivantes actives d'algues toxiques du genre ***Lingulodinium*** dont les séquences sont choisies parmi x éléments de l'un des ensembles suivants :
- (SEQ ID NO : 119, SEQ ID NO : 120 ou SEQ ID NO : 121)
- (SEQ ID NO : 122, SEQ ID NO : 123 ou SEQ ID NO : 124)
- (SEQ ID NO : 125, SEQ ID NO : 126 ou SEQ ID NO : 127)

x étant 3,
ou dont les séquences ont au moins 92% d'identité avec les susdites séquences SEQ ID NO : 119 à SEQ ID NO : 127.

Dans un mode de réalisation particulier, est décrit au moins un couple de sondes pour la détection de cellules vivantes actives d'algues toxiques du genre ***Lingulodinium,*** les séquences des sondes desdits couples sont les suivantes :
- (SEQ ID NO : 119 et SEQ ID NO : 120), (SEQ ID NO : 119 et SEQ ID NO : 121), (SEQ ID NO : 120 et SEQ ID NO : 121)
- (SEQ ID NO : 122 et SEQ ID NO : 123), (SEQ ID NO : 122 et SEQ ID NO : 124), (SEQ ID NO : 123 et SEQ ID NO : 124)
- (SEQ ID NO : 125 et SEQ ID NO : 126), (SEQ ID NO : 125 et SEQ ID NO : 127), (SEQ ID NO : 126 et SEQ ID NO : 127).

Dans ce second aspect, est décrit au moins un couple de sondes pour la détection de cellules vivantes actives d'algues toxiques du genre ***Heterosigma*** dont les séquences sont choisies parmi x éléments de l'un des ensembles suivants :
- (SEQ ID NO : 128 et SEQ ID NO : 129)

x étant 2,
ou dont les séquences ont au moins 92% d'identité avec les susdites séquences SEQ ID NO : 128 à SEQ ID NO : 129.

Selon tous les aspects de ce second aspect, une sonde dudit couple est une sonde capture liée à au moins une molécule d'attache positionnée en 3' ou en 5' de sa séquence et l'autre sonde dudit couple est une sonde signal liée à au moins une molécule de marquage positionnée en 3' ou en 5' de sa séquence.

Dans un mode de réalisation particulier de ce second aspect, ladite sonde capture est liée à au moins une molécule d'attache positionnée en 5' de sa séquence et ladite sonde signal est liée à au moins une molécule de marquage positionnée en 5' de sa séquence.

Dans un autre mode de réalisation particulier de ce second aspect, ladite sonde capture est liée à au moins une molécule d'attache positionnée en 5' de sa séquence et ladite sonde signal est liée à au moins une molécule de marquage positionnée en 3' de sa séquence.

Dans un autre mode de réalisation particulier de ce second aspect, ladite sonde capture est liée à au moins une molécule d'attache positionnée en 3' de sa séquence et ladite sonde signal est liée à au moins une molécule de marquage positionnée en 5' de sa séquence.

Dans un autre mode de réalisation particulier de ce second aspect, ladite sonde capture est liée à au moins une molécule d'attache positionnée en 3' de sa séquence et ladite sonde signal est liée à au moins une molécule de marquage positionnée en 3' de sa séquence.

Dans tous les aspects de ce second aspect, la molécule d'attache peut être choisie parmi une molécule de biotine, avidine, streptavidine, un groupe thiol, un groupe amine et un carbone.

Dans un mode de réalisation particulièrement préféré, la molécule d'attache est une molécule de biotine.

Dans tous les aspects de ce second aspect, la molécule de marquage peut être choisie parmi la un fluorochrome, une biotine, une molécule liée à une biotine, la digoxigénine, une enzyme utilisant un substrat chimioluminescent, une enzyme utilisant un substrat chromogène ou une enzyme utilisant un substrat à oxydation électrochimique.

Dans un mode de réalisation particulièrement préféré, la molécule de marquage est la digoxigénine.

Selon ce second aspect, ledit fluorochrome peut être choisi parmi le groupe consistant en : Alexa fluor, en particulier Alexa fluor 350, 405, 430, 488, 500, 514, 532, 546, 555, 568, 594, 610, 633, 647, 660, 680, 700, 750 ou 790, Isothiocyanate de fluorescéine (FITC), Rhodamine, Allophycocyanine (APC) et Phycoérythrine (PE).

Selon ce second aspect, ladite enzyme utilisant un substrat chimioluminescent peut être la péroxydase de raifort (HRP) et ledit substrat chimioluminescent peut être le luminol, ou encore, ladite enzyme utilisant un substrat chimioluminescent peut être la luciférase et ledit substrat chimioluminescent peut être la luciférine.

Selon ce second aspect, ladite enzyme utilisant un substrat chromogène peut être la phosphatase alcaline et ledit substrat chromogène peut être Nitrobleu de tetrazolium (NBT) ou le bromochlorylindolophosphate (BCIP), ou encore, ladite enzyme utilisant un substrat chromogène peut être la péroxydase de raifort (HRP) et ledit substrat chromogène peut être choisi parmi le 3,3'-Diaminobenzidine (DAB), le 3,3',5,5'-Tetramethylbenzidine (TMB), ou le 2,2'-azino-bis(acide 3-ethylbenzothiazoline-6-sulphonique) (ABTS).

Selon ce second aspect, ladite enzyme utilisant un substrat à oxydation électrochimique peut être la péroxydase de raifort (HRP) et ledit substrat à oxydation électrochimique peut être le 3,3',5,5'-Tetramethylbenzidine (TMB).

Dans un troisième aspect ne faisant pas partie de l'invention, sont décrites des sondes pour la détection de cellules vivantes actives d'algues toxiques.

Dans ce troisième aspect, est décrite au moins une sonde pour la détection de cellules vivantes actives d' algues toxiques du genre ***Alexandrium,*** ladite sonde ayant une séquence choisie parmi les séquences SEQ ID NO : 1, SEQ ID NO : 2, SEQ ID NO : 3, SEQ ID NO : 4, SEQ ID NO : 7, SEQ ID NO : 8, SEQ ID NO : 10, SEQ ID NO : 11, SEQ ID NO : 13, SEQ ID NO : 14, SEQ ID NO : 16, SEQ ID NO : 17, SEQ ID NO : 19, SEQ ID NO : 20, SEQ ID NO : 22, SEQ ID NO : 23, SEQ ID NO : 25, SEQ ID NO : 26, SEQ ID NO : 28 ou la séquence de ladite sonde ayant au moins 92% d'identité avec les susdites séquences SEQ ID NO : 1, SEQ ID NO: 2, SEQ ID NO : 3, SEQ ID NO : 4, SEQ ID NO : 7, SEQ ID NO : 8, SEQ ID NO : 10, SEQ ID NO : 11, SEQ ID NO : 13, SEQ ID NO : 14, SEQ ID NO : 16, SEQ ID NO : 17, SEQ ID NO : 19, SEQ ID NO : 20, SEQ ID NO : 22, SEQ ID NO : 23, SEQ ID NO : 25, SEQ ID NO : 26, SEQ ID NO : 28.

Dans ce troisième aspect, est décrite au moins une sonde pour la détection de cellules vivantes actives d'algues toxiques du genre ***Dinophysis**,* ladite sonde ayant une séquence choisie parmi les séquences SEQ ID NO : 30, SEQ ID NO : 31, SEQ ID NO : 33, SEQ ID NO : 35, SEQ ID NO : 37, SEQ ID NO : 38, SEQ ID NO : 39, SEQ ID NO : 41, SEQ ID NO : 42, SEQ ID NO : 44, SEQ ID NO : 45, SEQ ID NO : 47, SEQ ID NO : 48 ou la séquence de ladite sonde ayant au moins 92% d'identité avec les susdites séquences SEQ ID NO : 30, SEQ ID NO : 31, SEQ ID NO : 33, SEQ ID NO : 35, SEQ ID NO : 37, SEQ ID NO : 38, SEQ ID NO : 39, SEQ ID NO : 41, SEQ ID NO : 42, SEQ ID NO : 44, SEQ ID NO : 45, SEQ ID NO : 47, SEQ ID NO : 48.

Dans ce troisième aspect, est décrite au moins une sonde pour la détection de cellules vivantes actives d'algues toxiques du genre ***Pseudo-nitzschia**,* ladite sonde ayant une séquence choisie parmi les séquences SEQ ID NO : 49, SEQ ID NO : 51, SEQ ID NO : 52, SEQ ID NO : 54, SEQ ID NO : 55, SEQ ID NO : 57, SEQ ID NO : 58, SEQ ID NO : 60, SEQ ID NO : 61 ou la séquence de ladite sonde ayant au moins 92% d'identité avec les susdites séquences SEQ ID NO : 49, SEQ ID NO : 51, SEQ ID NO : 52, SEQ ID NO : 54, SEQ ID NO : 55, SEQ ID NO : 57, SEQ ID NO : 58, SEQ ID NO : 60, SEQ ID NO : 61.

Dans ce troisième aspect, est décrite au moins une sonde pour la détection de cellules vivantes actives d'algues toxiques du genre ***Prorocentrum**,* ladite sonde ayant une séquence choisie parmi les séquences SEQ ID NO : 63, SEQ ID NO : 64, SEQ ID NO : 66, SEQ ID NO : 67, SEQ ID NO : 69, SEQ ID NO : 70, SEQ ID NO : 72, SEQ ID NO : 73 ou la séquence de ladite sonde ayant au moins 92% d'identité avec les susdites séquences SEQ ID NO : 63, SEQ ID NO : 64, SEQ ID NO : 66, SEQ ID NO : 67, SEQ ID NO : 69, SEQ ID NO : 70, SEQ ID NO : 72, SEQ ID NO : 73.

Dans ce troisième aspect, est décrite au moins une sonde pour la détection de cellules vivantes actives d'algues toxiques du genre ***Chattonella**,* ladite sonde ayant une séquence choisie parmi les séquences SEQ ID NO : 75, SEQ ID NO : 76, SEQ ID NO : 77, SEQ ID NO : 79, SEQ ID NO : 80, SEQ ID NO : 82 ou la séquence de ladite sonde ayant au moins 92% d'identité avec les susdites séquences SEQ ID NO : 75, SEQ ID NO : 76, SEQ ID NO : 77, SEQ ID NO : 79, SEQ ID NO : 80, SEQ ID NO : 82.

Dans ce troisième aspect, est décrite au moins une sonde pour la détection de cellules vivantes actives d'algues toxiques du genre ***Gymnodinium,*** ladite sonde ayant une séquence choisie parmi les séquences SEQ ID NO : 84, SEQ ID NO : 85, SEQ ID NO : 87, SEQ ID NO : 88, SEQ ID NO : 90, SEQ ID NO : 91, SEQ ID NO : 93, SEQ ID NO : 94 ou la séquence de ladite sonde ayant au moins 92% d'identité avec les susdites séquences SEQ ID NO : 84, SEQ ID NO : 85, SEQ ID NO : 87, SEQ ID NO : 88, SEQ ID NO : 90, SEQ ID NO : 91, SEQ ID NO : 93, SEQ ID NO : 94.

Dans ce troisième aspect, est décrite au moins une sonde pour la détection de cellules vivantes actives d'algues toxiques du genre ***Karenia,*** ladite sonde ayant une séquence choisie parmi les séquences SEQ ID NO : 96, SEQ ID NO : 97, SEQ ID NO : 99, SEQ ID NO : 100, SEQ ID NO : 102, SEQ ID NO : 103, SEQ ID NO : 105, SEQ ID NO : 106, SEQ ID NO : 108, SEQ ID NO : 109, SEQ ID NO : 111, SEQ ID NO : 112, SEQ ID NO : 114, SEQ ID NO : 115, SEQ ID NO : 117, SEQ ID NO : 118 ou la séquence de ladite sonde ayant au moins 92% d'identité avec les susdites séquences SEQ ID NO : 96, SEQ ID NO : 97, SEQ ID NO : 99, SEQ ID NO : 100, SEQ ID NO : 102, SEQ ID NO : 103, SEQ ID NO : 105, SEQ ID NO : 106, SEQ ID NO : 108, SEQ ID NO : 109, SEQ ID NO : 111, SEQ ID NO : 112, SEQ ID NO : 114, SEQ ID NO : 115, SEQ ID NO : 117, SEQ ID NO : 118.

Dans ce troisième aspect, est décrite au moins une sonde pour la détection de cellules vivantes actives d'algues toxiques du genre ***Lingulodinium,*** ladite sonde ayant une séquence choisie parmi les séquences SEQ ID NO : 120, SEQ ID NO : 121, SEQ ID NO : 123, SEQ ID NO : 124, SEQ ID NO : 126, SEQ ID NO : 127 ou la séquence de ladite sonde ayant au moins 92% d'identité avec les susdites séquences SEQ ID NO : 120, SEQ ID NO : 121, SEQ ID NO : 123, SEQ ID NO : 124, SEQ ID NO : 126, SEQ ID NO : 127.

Dans ce troisième aspect, est décrite au moins une sonde pour la détection de cellules vivantes actives d'algues toxiques du genre ***Heterosigma,*** ladite sonde ayant une séquence choisie parmi les séquences SEQ ID NO : 128 ou la séquence de ladite sonde ayant au moins 92% d'identité avec les sus desdites séquences SEQ ID NO : 128.

Selon tous les aspects de ce troisième aspect, ladite sonde est liée à au moins une molécule d'attache en 3' ou en 5' de sa séquence ou à au moins une molécule de marquage en 3' ou en 5' de sa séquence.

Dans un aspect particulier, ladite sonde est liée à au moins une molécule d'attache en 3' de sa séquence.

Dans un autre aspect particulier, ladite sonde est liée à au moins une molécule d'attache en 5' de sa séquence.

Dans un autre aspect particulier, ladite sonde est liée à au moins une molécule de marquage en 3' de sa séquence.

Dans un autre aspect particulier, ladite sonde est liée à au moins une molécule de marquage en 5' de sa séquence.

Selon tous les aspects de ce troisième aspect, la « *au moins une molécule d'attache* » peut être choisie parmi une molécule de biotine, avidine, streptavidine, un groupe thiol, un groupe amine et un carbone.

Dans un mode de réalisation particulièrement préféré, la « *au moins une molécule d'attache »* est une molécule de biotine.

Selon tous les aspects de ce troisième aspect, la « *au moins une molécule de marquage »* peut être choisie parmi un fluorochrome, une biotine, une molécule liée à une biotine, la digoxigénine, une enzyme utilisant un substrat chimioluminescent, une enzyme utilisant un substrat chromogène ou une enzyme utilisant un substrat à oxydation électrochimique.

Dans un mode de réalisation particulièrement préféré, la *« au moins une molécule de marquage »* est la digoxigénine.

Selon ce troisième aspect, ledit fluorochrome peut être choisi parmi le groupe consistant en : Alexa fluor, en particulier Alexa fluor 350, 405, 430, 488, 500, 514, 532, 546, 555, 568, 594, 610, 633, 647, 660, 680, 700, 750 ou 790, Isothiocyanate de fluorescéine (FITC), Rhodamine, Allophycocyanine (APC) et Phycoérythrine (PE).

Selon ce troisième aspect, ladite enzyme utilisant un substrat chimioluminescent peut être la péroxydase de raifort (HRP) et ledit substrat chimioluminescent peut être le luminol, ou encore, ladite enzyme utilisant un substrat chimioluminescent peut être la luciférase et ledit substrat chimioluminescent peut être la luciférine.

Selon ce troisième aspect, ladite enzyme utilisant un substrat chromogène peut être la phosphatase alcaline et ledit substrat chromogène peut être Nitrobleu de tetrazolium (NBT) ou le bromochlorylindolophosphate (BCIP), ou encore, ladite enzyme utilisant un substrat chromogène peut être la péroxydase de raifort (HRP) et ledit substrat chromogène peut être choisi parmi le 3,3'-Diaminobenzidine (DAB), le 3,3',5,5'-Tetramethylbenzidine (TMB), ou le 2,2'-azino-bis(acide 3-ethylbenzothiazoline-6-sulphonique) (ABTS).

Selon ce troisième aspect, ladite enzyme utilisant un substrat à oxydation électrochimique peut être la péroxydase de raifort (HRP) et ledit substrat à oxydation électrochimique peut être le 3,3',5,5'-Tetramethylbenzidine (TMB).

Un quatrième aspect concerne un procédé de détection de cellules vivantes actives d'algues toxiques.

### Mode de réalisation A

Un mode de réalisation ne faisant pas partie de l'invention concerne un procédé de détection de cellules vivantes actives d'algues toxiques dans un échantillon susceptible de contenir au moins une algue toxique du genre ***Alexandrium*** comprenant les étapes suivantes :
a) hybridation éventuelle résultant de la mise en contact dudit échantillon avec une sonde capture et une sonde signal spécifiques d'algues toxiques du genre ***Alexandrium,*** la sonde capture et la sonde signal formant un couple de sondes, les séquences dudit couple de sondes étant choisies parmi x éléments de l'un des ensembles suivants :
   - (SEQ ID NO : 1, SEQ ID NO : 2 ou SEQ ID NO : 3)
   - (SEQ ID NO : 4 et SEQ ID NO : 5)
   - (SEQ ID NO : 6 et SEQ ID NO : 7)
   - (SEQ ID NO : 8, SEQ ID NO : 9 ou SEQ ID NO : 10)
   - (SEQ ID NO : 11, SEQ ID NO : 12 ou SEQ ID NO : 13)
   - (SEQ ID NO : 14, SEQ ID NO : 15 ou SEQ ID NO : 16)
   - (SEQ ID NO : 17, SEQ ID NO : 18 ou SEQ ID NO : 19)
   - (SEQ ID NO : 20, SEQ ID NO : 21 ou SEQ ID NO : 22),
   - (SEQ ID NO : 23, SEQ ID NO : 24 ou SEQ ID NO : 25),
   - (SEQ ID NO : 26, SEQ ID NO : 27 ou SEQ ID NO : 28)

   x étant 2 ou 3,
   ou les séquences desdites sondes ayant au moins 92% d'identité avec les susdites séquences SEQ ID NO : 1 à SEQ ID NO : 28
   ladite sonde capture et ladite sonde signal étant capables de s'hybrider avec l'acide nucléique ribosomique d'une algue toxique du genre ***Alexandrium*** éventuellement présent dans ledit échantillon afin de former un complexe
b) détection dudit complexe éventuel
   l'hybridation indiquant la présence d'algue toxique du genre ***Alexandrium**,*
   le seuil de détection minimal de l'algue toxique du genre ***Alexandrium*** étant
      - de 100 à 500 cellules vivantes actives par litre d'échantillon (cellules/L) et en particulier inférieur à 200 cellules vivantes actives par litre d'échantillon (cellules/L), ou
      - inférieur ou égal à 0,10 ng d'ARN par litre d'échantillon et en particulier compris de 0,01 à 0,09 ng d'ARN par litre d'échantillon.

Comme indiqué précédemment, un seuil de détection minimal de l'algue toxique du genre ***Alexandrium*** inférieur à 200 cellules vivantes actives par litre d'échantillon (cellules/L) correspond également à un seuil de détection minimal compris de 0,01 ng à 0,09 ng d'ARN par litre d'échantillon.

Est également décrit un procédé de détection de cellules vivantes actives d'algues toxiques dans un échantillon susceptible de contenir au moins une algue toxique du genre *Alexandrium,* tel que décrit ci-dessus, dans lequel la durée de la mise en œuvre dudit procédé de détection est inférieure à une heure.

Ainsi, un mode de réalisation ne faisant pas partie de l'invention concerne un procédé de détection de cellules vivantes actives d'algues toxiques dans un échantillon susceptible de contenir au moins une algue toxique du genre ***Alexandrium*** comprenant les étapes suivantes :
a) hybridation éventuelle résultant de la mise en contact dudit échantillon avec une sonde capture et une sonde signal spécifiques d'algues toxiques du genre ***Alexandrium**,* la sonde capture et la sonde signal formant un couple de sondes, les séquences dudit couple de sondes étant choisies parmi x éléments de l'un des ensembles suivants :
   - (SEQ ID NO : 1, SEQ ID NO : 2 ou SEQ ID NO : 3)
   - (SEQ ID NO : 4 et SEQ ID NO : 5)
   - (SEQ ID NO : 6 et SEQ ID NO : 7)
   - (SEQ ID NO : 8, SEQ ID NO : 9 ou SEQ ID NO : 10)
   - (SEQ ID NO : 11, SEQ ID NO : 12 ou SEQ ID NO : 13)
   - (SEQ ID NO : 14, SEQ ID NO : 15 ou SEQ ID NO : 16)
   - (SEQ ID NO : 17, SEQ ID NO : 18 ou SEQ ID NO : 19)
   - (SEQ ID NO : 20, SEQ ID NO : 21 ou SEQ ID NO : 22),
   - (SEQ ID NO : 23, SEQ ID NO : 24 ou SEQ ID NO : 25),
   - (SEQ ID NO : 26, SEQ ID NO : 27 ou SEQ ID NO : 28)

   x étant 2 ou 3,
   ou les séquences desdites sondes ayant au moins 92% d'identité avec les susdites séquences SEQ ID NO : 1 à SEQ ID NO : 28
   ladite sonde capture et ladite sonde signal étant capables de s'hybrider avec l'acide nucléique ribosomique d'une algue toxique du genre ***Alexandrium*** éventuellement présent dans ledit échantillon afin de former un complexe
b) détection dudit complexe éventuel
   l'hybridation indiquant la présence d'algue toxique du genre ***Alexandrium,***
   la durée de la mise en œuvre dudit procédé de détection étant inférieure à une heure.

Dans ce mode de réalisation, est décrit un procédé de détection de cellules vivantes actives d'algues toxiques dans un échantillon susceptible de contenir au moins une algue toxique du genre ***Alexandrium,*** tel que décrit précédement, dans lequel le seuil de détection minimal de l'algue toxique du genre ***Alexandrium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon,
et la durée de la mise en œuvre dudit procédé de détection est inférieure à une heure

Dans un mode de réalisation particulier, l'invention a pour objet un procédé de détection de cellules vivantes actives d'algues toxiques dans un échantillon naturel susceptible de contenir au moins une algue toxique du genre *Alexandrium* comprenant les étapes suivantes :
a) hybridation éventuelle résultant de la mise en contact des ARN extraits totaux dudit échantillon avec une sonde capture et une sonde signal spécifiques d'algues toxique du genre *Alexandrium,* la sonde capture et la sonde signal formant un couple de sondes, les séquences des sondes desdits couples étant les suivantes :
   - (SEQ ID NO : 1 et SEQ ID NO : 2),
   - (SEQ ID NO : 4 et SEQ ID NO : 5),
   - (SEQ ID NO : 11 et SEQ ID NO : 12), ou
   - (SEQ ID NO : 17 et SEQ ID NO : 18),
   ladite sonde capture et ladite sonde signal étant capables de s'hybrider avec l'ARN ribosomique d'une algue toxique du genre *Alexandrium* éventuellement présent dans ledit échantillon afin de former un complexe ;
b) détection dudit complexe éventuel
   l'hybridation indiquant la présence d'algue toxique du genre *Alexandrium,*
la durée de la mise en œuvre dudit procédé de détection étant en particulier inférieure à une heure.

Dans un mode de réalisation particulier, le procédé de détection de cellules vivantes actives d'algues toxiques dans un échantillon susceptible de contenir au moins une algue toxique du genre ***Alexandrium**,* tel que décrit précédemment, peut en outre comprendre, avant l'étape d'hybridation éventuelle, une étape de préparation dudit échantillon à analyser afin d'obtenir un échantillon préparé.

Dans un mode de réalisation particulier, le procédé de détection de cellules vivantes actives d'algues toxiques dans un échantillon susceptible de contenir au moins une algue toxique du genre ***Alexandrium**,* tel que décrit préécdemment, peut en outre comprendre une étape de quantification des algues toxiques du genre ***Alexandrium*** dans le cas d'une hybridation indiquant la présence d'algue toxique du genre ***Alexandrium**.*

Ainsi, un mode de réalisation ne faisant pas partie de l'invention concerne un procédé de détection de cellules vivantes actives d'algues toxiques dans un échantillon susceptible de contenir au moins une algue toxique du genre ***Alexandrium*** comprenant les étapes suivantes :
a) préparation dudit échantillon à analyser afin d'obtenir un échantillon préparé
b) hybridation éventuelle résultant de la mise en contact dudit échantillon préparé avec une sonde capture et une sonde signal spécifiques d'algues toxiques du genre ***Alexandrium,*** la sonde capture et la sonde signal formant un couple de sondes, les séquences dudit couple de sondes étant choisies parmi x éléments de l'un des ensembles suivants :
   - (SEQ ID NO : 1, SEQ ID NO : 2 ou SEQ ID NO : 3)
   - (SEQ ID NO : 4 et SEQ ID NO : 5)
   - (SEQ ID NO : 6 et SEQ ID NO : 7)
   - (SEQ ID NO : 8, SEQ ID NO : 9 ou SEQ ID NO : 10)
   - (SEQ ID NO : 11, SEQ ID NO : 12 ou SEQ ID NO : 13)
   - (SEQ ID NO : 14, SEQ ID NO : 15 ou SEQ ID NO : 16)
   - (SEQ ID NO : 17, SEQ ID NO : 18 ou SEQ ID NO : 19)
   - (SEQ ID NO : 20, SEQ ID NO : 21 ou SEQ ID NO : 22),
   - (SEQ ID NO : 23, SEQ ID NO : 24 ou SEQ ID NO : 25),
   - (SEQ ID NO : 26, SEQ ID NO : 27 ou SEQ ID NO : 28)

   x étant 2 ou 3,
   ou les séquences desdites sondes ayant au moins 92% d'identité avec les susdites séquences SEQ ID NO : 1 à SEQ ID NO : 28
   ladite sonde capture et ladite sonde signal étant capables de s'hybrider avec l'acide nucléique ribosomique d'une algue toxique du genre ***Alexandrium*** éventuellement présent dans ledit échantillon afin de former un complexe
c) détection dudit complexe éventuel
d) quantification des algues toxiques du genre ***Alexandrium,*** dans le cas d'une hybridation
   l'hybridation indiquant la présence d'algue toxique du genre ***Alexandrium,***
   le seuil de détection minimal de l'algue toxique du genre ***Alexandrium*** étant
      - de 100 à 500 cellules vivantes actives par litre d'échantillon (cellules/L) et en particulier inférieur à 200 cellules vivantes actives par litre d'échantillon (cellules/L), ou
      - inférieur ou égal à 0,10 ng d'ARN par litre d'échantillon et en particulier compris de 0,01 à 0,09 ng d'ARN par litre d'échantillon.

Comme indiqué précédemment, un seuil de détection minimal de l'algue toxique du genre ***Alexandrium*** inférieur à 200 cellules vivantes actives par litre d'échantillon (cellules/L) correspond également à un seuil de détection minimal compris de 0,01 ng à 0,09 ng d'ARN par litre d'échantillon.

Un autre mode de réalisation ne faisant pas partie de l'invention concerne un procédé de détection de cellules vivantes actives d'algues toxiques dans un échantillon susceptible de contenir au moins une algue toxique du genre ***Alexandrium*** comprenant les étapes suivantes :
a) préparation dudit échantillon à analyser afin d'obtenir un échantillon préparé
b) hybridation éventuelle résultant de la mise en contact dudit échantillon préparé avec une sonde capture et une sonde signal spécifiques d'algues toxiques du genre ***Alexandrium**,* la sonde capture et la sonde signal formant un couple de sondes, les séquences dudit couple de sondes étant choisies parmi x éléments de l'un des ensembles suivants :
   - (SEQ ID NO : 1, SEQ ID NO : 2 ou SEQ ID NO : 3)
   - (SEQ ID NO : 4 et SEQ ID NO : 5)
   - (SEQ ID NO : 6 et SEQ ID NO : 7)
   - (SEQ ID NO : 8, SEQ ID NO : 9 ou SEQ ID NO : 10)
   - (SEQ ID NO : 11, SEQ ID NO : 12 ou SEQ ID NO : 13)
   - (SEQ ID NO : 14, SEQ ID NO : 15 ou SEQ ID NO : 16)
   - (SEQ ID NO : 17, SEQ ID NO : 18 ou SEQ ID NO : 19)
   - (SEQ ID NO : 20, SEQ ID NO : 21 ou SEQ ID NO : 22),
   - (SEQ ID NO : 23, SEQ ID NO : 24 ou SEQ ID NO : 25),
   - (SEQ ID NO : 26, SEQ ID NO : 27 ou SEQ ID NO : 28)

   x étant 2 ou 3,
   ou les séquences desdites sondes ayant au moins 92% d'identité avec les susdites séquences SEQ ID NO : 1 à SEQ ID NO : 28
   ladite sonde capture et ladite sonde signal étant capables de s'hybrider avec l'acide nucléique ribosomique d'une algue toxique du genre ***Alexandrium*** éventuellement présent dans ledit échantillon afin de former un complexe
c) détection dudit complexe éventuel
d) quantification des algues toxiques du genre ***Alexandrium,*** dans le cas d'une hybridation
   l'hybridation indiquant la présence d'algue toxique du genre ***Alexandrium***
   la durée de la mise en œuvre des étapes b) et c) étant inférieure à une heure.

Dans un mode de réalisation particulier, est décrit un procédé de détection de cellules vivantes actives d'algues toxiques dans un échantillon susceptible de contenir au moins une algue toxique du genre ***Alexandrium,*** tel que décrit ci-dessus, dans lequel le seuil de détection minimal de l'algue toxique du genre ***Alexandrium*** est inférieur à 200 cellules vivantes actives par litre d'échantillon (cellules/L) ou inférieur ou égal à 0,10 ng d'ARN par litre d'échantillon et la mise en œuvre des étapes b) et c) est inférieure à une heure.

Ainsi, un mode de réalisation ne faisant pas partie de l'invention concerne un procédé de détection de cellules vivantes actives d'algues toxiques dans un échantillon susceptible de contenir au moins une algue toxique du genre *Alexandrium* comprenant les étapes suivantes :
a) préparation dudit échantillon à analyser afin d'obtenir un échantillon préparé
b) hybridation éventuelle résultant de la mise en contact dudit échantillon préparé avec une sonde capture et une sonde signal spécifiques d'algues toxiques du genre *Alexandrium,* la sonde capture et la sonde signal formant un couple de sondes, les séquences dudit couple de sondes étant choisies parmi x éléments de l'un des ensembles suivants :
   - (SEQ ID NO : 1, SEQ ID NO : 2 ou SEQ ID NO : 3)
   - (SEQ ID NO : 4 et SEQ ID NO : 5)
   - (SEQ ID NO : 6 et SEQ ID NO : 7)
   - (SEQ ID NO : 8, SEQ ID NO : 9 ou SEQ ID NO : 10)
   - (SEQ ID NO : 11, SEQ ID NO : 12 ou SEQ ID NO : 13)
   - (SEQ ID NO : 14, SEQ ID NO : 15 ou SEQ ID NO : 16)
   - (SEQ ID NO : 17, SEQ ID NO : 18 ou SEQ ID NO : 19)
   - (SEQ ID NO : 20, SEQ ID NO : 21 ou SEQ ID NO : 22),
   - (SEQ ID NO : 23, SEQ ID NO : 24 ou SEQ ID NO : 25),
   - (SEQ ID NO : 26, SEQ ID NO : 27 ou SEQ ID NO : 28)

   x étant 2 ou 3,
   ou les séquences desdites sondes ayant au moins 92% d'identité avec les susdites séquences SEQ ID NO : 1 à SEQ ID NO : 28,
   ladite sonde capture et ladite sonde signal étant capables de s'hybrider avec l'acide nucléique ribosomique d'une algue toxique du genre *Alexandrium* éventuellement présent dans ledit échantillon afin de former un complexe
c) détection dudit complexe éventuel
d) quantification des algues toxiques du genre ***Alexandrium,*** dans le cas d'une hybridation,
   l'hybridation indiquant la présence d'algue toxique du genre ***Alexandrium,***
   le seuil de détection minimal de l'algue toxique du genre ***Alexandrium*** étant
      - de 100 à 500 cellules vivantes actives par litre d'échantillon (cellules/L) et en particulier inférieur à 200 cellules vivantes actives par litre d'échantillon (cellules/L), ou
      - inférieur ou égal à 0,10 ng d'ARN par litre d'échantillon et en particulier compris de 0,01 à 0,09 ng d'ARN par litre d'échantillon,
   la durée de la mise en œuvre des étapes b) et c) étant inférieure à une heure.

Dans un mode de réalisation particulier, est décrit un procédé de détection de cellules vivantes actives d'algues toxiques dans un échantillon susceptible de contenir au moins une algue toxique du genre ***Alexandrium*** tel que décrit ci-dessus dans lequel ladite étape de préparation dudit échantillon à analyser comprend les étapes suivantes :
a) une étape de concentration dudit l'échantillon afin d'obtenir un échantillon concentré
b) une étape de lyse des algues toxiques éventuellement présentes dans ledit échantillon, résultant en la libération des acides nucléiques ribosomique des algues toxiques du genre ***Alexandrium*** susceptibles d'être contenus dans ledit échantillon.

Dans un mode de réalisation particulier, est décrit un procédé de détection de cellules vivantes actives d'algues toxiques dans un échantillon susceptible de contenir au moins une algue toxique du genre ***Alexandrium*** tel que décrit ci-dessus, pouvant en outre comprendre une étape d'extraction et de purification des acides nucléiques ribosomique obtenus suite à l'étape de lyse b) à l'aide de protocole d'extraction et purification des acides nucléiques connu de l'homme de l'art.

Dans un mode de réalisation particulier, est décrit un procédé de détection de cellules vivantes actives d'algues toxiques dans un échantillon susceptible de contenir au moins une algue toxique du genre ***Alexandrium*** tel que décrit ci-dessus, pouvant en outre comprendre une étape de fragmentation des acides nucléiques ribosomique obtenus suite à l'étape de lyse b) afin d'homogénéiser la taille des acides nucléiques extraits et purifiés.

Dans un mode de réalisation particulier, ladite étape de concentration dudit échantillon peut être une étape de centrifugation ou de filtration.

Dans un mode de réalisation particulier, ladite filtration peut être effectuée sur des filtres de nylon ou de polycarbonates. Lesdits filtres peuvent par exemple avoir une porosité de 0,2 à 100 µm.

Dans un mode de réalisation particulier, ladite étape de lyse peut être une étape de lyse chimique comprenant l'ajout d'une solution de lyse audit échantillon concentré obtenu à l'étape a) décrite précédemment.

Dans un mode de réalisation particulier, ladite solution de lyse peut comprendre un tampon neutre, un agent chaotropique, un détergent ionique ou non ionique, un agent réducteur et un agent chélateur.

Le tampon neutre peut par exemple être du phosphate, du Saline Sodium Citrate (SSC) ou du Tris. L'agent chaotropique peut par exemple être du guanidium chloride. Le détergent ionique ou non ionique peut par exemple être du sodium dodecyl sulfate (SDS) ou du Triton^{®} X100. L'agent réducteur peut par exemple être du b-mercaptoethanol ou du DiThioTreitol. L'agent chélateur peut par exemple être de l'acide Éthylène Diamine Tétra Acétique (EDTA) ou de l'acide Éthylène Glycol Tétra Acétique (EGTA).

Dans un mode de réalisation particulier, ladite étape de lyse chimique peut être accompagnée d'une lyse thermique, d'une lyse sonique et/ou d'une lyse mécanique. La lyse thermique peut par exemple pêtre effectuée avec de l'azote liquide ou en chauffant ledit échantillon. La lyse sonique peut par exemple être effectuée à l'aide d'ultrason ou de vibrations. La lyse mécanique peut par exemple être effectuée à l'aide d'un vortex ou d'un broyage.

Dans un mode de réalisation particulier, est décrit un procédé de détection de cellules vivantes actives d'algues toxiques dans un échantillon susceptible de contenir au moins une algue toxique du genre ***Alexandrium*** tel que décrit précédemment dans lequel ladite sonde capture est liée à au moins une molécule d'attache positionnée en 5' de sa séquence et ladite sonde signal est liée à au moins une molécule de marquage positionnée en 5' de sa séquence.

Dans un autre mode de réalisation particulier, est décrit un procédé de détection de cellules vivantes actives d'algues toxiques dans un échantillon susceptible de contenir au moins une algue toxique du genre ***Alexandrium*** tel que décrit précédemment dans lequel ladite sonde capture est liée à au moins une molécule d'attache positionnée en 5' de sa séquence et ladite sonde signal est liée à au moins une molécule de marquage positionnée en 3' de sa séquence. Dans un autre mode de réalisation particulier, est décrit un procédé de détection de cellules vivantes actives d'algues toxiques dans un échantillon susceptible de contenir au moins une algue toxique du genre ***Alexandrium*** tel que décrit précédemment dans lequel ladite sonde capture est liée à au moins une molécule d'attache positionnée en 3' de sa séquence et ladite sonde signal est liée à au moins une molécule de marquage positionnée en 5' de sa séquence. Dans un autre mode de réalisation particulier, est décrit un procédé de détection de cellules vivantes actives d'algues toxiques dans un échantillon susceptible de contenir au moins une algue toxique du genre ***Alexandrium*** tel que décrit précédemment dans lequel ladite sonde capture est liée à au moins une molécule d'attache positionnée en 3' de sa séquence et ladite sonde signal est liée à au moins une molécule de marquage positionnée en 3' de sa séquence. Dans un mode de réalisation particulier, la molécule d'attache peut être choisie parmi une molécule de biotine, avidine, streptavidine, un groupe thiol, un groupe amine et un groupe carbone.

Dans un mode de réalisation particulièrement préféré, ladite molécule d'attache est une molécule de biotine.

Dans un mode de réalisation particulier, la molécule de marquage peut être choisie parmi un fluorochrome, une biotine, une molécule liée à une biotine, la digoxigénine, une enzyme utilisant un substrat chimioluminescent, une enzyme utilisant un substrat chromogène ou une enzyme utilisant un substrat à oxydation électrochimique.

Dans un mode de réalisation particulièrement préféré, la molécule de marquage est la digoxigénine.

Dans un mode de réalisation du procédé de détection tel que décrit ci-dessus, dans lequel ladite sonde capture est liée à au moins une molécule d'attache positionnée en 5' de sa séquence et ladite sonde signal est liée à au moins une molécule de marquage positionnée en 5' de sa séquence, ou
ladite sonde capture est liée à au moins une molécule d'attache positionnée en 5' de sa séquence et ladite sonde signal est liée à au moins une molécule de marquage positionnée en 3' de sa séquence, ou
ladite sonde capture est liée à au moins une molécule d'attache positionnée en 3' de sa séquence et ladite sonde signal est liée à au moins une molécule de marquage positionnée en 5' de sa séquence, ou
ladite sonde capture est liée à au moins une molécule d'attache positionnée en 3' de sa séquence et ladite sonde signal est liée à au moins une molécule de marquage positionnée en 3' de sa séquence,
ladite « au moins une molécule d'attache »étant notamment choisie parmi une molécule de biotine, avidine, streptavidine, un groupe thiol, un groupe amine et un groupe carbone, de préférence une molécule de biotine,
ladite « au moins une molécule de marquage »étant notamment choisie parmi un fluorochrome, une biotine, une molécule liée à une biotine, la digoxigénine, une enzyme utilisant un substrat chimioluminescent, une enzyme utilisant un substrat chromogène ou une enzyme utilisant un substrat à oxydation électrochimique, de préférence la digoxigénine.

Dans un mode de réalisation particulier, ledit fluorochrome peut être choisi parmi le groupe consistant en : Alexa fluor, en particulier Alexa fluor 350, 405, 430, 488, 500, 514, 532, 546, 555, 568, 594, 610, 633, 647, 660, 680, 700, 750 ou 790, Isothiocyanate de fluorescéine (FITC), Rhodamine, Allophycocyanine (APC) et Phycoérythrine (PE).

Dans un mode de réalisation particulier, ladite enzyme utilisant un substrat chimioluminescent peut être la péroxidase de raifort (HRP) et ledit substrat chimioluminescent peut être le luminol, ou encore, ladite enzyme utilisant un substrat chimioluminescent peut être la luciférase et ledit substrat chimioluminescent peut être la luciférine.

Dans un mode de réalisation particulier, ladite enzyme utilisant un substrat chromogène peut être la phosphatase alcaline et ledit substrat chromogène peut être le Nitrobleu de tetrazolium (NBT) ou le bromochlorylindolophosphate (BCIP), ou encore, ladite enzyme utilisant un substrat chromogène peut être la peroxydase de raifort (HRP) et ledit substrat chromogène peut être choisi parmi le 3,3'-Diaminobenzidine (DAB), le 3,3',5,5'-Tetramethylbenzidine (TMB), ou le 2,2'-azino-bis(acide 3-ethylbenzothiazoline-6-sulphonique) (ABTS).

Dans un mode de réalisation particulier, ladite enzyme utilisant un substrat à oxydation électrochimique peut être la péroxydase de raifort (HRP) et ledit substrat à oxydation électrochimique peut être le 3,3',5,5'-Tetramethylbenzidine (TMB).

Un mode de réalisation particulier concerne un procédé de détection de cellules vivantes actives d'algues toxiques dans un échantillon susceptible de contenir au moins une algue toxique du genre ***Alexandrium*** tel que décrit précédemment dans lequel ladite hybridation peut être effectuée dans une solution d'hybridation.

Dans un mode de réalisation particulier, ladite solution d'hybridation comprend 0 à 0,3 M de NaCl, 0 à 0,1 M de tampon choisi parmi du citrate, du Tris-HCl, du PIPES, de l'HEPES ou du phosphate, 0,001 à 0,05 % d'agent détergent choisi parmi le SDS, le triton^{®}, le TWEEN^{®}20, éventuellement 0,001 à 0,5 M d'agent chélateur choisi parmi l'EDTA ou l'EGTA, éventuellement 0,1 à 30% d'agent bloquant choisi parmi la BSA, l'ADN de Hareng, l'ADN de saumon, l'ADN de veau, l'ADN de levure ou une protéine exogène et éventuellement un autre agent chimique choisi parmi le MgCl₂, le KCl et la CaCl₂, de préférence le MgCl₂.

Dans un autre mode de réalisation, ladite solution d'hybridation comprend 0,1 M à 1 M de NaCl ou de KCl, 0,01 M à 1 M de Tris-HCl, d'HEPES, de PBS, de KH₂PO₄ ou de SSC d'un pH allant de 6,0 à 9,0, 0,01 à 0,05% d'agent détergent choisi parmi du SDS ou du N-Lauroylsarcosine, éventuellement 0,01 et 0,1 M d'agent chélateur choisi parmi l'EDTA, l'EGTA ou un agent chélateur similaire choisi parmi le citrate de calcium ou l'hexamétaphosphate de sodium et éventuellement 0,1 et 30% d'agent bloquant choisi parmi une protéine telle que la protéine Bovine Serum Albumin (BSA) ou un acide nucléique tel que l'ADN d'Hareng.

Dans un mode de réalisation particulièrement préféré, ladite solution d'hybridation est constituée de 0,3 M de NaCl, de 0,08 M de Tris-HCl et de 0,04 % de SDS et est de pH 8.

Dans un mode de réalisation particulier, ladite hybridation est effectuée à une température allant de 37°C à 70°C. Dans un mode de réalisation particulièrement préféré, ladite hybridation est effectuée à une température de 60°C.

Dans un mode de réalisation particulier, le temps de contact dudit échantillon avec ladite sonde capture et ladite sonde signal est compris entre 10 et 60 minutes. Dans un mode de réalisation particulièrement préféré, le temps de contact dudit échantillon avec ladite sonde capture et ladite sonde signal est de 10 minutes.

Dans un mode de réalisation particulier, ladite étape de détection peut être suivie d'une ou plusieurs étapes de lavage avec une solution de lavage. Dans un mode de réalisation particulièrement préféré, trois étapes de lavage sont réalisées.

Dans un mode de réalisation particulier, chaque étape de lavage peut avoir une durée allant de 1 à 60 minutes.

Dans un mode de réalisation particulier, ladite solution de lavage comprend 0 à 0,3 M de NaCl, 0 à 0,1 M de tampon choisi parmi du citrate, du Tris-HCl, du PIPES, de l'HEPES ou du phosphate, 0,001 à 0,05 % d'agent détergent choisi parmi le SDS, le triton^{®}, le TWEEN^{®}20, éventuellement 0,001 à 0,5 M d'agent chélateur choisi parmi l'EDTA ou l'EGTA, éventuellement 0,1 à 30% d'agent bloquant choisi parmi la BSA, l'ADN de Hareng, l'ADN de saumon, l'ADN de veau, l'ADN de levure ou une protéine exogène et éventuellement un autre agent chimique choisi parmi le MgCl₂, le KCl et la CaCl₂, de préférence le MgCl₂.

Dans un autre mode de réalisation, ladite solution de lavage comprend 0,1 M à 1 M de NaCl ou de KCl, 0,01 M à 1 M de Tris-HCl, d'HEPES, de PBS, de KH₂PO₄ ou de SSC d'un pH allant de 6,0 à 9,0, 0,01 et 0,05% d'agent détergent choisi parmi du SDS ou du N-Lauroylsarcosine, éventuellement 0,01 et 0,1 M d'agent chélateur choisi parmi l'EDTA, l'EGTA ou un agent chélateur similaire choisi parmi le citrate de calcium ou l'hexamétaphosphate de sodium et éventuellement 0,1 et 30% d'agent bloquant choisi parmi une protéine telle que la protéine Bovine Serum Albumin (BSA) ou un acide nucléique tel que l'ADN de Hareng.

Dans un mode de réalisation particulièrement préféré, ladite solution de lavage comprend 0,01 et 0,7 M de PBS, de Na2HPO4, de KH₂PO₄, de K₂PO₄ et/ou de SSC, et 0,1 et 0,4 M de NaCl ou de KCl.

Dans un mode de réalisation particulièrement préféré, ladite solution de lavage est constituée de 0,1M de K₂PO₄, de 0,1M de KH₂PO₄ et de 0,1M de KCl et est de pH 7,6.

Un mode de réalisation particulier concerne également un procédé de détection de cellules vivantes actives d'algues toxiques dans un échantillon susceptible de contenir au moins une algue toxique du genre ***Alexandrium*** tel que décrit précédemment, dans lequel ladite étape de détection dudit complexe peut être réalisée par lecteur de fluorescence, ou lecteur de luminescence, par lecteur d'absorbance, par gamma-caméra, par béta-caméra ou à l'aide d'un ampèremètre ou d'un potentiomètre.

Ladite étape de détection dudit complexe peut être réalisée par microscopie à fluorescence ou lecteur de fluorescence lorsque :
- la molécule de marquage est un fluorochrome
- lorsque la molécule de marquage est la biotine et est détectée *via* un fluorochrome conjugué à de la streptavidine ou de l'avidine
- lorsque la molécule de marquage est conjuguée à la biotine et est détectée *via* un fluorochrome conjugué à de la streptavidine ou de l'avidine
- lorsque la molécule de marquage est la digoxigénine et est détectée *via* un fluorochrome conjugué à un anticorps anti-digoxigénine.

Ladite étape de détection dudit complexe peut être réalisée par lecteur de luminescence lorsque :
- la molécule de marquage est une enzyme utilisant un substrat chimioluminescent
- la molécule de marquage est la biotine et est détectée *via* une enzyme utilisant un substrat chimioluminescent conjuguée à la streptavidine ou à l'avidine
- la molécule de marquage est conjuguée à la biotine et est détectée *via* une enzyme utilisant un substrat chimioluminescent conjuguée à la streptavidine ou à l'avidine
- la molécule de marquage est la digoxigénine et est détectée *via* une enzyme utilisant un substrat chimioluminescent conjuguée à un anticorps anti-digoxigénine.

Ladite étape de détection dudit complexe peut être réalisée par lecteur d'absorbance lorsque :
- la molécule de marquage est une enzyme utilisant un substrat chromogène
- la molécule de marquage est la biotine et est détectée *via* une enzyme utilisant un substrat chromogène conjuguée à la streptavidine ou à l'avidine
- la molécule de marquage est conjuguée à la biotine et est détectée *via* une enzyme utilisant un substrat chromogène conjuguée à la streptavidine ou à l'avidine
- la molécule de marquage est la digoxigénine et est détectée *via* une enzyme utilisant un substrat chromogène conjuguée à un anticorps anti-digoxigénine.

Ladite étape de détection dudit complexe peut être réalisée à l'aide d'un ampèremètre ou potentiomètre lorsque :
- la molécule de marquage est une enzyme utilisant un substrat à oxydation electrochimique
- la molécule de marquage est la biotine et est détectée *via* une enzyme utilisant un substrat à oxydation electrochimique conjuguée à la streptavidine ou à l'avidine
- la molécule de marquage est conjuguée à la biotine et est détectée *via* une enzyme utilisant un substrat à oxydation electrochimique conjuguée à la streptavidine ou à l'avidine
- la molécule de marquage est la digoxigénine et est détectée *via* une enzyme utilisant un substrat à oxydation electrochimique conjuguée à un anticorps anti-digoxigénine,
ladite enzyme utilisant un substrat à oxydation électrochimique réagissant en présence d'H₂O₂ et oxyde ledit substrat à oxydation electrochimique qui, une fois réduit, génère alors une différence de potentiel électrique mesurée par l'électrode.

Dans un mode de réalisation, ledit florochrome peut être choisi parmi le groupe consistant en : Alexa fluor, en particulier Alexa fluor 350, 405, 430, 488, 500, 514, 532, 546, 555, 568, 594, 610, 633, 647, 660, 680, 700, 750 ou 790, Isothiocyanate de fluorescéine (FITC), Rhodamine, Allophycocyanine (APC) et Phycoérythrine (PE).

Dans un mode de réalisation, ladite enzyme utilisant un substrat chimioluminescent peut être la péroxidase de raifort (HRP) et ledit substrat chimioluminescent peut être le luminol, ou encore, ladite enzyme utilisant un substrat chimioluminescent peut être la luciférase et ledit substrat chimioluminescent peut être la luciférine.

Dans un mode de réalisation, ladite enzyme utilisant un substrat chromogène peut être la phosphatase alcaline et ledit substrat chromogène peut être Nitrobleu de tetrazolium (NBT) ou le bromochlorylindolophosphate (BCIP), ou encore, ladite enzyme utilisant un substrat chromogène peut être la péroxydase de raifort (HRP) et ledit substrat chromogène peut être choisi parmi le 3,3'-Diaminobenzidine (DAB), le 3,3',5,5'-Tetramethylbenzidine (TMB), ou le 2,2'-azino-bis(acide 3-ethylbenzothiazoline-6-sulphonique) (ABTS).

Dans un mode de réalisation, ladite enzyme utilisant un substrat à oxydation électrochimique peut être la péroxydase de raifort (HRP) et ledit substrat à oxydation électrochimique peut être le 3,3',5,5'-Tetramethylbenzidine (TMB).

Concernant l'étape de quantification, les résultats peuvent être exprimés en absorbance à 630 nm ou à 450 nm après lecture à l'aide d'un lecteur de microplaque, ou en intensité de courant après lecture à l'aide d'un ampèremètre ou potentiomètre. Pour chaque genre d'algues toxiques à détecter, une courbe de calibration est réalisée à l'aide de standards synthétiques de concentrations croissantes connues. La quantification est déterminée en rapportant les valeurs moyennes d'absorbance ou de courant issues de chaque échantillon sur la courbe de calibration. Les standards sont rapportés aux valeurs de quantité d'ARN ou d'équivalents cellulaires établis à partir de nombres connus de cellules issues de cultures et ajoutées à un échantillon environnemental non contaminé.

Comme indiqué précédemment, selon la présente invention, la limite de quantification des algues listées ci-dessus est de 0,04 à 0,12 ng d'ARN par litre d'échantillon selon le type d'algue. Selon ce mode de réalisation, ledit échantillon peut être un échantillon d'eau de mer, d'eau saumâtre, de milieux de cultures ou de cultures de microalgues produites à des fins commerciales.

### Mode de réalisation B

L'invention concerne également un procédé de détection de cellules vivantes actives d'algues toxiques, tel que décrit dans la revendication 5, dans un échantillon susceptible de contenir en plus au moins une algue toxique du genre ***Dinophysis,*** comprenant en plus de l'étape d'hybridation éventuelle résultant de la mise en contact dudit échantillon avec une sonde capture et une sonde signal spécifiques d'algues toxique du genre ***Alexandrium,*** une étape d'hybridation éventuelle résultant de la mise en contact dudit échantillon avec une sonde capture et une sonde signal spécifiques d'algues toxique du genre ***Dinophysis,*** la sonde capture et la sonde signal formant un couple de sondes, les séquences dudit couple de sondes étant choisies parmi x éléments de l'un des ensembles suivants :
- (SEQ ID NO : 29, SEQ ID NO : 30 ou SEQ ID NO : 31)
- (SEQ ID NO : 32 et SEQ ID NO : 33)
- (SEQ ID NO : 34 et SEQ ID NO : 35)
- (SEQ ID NO : 36 et SEQ ID NO : 37)
- (SEQ ID NO : 38 et SEQ ID NO : 39)
- (SEQ ID NO : 40, SEQ ID NO : 41 ou SEQ ID NO : 42)
- (SEQ ID NO : 43, SEQ ID NO : 44 ou SEQ ID NO : 44), ou
- (SEQ ID NO : 46, SEQ ID NO : 47 ou SEQ ID NO : 48)

x étant 2 ou 3,
ou les séquences desdites sondes ayant au moins 92% d'identité avec les susdites séquences SEQ ID NO : 29 à SEQ ID NO : 48
l'hybridation indiquant la présence d'algue toxique du genre ***Dinophysis**.*

Tous les différents modes de réalisation décrits pour le procédé de détection selon le mode de réalisation A peuvent s'appliquer pour le mode de réalisation B.

Comme précédemment pour la détection *d'Alexandrium* selon le mode de réalisation A, dans un mode de réalisation particulier, le seuil de détection minimal de l'algue toxique du genre ***Dinophysis*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation C

De la même façon, est décrit un des procédés de détection de cellules vivantes actives d'algues toxiques, tel que décrit précédemment selon le mode de réalisation A ou selon le mode de réalisation B, dans un échantillon susceptible de contenir en plus au moins une algue toxique du genre ***Pseudo-Nitzschia**,* comprenant en plus de l'étape d'hybridation éventuelle résultant de la mise en contact dudit échantillon avec une sonde capture et une sonde signal spécifiques d'algues toxique du genre ***Alexandrium,***
une étape d'hybridation éventuelle résultant de la mise en contact dudit échantillon avec une sonde capture et une sonde signal spécifiques d'algues toxique du genre ***Pseudo-nitzschia,*** la sonde capture et la sonde signal formant un couple de sondes, les séquences dudit couple de sondes étant choisies parmi x éléments de l'un des ensembles suivants :
   - (SEQ ID NO : 49 et SEQ ID NO : 50)
   - (SEQ ID NO : 51 et SEQ ID NO : 52)
   - (SEQ ID NO : 53, SEQ ID NO : 54 ou SEQ ID NO : 55)
   - (SEQ ID NO : 56, SEQ ID NO : 57 ou SEQ ID NO : 58), ou
   - (SEQ ID NO : 59, SEQ ID NO : 60 ou SEQ ID NO : 61)
x étant 2 ou 3,
ou les séquences desdites sondes ayant au moins 92% d'identité avec les susdites séquences SEQ ID NO : 49 à SEQ ID NO : 61,
l'hybridation indiquant la présence d'algue toxique du genre ***Pseudo nitzschia.***

Tous les différents modes de réalisation décrits pour le procédé de détection selon le mode de réalisation A peuvent s'appliquer pour le mode de réalisation C.

Comme précédemment pour la détection d'*Alexandrium* selon le mode de réalisation A, dans un mode de réalisation particulier, le seuil de détection minimal de l'algue toxique du genre *Pseudo-nitzschia* est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation D

De la même façon, est décrit un des procédés de détection de cellules vivantes actives d'algues toxiquesl'un des procédés de détection de cellules vivantes actives d'algues toxiques, tel que décrit précédemment selon les modes de réalisation A, B ou C, dans un échantillon susceptible de contenir en plus au moins une algue toxique du genre ***Prorocentrum,*** comprenant en plus de l'étape d'hybridation éventuelle résultant de la mise en contact dudit échantillon avec une sonde capture et une sonde signal spécifiques d'algues toxique du genre ***Alexandrium,***
une étape d'hybridation éventuelle résultant de la mise en contact dudit échantillon avec une sonde capture et une sonde signal spécifiques d'algues toxique du genre ***Prorocentrum,*** la sonde capture et la sonde signal formant un couple de sondes, les séquences dudit couple de sondes étant choisies parmi x éléments de l'un des ensembles suivants :
   - (SEQ ID NO : 62, SEQ ID NO : 63 ou SEQ ID NO : 64)
   - (SEQ ID NO : 65, SEQ ID NO : 66 ou SEQ ID NO : 67)
   - (SEQ ID NO : 68, SEQ ID NO : 69 ou SEQ ID NO : 70)
   - (SEQ ID NO : 71, SEQ ID NO : 72 ou SEQ ID NO : 73)
x étant 2 ou 3,
ou les séquences desdites sondes ayant au moins 92% d'identité avec les susdites séquences SEQ ID NO : 62 à SEQ ID NO : 73
l'hybridation indiquant la présence d'algue toxique du genre ***Prorocentrum.***

Tous les différents modes de réalisation décrits pour le procédé de détection selon le mode de réalisation A peuvent s'appliquer pour le mode de réalisation D.

Comme précédemment pour la détection d'*Alexandrium* selon le mode de réalisation A, dans un mode de réalisation particulier, le seuil de détection minimal de l'algue toxique du genre *Prorocentrum* est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation E

De la même façon, est décrit l'un des procédés de détection de cellules vivantes actives d'algues toxiques, tel que décrit précédemment selon les modes de réalisation A, B, C, ou D, dans un échantillon susceptible de contenir en plus au moins une algue toxique du genre ***Chattonella,*** comprenant en plus de l'étape d'hybridation éventuelle résultant de la mise en contact dudit échantillon avec une sonde capture et une sonde signal spécifiques d'algues toxique du genre ***Alexandrium,***
une étape d'hybridation éventuelle résultant de la mise en contact dudit échantillon avec une sonde capture et une sonde signal spécifiques d'algues toxique du genre ***Chattonella,*** la sonde capture et la sonde signal formant un couple de sondes, les séquences dudit couple de sondes étant choisies parmi x éléments de l'un des ensembles suivants :
   - (SEQ ID NO : 74, SEQ ID NO : 75 ou SEQ ID NO : 76)
   - (SEQ ID NO : 77, SEQ ID NO : 78 ou SEQ ID NO : 79)
   - (SEQ ID NO : 80, SEQ ID NO : 81 ou SEQ ID NO : 82)
x étant 2 ou 3,
ou les séquences desdites sondes ayant au moins 92% d'identité avec les susdites séquences SEQ ID NO : 74 à SEQ ID NO : 82
l'hybridation indiquant la présence d'algue toxique du genre ***Chattonella.***

Tous les différents modes de réalisation décrits pour le procédé de détection selon le mode de réalisation A peuvent s'appliquer pour le mode de réalisation E.

Comme précédemment pour la détection d'*Alexandrium* selon le mode de réalisation A, dans un mode de réalisation particulier, le seuil de détection minimal de l'algue toxique du genre *Chattonella* est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation F

De la même façon, est décrit un des procédés de détection de cellules vivantes actives d'algues toxiquesl'un des procédés de détection de cellules vivantes actives d'algues toxiques, tel que décrit précédemment selon les modes de réalisation A, B, C, D ou E, dans un échantillon susceptible de contenir en plus au moins une algue toxique du genre ***Gymnodinium,*** comprenant en plus de l'étape d'hybridation éventuelle résultant de la mise en contact dudit échantillon avec une sonde capture et une sonde signal spécifiques d'algues toxique du genre ***Alexandrium,***
une étape d'hybridation éventuelle résultant de la mise en contact dudit échantillon avec une sonde capture et une sonde signal spécifiques d'algues toxique du genre ***Gymnodinium,*** la sonde capture et la sonde signal formant un couple de sondes, les séquences dudit couple de sondes étant choisies parmi x éléments de l'un des ensembles suivants :
   - (SEQ ID NO : 83, SEQ ID NO : 84 ou SEQ ID NO : 85)
   - (SEQ ID NO : 86, SEQ ID NO : 87 ou SEQ ID NO : 88)
   - (SEQ ID NO : 89, SEQ ID NO : 90 ou SEQ ID NO : 91)
   - (SEQ ID NO : 92, SEQ ID NO : 93 ou SEQ ID NO : 94)
x étant 2 ou 3,
ou les séquences desdites sondes ayant au moins 92% d'identité avec les susdites séquences SEQ ID NO : 83 à SEQ ID NO : 94
l'hybridation indiquant la présence d'algue toxique du genre ***Gymnodinium.***

Tous les différents modes de réalisation décrits pour le procédé de détection selon le mode de réalisation A peuvent s'appliquer pour le mode de réalisation F.

Comme précédemment pour la détection d'*Alexandrium* selon le mode de réalisation A, dans un mode de réalisation particulier, le seuil de détection minimal de l'algue toxique du genre ***Gymnodinium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation G

De la même façon, est décrit un des procédés de détection de cellules vivantes actives d'algues toxiquesl'un des procédés de détection de cellules vivantes actives d'algues toxiques, tel que décrit précédemment selon les modes de réalisation A, B, C, D, E ou F, dans un échantillon susceptible de contenir en plus au moins une algue toxique du genre ***Karenia,*** comprenant en plus de l'étape d'hybridation éventuelle résultant de la mise en contact dudit échantillon avec une sonde capture et une sonde signal spécifiques d'algues toxique du genre ***Alexandrium,***
une étape d'hybridation éventuelle résultant de la mise en contact dudit échantillon avec une sonde capture et une sonde signal spécifiques d'algues toxique du genre ***Karenia,*** la sonde capture et la sonde signal formant un couple de sondes, les séquences dudit couple de sondes étant choisies parmi x éléments de l'un des ensembles suivants :
   - (SEQ ID NO : 95, SEQ ID NO : 96 ou SEQ ID NO : 97)
   - (SEQ ID NO : 98, SEQ ID NO : 99 ou SEQ ID NO : 100)
   - (SEQ ID NO : 101, SEQ ID NO : 102 ou SEQ ID NO : 103)
   - (SEQ ID NO : 104, SEQ ID NO : 105 ou SEQ ID NO : 106)
   - (SEQ ID NO : 107, SEQ ID NO : 108 ou SEQ ID NO : 109)
   - (SEQ ID NO : 110, SEQ ID NO : 111 ou SEQ ID NO : 112)
   - (SEQ ID NO : 113, SEQ ID NO : 114 ou SEQ ID NO : 115)
   - (SEQ ID NO : 116, SEQ ID NO : 117 ou SEQ ID NO : 118)
x étant 2 ou 3,
ou les séquences desdites sondes ayant au moins 92% d'identité avec les susdites séquences SEQ ID NO : 95 à SEQ ID NO : 118
l'hybridation indiquant la présence d'algue toxique du genre ***Karenia.***

Tous les différents modes de réalisation décrits pour le procédé de détection selon le mode de réalisation A peuvent s'appliquer pour le mode de réalisation G.

Comme précédemment pour la détection d'*Alexandrium* selon le mode de réalisation A, dans un mode de réalisation particulier, le seuil de détection minimal de l'algue toxique du genre ***Karenia*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation H

De la même façon, est décrit un des procédés de détection de cellules vivantes actives d'algues toxiquesl'un des procédés de détection de cellules vivantes actives d'algues toxiques, tel que décrit précédemment selon les modes de réalisation A, B, C, D, E, F ou G dans un échantillon susceptible de contenir en plus au moins une algue toxique du genre ***Lingulodinium,*** comprenant en plus de l'étape d'hybridation éventuelle résultant de la mise en contact dudit échantillon avec une sonde capture et une sonde signal spécifiques d'algues toxique du genre ***Alexandrium,***
une étape d'hybridation éventuelle résultant de la mise en contact dudit échantillon avec une sonde capture et une sonde signal spécifiques d'algues toxique du genre ***Lingulodinium,*** la sonde capture et la sonde signal formant un couple de sondes, les séquences dudit couple de sondes étant choisies parmi x éléments de l'un des ensembles suivants :
   - (SEQ ID NO : 119, SEQ ID NO : 120 ou SEQ ID NO : 121)
   - (SEQ ID NO : 122, SEQ ID NO : 123 ou SEQ ID NO : 124)
   - (SEQ ID NO : 125, SEQ ID NO : 126 ou SEQ ID NO : 127)
x étant 2 ou 3,
ou les séquences desdites sondes ayant au moins 92% d'identité avec les susdites séquences SEQ ID NO : 119 à SEQ ID NO : 127
l'hybridation indiquant la présence d'algue toxique du genre ***Lingulodinium.***

Tous les différents modes de réalisation décrits pour le procédé de détection selon le mode de réalisation A peuvent s'appliquer pour le mode de réalisation H.

Comme précédemment pour la détection *d'Alexandrium* selon le mode de réalisation A, dans un mode de réalisation particulier, le seuil de détection minimal de l'algue toxique du genre *Lingulodinium* est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation I

De la même façon, est décrit un des procédés de détection de cellules vivantes actives d'algues toxiquesl'un des procédés de détection de cellules vivantes actives d'algues toxiques, tel que décrit précédemment selon les modes de réalisation A, B, C, D, E, F, G ou H, dans un échantillon susceptible de contenir en plus au moins une algue toxique du genre ***Heterosigma,*** comprenant en plus de l'étape d'hybridation éventuelle résultant de la mise en contact dudit échantillon avec une sonde capture et une sonde signal spécifiques d'algues toxique du genre ***Alexandrium,***
une étape d'hybridation éventuelle résultant de la mise en contact dudit échantillon avec une sonde capture et une sonde signal spécifiques d'algues toxique du genre ***Heterosigma,*** la sonde capture et la sonde signal formant un couple de sondes, les séquences dudit couple de sondes étant choisies parmi x éléments de l'un des ensembles suivants :
   - (SEQ ID NO : 128 et SEQ ID NO : 129)
x étant 2 ou 3,
ou les séquences desdites sondes ayant au moins 92% d'identité avec les susdites séquences SEQ ID NO : 128 à SEQ ID NO : 129
l'hybridation indiquant la présence d'algue toxique du genre ***Heterosigma.***

Tous les différents modes de réalisation décrits pour le procédé de détection selon le mode de réalisation A peuvent s'appliquer pour le mode de réalisation I.

Comme précédemment pour la détection *d'Alexandrium* selon le mode de réalisation A, dans un mode de réalisation particulier, le seuil de détection minimal de l'algue toxique du genre ***Heterosigma*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

Comme pour le premier aspect relatif à l'utilisation, toutes les combinaisons des modes de réalisation A, B, C, D, E, F, G, H et/ou I de ce quatrième aspect peuvent être envisagées. De cette façon, l'ensemble des combinaisons d'algues toxiques B à I128 décrites dans le premier aspect peuvent être détectées par les procédés tels que décrits dans ce quatrième aspect. L'étape d'hybridation décrite précédemment entre l'acide nucléique ribosomique de l'algue toxiques à détecter et les sondes capture et signal peut être effectuée de plusieurs manières. Dans une première possibilité, les sondes capture sont incubées sur le support et les sondes signal sont incubées avec les acides nucléiques ribosomiques de l'algue toxique à détecter. Ensuite, les couples éventuellement formés entre les sondes signal et les acides nucléiques ribosomiques de l'algue toxique à détecter sont incubés avec le support contenant les sondes capture.

Dans une seconde posibilité, les sondes capture sont incubées sur le support. Ensuite, les sondes signal, les acides nucléiques ribosomiques de l'algue toxique à détecter et le support contenant les sondes captures sont incubés ensemble.

Dans un troisième aspect, les sondes capture et signal sont incubées avec les acides nucléiques ribosomiques de l'algue toxique à détecter. Ce mélange est ensuite incubé avec le support. Dans un quatrième aspect, les sondes capture sont incubées avec les acides nucléiques ribosomiques de l'algue toxique à détecter. Ce mélange est ensuite incubé avec le support et les sondes signal.

### Mode de réalisation A1

Ainsi, un mode de réalisation particulier de ce quatrième aspect, décrit mais ne faisant pas partie de l'invention, concerne un procédé de détection de cellules vivantes actives d'algues toxiques dans un échantillon susceptible de contenir au moins une algue toxique du genre ***Alexandrium,*** comprenant les étapes suivantes :
a) addition d'un complexe éventuel formé entre l'acide nucléique ribosomique d'une algue toxique du genre ***Alexandrium,*** et une sonde signal sur un support contenant une sonde capture,
b) détection de l'hybridation éventuelle du susdit complexe avec ladite sonde capture, l'hybridation ayant lieu entre la sonde capture et l'acide nucléique ribosomique du susdit complexe,
   l'hybridation indiquant la présence d'algue toxique du genre ***Alexandrium,***
   ladite sonde capture et ladite sonde signal formant un couple de sondes, les séquences dudit couple de sondes étant choisies parmi x éléments de l'un des ensembles suivants :
      - (SEQ ID NO : 1, SEQ ID NO : 2 ou SEQ ID NO : 3)
      - (SEQ ID NO : 4 et SEQ ID NO : 5)
      - (SEQ ID NO : 6 et SEQ ID NO : 7)
      - (SEQ ID NO : 8, SEQ ID NO : 9 ou SEQ ID NO : 10)
      - (SEQ ID NO : 11, SEQ ID NO : 12 ou SEQ ID NO : 13)
      - (SEQ ID NO : 14, SEQ ID NO : 15 ou SEQ ID NO : 16)
      - (SEQ ID NO : 17, SEQ ID NO : 18 ou SEQ ID NO : 19)
      - (SEQ ID NO : 20, SEQ ID NO : 21 ou SEQ ID NO : 22),
      - (SEQ ID NO : 23, SEQ ID NO : 24 ou SEQ ID NO : 25),
      - (SEQ ID NO : 26, SEQ ID NO : 27 ou SEQ ID NO : 28)
   x étant 2 ou 3,
   ou les séquences desdites sondes ayant au moins 92% d'identité avec les susdites séquences SEQ ID NO : 1 à SEQ ID NO : 28.

Tous les différents modes de réalisation décrits pour le procédé de détection selon le mode de réalisation A peuvent s'appliquer pour le mode de réalisation A1.

Comme précédemment, dans un mode de réalisation particulier, le seuil de détection minimal de l'algue toxique du genre ***Alexandrium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation B1

Est décrit un procédé de détection de cellules vivantes actives d'algues toxiques tel que décrit précédemment selon le mode de réalisation A1, dans un échantillon susceptible de contenir en plus au moins une algue toxique du genre ***Dinophysis,*** comprenant, en plus, l'addition d'un complexe éventuel formé entre l'acide nucléique ribosomique d'une algue toxique du genre ***Dinophysis*** et une sonde signal sur un support contenant une sonde capture,
ladite sonde capture et ladite sonde signal formant un couple de sondes, les séquences dudit couple de sondes étant choisies parmi x éléments de l'un des ensembles suivants :
   - (SEQ ID NO : 29, SEQ ID NO : 30 ou SEQ ID NO : 31)
   - (SEQ ID NO : 32 et SEQ ID NO : 33)
   - (SEQ ID NO : 34 et SEQ ID NO : 35)
   - (SEQ ID NO : 36 et SEQ ID NO : 37)
   - (SEQ ID NO : 38 et SEQ ID NO : 39)
   - (SEQ ID NO : 40, SEQ ID NO : 41 ou SEQ ID NO : 42)
   - (SEQ ID NO : 3, SEQ ID NO : 44 ou SEQ ID NO : 45), ou
   - (SEQ ID NO : 46, SEQ ID NO : 47 ou SEQ ID NO : 48)
x étant 2 ou 3,
ou les séquences desdites sondes ayant au moins 92% d'identité avec les susdites séquences SEQ ID NO : 29 à SEQ ID NO : 48
l'hybridation indiquant la présence d'algue toxique du genre ***Dinophysis.***

Tous les différents modes de réalisation décrits pour le procédé de détection selon le mode de réalisation A peuvent s'appliquer pour le mode de réalisation B1.

Comme précédemment, dans un mode de réalisation particulier, le seuil de détection minimal de l'algue toxique du genre ***Dinophysis*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation C1

Est décrit un des procédés de détection de cellules vivantes actives d'algues toxiques tels que décrits précédemment selon le mode de réaliation A1 ou selon le mode de réalisation B1, dans un échantillon susceptible de contenir en plus au moins une algue toxique du genre ***Pseudo-nitzschia**,* comprenant en plus l'addition d'un complexe éventuel formé entre l'acide nucléique ribosomique d'une algue toxique du genre ***Pseudo-nitzschia*** et une sonde signal sur un support contenant une sonde capture,
ladite sonde capture et ladite sonde signal formant un couple de sondes, les séquences dudit couple de sondes étant choisies parmi x éléments de l'un des ensembles suivants :
   - (SEQ ID NO : 49 et SEQ ID NO : 50)
   - (SEQ ID NO : 51 et SEQ ID NO : 52)
   - (SEQ ID NO : 53, SEQ ID NO : 54 ou SEQ ID NO : 55)
   - (SEQ ID NO : 56, SEQ ID NO : 57 ou SEQ ID NO : 58), ou
   - (SEQ ID NO : 59, SEQ ID NO : 60 ou SEQ ID NO : 61)
x étant 2 ou 3,
ou les séquences desdites sondes ayant au moins 92% d'identité avec les susdites séquences SEQ ID NO : 49 à SEQ ID NO : 61
l'hybridation indiquant la présence d'algue toxique du genre ***Pseudo-nitzschia.***

Tous les différents modes de réalisation décrits pour le procédé de détection selon le mode de réalisation A peuvent s'appliquer pour le mode de réalisation C1.

Comme précédemment, dans un mode de réalisation particulier, le seuil de détection minimal de l'algue toxique du genre ***Pseudo-nitzschia*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation D1

Est décrit un des procédés de détection de cellules vivantes actives d'algues toxiques tels que décrits précédemment selon les modes de réaliation A1, B1 ou C1, dans un échantillon susceptible de contenir en plus au moins une algue toxique du genre ***Prorocentrum,*** comprenant en plus l'addition d'un complexe éventuel formé entre l'acide nucléique ribosomique d'une algue toxique du genre ***Prorocentrum*** et une sonde signal sur un support contenant une sonde capture,
ladite sonde capture et ladite sonde signal formant un couple de sondes, les séquences dudit couple de sondes étant choisies parmi x éléments de l'un des ensembles suivants :
   - (SEQ ID NO : 62, SEQ ID NO : 63 ou SEQ ID NO : 64)
   - (SEQ ID NO : 65, SEQ ID NO : 66 ou SEQ ID NO : 67)
   - (SEQ ID NO : 68, SEQ ID NO : 69 ou SEQ ID NO : 70)
   - (SEQ ID NO : 71, SEQ ID NO : 72 ou SEQ ID NO : 73)
x étant 2 ou 3,
ou les séquences desdites sondes ayant au moins 92% d'identité avec les susdites séquences SEQ ID NO : 62 à SEQ ID NO : 73
l'hybridation indiquant la présence d'algue toxique du genre ***Prorocentrum.***

Tous les différents modes de réalisation décrits pour le procédé de détection selon le mode de réalisation A peuvent s'appliquer pour le mode de réalisation D1.

Comme précédemment, dans un mode de réalisation particulier, le seuil de détection minimal de l'algue toxique du genre ***Prorocentrum*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation E1

Est décrit un des procédés de détection de cellules vivantes actives d'algues toxiques tels que décrits précédemment selon les modes de réaliation A1, B1, C1, ou D1, dans un échantillon susceptible de contenir en plus au moins une algue toxique du genre ***Chattonella,*** comprenant en plus l'addition d'un complexe éventuel formé entre l'acide nucléique ribosomique d'une algue toxique du genre ***Chattonella*** et une sonde signal sur un support contenant une sonde capture,
ladite sonde capture et ladite sonde signal formant un couple de sondes, les séquences dudit couple de sondes étant choisies parmi x éléments de l'un des ensembles suivants :
   - (SEQ ID NO : 74, SEQ ID NO : 75 ou SEQ ID NO : 76)
   - (SEQ ID NO : 77, SEQ ID NO : 78 ou SEQ ID NO : 79)
   - (SEQ ID NO : 80, SEQ ID NO : 81 ou SEQ ID NO : 82)
x étant 2 ou 3,
ou les séquences desdites sondes ayant au moins 92% d'identité avec les susdites séquences SEQ ID NO : 74 à SEQ ID NO : 82
l'hybridation indiquant la présence d'algue toxique du genre ***Chattonella.***

Tous les différents modes de réalisation décrits pour le procédé de détection selon le mode de réalisation A peuvent s'appliquer pour le mode de réalisation E1.

Comme précédemment, dans un mode de réalisation particulier, le seuil de détection minimal de l'algue toxique du genre ***Chatonella*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation F1

Est décrit un des procédés de détection de cellules vivantes actives d'algues toxiques tels que décrits précédemment selon les modes de réaliation A1, B1, C1, D1 ou E1, dans un échantillon susceptible de contenir en plus au moins une algue toxique du genre ***Gymnodinium,*** comprenant en plus l'addition d'un complexe éventuel formé entre l'acide nucléique ribosomique d'une algue toxique du genre ***Gymnodinium*** et une sonde signal sur un support contenant une sonde capture,
la sonde capture et la sonde signal formant un couple de sondes, les séquences dudit couple de sondes étant choisies parmi x éléments de l'un des ensembles suivants :
   - (SEQ ID NO : 83, SEQ ID NO : 84 ou SEQ ID NO : 85)
   - (SEQ ID NO : 86, SEQ ID NO : 87 ou SEQ ID NO : 88)
   - (SEQ ID NO : 89, SEQ ID NO : 90 ou SEQ ID NO : 91)
   - (SEQ ID NO : 92, SEQ ID NO : 93 ou SEQ ID NO : 94)
x étant 2 ou 3,
ou les séquences desdites sondes ayant au moins 92% d'identité avec les susdites séquences SEQ ID NO : 83 à SEQ ID NO : 94
l'hybridation indiquant la présence d'algue toxique du genre ***Gymnodinium.***

Tous les différents modes de réalisation décrits pour le procédé de détection selon le mode de réalisation A peuvent s'appliquer pour le mode de réalisation F1.

Comme précédemment, dans un mode de réalisation particulier, le seuil de détection minimal de l'algue toxique du genre ***Gymnodinium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation G1

Est décrit un des procédés de détection de cellules vivantes actives d'algues toxiques tels que décrits précédemment selon les modes de réaliation A1, B1, C1, D1, E1 ou F1, dans un échantillon susceptible de contenir en plus au moins une algue toxique du genre ***Karenia,*** comprenant en plus l'addition d'un complexe éventuel formé entre l'acide nucléique ribosomique d'une algue toxique du genre ***Karenia*** et une sonde signal sur un support contenant une sonde capture,
la sonde capture et la sonde signal formant un couple de sondes, les séquences dudit couple de sondes étant choisies parmi x éléments de l'un des ensembles suivants :
   - (SEQ ID NO : 95, SEQ ID NO : 96 ou SEQ ID NO : 97)
   - (SEQ ID NO : 98, SEQ ID NO : 99 ou SEQ ID NO : 100)
   - (SEQ ID NO : 101, SEQ ID NO : 102 ou SEQ ID NO : 103)
   - (SEQ ID NO : 104, SEQ ID NO : 105 ou SEQ ID NO : 106)
   - (SEQ ID NO : 107, SEQ ID NO : 108 ou SEQ ID NO : 109)
   - (SEQ ID NO : 110, SEQ ID NO : 111 ou SEQ ID NO : 112)
   - (SEQ ID NO : 113, SEQ ID NO : 114 ou SEQ ID NO : 115)
   - (SEQ ID NO : 116, SEQ ID NO : 117 ou SEQ ID NO : 118)
x étant 2 ou 3,
ou les séquences desdites sondes ayant au moins 92% d'identité avec les susdites séquences SEQ ID NO : 95 à SEQ ID NO : 118
l'hybridation indiquant la présence d'algue toxique du genre ***Karenia***

Tous les différents modes de réalisation décrits pour le procédé de détection selon le mode de réalisation A peuvent s'appliquer pour le mode de réalisation G1.

Comme précédemment, dans un mode de réalisation particulier, le seuil de détection minimal de l'algue toxique du genre ***Karenia*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation H1

Est décrit un des procédés de détection de cellules vivantes actives d'algues toxiques tels que décrits précédemment selon les modes de réaliation A1, B1, C1, D1, E1, F1, ou G1, dans un échantillon susceptible de contenir en plus au moins une algue toxique du genre ***Lingulodinium,*** comprenant en plus l'addition d'un complexe éventuel formé entre l'acide nucléique ribosomique d'une algue toxique du genre ***Lingulodinium*** et une sonde signal sur un support contenant une sonde capture,
la sonde capture et la sonde signal formant un couple de sondes, les séquences dudit couple de sondes étant choisies parmi x éléments de l'un des ensembles suivants :
   - (SEQ ID NO : 119, SEQ ID NO : 120 ou SEQ ID NO : 121)
   - (SEQ ID NO : 122, SEQ ID NO : 123 ou SEQ ID NO : 124)
   - (SEQ ID NO : 125, SEQ ID NO : 126 ou SEQ ID NO : 127)
x étant 2 ou 3,
ou les séquences desdites sondes ayant au moins 92% d'identité avec les susdites séquences SEQ ID NO : 119 à SEQ ID NO : 127
l'hybridation indiquant la présence d'algue toxique du genre ***Lingulodinium.***

Tous les différents modes de réalisation décrits pour le procédé de détection selon le mode de réalisation A peuvent s'appliquer pour le mode de réalisation H1.

Comme précédemment, dans un mode de réalisation particulier, le seuil de détection minimal de l'algue toxique du genre ***Lingulodinium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation I1

Est décrit un des procédés de détection de cellules vivantes actives d'algues toxiques tels que décrits précédemment selon les modes de réaliation A1, B1, C1, D1, E1, F1, G1 ou H1, dans un échantillon susceptible de contenir en plus au moins une algue toxique du genre ***Heterosigma,*** comprenant en plus l'addition d'un complexe éventuel formé entre l'acide nucléique ribosomique d'une algue toxique du genre ***Heterosigma*** et une sonde signal sur un support contenant une sonde capture,
la sonde capture et la sonde signal formant un couple de sondes, les séquences dudit couple de sondes étant choisies parmi x éléments de l'un des ensembles suivants :
   - (SEQ ID NO : 128 et SEQ ID NO : 129)
x étant 2 ou 3,
ou les séquences desdites sondes ayant au moins 92% d'identité avec les susdites séquences SEQ ID NO : 128 à SEQ ID NO : 129
l'hybridation indiquant la présence d'algue toxique du genre ***Heterosigma***

Tous les différents modes de réalisation décrits pour le procédé de détection selon le mode de réalisation A peuvent s'appliquer pour le mode de réalisation I1.

Comme précédemment, dans un mode de réalisation particulier, le seuil de détection minimal de l'algue toxique du genre ***Heterosigma*** est de
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

Comme pour le premier aspect relatif à l'utilisation, toutes les combinaisons des modes de réalisation A1, B1, C1, D1, E1, F1, G1, H1 et/ou I1 de ce quatrième aspect peuvent être envisagées. De cette façon, l'ensemble des combinaisons d'algues toxiques B à I128 décrites dans le premier aspect peuvent être détectées par les procédés tels que décrits dans ce quatrième aspect.

### Mode de réalisation A2

Ainsi, un autre mode de réalisation particulier de ce quatrième aspect, décrit mais ne faisant pas partie de l'invention, concerne un procédé de détection de cellules vivantes actives d'algues toxiques dans un échantillon susceptible de contenir au moins une algue toxique du genre ***Alexandrium*** comprenant les étapes suivantes :
a) addition de l'acide nucléique ribosomique d'une algue toxique du genre ***Alexandrium*** et d'une sonde signal sur un support contenant une sonde capture,
b) détection de l'hybridation éventuelle d'un complexe formé entre ladite sonde capture, ledit acide nucléique ribosomique et ladite sonde signal, l'hybridation ayant lieu entre la sonde capture, l'acide nucléique ribosomique et la sonde signal, l'hybridation indiquant la présence d'algue toxique du genre ***Alexandrium*** ladite sonde capture et ladite sonde signal formant un couple de sondes, les séquences dudit couple de sondes étant choisies parmi x éléments de l'un des ensembles suivants :
   - (SEQ ID NO : 1, SEQ ID NO : 2 ou SEQ ID NO : 3)
   - (SEQ ID NO : 4 et SEQ ID NO : 5)
   - (SEQ ID NO : 6 et SEQ ID NO : 7)
   - (SEQ ID NO : 8, SEQ ID NO : 9 ou SEQ ID NO : 10)
   - (SEQ ID NO : 11, SEQ ID NO : 12 ou SEQ ID NO : 13)
   - (SEQ ID NO : 14, SEQ ID NO : 15 ou SEQ ID NO : 16)
   - (SEQ ID NO : 17, SEQ ID NO : 18 ou SEQ ID NO : 19)
   - (SEQ ID NO : 20, SEQ ID NO : 21 ou SEQ ID NO : 22),
   - (SEQ ID NO : 23, SEQ ID NO : 24 ou SEQ ID NO : 25),
   - (SEQ ID NO : 26, SEQ ID NO : 27 ou SEQ ID NO : 28)

   x étant 2 ou 3,
   ou les séquences desdites sondes ayant au moins 92% d'identité avec les susdites séquences SEQ ID NO : 1 à SEQ ID NO : 28.

Tous les différents modes de réalisation décrits pour le procédé de détection selon le mode de réalisation A peuvent s'appliquer pour le mode de réalisation A2.

Comme précédemment, dans un mode de réalisation particulier, le seuil de détection minimal de l'algue toxique du genre ***Alexandrium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation B2

Est décrit un procédé de détection de cellules vivantes actives d'algues toxiques tel que décrit précédemment selon le mode de réalisation A2, dans un échantillon susceptible de contenir en plus au moins une algue toxique du genre ***Dinophysis,*** comprenant, en plus, l'addition de l'acide nucléique ribosomique d'une algue toxique du genre ***Dinophysis*** et d'une sonde signal sur un support contenant une sonde capture,
ladite sonde capture et ladite sonde signal formant un couple de sondes, les séquences dudit couple de sondes étant choisies parmi x éléments de l'un des ensembles suivants :
   - (SEQ ID NO : 29, SEQ ID NO : 30 ou SEQ ID NO : 31)
   - (SEQ ID NO : 32 et SEQ ID NO : 33)
   - (SEQ ID NO : 34 et SEQ ID NO : 35)
   - (SEQ ID NO : 36 et SEQ ID NO : 37)
   - (SEQ ID NO : 38 et SEQ ID NO : 39)
   - (SEQ ID NO : 40, SEQ ID NO : 41 ou SEQ ID NO : 42)
   - (SEQ ID NO : 43, SEQ ID NO : 44 ou SEQ ID NO : 45), ou
   - (SEQ ID NO : 46, SEQ ID NO : 47 ou SEQ ID NO : 48)
x étant 2 ou 3,
ou les séquences desdites sondes ayant au moins 92% d'identité avec les susdites séquences SEQ ID NO : 29 à SEQ ID NO : 48
l'hybridation indiquant la présence d'algue toxique du genre ***Dinophysis.***

Tous les différents modes de réalisation décrits pour le procédé de détection selon le mode de réalisation A peuvent s'appliquer pour le mode de réalisation B2.

Comme précédemment, dans un mode de réalisation particulier, le seuil de détection minimal de l'algue toxique du genre ***Dinophysis*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation C2

Est décrit un des procédés de détection de cellules vivantes actives d'algues toxiques tels que décrits précédemment selon les modes de réalisation A2 ou B2, dans un échantillon susceptible de contenir en plus au moins une algue toxique du genre ***Pseudo-nitzschia**,* comprenant en plus l'addition de l'acide nucléique ribosomique d'une algue toxique du genre ***Pseudo-nitzschia*** et d'une sonde signal sur un support contenant une sonde capture,
ladite sonde capture et ladite sonde signal formant un couple de sondes, les séquences dudit couple de sondes étant choisies parmi x éléments de l'un des ensembles suivants :
   - (SEQ ID NO : 49 et SEQ ID NO : 50)
   - (SEQ ID NO : 51 et SEQ ID NO : 52)
   - (SEQ ID NO : 53, SEQ ID NO : 54 ou SEQ ID NO : 55)
   - (SEQ ID NO : 56, SEQ ID NO : 57 ou SEQ ID NO : 58), ou
   - (SEQ ID NO : 59, SEQ ID NO : 60 ou SEQ ID NO : 61)
x étant 2 ou 3,
ou les séquences desdites sondes ayant au moins 92% d'identité avec les susdites séquences SEQ ID NO : 49 à SEQ ID NO : 61
l'hybridation indiquant la présence d'algue toxique du genre ***Pseudo-nitzschia.***

Tous les différents modes de réalisation décrits pour le procédé de détection selon le mode de réalisation A peuvent s'appliquer pour le mode de réalisation C2.

Comme précédemment, dans un mode de réalisation particulier, le seuil de détection minimal de l'algue toxique du genre ***Pseudo-nitzschia*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation D2

Est décrit un des procédés de détection de cellules vivantes actives d'algues toxiques tels que décrits précédemment selon les modes de réalisation A2, B2 ou C2, dans un échantillon susceptible de contenir en plus au moins une algue toxique du genre ***Prorocentrum,*** comprenant en plus l'addition de l'acide nucléique ribosomique d'une algue toxique du genre ***Prorocentrum*** et d'une sonde signal sur un support contenant une sonde capture,
ladite sonde capture et ladite sonde signal formant un couple de sondes, les séquences dudit couple de sondes étant choisies parmi x éléments de l'un des ensembles suivants :
   - (SEQ ID NO : 62, SEQ ID NO : 63 ou SEQ ID NO : 64)
   - (SEQ ID NO : 65, SEQ ID NO : 66 ou SEQ ID NO : 67)
   - (SEQ ID NO : 68, SEQ ID NO : 69 ou SEQ ID NO : 70)
   - (SEQ ID NO : 71, SEQ ID NO : 72 ou SEQ ID NO : 73)
x étant 2 ou 3,
ou les séquences desdites sondes ayant au moins 92% d'identité avec les susdites séquences SEQ ID NO : 62 à SEQ ID NO : 73
l'hybridation indiquant la présence d'algue toxique du genre ***Prorocentrum.***

Tous les différents modes de réalisation décrits pour le procédé de détection selon le mode de réalisation A peuvent s'appliquer pour le mode de réalisation D2.

Comme précédemment, dans un mode de réalisation particulier, le seuil de détection minimal de l'algue toxique du genre ***Prorocentrum*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation E2

Est décrit un des procédés de détection de cellules vivantes actives d'algues toxiques tels que décrits précédemment selon les modes de réalisation A2, B2, C2 ou D2, dans un échantillon susceptible de contenir en plus au moins une algue toxique du genre ***Chattonella,*** comprenant en plus l'addition de l'acide nucléique ribosomique d'une algue toxique du genre ***Chattonella*** et d'une sonde signal sur un support contenant une sonde capture,
ladite sonde capture et ladite sonde signal formant un couple de sondes, les séquences dudit couple de sondes étant choisies parmi x éléments de l'un des ensembles suivants :
   - (SEQ ID NO : 74, SEQ ID NO : 75 ou SEQ ID NO : 76)
   - (SEQ ID NO : 77, SEQ ID NO : 78 ou SEQ ID NO : 79)
   - (SEQ ID NO : 80, SEQ ID NO : 81 ou SEQ ID NO : 82)
x étant 2 ou 3,
ou les séquences desdites sondes ayant au moins 92% d'identité avec les susdites séquences SEQ ID NO : 74 à SEQ ID NO : 82
l'hybridation indiquant la présence d'algue toxique du genre ***Chattonella.***

Tous les différents modes de réalisation décrits pour le procédé de détection selon le mode de réalisation A peuvent s'appliquer pour le mode de réalisation E2.

Comme précédemment, dans un mode de réalisation particulier, le seuil de détection minimal de l'algue toxique du genre ***Chattonella*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation F2

Est décrit un des procédés de détection de cellules vivantes actives d'algues toxiques tels que décrits précédemment selon les modes de réalisation A2, B2, C2, D2 ou E2, dans un échantillon susceptible de contenir en plus au moins une algue toxique du genre ***Gymnodinium,*** comprenant en plus l'addition de l'acide nucléique ribosomique d'une algue toxique du genre ***Gymnodinium*** et d'une sonde signal sur un support contenant une sonde capture,
la sonde capture et la sonde signal formant un couple de sondes, les séquences dudit couple de sondes étant choisies parmi x éléments de l'un des ensembles suivants :
   - (SEQ ID NO : 83, SEQ ID NO : 84 ou SEQ ID NO : 85)
   - (SEQ ID NO : 86, SEQ ID NO : 87 ou SEQ ID NO : 88)
   - (SEQ ID NO : 89, SEQ ID NO : 90 ou SEQ ID NO : 91)
   - (SEQ ID NO : 92, SEQ ID NO : 93 ou SEQ ID NO : 94)
x étant 2 ou 3,
ou les séquences desdites sondes ayant au moins 92% d'identité avec les susdites séquences SEQ ID NO : 83 à SEQ ID NO : 94
l'hybridation indiquant la présence d'algue toxique du genre ***Gymnodinium.***

Tous les différents modes de réalisation décrits pour le procédé de détection selon le mode de réalisation A peuvent s'appliquer pour le mode de réalisation F2.

Comme précédemment, dans un mode de réalisation particulier, le seuil de détection minimal de l'algue toxique du genre ***Gymnodinium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation G2

Est décrit un des procédés de détection de cellules vivantes actives d'algues toxiques tels que décrits précédemment selon les modes de réalisation A2, B2, C2, D2, E2 ou F2, dans un échantillon susceptible de contenir en plus au moins une algue toxique du genre ***Karenia,*** comprenant en plus l'addition de l'acide nucléique ribosomique d'une algue toxique du genre ***Karenia*** et d'une sonde signal sur un support contenant une sonde capture,
la sonde capture et la sonde signal formant un couple de sondes, les séquences dudit couple de sondes étant choisies parmi x éléments de l'un des ensembles suivants :
   - (SEQ ID NO : 95, SEQ ID NO : 96 ou SEQ ID NO : 97)
   - (SEQ ID NO : 98, SEQ ID NO : 99 ou SEQ ID NO : 100)
   - (SEQ ID NO : 101, SEQ ID NO : 102 ou SEQ ID NO : 103)
   - (SEQ ID NO : 104, SEQ ID NO : 105 ou SEQ ID NO : 106)
   - (SEQ ID NO : 107, SEQ ID NO : 108 ou SEQ ID NO : 109)
   - (SEQ ID NO : 110, SEQ ID NO : 111 ou SEQ ID NO : 112)
   - (SEQ ID NO : 113, SEQ ID NO : 114 ou SEQ ID NO : 115)
   - (SEQ ID NO : 116, SEQ ID NO : 117 ou SEQ ID NO : 118)
x étant 2 ou 3,
ou les séquences desdites sondes ayant au moins 92% d'identité avec les susdites séquences SEQ ID NO : 95 à SEQ ID NO : 118
l'hybridation indiquant la présence d'algue toxique du genre ***Karenia.***

Tous les différents modes de réalisation décrits pour le procédé de détection selon le mode de réalisation A peuvent s'appliquer pour le mode de réalisation G2.

Comme précédemment, dans un mode de réalisation particulier, le seuil de détection minimal de l'algue toxique du genre *Karenia* est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation H2

Est décrit un des procédés de détection de cellules vivantes actives d'algues toxiques tels que décrits précédemment selon les modes de réalisation A2, B2, C2, D2, E2, F2 ou G2, dans un échantillon susceptible de contenir en plus au moins une algue toxique du genre ***Lingulodinium,*** comprenant en plus l'addition de l'acide nucléique ribosomique d'une algue toxique du genre ***Lingulodinium*** et d'une sonde signal sur un support contenant une sonde capture,
la sonde capture et la sonde signal formant un couple de sondes, les séquences dudit couple de sondes étant choisies parmi x éléments de l'un des ensembles suivants :
   - (SEQ ID NO : 119, SEQ ID NO : 120 ou SEQ ID NO : 121)
   - (SEQ ID NO : 122, SEQ ID NO : 123 ou SEQ ID NO : 124)
   - (SEQ ID NO : 125, SEQ ID NO : 126 ou SEQ ID NO : 127)
x étant 2 ou 3,
ou les séquences desdites sondes ayant au moins 92% d'identité avec les susdites séquences SEQ ID NO : 119 à SEQ ID NO : 127
l'hybridation indiquant la présence d'algue toxique du genre ***Lingulodinium.***

Tous les différents modes de réalisation décrits pour le procédé de détection selon le mode de réalisation A peuvent s'appliquer pour le mode de réalisation H2.

Comme précédemment, dans un mode de réalisation particulier, le seuil de détection minimal de l'algue toxique du genre ***Lingulodinium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation I2

Est décrit un des procédés de détection de cellules vivantes actives d'algues toxiques tels que décrits précédemment selon les modes de réalisation A2, B2, C2, D2, E2, F2, G2 ou H2 dans un échantillon susceptible de contenir en plus au moins une algue toxique du genre ***Heterosigma,*** comprenant en plus l'addition de l'acide nucléique ribosomique d'une algue toxique du genre ***Heterosigma*** et d'une sonde signal sur un support contenant une sonde capture,
la sonde capture et la sonde signal formant un couple de sondes, les séquences dudit couple de sondes étant choisies parmi x éléments de l'un des ensembles suivants :
   - (SEQ ID NO : 128 et SEQ ID NO : 129)
x étant 2 ou 3,
ou les séquences desdites sondes ayant au moins 92% d'identité avec les susdites séquences SEQ ID NO : 128 à SEQ ID NO : 129
l'hybridation indiquant la présence d'algue toxique du genre ***Heterosigma.***

Tous les différents modes de réalisation décrits pour le procédé de détection selon le mode de réalisation A peuvent s'appliquer pour le mode de réalisation I2.

Comme précédemment, dans un mode de réalisation particulier, le seuil de détection minimal de l'algue toxique du genre ***Heterosigma*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

Comme pour le premier aspect relatif à l'utilisation, toutes les combinaisons des modes de réalisation A2, B2, C2, D2, E2, F2, G2, H2 et/ou I2 de ce quatrième aspect peuvent être envisagées. De cette façon, l'ensemble des combinaisons d'algues toxiques B à I128 décrites dans le premier aspect peuvent être détectées par les procédés tels que décrits dans ce quatrième aspect.

### Mode de réalisation A3

Ainsi, un autre mode de réalisation particulier de ce quatrième aspect, décrit mais ne faisant pas partie de l'invention, concerne un procédé de détection de cellules vivantes actives d'algues toxiques dans un échantillon susceptible de contenir au moins une algue toxique du genre ***Alexandrium*** comprenant les étapes suivantes :
a) addition d'un complexe formé entre :
   - l'acide nucléique ribosomique d'une algue toxique du genre ***Alexandrium***
   - une sonde signal
   - et une sonde capture
   sur un support,
b) détection de l'hybridation éventuelle susdit complexe, l'hybridation ayant lieu entre la sonde capture, l'acide nucléique ribosomique et la sonde signal,
   l'hybridation indiquant la présence d'algue toxique du genre ***Alexandrium*** ladite sonde capture et ladite sonde signal formant un couple de sondes, les séquences dudit couple de sondes étant choisies parmi x éléments de l'un des ensembles suivants :
      - (SEQ ID NO : 1, SEQ ID NO : 2 ou SEQ ID NO : 3)
      - (SEQ ID NO : 4 et SEQ ID NO : 5)
      - (SEQ ID NO : 6 et SEQ ID NO : 7)
      - (SEQ ID NO : 8, SEQ ID NO : 9 ou SEQ ID NO : 10)
      - (SEQ ID NO : 11, SEQ ID NO : 12 ou SEQ ID NO : 13)
      - (SEQ ID NO : 14, SEQ ID NO : 15 ou SEQ ID NO : 16)
      - (SEQ ID NO : 17, SEQ ID NO : 18 ou SEQ ID NO : 19)
      - (SEQ ID NO : 20, SEQ ID NO : 21 ou SEQ ID NO : 22),
      - (SEQ ID NO : 23, SEQ ID NO : 24 ou SEQ ID NO : 25),
      - (SEQ ID NO : 26, SEQ ID NO : 27 ou SEQ ID NO : 28)
   x étant 2 ou 3,
   ou les séquences desdites sondes ayant au moins 92% d'identité avec les susdites séquences SEQ ID NO : 1 à SEQ ID NO : 28.

Tous les différents modes de réalisation décrits pour le procédé de détection selon le mode de réalisation A peuvent s'appliquer pour le mode de réalisation A3.

Comme précédemment, dans un mode de réalisation particulier, le seuil de détection minimal de l'algue toxique du genre ***Alexandrium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation B3

Est décrit un procédé de détection de cellules vivantes actives d'algues toxiques tels que décrit précédemment selon le mode de réalisation A3, dans un échantillon susceptible de contenir en plus au moins une algue toxique du genre ***Dinophysis,*** comprenant, en plus, l'addition d'un complexe formé entre :
- l'acide nucléique ribosomique d'une algue toxique du genre ***Dinophysis***
- une sonde signal
- et une sonde capture

sur un support,
ladite sonde capture et ladite sonde signal formant un couple de sondes, les séquences dudit couple de sondes étant choisies parmi x éléments de l'un des ensembles suivants :
   - (SEQ ID NO : 29, SEQ ID NO : 30 ou SEQ ID NO : 31)
   - (SEQ ID NO : 32 et SEQ ID NO : 33)
   - (SEQ ID NO : 34 et SEQ ID NO : 35)
   - (SEQ ID NO : 36 et SEQ ID NO : 37)
   - (SEQ ID NO : 38 et SEQ ID NO : 39)
   - (SEQ ID NO : 40, SEQ ID NO : 41 ou SEQ ID NO : 42)
   - (SEQ ID NO : 43, SEQ ID NO : 44 ou SEQ ID NO : 45), ou
   - (SEQ ID NO : 46, SEQ ID NO : 47 ou SEQ ID NO : 48)
x étant 2 ou 3,
ou les séquences desdites sondes ayant au moins 92% d'identité avec les susdites séquences SEQ ID NO : 29 à SEQ ID NO : 48
l'hybridation indiquant la présence d'algue toxique du genre ***Dinophysis.***

Tous les différents modes de réalisation décrits pour le procédé de détection selon le mode de réalisation A peuvent s'appliquer pour le mode de réalisation B3.

Comme précédemment, dans un mode de réalisation particulier, le seuil de détection minimal de l'algue toxique du genre ***Dinophysis*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation C3

Est décrit un des procédés de détection de cellules vivantes actives d'algues toxiques tels que décrits précédemment selon les modes de réalisation A3 ou B3, dans un échantillon susceptible de contenir en plus au moins une algue toxique du genre ***Pseudo-nitzschia**,* comprenant en plus l'addition d'un complexe formé entre :
- l'acide nucléique ribosomique d'une algue toxique du genre ***Pseudo-nitzschia***
- une sonde signal
- et une sonde capture

sur un support,
ladite sonde capture et ladite sonde signal formant un couple de sondes, les séquences dudit couple de sondes étant choisies parmi x éléments de l'un des ensembles suivants :
   - (SEQ ID NO : 49 et SEQ ID NO : 50)
   - (SEQ ID NO : 51 et SEQ ID NO : 52)
   - (SEQ ID NO : 53, SEQ ID NO : 54 ou SEQ ID NO : 55)
   - (SEQ ID NO : 56, SEQ ID NO : 57 ou SEQ ID NO : 58), ou
   - (SEQ ID NO : 59, SEQ ID NO : 60 ou SEQ ID NO : 61)
x étant 2 ou 3,
ou les séquences desdites sondes ayant au moins 92% d'identité avec les susdites séquences SEQ ID NO : 49 à SEQ ID NO : 61
l'hybridation indiquant la présence d'algue toxique du genre ***Pseudo-nitzschia.***

Tous les différents modes de réalisation décrits pour le procédé de détection selon le mode de réalisation A peuvent s'appliquer pour le mode de réalisation C3.

Comme précédemment, dans un mode de réalisation particulier, le seuil de détection minimal de l'algue toxique du genre ***Pseudo-nitzschia*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation D3

Est décrit un des procédés de détection de cellules vivantes actives d'algues toxiques tels que décrits précédemment selon les modes de réalisation A3, B3 ou C3, dans un échantillon susceptible de contenir en plus au moins une algue toxique du genre ***Prorocentrum,*** comprenant en plus l'addition d'un complexe formé entre :
- l'acide nucléique ribosomique d'une algue toxique du genre ***Prorocentrum***
- une sonde signal
- et une sonde capture

sur un support,
ladite sonde capture et ladite sonde signal formant un couple de sondes, les séquences dudit couple de sondes étant choisies parmi x éléments de l'un des ensembles suivants :
   - (SEQ ID NO : 62, SEQ ID NO : 63 ou SEQ ID NO : 64)
   - (SEQ ID NO : 65, SEQ ID NO : 66 ou SEQ ID NO : 67)
   - (SEQ ID NO : 68, SEQ ID NO : 69 ou SEQ ID NO : 70)
   - (SEQ ID NO : 71, SEQ ID NO : 72 ou SEQ ID NO : 73)
x étant 2 ou 3,
ou les séquences desdites sondes ayant au moins 92% d'identité avec les susdites séquences SEQ ID NO : 62 à SEQ ID NO : 73
l'hybridation indiquant la présence d'algue toxique du genre ***Prorocentrum.***

Tous les différents modes de réalisation décrits pour le procédé de détection selon le mode de réalisation A peuvent s'appliquer pour le mode de réalisation D3.

Comme précédemment, dans un mode de réalisation particulier, le seuil de détection minimal de l'algue toxique du genre ***Prorocentrum*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation E3

Est décrit un des procédés de détection de cellules vivantes actives d'algues toxiques tels que décrits précédemment selon les modes de réalisation A3, B3, C3 ou D3, dans un échantillon susceptible de contenir en plus au moins une algue toxique du genre ***Chattonella,*** comprenant en plus l'addition d'un complexe formé entre :
- l'acide nucléique ribosomique d'une algue toxique du genre ***Chattonella***
- une sonde signal
- et une sonde capture

sur un support,
ladite sonde capture et ladite sonde signal formant un couple de sondes, les séquences dudit couple de sondes étant choisies parmi x éléments de l'un des ensembles suivants :
   - (SEQ ID NO : 74, SEQ ID NO : 75 ou SEQ ID NO : 76)
   - (SEQ ID NO : 77, SEQ ID NO : 78 ou SEQ ID NO : 79)
   - (SEQ ID NO : 80, SEQ ID NO : 81 ou SEQ ID NO : 82)
x étant 2 ou 3,
ou les séquences desdites sondes ayant au moins 92% d'identité avec les susdites séquences SEQ ID NO : 74 à SEQ ID NO : 82
l'hybridation indiquant la présence d'algue toxique du genre ***Chattonella.***

Tous les différents modes de réalisation décrits pour le procédé de détection selon le mode de réalisation A peuvent s'appliquer pour le mode de réalisation E3.

Comme précédemment, dans un mode de réalisation particulier, le seuil de détection minimal de l'algue toxique du genre ***Chatonella*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation F3

Est décrit un des procédés de détection de cellules vivantes actives d'algues toxiques tels que décrits précédemment selon les modes de réalisation A3, B3, C3, D3 ou E3, dans un échantillon susceptible de contenir en plus au moins une algue toxique du genre ***Gymnodinium,*** comprenant en plus l'addition d'un complexe formé entre :
- l'acide nucléique ribosomique d'une algue toxique du genre ***Gymnodinium***
- une sonde signal
- et une sonde capture

sur un support,
la sonde capture et la sonde signal formant un couple de sondes, les séquences dudit couple de sondes étant choisies parmi x éléments de l'un des ensembles suivants :
   - (SEQ ID NO : 83, SEQ ID NO : 84 ou SEQ ID NO : 85)
   - (SEQ ID NO : 86, SEQ ID NO : 87 ou SEQ ID NO : 88)
   - (SEQ ID NO : 89, SEQ ID NO : 90 ou SEQ ID NO : 91)
   - (SEQ ID NO : 92, SEQ ID NO : 93 ou SEQ ID NO : 94)
x étant 2 ou 3,
ou les séquences desdites sondes ayant au moins 92% d'identité avec les susdites séquences SEQ ID NO : 83 à SEQ ID NO : 94
l'hybridation indiquant la présence d'algue toxique du genre ***Gymnodinium.***

Tous les différents modes de réalisation décrits pour le procédé de détection selon le mode de réalisation A peuvent s'appliquer pour le mode de réalisation F3.

Comme précédemment, dans un mode de réalisation particulier, le seuil de détection minimal de l'algue toxique du genre ***Gymnodinium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation G3

Est décrit un des procédés de détection de cellules vivantes actives d'algues toxiques tels que décrits précédemment selon les modes de réalisation A3, B3, C3, D3, E3 ou F3, dans un échantillon susceptible de contenir en plus au moins une algue toxique du genre ***Karenia,*** comprenant en plus l'addition d'un complexe formé entre :
- l'acide nucléique ribosomique d'une algue toxique du genre ***Karenia***
- une sonde signal
- et une sonde capture

sur un support,
la sonde capture et la sonde signal formant un couple de sondes, les séquences dudit couple de sondes étant choisies parmi x éléments de l'un des ensembles suivants :
   - (SEQ ID NO : 95, SEQ ID NO : 96 ou SEQ ID NO : 97)
   - (SEQ ID NO : 98, SEQ ID NO : 99 ou SEQ ID NO : 100)
   - (SEQ ID NO : 101, SEQ ID NO : 102 ou SEQ ID NO : 103)
   - (SEQ ID NO : 104, SEQ ID NO : 105 ou SEQ ID NO : 106)
   - (SEQ ID NO : 107, SEQ ID NO : 108 ou SEQ ID NO : 109)
   - (SEQ ID NO : 110, SEQ ID NO : 111 ou SEQ ID NO : 112)
   - (SEQ ID NO : 113, SEQ ID NO : 114 ou SEQ ID NO : 115)
   - (SEQ ID NO : 116, SEQ ID NO : 117 ou SEQ ID NO : 118)
x étant 2 ou 3,
ou les séquences desdites sondes ayant au moins 92% d'identité avec les susdites séquences SEQ ID NO : 95 à SEQ ID NO : 118
l'hybridation indiquant la présence d'algue toxique du genre ***Karenia.***

Tous les différents modes de réalisation décrits pour le procédé de détection selon le mode de réalisation A peuvent s'appliquer pour le mode de réalisation G3.

Comme précédemment, dans un mode de réalisation particulier, le seuil de détection minimal de l'algue toxique du genre ***Karenia*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation H3

Est décrit un des procédés de détection de cellules vivantes actives d'algues toxiques tels que décrits précédemment selon les modes de réalisation A3, B3, C3, D3, E3, F3 ou G3, dans un échantillon susceptible de contenir en plus au moins une algue toxique du genre ***Lingulodinium,*** comprenant en plus l'addition d'un complexe formé entre :
- l'acide nucléique ribosomique d'une algue toxique du **genre *Lingulodinium***
- une sonde signal
- et une sonde capture

sur un support,
la sonde capture et la sonde signal formant un couple de sondes, les séquences dudit couple de sondes étant choisies parmi x éléments de l'un des ensembles suivants :
   - (SEQ ID NO : 119, SEQ ID NO : 120 ou SEQ ID NO : 121)
   - (SEQ ID NO : 122, SEQ ID NO : 123 ou SEQ ID NO : 124)
   - (SEQ ID NO : 125, SEQ ID NO : 126 ou SEQ ID NO : 127)
x étant 2 ou 3,
ou les séquences desdites sondes ayant au moins 92% d'identité avec les susdites séquences SEQ ID NO : 119 à SEQ ID NO : 127
l'hybridation indiquant la présence d'algue toxique du genre ***Lingulodinium.***

Tous les différents modes de réalisation décrits pour le procédé de détection selon le mode de réalisation A peuvent s'appliquer pour le mode de réalisation H3.

Comme précédemment, dans un mode de réalisation particulier, le seuil de détection minimal de l'algue toxique du genre ***Lingulodinium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation 13

Est décrit un des procédés de détection de cellules vivantes actives d'algues toxiques tels que décrits précédemment selon les modes de réalisation A3, B3, C3, D3, E3, F3, G3 ou H3, dans un échantillon susceptible de contenir en plus au moins une algue toxique du genre ***Heterosigma,*** comprenant en plus l'addition d'un complexe formé entre :
- l'acide nucléique ribosomique d'une algue toxique du genre ***Heterosigma***
- une sonde signal
- et une sonde capture

sur un support,
la sonde capture et la sonde signal formant un couple de sondes, les séquences dudit couple de sondes étant choisies parmi x éléments de l'un des ensembles suivants :
   - (SEQ ID NO : 128 et SEQ ID NO : 129)
x étant 2 ou 3,
ou les séquences desdites sondes ayant au moins 92% d'identité avec les susdites séquences SEQ ID NO : 128 à SEQ ID NO : 129
l'hybridation indiquant la présence d'algue toxique du genre ***Heterosigma.***

Tous les différents modes de réalisation décrits pour le procédé de détection selon le mode de réalisation A peuvent s'appliquer pour le mode de réalisation I3.

Comme précédemment, dans un mode de réalisation particulier, le seuil de détection minimal de l'algue toxique du genre ***Heterosigma*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

Comme pour le premier aspect relatif à l'utilisation, toutes les combinaisons des modes de réalisation A3, B3, C3, D3, E3, F3, G3, H3 et/ou I3 de ce quatrième aspect peuvent être envisagées. De cette façon, l'ensemble des combinaisons d'algues toxiques B à I128 décrites dans le premier aspect peuvent être détectées par les procédés tels que décrits dans ce quatrième aspect.

### Mode de réalisation A4

Un autre mode de réalisation particulier de ce quatrième aspect, décrit mais ne faisant pas partie de l'invention, concerne un procédé de détection de cellules vivantes actives d'algues toxiques dans un échantillon susceptible de contenir au moins une algue toxique du genre *Alexandrium* comprenant les étapes suivantes :
a) addition d'une sonde signal et d'un complexe éventuel formé entre l'acide nucléique ribosomique d'une algue toxique du genre ***Alexandrium*** et une sonde capture sur un support,
b) détection de l'hybridation éventuelle du susdit complexe avec ladite sonde signal, l'hybridation ayant lieu entre la sonde signal et l'acide nucléique ribosomique du susdit complexe,
   l'hybridation indiquant la présence d'algue toxique du genre ***Alexandrium*** ladite sonde capture et ladite sonde signal formant un couple de sondes, les séquences dudit couple de sondes étant choisies parmi x éléments de l'un des ensembles suivants :
      - (SEQ ID NO: 1, SEQ ID NO : 2 ou SEQ ID NO : 3)
      - (SEQ ID NO : 4 et SEQ ID NO : 5)
      - (SEQ ID NO : 6 et SEQ ID NO : 7)
      - (SEQ ID NO : 8, SEQ ID NO : 9 ou SEQ ID NO : 10)
      - (SEQ ID NO : 11, SEQ ID NO : 12 ou SEQ ID NO : 13)
      - (SEQ ID NO : 14, SEQ ID NO : 15 ou SEQ ID NO : 16)
      - (SEQ ID NO : 17, SEQ ID NO : 18 ou SEQ ID NO : 19)
      - (SEQ ID NO : 20, SEQ ID NO : 21 ou SEQ ID NO : 22),
      - (SEQ ID NO : 23, SEQ ID NO : 24 ou SEQ ID NO : 25),
      - (SEQ ID NO : 26, SEQ ID NO : 27ou SEQ ID NO : 28)
   x étant 2 ou 3,
   ou les séquences desdites sondes ayant au moins 92% d'identité avec les susdites séquences SEQ ID NO : 1 à SEQ ID NO : 28.

Tous les différents modes de réalisation décrits pour le procédé de détection selon le mode de réalisation A peuvent s'appliquer pour le mode de réalisation A4.

Comme précédemment, dans un mode de réalisation particulier, le seuil de détection minimal de l'algue toxique du genre ***Alexandrium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation B4

Est décrit un procédé de détection de cellules vivantes actives d'algues toxiques tel que décrit précédemment selon le mode de réalisation A4, dans un échantillon susceptible de contenir en plus au moins une algue toxique du genre ***Dinophysis,*** comprenant, en plus, l'addition d'une sonde signal et d'un complexe éventuel formé entre l'acide nucléique ribosomique d'une algue toxique du genre ***Dinophysis*** et une sonde capture sur un support,
ladite sonde capture et ladite sonde signal formant un couple de sondes, les séquences dudit couple de sondes étant choisies parmi x éléments de l'un des ensembles suivants :
   - (SEQ ID NO : 29, SEQ ID NO : 30 ou SEQ ID NO : 31)
   - (SEQ ID NO : 32 et SEQ ID NO : 33)
   - (SEQ ID NO : 34 et SEQ ID NO : 35)
   - (SEQ ID NO : 36 et SEQ ID NO : 37)
   - (SEQ ID NO : 38 et SEQ ID NO : 39)
   - (SEQ ID NO : 40, SEQ ID NO : 41 ou SEQ ID NO : 42)
   - (SEQ ID NO : 43, SEQ ID NO : 44 ou SEQ ID NO : 45), ou
   - (SEQ ID NO : 46, SEQ ID NO : 47 ou SEQ ID NO : 48)
x étant 2 ou 3,
ou les séquences desdites sondes ayant au moins 92% d'identité avec les susdites séquences SEQ ID NO : 29 à SEQ ID NO : 48
l'hybridation indiquant la présence d'algue toxique du genre ***Dinophysis.***

Tous les différents modes de réalisation décrits pour le procédé de détection selon le mode de réalisation A peuvent s'appliquer pour le mode de réalisation B4.

Comme précédemment, dans un mode de réalisation particulier, le seuil de détection minimal de l'algue toxique du genre ***Dinophysis*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation C4

Est décrit un des procédés de détection de cellules vivantes actives d'algues toxiques tels que décrits précédemment selon les modes de réalisation A4 ou B4, dans un échantillon susceptible de contenir en plus au moins une algue toxique du genre ***Pseudo-nitzschia,*** comprenant en plus l'addition d'une sonde signal et d'un complexe éventuel formé entre l'acide nucléique ribosomique d'une algue toxique du genre ***Pseudo-nitzschia*** une sonde capture sur un support, ladite sonde capture et ladite sonde signal formant un couple de sondes, les séquences dudit couple de sondes étant choisies parmi x éléments de l'un des ensembles suivants :
- (SEQ ID NO : 49 et SEQ ID NO : 50)
- (SEQ ID NO : 51 et SEQ ID NO : 52)
- (SEQ ID NO : 53, SEQ ID NO : 54 ou SEQ ID NO : 55)
- (SEQ ID NO : 56, SEQ ID NO : 57 ou SEQ ID NO : 58), ou
- (SEQ ID NO : 59, SEQ ID NO : 60 ou SEQ ID NO : 61)

x étant 2 ou 3,
ou les séquences desdites sondes ayant au moins 92% d'identité avec les susdites séquences SEQ ID NO : 49 à SEQ ID NO : 61
l'hybridation indiquant la présence d'algue toxique du genre ***Pseudo-nitzschia.***

Tous les différents modes de réalisation décrits pour le procédé de détection selon le mode de réalisation A peuvent s'appliquer pour le mode de réalisation C4.

Comme précédemment, dans un mode de réalisation particulier, le seuil de détection minimal de l'algue toxique du genre ***Pseudo-nitzschia*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation D4

Est décrit un des procédés de détection de cellules vivantes actives d'algues toxiques tels que décrits précédemment selon les modes de réalisation A4, B4 ou C4, dans un échantillon susceptible de contenir en plus au moins une algue toxique du genre ***Prorocentrum,*** comprenant en plus l'addition d'une sonde signal et d'un complexe éventuel formé entre l'acide nucléique ribosomique d'une algue toxique du genre ***Prorocentrum*** et une sonde capture sur un support, ladite sonde capture et ladite sonde signal formant un couple de sondes, les séquences dudit couple de sondes étant choisies parmi x éléments de l'un des ensembles suivants :
- (SEQ ID NO : 62, SEQ ID NO : 63 ou SEQ ID NO : 64)
- (SEQ ID NO : 65, SEQ ID NO : 66 ou SEQ ID NO : 67)
- (SEQ ID NO : 68, SEQ ID NO : 69 ou SEQ ID NO : 70)
- (SEQ ID NO : 71, SEQ ID NO : 72 ou SEQ ID NO : 73)

x étant 2 ou 3,
ou les séquences desdites sondes ayant au moins 92% d'identité avec les susdites séquences SEQ ID NO : 62 à SEQ ID NO : 73
l'hybridation indiquant la présence d'algue toxique du genre ***Prorocentrum.***

Tous les différents modes de réalisation décrits pour le procédé de détection selon le mode de réalisation A peuvent s'appliquer pour le mode de réalisation D4.

Comme précédemment, dans un mode de réalisation particulier, le seuil de détection minimal de l'algue toxique du genre ***Prorocentrum*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation E4

Est décrit un des procédés de détection de cellules vivantes actives d'algues toxiques tels que décrits précédemment selon les modes de réalisation A4, B4, C4 ou D4, dans un échantillon susceptible de contenir en plus au moins une algue toxique du genre ***Chattonella,*** comprenant en plus l'addition d'une sonde signal et d'un complexe éventuel formé entre l'acide nucléique ribosomique d'une algue toxique du genre ***Chattonella*** et une sonde capture sur un support,
ladite sonde capture et ladite sonde signal formant un couple de sondes, les séquences dudit couple de sondes étant choisies parmi x éléments de l'un des ensembles suivants :
   - (SEQ ID NO : 74, SEQ ID NO : 75 ou SEQ ID NO : 76)
   - (SEQ ID NO : 77, SEQ ID NO : 78 ou SEQ ID NO : 79)
   - (SEQ ID NO : 80, SEQ ID NO : 81 ou SEQ ID NO : 82)
x étant 2 ou 3,
ou les séquences desdites sondes ayant au moins 92% d'identité avec les susdites séquences SEQ ID NO : 74 à SEQ ID NO : 82
l'hybridation indiquant la présence d'algue toxique du genre ***Chattonella.***

Tous les différents modes de réalisation décrits pour le procédé de détection selon le mode de réalisation A peuvent s'appliquer pour le mode de réalisation E4.

Comme précédemment, dans un mode de réalisation particulier, le seuil de détection minimal de l'algue toxique du genre ***Chattonella*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation F4

Est décrit un des procédés de détection de cellules vivantes actives d'algues toxiques tels que décrits précédemment selon les modes de réalisation A4, B4, C4, D4 ou E4, dans un échantillon susceptible de contenir en plus au moins une algue toxique du genre ***Gymnodinium,*** comprenant en plus l'addition d'une sonde signal et d'un complexe éventuel formé entre l'acide nucléique ribosomique d'une algue toxique du genre ***Gymnodinium*** et une sonde capture sur un support,
la sonde capture et la sonde signal formant un couple de sondes, les séquences dudit couple de sondes étant choisies parmi x éléments de l'un des ensembles suivants :
   - (SEQ ID NO : 83, SEQ ID NO : 84 ou SEQ ID NO : 85)
   - (SEQ ID NO : 86, SEQ ID NO : 87 ou SEQ ID NO : 88)
   - (SEQ ID NO : 89, SEQ ID NO : 90 ou SEQ ID NO : 91)
   - (SEQ ID NO : 92, SEQ ID NO : 93 ou SEQ ID NO : 94)
x étant 2 ou 3,
ou les séquences desdites sondes ayant au moins 92% d'identité avec les susdites séquences SEQ ID NO : 83 à SEQ ID NO : 94
l'hybridation indiquant la présence d'algue toxique du genre ***Gymnodinium.***

Tous les différents modes de réalisation décrits pour le procédé de détection selon le mode de réalisation A peuvent s'appliquer pour le mode de réalisation F4.

Comme précédemment, dans un mode de réalisation particulier, le seuil de détection minimal de l'algue toxique du genre ***Gymnodinium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation G4

Est décrit un des procédés de détection de cellules vivantes actives d'algues toxiques tels que décrits précédemment selon les modes de réalisation A4, B4, C4, D4, E4 ou F4, dans un échantillon susceptible de contenir en plus au moins une algue toxique du genre ***Karenia,*** comprenant en plus l'addition d'une sonde signal et d'un complexe éventuel formé entre l'acide nucléique ribosomique d'une algue toxique du genre ***Karenia*** et une sonde capture sur un support,
la sonde capture et la sonde signal formant un couple de sondes, les séquences dudit couple de sondes étant choisies parmi x éléments de l'un des ensembles suivants :
   - (SEQ ID NO : 95, SEQ ID NO : 96 ou SEQ ID NO : 97)
   - (SEQ ID NO : 98, SEQ ID NO : 99 ou SEQ ID NO : 100)
   - (SEQ ID NO : 101, SEQ ID NO : 102 ou SEQ ID NO : 103)
   - (SEQ ID NO : 104, SEQ ID NO : 105 ou SEQ ID NO : 106)
   - (SEQ ID NO : 107, SEQ ID NO : 108 ou SEQ ID NO : 109)
   - (SEQ ID NO : 110, SEQ ID NO : 111 ou SEQ ID NO : 112)
   - (SEQ ID NO : 113, SEQ ID NO : 114 ou SEQ ID NO : 115)
   - (SEQ ID NO : 116, SEQ ID NO : 117 ou SEQ ID NO : 118)
x étant 2 ou 3,
ou les séquences desdites sondes ayant au moins 92% d'identité avec les susdites séquences SEQ ID NO : 95 à SEQ ID NO : 118
l'hybridation indiquant la présence d'algue toxique du genre *Karenia.*

Tous les différents modes de réalisation décrits pour le procédé de détection selon le mode de réalisation A peuvent s'appliquer pour le mode de réalisation G4.

Comme précédemment, dans un mode de réalisation particulier, le seuil de détection minimal de l'algue toxique du genre *Karenia* est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation H4

Est décrit un des procédés de détection de cellules vivantes actives d'algues toxiques tels que décrits précédemment selon les modes de réalisation A4, B4, C4, D4, E4, F4 ou G4, dans un échantillon susceptible de contenir en plus au moins une algue toxique du genre ***Lingulodinium,*** comprenant en plus l'addition d'une sonde signal et d'un complexe éventuel formé entre l'acide nucléique ribosomique d'une algue toxique du genre ***Lingulodinium*** et une sonde capture sur un support,
la sonde capture et la sonde signal formant un couple de sondes, les séquences dudit couple de sondes étant choisies parmi x éléments de l'un des ensembles suivants :
   - (SEQ ID NO : 119, SEQ ID NO : 120 ou SEQ ID NO : 121)
   - (SEQ ID NO : 122, SEQ ID NO : 123 ou SEQ ID NO : 124)
   - (SEQ ID NO : 125, SEQ ID NO : 126 ou SEQ ID NO : 127)
x étant 2 ou 3,
ou les séquences desdites sondes ayant au moins 92% d'identité avec les susdites séquences SEQ ID NO : 119 à SEQ ID NO : 127
l'hybridation indiquant la présence d'algue toxique du genre ***Lingulodinium.***

Tous les différents modes de réalisation décrits pour le procédé de détection selon le mode de réalisation A peuvent s'appliquer pour le mode de réalisation H4.

Comme précédemment, dans un mode de réalisation particulier, le seuil de détection minimal de l'algue toxique du genre ***Lingulodinium*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

### Mode de réalisation I4

Est décrit un des procédés de détection de cellules vivantes actives d'algues toxiques tels que décrits précédemment selon les modes de réalisation A4, B4, C4, D4, E4, F4, G4 ou H4, dans un échantillon susceptible de contenir en plus au moins une algue toxique du genre ***Heterosigma,*** comprenant en plus l'addition d'une sonde signal et d'un complexe éventuel formé entre l'acide nucléique ribosomique d'une algue toxique du genre ***Heterosigma*** et une sonde capture sur un support,
la sonde capture et la sonde signal formant un couple de sondes, les séquences dudit couple de sondes étant choisies parmi x éléments de l'un des ensembles suivants :
   - (SEQ ID NO : 128 et SEQ ID NO : 129)
x étant 2 ou 3,
ou les séquences desdites sondes ayant au moins 92% d'identité avec les susdites séquences SEQ ID NO : 128 à SEQ ID NO : 129
l'hybridation indiquant la présence d'algue toxique du genre ***Heterosigma.***

Tous les différents modes de réalisation décrits pour le procédé de détection selon le mode de réalisation A peuvent s'appliquer pour le mode de réalisation I4.

Comme précédemment, dans un mode de réalisation particulier, le seuil de détection minimal de l'algue toxique du genre ***Heterosigma*** est
- de 100 à 500 cellules vivantes actives par litre d'échantillon (cellules/L) et en particulier inférieur à 200 cellules vivantes actives par litre d'échantillon (cellules/L), ou
- inférieur ou égal à 0,10 ng d'ARN par litre d'échantillon et en particulier compris de 0,01 à 0,09 ng d'ARN par litre d'échantillon.

De la même façon, dans un mode de réalisation particulier, la durée de la mise en œuvre dudit procédé de détection est inférieure à une heure.

Comme pour le premier aspect relatif à l'utilisation, toutes les combinaisons des modes de réalisation A4, B4, C4, D4, E4, F4, G4, H4 et/ou I4 de ce quatrième aspect peuvent être envisagées. De cette façon, l'ensemble des combinaisons d'algues toxiques B à I128 décrites dans le premier aspect peuvent être détectées par les procédés tels que décrits dans ce quatrième aspect.

Selon un mode de réalisation, et dans tous les aspects de ce quatrième aspect, un contrôle positif peut être utilisé. Le contrôle positif peut par exemple être un acide nucléique synthétique complémentaire de la sonde capture et de la sonde signal. Le contrôle positif peut également être utilisé comme standard.

Selon un mode de réalisation, et dans tous les aspects de ce quatrième aspect, un contrôle négatif peut être utilisé. Le contrôle négatif peut par exemple être un acide nucléique synthétique non complémentaire de la sonde capture et de la sonde signal.

Selon un mode de réalisation, et dans tous les aspects de ce quatrième aspect, la détection simultanée de plusieurs algues est possible. Dans ce cas, la détection simultanée est effectuée sur le même support, mais de façon distincte. Par exemple, dans le cas où le support est une microplaque, la détection de chaque algue à détecter se fait dans des puits séparés de la microplaque.

Un cinquième aspect concerne des kits pour la détection de cellules vivantes actives d'algues toxiques.

### Mode de réalisation A

Un mode de réalisation décrit mais ne faisant pas partie de l'invention concerne un kit pour la détection de cellules vivantes actives d'algues toxiques d'algues toxiques du genre ***Alexandrium,*** ledit kit contenant :
a) au moins un couple de sondes spécifiques d'algues toxiques du genre ***Alexandrium,*** les séquences desdites sondes étant choisies parmi x éléments de l'un des ensembles suivants :
   - (SEQ ID NO: 1, SEQ ID NO : 2 ou SEQ ID NO : 3)
   - (SEQ ID NO : 4 et SEQ ID NO : 5)
   - (SEQ ID NO : 6 et SEQ ID NO : 7)
   - (SEQ ID NO : 8, SEQ ID NO : 9 ou SEQ ID NO : 10)
   - (SEQ ID NO : 11, SEQ ID NO : 12 ou SEQ ID NO : 13)
   - (SEQ ID NO : 14, SEQ ID NO : 15 ou SEQ ID NO : 16)
   - (SEQ ID NO : 17, SEQ ID NO : 18 ou SEQ ID NO : 19)
   - (SEQ ID NO : 20, SEQ ID NO : 21 ou SEQ ID NO : 22)
   - (SEQ ID NO : 23, SEQ ID NO : 24 ou SEQ ID NO : 25)
   - (SEQ ID NO : 26, SEQ ID NO : 27 ou SEQ ID NO : 28)

   x étant 2 ou 3,
   ou les séquences desdites sondes ayant au moins 92% d'identité avec les susdites séquences SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 25, SEQ ID NO : 26, SEQ ID NO : 27, SEQ ID NO : 28
   une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence
   ladite sonde capture et ladite sonde signal étant capables de s'hybrider avec l'acide nucléique ribosomique d'une algue toxique du genre ***Alexandrium,***
b) éventuellement une solution d'hybridation
c) éventuellement une solution de lavage, et
d) éventuellement une solution de révélation.

En particulier, l'invention a pour objet un kit pour la détection de cellules vivantes actives d'algues toxiques d'algues toxiques du genre *Alexandrium,* ledit kit contenant :
a) au moins un couple de sondes spécifiques d'algues toxiques du genre *Alexandrium,* les séquences des sondes desdits couples étant les suivantes :
   - (SEQ ID NO : 1 et SEQ ID NO : 2),
   - (SEQ ID NO : 4 et SEQ ID NO : 5),
   - (SEQ ID NO : 11 et SEQ ID NO : 12), ou
   - (SEQ ID NO : 17 et SEQ ID NO : 18),

   une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
   ladite sonde capture et ladite sonde signal étant capables de s'hybrider avec l'ARN ribosomique d'une algue toxique du genre *Alexandrium,*
b) éventuellement une solution d'hybridation,
c) éventuellement une solution de lavage, et
d) éventuellement une solution de révélation.

### Mode de réalisation B

L'invention concerne également un kit tel que décrit dans la revendication 9 pour la détection de cellules vivantes actives d'algues toxiques d'algues toxiques du genre ***Alexandrium*** et/ou ***Dinophysis,*** ledit kit contenant **en plus :**
a) au moins un couple de sondes spécifiques d'algues toxiques du genre ***Dinophysis,*** les séquences desdites sondes étant choisies parmi x éléments de l'un des ensembles suivants :
   - (SEQ ID NO : 29, SEQ ID NO : 30 ou SEQ ID NO : 31)
   - (SEQ ID NO : 32 et SEQ ID NO : 33)
   - (SEQ ID NO : 34 et SEQ ID NO : 35)
   - (SEQ ID NO : 36 et SEQ ID NO : 37)
   - (SEQ ID NO : 38 et SEQ ID NO : 39)
   - (SEQ ID NO : 40, SEQ ID NO : 41 ou SEQ ID NO : 42)
   - (SEQ ID NO : 3, SEQ ID NO : 44 ou SEQ ID NO : 45), ou
   - (SEQ ID NO : 46, SEQ ID NO : 47 ou SEQ ID NO : 48)

   x étant 2 ou 3,
   ou les séquences desdites sondes ayant au moins 92% d'identité avec les susdites séquences SEQ ID NO : 29, SEQ ID NO : 30, SEQ ID NO : 31, SEQ ID NO : 32, SEQ ID NO: 33, SEQ ID NO: 34, SEQ ID NO: 35, SEQ ID NO: 36, SEQ ID NO : 37, SEQ ID NO : 38, SEQ ID NO : 39, SEQ ID NO: 40, SEQ ID NO : 41, SEQ ID NO : 42, SEQ ID NO : 43, SEQ ID NO: 44, SEQ ID NO : 45, SEQ ID NO : 46, SEQ ID NO : 47 ou SEQ ID NO : 48
   une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence
   ladite sonde capture et ladite sonde signal étant capables de s'hybrider avec l'acide nucléique ribosomique d'une algue toxique du genre ***Dinophysis.***

### Mode de réalisation C

Est décrit un kit tel que décrit précédemment selon le mode de réalisation A ou selon le mode de réalisation B, pour la détection de cellules vivantes actives d'algues toxiques d'algues toxiques du genre ***Alexandrium*** et/ou ***Pseudo-nitzschia,*** ledit kit contenant **en plus :**
a) au moins un couple de sondes spécifiques d'algues toxiques du genre ***Pseudo-nitzschia,*** les séquences desdites sondes étant choisies parmi x éléments de l'un des ensembles suivants :
   - (SEQ ID NO : 49 et SEQ ID NO : 50)
   - (SEQ ID NO : 51 et SEQ ID NO : 52)
   - (SEQ ID NO : 53, SEQ ID NO : 54 ou SEQ ID NO : 55)
   - (SEQ ID NO : 56, SEQ ID NO : 57, SEQ ID NO : 58)
   - (SEQ ID NO : 59, SEQ ID NO : 60, SEQ ID NO : 61)

   x étant 2 ou 3,
   ou les séquences desdites sondes ayant au moins 92% d'identité avec les susdites séquences SEQ ID NO : 49, SEQ ID NO : 50, SEQ ID NO : 51, SEQ ID NO : 52, SEQ ID NO: 53, SEQ ID NO: 54, SEQ ID NO: 55, SEQ ID NO: 56, SEQ ID NO : 57, SEQ ID NO : 58, SEQ ID NO : 59, SEQ ID NO: 60, SEQ ID NO : 61,
   une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
   ladite sonde capture et ladite sonde signal étant capables de s'hybrider avec l'acide nucléique ribosomique d'une algue toxique du genre ***Pseudo-nitzschia.***

### Mode de réalisation D

Est décrit un kit tel que décrit précédemment selon le mode de réalisation A, B ou C, pour la détection de cellules vivantes actives d'algues toxiques d'algues toxiques du genre ***Alexandrium*** et/ou ***Prorocentrum,*** ledit kit contenant **en plus :**
a) au moins un couple de sondes spécifiques d'algues toxiques du genre ***Prorocentrum,*** les séquences desdites sondes étant choisies parmi x éléments de l'un des ensembles suivants :
   - (SEQ ID NO : 62, SEQ ID NO : 63 ou SEQ ID NO : 64)
   - (SEQ ID NO : 65, SEQ ID NO : 66 ou SEQ ID NO : 67)
   - (SEQ ID NO : 68, SEQ ID NO : 69 ou SEQ ID NO : 70)
   - (SEQ ID NO : 71, SEQ ID NO : 72 ou SEQ ID NO : 73)

   x étant 2 ou 3,
   ou les séquences desdites sondes ayant au moins 92% d'identité avec les susdites séquences SEQ ID NO : 62, SEQ ID NO : 63, SEQ ID NO : 64, SEQ ID NO : 65, SEQ ID NO: 66, SEQ ID NO: 67, SEQ ID NO: 68, SEQ ID NO: 69, SEQ ID NO : 70, SEQ ID NO : 71, SEQ ID NO : 72 ou SEQ ID NO : 73,
   une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
   ladite sonde capture et ladite sonde signal étant capables de s'hybrider avec l'acide nucléique ribosomique d'une algue toxique du genre ***Prorocentrum.***

### Mode de réalisation E

Est décrit un kit tel que décrit précédemment selon le mode de réalisation A, B, C ou D, pour la détection de cellules vivantes actives d'algues toxiques d'algues toxiques du genre ***Alexandrium*** et/ou ***Chattonella,*** ledit kit contenant **en plus** :
a) au moins un couple de sondes spécifiques d'algues toxiques du genre *Chattonella,* les séquences desdites sondes étant choisies parmi x éléments de l'un des ensembles suivants :
   - (SEQ ID NO : 74, SEQ ID NO : 75 ou SEQ ID NO : 76)
   - (SEQ ID NO : 77, SEQ ID NO : 78 ou SEQ ID NO : 79)
   - (SEQ ID NO : 80, SEQ ID NO : 81 ou SEQ ID NO : 82)

   x étant 3,
   ou les séquences desdites sondes ayant au moins 92% d'identité avec les susdites séquences SEQ ID NO : 74, SEQ ID NO : 75, SEQ ID NO : 76, SEQ ID NO : 77, SEQ ID NO: 78, SEQ ID NO: 79, SEQ ID NO: 80, SEQ ID NO: 81 ou SEQ ID NO : 82,
   une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
   ladite sonde capture et ladite sonde signal étant capables de s'hybrider avec l'acide nucléique ribosomique d'une algue toxique du genre ***Chattonella.***

### Mode de réalisation F

Est décrit un kit tel que décrit précédemment selon le mode de réalisation A, B, C, D ou E, pour la détection de cellules vivantes actives d'algues toxiques d'algues toxiques du genre ***Alexandrium*** et/ou ***Gymnodinium,*** ledit kit contenant **en plus** :
a) au moins un couple de sondes spécifiques d'algues toxiques du genre ***Gymnodinium,*** les séquences desdites sondes étant choisies parmi x éléments de l'un des ensembles suivants :
   - (SEQ ID NO : 83, SEQ ID NO : 84 ou SEQ ID NO : 85)
   - (SEQ ID NO : 86, SEQ ID NO : 87 ou SEQ ID NO : 88)
   - (SEQ ID NO : 89, SEQ ID NO : 90 ou SEQ ID NO : 91)
   - (SEQ ID NO : 92, SEQ ID NO : 93 ou SEQ ID NO : 94)

   x étant 3,
   ou les séquences desdites sondes ayant au moins 92% d'identité avec les susdites séquences SEQ ID NO : 83, SEQ ID NO : 84, SEQ ID NO : 85, SEQ ID NO : 6, SEQ ID NO : 87, SEQ ID NO : 88, SEQ ID NO : 89, SEQ ID NO: 90, SEQ ID NO : 91, SEQ ID NO : 92, SEQ ID NO : 93 ou SEQ ID NO : 94,
   une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
   ladite sonde capture et ladite sonde signal étant capables de s'hybrider avec l'acide nucléique ribosomique d'une algue toxique du genre *Gymnodinium.*

### Mode de réalisation G

Est décrit un kit tel que décrit précédemment selon le mode de réalisation A, B, C, D, E ou F, pour la détection de cellules vivantes actives d'algues toxiques d'algues toxiques du genre *Alexandrium* et/ou *Karenia,* ledit kit contenant en plus :
a) au moins un couple de sondes spécifiques d'algues toxiques du genre *Karenia,* les séquences desdites sondes étant choisies parmi x éléments de l'un des ensembles suivants :
   - (SEQ ID NO : 95, SEQ ID NO : 96 ou SEQ ID NO : 97)
   - (SEQ ID NO : 98, SEQ ID NO : 99 ou SEQ ID NO : 100)
   - (SEQ ID NO : 101, SEQ ID NO : 102 ou SEQ ID NO : 103)
   - (SEQ ID NO : 104, SEQ ID NO : 105 ou SEQ ID NO : 106)
   - (SEQ ID NO : 107, SEQ ID NO : 108 ou SEQ ID NO : 109)
   - (SEQ ID NO : 110, SEQ ID NO : 111 ou SEQ ID NO : 112)
   - (SEQ ID NO : 113, SEQ ID NO : 114 ou SEQ ID NO : 115)
   - (SEQ ID NO : 116, SEQ ID NO : 117 ou SEQ ID NO : 118)

   x étant 3,
   ou les séquences desdites sondes ayant au moins 92% d'identité avec les susdites séquences SEQ ID NO : 95, SEQ ID NO : 96, SEQ ID NO : 97, SEQ ID NO : 98, SEQ ID NO: 99, SEQ ID NO: 100, SEQ ID NO: 101, SEQ ID NO: 102, SEQ ID NO : 103, SEQ ID NO : 104, SEQ ID NO : 105, SEQ ID NO : 106, SEQ ID NO : 107, SEQ ID NO : 108, SEQ ID NO : 109, SEQ ID NO : 110, SEQ ID NO : 111, SEQ ID NO : 112, SEQ ID NO : 113, SEQ ID NO : 114, SEQ ID NO : 115, SEQ ID NO : 116, SEQ ID NO : 117 ou SEQ ID NO : 118,
   une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
   ladite sonde capture et ladite sonde signal étant capables de s'hybrider avec l'acide nucléique ribosomique d'une algue toxique du genre *Karenia.*

### Mode de réalisation H

Est décrit un kit tel que décrit précédemment selon le mode de réalisation A, B, C, D, E, F ou G, pour la détection de cellules vivantes actives d'algues toxiques d'algues toxiques du genre *Alexandrium* et/ou *Lingulodinium,* ledit kit contenant en plus :
a) au moins un couple de sondes spécifiques d'algues toxiques du genre *Lingulodinium,* les séquences desdites sondes étant choisies parmi x éléments de l'un des ensembles suivants :
   - (SEQ ID NO : 119, SEQ ID NO : 120 ou SEQ ID NO : 121)
   - (SEQ ID NO : 122, SEQ ID NO : 123 ou SEQ ID NO : 124)
   - (SEQ ID NO : 125, SEQ ID NO : 126 ou SEQ ID NO : 127)

   x étant 3,
   ou les séquences desdites sondes ayant au moins 92% d'identité avec les susdites séquences SEQ ID NO : 119, SEQ ID NO : 120, SEQ ID NO : 121, SEQ ID NO : 122, SEQ ID NO : 123, SEQ ID NO : 124, SEQ ID NO : 125, SEQ ID NO : 126 ou SEQ ID NO : 127,
   une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
   ladite sonde capture et ladite sonde signal étant capables de s'hybrider avec l'acide nucléique ribosomique d'une algue toxique du genre ***Lingulodinium.***

### Mode de réalisation I

Est décrit un kit tel que décrit précédemment selon le mode de réalisation A, B, C, D, E, F, G ou H, pour la détection de cellules vivantes actives d'algues toxiques d'algues toxiques du genre ***Alexandrium*** et/ou ***Heterosigma,*** ledit kit contenant en **plus** :
a) au moins un couple de sondes spécifiques d'algues toxiques du genre ***Heterosigma,*** les séquences desdites sondes étant choisies parmi x éléments de l'un des ensembles suivants :
   - (SEQ ID NO : 128 et SEQ ID NO : 129)

   x étant 2,
   ou les séquences desdites sondes ayant au moins 92% d'identité avec les susdites séquences SEQ ID NO : 128 ou SEQ ID NO : 129,
   une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
   ladite sonde capture et ladite sonde signal étant capables de s'hybrider avec l'acide nucléique ribosomique d'une algue toxique du genre ***Heterosigma.***

Comme pour le premier aspect relatif à l'utilisation, toutes les combinaisons des modes de réalisation A, B, C, D, E, F, G, H et/ou I de ce cinquième aspect peuvent être envisagées. De cette façon, l'ensemble des combinaisons d'algues toxiques B à I128 décrites dans le premier aspect peuvent être détectées par les kits tels que décrits dans ce cinquième aspect.

Dans l'ensemble des modes de réalisation de ce cinquième aspect et selon un mode de réalisation particulier, ladite sonde capture est liée à au moins une molécule d'attache positionnée en 5' de sa séquence et ladite sonde signal est liée à au moins une molécule de marquage positionnée en 5' de sa séquence.

Dans l'ensemble des modes de réalisation de ce cinquième aspect et selon un autre mode de réalisation particulier, ladite sonde capture est liée à au moins une molécule d'attache positionnée en 5' de sa séquence et ladite sonde signal est liée à au moins une molécule de marquage positionnée en 3' de sa séquence.

Dans l'ensemble des modes de réalisation de ce cinquième aspect et selon un autre mode de réalisation particulier, ladite sonde capture est liée à au moins une molécule d'attache positionnée en 3' de sa séquence et ladite sonde signal est liée à au moins une molécule de marquage positionnée en 5' de sa séquence.

Dans l'ensemble des modes de réalisation de ce cinquième aspect et selon un autre mode de réalisation particulier, ladite sonde capture est liée à une molécule d'attache positionnée en 3' de sa séquence et ladite sonde signal est liée à une ou plusieurs molécules de marquage positionnées en 3' de sa séquence.

Dans l'ensemble des modes de réalisation de ce cinquième aspect, ladite « au moins une molécule d'attache »peut être choisie parmi une molécule de biotine, avidine, streptavidine, un groupe thiol, un groupe amine et un groupe carbone.

Dans l'ensemble des modes de réalisation de ce cinquième aspect et dans un mode de réalisation particulier, ladite « au moins une molécule d'attache »est une molécule de biotine

Dans l'ensemble des modes de réalisation de ce cinquième aspect, ladite « au moins une molécule de marquage »peut être choisie parmi un fluorochrome, une biotine, une molécule liée à une biotine, la digoxigénine, une enzyme utilisant un substrat chimioluminescent, une enzyme utilisant un substrat chromogène ou une enzyme utilisant un substrat à oxydation électrochimique.

Dans l'ensemble des modes de réalisation de ce cinquième aspect et dans un mode de réalisation particulier, ladite au moins une molécule de marquage est la digoxigénine.

Dans l'ensemble des modes de réalisation de ce cinquième aspect, ledit fluorochrome peut être choisi parmi le groupe consistant en : Alexa fluor, en particulier Alexa fluor 350, 405, 430, 488, 500, 514, 532, 546, 555, 568, 594, 610, 633, 647, 660, 680, 700, 750 ou 790, Isothiocyanate de fluorescéine (FITC), Rhodamine, Allophycocyanine (APC) et Phycoérythrine (PE).

Dans l'ensemble des modes de réalisation de ce cinquième aspect, ladite enzyme utilisant un substrat chimioluminescent peut être la péroxydasede raifort (HRP) et ledit substrat chimioluminescent peut être le luminol, ou encore, ladite enzyme utilisant un substrat chimioluminescent peut être la luciférase et ledit substrat chimioluminescent peut être la luciférine.

Dans l'ensemble des modes de réalisation de ce cinquième aspect, ladite enzyme utilisant un substrat chromogène peut être la phosphatase alcaline et ledit substrat chromogène peut être Nitrobleu de tetrazolium (NBT) ou le bromochlorylindolophosphate (BCIP), ou encore, ladite enzyme utilisant un substrat chromogène peut être la péroxidase de raifort (HRP) et ledit substrat chromogène peut être choisi parmi le 3,3'-Diaminobenzidine (DAB), le 3,3',5,5'-Tetramethylbenzidine (TMB), ou le 2,2'-azino-bis(acide 3-ethylbenzothiazoline-6-sulphonique) (ABTS).

Dans l'ensemble des modes de réalisation de ce cinquième aspect, ladite enzyme utilisant un substrat à oxydation électrochimique peut être la péroxydase de raifort (HRP) et ledit substrat à oxydation électrochimique peut être le 3,3',5,5'-Tetramethylbenzidine (TMB).

Dans l'ensemble des modes de réalisation de ce cinquième aspect et dans un mode de réalisation particulier, ladite solution d'hybridation peut comprendre 0 à 0,3 M de NaCl, 0 à 0,1 M de tampon choisi parmi du citrate, du Tris-HCl, du PIPES, de l'HEPES ou du phosphate, 0,001 à 0,05 % d'agent détergent choisi parmi le SDS, le triton^{®}, le TWEEN^{®}20, éventuellement 0,001 à 0,5 M d'agent chélateur choisi parmi l'EDTA ou l'EGTA, éventuellement 0,1 à 30% d'agent bloquant choisi parmi la BSA, l'ADN de Hareng, l'ADN de saumon, l'ADN de veau, l'ADN de levure ou une protéine exogène et éventuellement un autre agent chimique choisi parmi le MgCl₂, le CaCl₂ et le KCl, de préférence le MgCl₂.

Dans l'ensemble des modes de réalisation de ce cinquième aspect et dans un autre mode de réalisation particulier, ladite solution d'hybridation peut comprendre 0,1 M à 1 M de NaCl ou de KCl, 0,01 M à 1 M de Tris-HCl, d'HEPES, de PBS, de KH₂PO₄ ou de SSC d'un pH allant de 6,0 à 9,0, 0,01 et 0,05% d'agent détergent choisi parmi du SDS ou du N-Lauroylsarcosine, éventuellement 0,01 et 0,1 M d'agent chélateur choisi parmi l'EDTA, l'EGTA ou un agent chélateur similaire choisi parmi le citrate de calcium ou l'hexamétaphosphate de sodium et éventuellement 0,1 et 30% d'agent bloquant choisi parmi une protéine telle que la protéine Bovine Serum Albumin (BSA) ou un acide nucléique tels que l'ADN de Hareng.

Dans l'ensemble des modes de réalisation de ce cinquième aspect et dans un autre mode de réalisation particulier, ladite solution d'hybridation peut être constituée de 0,3 M de NaCl, de 0,08 M de Tris-HCl et de 0,04% de SDS et est de pH 8.

Dans l'ensemble des modes de réalisation de ce cinquième aspect et dans un mode de réalisation particulier, ladite solution de lavage peut comprendre 0 à 0,3 M de NaCl, 0 à 0,1 M de tampon choisi parmi du citrate, du Tris-HCl, du PIPES, de l'HEPES ou du phosphate, 0,001 à 0,05 % d'agent détergent choisi parmi le SDS, le triton^{®}, le TWEEN^{®}20, éventuellement 0,001 à 0,5 M d'agent chélateur choisi parmi l'EDTA ou l'EGTA, éventuellement 0,1 à 30% d'agent bloquant choisi parmi la BSA, l'ADN de Hareng, l'ADN de saumon, l'ADN de veau, l'ADN de levure ou une protéine exogène et éventuellement un autre agent chimique choisi parmi le MgCl₂, le CaCl₂ et le KCl, de préférence le MgCl₂.

Dans l'ensemble des modes de réalisation de ce cinquième aspect et dans un autre mode de réalisation particulier, ladite solution de lavage peut comprendre 0,1 M à 1 M de NaCl ou de KCl, 0,01 M à 1 M de Tris-HCl, d'HEPES, de PBS, de KH₂PO₄ ou de SSC d'un pH allant de 6,0 à 9,0, 0,01 et 0,05% d'agent détergent choisi parmi du SDS ou du N-Lauroylsarcosine, éventuellement 0,01 et 0,1 M d'agent chélateur choisi parmi l'EDTA, l'EGTA ou un agent chélateur similaire choisi parmi le citrate de calcium ou l'hexamétaphosphate de sodium et éventuellement 0,1 et 30% d'agent bloquant choisi parmi une protéine telle que la protéine Bovine Serum Albumin (BSA) ou un acide nucléique tels que l'ADN de Hareng.

Dans l'ensemble des modes de réalisation de ce cinquième aspect et dans un autre mode de réalisation particulier, ladite solution de lavage peut comprendre 0,01 et 0,7 M de PBS, de Na2HPO4, de KH₂PO₄, de K₂PO₄ et/ou de SSC, et 0,1 et 0,4 M de NaCl ou de KCl.

Dans l'ensemble des modes de réalisation de ce cinquième aspect et dans un autre mode de réalisation particulier, ladite solution de lavage peut être constituée de 0,1M de K₂PO₄, de 0,1M de KH₂PO₄ et de 0,1M de KCl et est de pH 7,6.

Dans l'ensemble des modes de réalisation de ce cinquième aspect, on entend par *« solution de révélation »,* toute solution contenant les moyens nécessaires à la révélation de l'hybridation éventuelle entre la sonde capture, l'acide nucléique ribosomique et la sonde signal. Selon la molécule de marquage utilisée, le kit peut comprendre une ou plusieurs solutions de révélation.

Par exemple, lorsque la molécule de marquage est une molécule de biotine ou est conjuguée à une molécule de biotine, la solution de révélation peut contenir un fluorochrome conjugué à de la streptavidine ou de l'avidine.

Par exemple, lorsque la molécule de marquage est une molécule de digoxigénine, la solution de révélation peut contenir un fluorochrome conjugué à un anticorps anti-digoxigénine.

Par exemple, lorsque la molécule de marquage est une enzyme utilisant un substrat chimioluminescent, comme par exemple la peroxydase de raifort ou la luciférase, la solution de révélation peut contenir le substrat chimioluminescent correspondant, comme par exemple le luminol lorsque l'enzyme utilisant un substrat chimioluminescent est la peroxydase de raifort ou la luciférine lorsque l'enzyme utilisant un substrat chimioluminescent est la luciférase.

Par exemple, lorsque la molécule de marquage est une enzyme utilisant un substrat chromogène, comme par exemple la phosphatase alcaline ou la péroxydase de raifort, la solution de révélation peut contenir le substrat chromogène correspondant, comme par exemple le Nitrobleu de tetrazolium (NBT) ou le bromochlorylindolophosphate (BCIP) lorsque l'enzyme utilisant un substrat chromogène est la phosphatase alcaline ou le 3,3'-Diaminobenzidine (DAB), le 3,3',5,5'-Tetramethylbenzidine (TMB), ou le 2,2'-azino-bis(acide 3-ethylbenzothiazoline-6-sulphonique) (ABTS) lorsque l'enzyme utilisant un substrat chimioluminescent est la peroxydase de raifort.

Par exemple, lorsque la molécule de marquage est une enzyme utilisant un substrat à oxydation électrochimique, comme par exemple la péroxydase de raifort, la solution de révélation peut contenir le substrat à oxydation électrochimique, comme par exemple le 3,3',5,5'-Tetramethylbenzidine (TMB).

Par exemple, lorsque la molécule de marquage est une molécule de biotine ou est conjuguée à une molécule de biotine, le kit peut comprendre deux solutions de révélation.

Une des solutions de révélation peut, par exemple, comprendre :
- une enzyme utilisant un substrat chimioluminescent, comme par exemple la peroxydase de raifort ou la luciférase, couplée à la streptavidine ou à l'avidine, ou
- une enzyme utilisant un substrat chromogène, comme par exemple la phosphatase alcaline ou la peroxydase de raifort, couplée à la streptavidine ou à l'avidine, ou
- une enzyme utilisant un substrat à oxydation électrochimique, comme par exemple la peroxydase de raifort.

L'autre solution de révélation peut comprendre :
- un substrat chimioluminescent, comme par exemple le luminol lorsque l'enzyme utilisant un substrat chimioluminescent est la peroxydase de raifort ou la luciférine lorsque l'enzyme utilisant un substrat chimioluminescent est la luciférase, ou
- un substrat chromogène, comme par exemple le Nitrobleu de tetrazolium (NBT) ou le bromochlorylindolophosphate (BCIP) lorsque l'enzyme utilisant un substrat chromogène est la phosphatase alcaline ou le 3,3'-Diaminobenzidine (DAB), le 3,3',5,5'-Tetramethylbenzidine (TMB), ou le 2,2'-azino-bis(acide 3-ethylbenzothiazoline-6-sulphonique) (ABTS) lorsque l'enzyme utilisant un substrat chimioluminescent est la peroxydase de raifort, ou
- un substrat à oxydation électrochimique, comme par exemple le 3,3',5,5'-Tetramethylbenzidine (TMB).

Par exemple, lorsque la molécule de marquage est une molécule de digoxigénine le kit peut comprendre deux solutions de révélation. Une des solutions de révélation peut, par exemple, contenir :
- une enzyme utilisant un substrat chimioluminescent conjuguée à un anticorps anti-digoxigénine, ou
- une enzyme utilisant un substrat chromogène conjuguée à un anticorps anti-digoxigénine, ou
- une enzyme utilisant un substrat à oxydation électrochimique conjuguée à un anticorps anti-digoxigénine, ou

L'autre solution de révélation peut comprendre :
- un substrat chimioluminescent, comme par exemple le luminol lorsque l'enzyme utilisant un substrat chimioluminescent est la peroxydase de raifort ou la luciférine lorsque l'enzyme utilisant un substrat chimioluminescent est la luciférase, ou
- un substrat chromogène, comme par exemple le Nitrobleu de tetrazolium (NBT) ou le bromochlorylindolophosphate (BCIP) lorsque l'enzyme utilisant un substrat chromogène est la phosphatase alcaline ou le 3,3'-Diaminobenzidine (DAB), le 3,3',5,5'-Tetramethylbenzidine (TMB), ou le 2,2'-azino-bis(acide 3-ethylbenzothiazoline-6-sulphonique) (ABTS) lorsque l'enzyme utilisant un substrat chimioluminescent est la peroxydase de raifort, ou
- un substrat à oxydation électrochimique, comme par exemple le 3,3',5,5'-Tetramethylbenzidine (TMB).

Dans l'ensemble des modes de réalisation de ce cinquième aspect et dans un mode de réalisation particulier, ledit kit peut comprendre en plus une solution de lyse.

Selon ce mode de réalisation particulier, ladite solution de lyse peut comprendre un tampon neutre choisi parmi du phosphate, du SSC ou du Tris, un agent chaotropique choisi parmi du guanidium chloride, un détergent ionique ou non ionique tel que le sodium dodecyl sulfate (SDS) ou le Triton^{®} X100, un agent réducteur choisi parmi du b-mercaptoethanol ou du DiThioTreitol et un agent chélateur choisi parmi de l'acide Éthylène Diamine Tétra Acétique (EDTA) ou de l'acide Éthylène Glycol Tétra Acétique (EGTA).

Dans l'ensemble des modes de réalisation de ce cinquième aspect et dans un mode de réalisation particulier, ledit kit peut comprendre en plus un substrat chromogène lorsque :
- la molécule de marquage est une enzyme utilisant un substrat chromogène,
- la molécule de marquage est la biotine et est détectée *via* une enzyme utilisant un substrat chromogène conjuguée à de la streptavidine ou de l'avidine
- la molécule de marquage est conjuguée à la biotine et est détectée *via* une enzyme utilisant un substrat chromogène conjuguée à de la streptavidine ou de l'avidine, ou
- la molécule de marquage est la digoxigénine et est détectée *via* une enzyme utilisant un substrat chromogène conjuguée à un anticorps anti-digoxigénine.

Selon ce mode de réalisation particulier, ladite enzyme utilisant un substrat chromogène peut être la phosphatase alcaline et ledit substrat chromogène peut être Nitrobleu de tetrazolium (NBT) ou le bromochlorylindolophosphate (BCIP), ou ladite enzyme utilisant un substrat chromogène peut être la péroxidase de raifort (HRP) et ledit substrat chromogène peut être choisi parmi le 3,3'-Diaminobenzidine (DAB), le 3,3',5,5'-Tetramethylbenzidine (TMB), ou le 2,2'-azino-bis(acide 3-ethylbenzothiazoline-6-sulphonique) (ABTS).

Dans l'ensemble des modes de réalisation de ce cinquième aspect et dans un mode de réalisation particulier, ledit kit peut comprendre en plus un substrat chimioluminescent lorsque :
- la molécule de marquage est une enzyme utilisant un substrat chimioluminescent
- la molécule de marquage est la biotine et est détectée *via* une enzyme utilisant un substrat chimioluminescent conjuguée à de la streptavidine ou de l'avidine
- la molécule de marquage est conjuguée à la biotine et est détectée *via* une enzyme utilisant un substrat chimioluminescent conjuguée à de la streptavidine ou de l'avidine, ou
- la molécule de marquage est la digoxigénine et est détectée *via* une enzyme utilisant un substrat chimioluminescent conjuguée à un anticorps anti-digoxigénine.

Selon ce mode de réalisation particulier, ladite enzyme utilisant un substrat chimioluminescent peut être la péroxidase de raifort (HRP) et ledit substrat chimioluminescent peut être le luminol, ou ladite enzyme utilisant un substrat chimioluminescent peut être la luciférase et ledit substrat chimioluminescent peut être la luciférine.

Dans l'ensemble des modes de réalisation de ce cinquième aspect et dans un mode de réalisation particulier, ledit kit peut comprendre en plus un substrat à oxydation électrochimique lorsque :
- la molécule de marquage est une enzyme utilisant un substrat à oxydation électrochimique
- la molécule de marquage est la biotine et est détectée *via* une enzyme utilisant un substrat à oxydation électrochimique conjuguée à de la streptavidine ou de l'avidine
- la molécule de marquage est conjuguée à la biotine et est détectée *via* une enzyme utilisant un substrat à oxydation électrochimique conjuguée à de la streptavidine ou de l'avidine, ou
- la molécule de marquage est la digoxigénine et est détectée *via* une enzyme utilisant un substrat à oxydation électrochimique conjuguée à un anticorps anti-digoxigénine.

Selon ce mode de réalisation particulier, ladite enzyme utilisant un substrat à oxydation électrochimique peut être la péroxydase de raifort (HRP) et ledit substrat à oxydation électrochimique peut être le 3,3',5,5'-Tetramethylbenzidine (TMB).

Dans l'ensemble des modes de réalisation de ce cinquième aspect et dans un mode de réalisation particulier, ledit kit peut comprendre en plus une solution contenant un anticorps anti-digoxigénine lorsque la molécule de marquage est la digoxigénine

Selon ce mode de réalisation particulier, ledit anticorps anti-digoxigénine peut être conjugué :
- à un fluorochrome
- à un enzyme utilisant un substrat chromogène
- à un enzyme utilisant un substrat chimioluminescent
- à un enzyme utilisant un substrat à oxydation électrochimique.

Dans l'ensemble des modes de réalisation de ce cinquième aspect, ledit fluorochrome peut être choisi parmi le groupe consistant en : Alexa fluor, en particulier Alexa fluor 350, 405, 430, 488, 500, 514, 532, 546, 555, 568, 594, 610, 633, 647, 660, 680, 700, 750 ou 790, Isothiocyanate de fluorescéine (FITC), Rhodamine, Allophycocyanine (APC) et Phycoérythrine (PE).

Dans l'ensemble des modes de réalisation de ce cinquième aspect, ladite enzyme utilisant un substrat chromogène peut être la phosphatase alcaline et ledit substrat chromogène peut être Nitrobleu de tetrazolium (NBT) ou le bromochlorylindolophosphate (BCIP), ou ladite enzyme utilisant un substrat chromogène peut être la péroxidase de raifort (HRP) et ledit substrat chromogène peut être choisi parmi le 3,3'-Diaminobenzidine (DAB), le 3,3',5,5'-Tetramethylbenzidine (TMB), ou le 2,2'-azino-bis(acide 3-ethylbenzothiazoline-6-sulphonique) (ABTS).

Dans l'ensemble des modes de réalisation de ce cinquième aspect, ladite enzyme utilisant un substrat chimioluminescent peut être la péroxydase de raifort (HRP) et ledit substrat chimioluminescent peut être le luminol, ou ladite enzyme utilisant un substrat chimioluminescent peut être la luciférase et ledit substrat chimioluminescent peut être la luciférine.

Dans l'ensemble des modes de réalisation de ce cinquième aspect, ladite enzyme utilisant un substrat à oxydation électrochimique peut être la péroxydase de raifort (HRP) et ledit substrat à oxydation électrochimique peut être le 3,3',5,5'-Tetramethylbenzidine (TMB).

Dans l'ensemble des modes de réalisation de ce cinquième aspect, et selon un mode de réalisation particulier, ledit kit peut comprendre en plus un support.

Selon ce mode de réalisation particulier, ledit support peut être choisi parmi choisi parmi le groupe consistant en : une microplaque, une lame de verre, des billes magnétiques, des électrodes imprimées en différente matières comme le carbone ou l'or.

Selon ce mode de réalisation particulier, ledit support peut être fonctionnalisé avec de la streptavidine, de l'avidine, un groupement aldéhyde, un groupement epoxy, un groupement carboxyl, un groupement isothiocyanate, de l'or, du mercaptosilane ou un groupement maléimide.

Ainsi, est décrit également un kit tel que décrit ci-dessus, ledit kit comprenant en plus un support,
ledit support étant notamment choisi parmi le groupe consistant en : une microplaque, une lame de verre, des billes magnétiques, des électrodes imprimées en différente matières comme le carbone ou l'or,
de préférence une microplaque ou des billes magnétiques.

Dans l'ensemble des modes de réalisation de ce cinquième aspect, et selon un mode de réalisation particulier, ledit kit peut comprendre en plus un contrôle positif, le contrôle positif étant une molécule d'acide nucléique synthétique complémentaire de ladite sonde signal et de ladite sonde capture.

Dans l'ensemble des modes de réalisation de ce cinquième aspect, et selon un mode de réalisation particulier, ledit kit peut comprendre en plus un contrôle négatif, le contrôle négatif étant une molécule d'acide nucléique synthétique non complémentaire de ladite sonde signal et de ladite sonde capture.

Dans l'ensemble des modes de réalisation de ce cinquième aspect et dans un mode de réalisation particulier, lesdites sondes signal peuvent être conservées dans une des solutions d'hybridation telles que définies précédemment.

Dans l'ensemble des modes de réalisation de ce cinquième aspect, et dans un mode de réalisation particulier, lesdites sondes captures peuvent être conservées sur un support tel que défini précédemment.

Dans l'ensemble des modes de réalisation de ce cinquième aspect, et dans un mode de réalisation particulier, ledit support contenant lesdites sondes captures peut être conservé dans une solution de conservation comme par exemple la solution commerciale ProClin^{®} (Sigma-Aldrich^{®}, 48912-U)

Dans l'ensemble des modes de réalisation de ce cinquième aspect, et dans un autre mode de réalisation particulier, ledit support contenant lesdites sondes captures peut être conservé de préférence lyophilisées sur le support.

Dans un aspect particulier, ledit kit est conservé de préférence à 4°C.

Dans l'ensemble des modes de réalisation de ce cinquième aspect, et dans un mode de réalisation particulier, ledit kit peut contenir en plus une procédure d'utilisation dudit kit.

Un autre aspect de ce cinquième aspect, décrit mais ne faisant pas partie de l'invention, concerne un kit pour la détection de cellules vivantes actives d'algues toxiques d'algues toxiques du genre *Alexandrium,* ledit kit contenant :
a) au moins un couple de sondes spécifiques d'algues toxiques du genre *Alexandrium,* les séquences desdites sondes étant choisies parmi x éléments de l'un des ensembles suivants :
   - (SEQ ID NO : 1, SEQ ID NO : 2 ou SEQ ID NO : 3)
   - (SEQ ID NO : 4 et SEQ ID NO : 5)
   - (SEQ ID NO : 6 et SEQ ID NO : 7)
   - (SEQ ID NO : 8, SEQ ID NO : 9 ou SEQ ID NO : 10)
   - (SEQ ID NO : 11, SEQ ID NO : 12 ou SEQ ID NO : 13)
   - (SEQ ID NO : 14, SEQ ID NO : 15 ou SEQ ID NO : 16)
   - (SEQ ID NO : 17, SEQ ID NO : 18 ou SEQ ID NO : 19)
   - (SEQ ID NO : 20, SEQ ID NO : 21 ou SEQ ID NO : 22)
   - (SEQ ID NO : 23, SEQ ID NO : 24 ou SEQ ID NO : 25)
   - (SEQ ID NO : 26, SEQ ID NO : 27 ou SEQ ID NO : 28)

   x étant 2 ou 3,
   ou les séquences desdites sondes ayant au moins 92% d'identité avec les susdites séquences SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO: 10, SEQ ID NO: 11, SEQ ID NO: 12, SEQ ID NO: 13, SEQ ID NO : 14, SEQ ID NO : 15, SEQ ID NO : 16, SEQ ID NO: 17, SEQ ID NO : 18, SEQ ID NO : 19, SEQ ID NO : 20, SEQ ID NO: 21, SEQ ID NO : 22, SEQ ID NO : 23, SEQ ID NO : 24, SEQ ID NO: 25, SEQ ID NO : 26, SEQ ID NO : 27, SEQ ID NO : 28,
   une sonde dudit couple étant une sonde capture liée à au moins une molécule de d'attache positionnée en 3' ou en 5' de sa séquence et l'autre sonde dudit couple étant une sonde signal liée à au moins une molécule signal positionnée en 3' ou en 5' de sa séquence,
   ladite sonde capture et ladite sonde signal étant capables de s'hybrider avec l'acide nucléique ribosomique d'une algue toxique du genre ***Alexandrium,***
   ladite molécule d'attache étant une molécule de biotine,
   ladite molécule signal étant la digoxigénine
b) une solution d'hybridation contenant 0,3M de NaCl, 0,08M de Tris-HCl et 0,04% SDS et de pH 8,
c) une solution de lavage contenant 0,1M de K₂PO₄, de 0,1M de KH₂PO₄ et de 0,1M de KCl et est de pH 7,6,
d) une solution de lyse étant une solution commerciale du kit Quick-RNA^{™} MiniPrep (Zymo Research^{®}, USA),
e) un support, ledit support étant une microplaque fonctionnalisée avec de la streptavidine ou de l'avidine
f) une solution contenant un anticorps anti-digoxigénine, ledit anticorps anti-digoxigénine étant lié à la péroxidase de raifort (HRP)
g) un substrat chromogène, ledit substrat chromogène étant le 3,3', 5, 5'-Tetramethylbenzidine (TMB),
h) un contrôle positif, ledit contrôle positif étant une molécule d'acide nucléique synthétique complémentaire de ladite sonde signal et de ladite sonde capture
i) un contrôle négatif, ledit contrôle négatif étant une molécule d'acide nucléique synthétique non complémentaire de ladite sonde signal et de ladite sonde capture
   lesdites sondes captures étant conservées lyophilisées sur ledit support,
   lesdites sondes signal étant conservées dans ladite solution d'hybridation.

Un autre aspect de ce cinquième aspect, décrit mais ne faisant pas partie de l'invention, concerne un kit pour la détection de cellules vivantes actives d'algues toxiques d'algues toxiques du genre ***Alexandrium,*** ledit kit contenant :
a) au moins un couple de sondes spécifiques d'algues toxiques du genre ***Alexandrium,*** les séquences desdites sondes étant choisies parmi x éléments de l'un des ensembles suivants :
   - (SEQ ID NO: 1, SEQ ID NO : 2 ou SEQ ID NO : 3)
   - (SEQ ID NO : 4 et SEQ ID NO : 5)
   - (SEQ ID NO : 6 et SEQ ID NO : 7)
   - (SEQ ID NO : 8, SEQ ID NO : 9 ou SEQ ID NO : 10)
   - (SEQ ID NO : 11, SEQ ID NO : 12 ou SEQ ID NO : 13)
   - (SEQ ID NO : 14, SEQ ID NO : 15 ou SEQ ID NO : 16)
   - (SEQ ID NO : 17, SEQ ID NO : 18 ou SEQ ID NO : 19)
   - (SEQ ID NO : 20, SEQ ID NO : 21 ou SEQ ID NO : 22)
   - (SEQ ID NO : 23, SEQ ID NO : 24 ou SEQ ID NO : 25)
   - (SEQ ID NO : 26, SEQ ID NO : 27 ou SEQ ID NO : 28)

   x étant 2 ou 3,
   ou les séquences desdites sondes ayant au moins 92% d'identité avec les susdites séquences SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO: 10, SEQ ID NO: 11, SEQ ID NO: 12, SEQ ID NO: 13, SEQ ID NO : 14, SEQ ID NO : 15, SEQ ID NO : 16, SEQ ID NO: 17, SEQ ID NO : 18, SEQ ID NO : 19, SEQ ID NO : 20, SEQ ID NO: 21, SEQ ID NO : 22, SEQ ID NO : 23, SEQ ID NO : 24, SEQ ID NO: 25, SEQ ID NO : 26, SEQ ID NO : 27, SEQ ID NO : 28,
   une sonde dudit couple étant une sonde capture liée à au moins une molécule de d'attache positionnée en 3' ou en 5' de sa séquence et l'autre sonde dudit couple étant une sonde signal liée à au moins une molécule signal positionnée en 3' ou en 5' de sa séquence,
   ladite sonde capture et ladite sonde signal étant capables de s'hybrider avec l'acide nucléique ribosomique d'une algue toxique du genre ***Alexandrium,***
   ladite molécule d'attache étant une molécule de biotine,
   ladite molécule signal étant la digoxigénine
b) au moins un couple de sondes spécifiques d'algues toxiques du genre ***Dinophysis,*** les séquences desdites sondes étant choisies parmi x éléments de l'un des ensembles suivants :
   - (SEQ ID NO : 29, SEQ ID NO : 30 ou SEQ ID NO : 31)
   - (SEQ ID NO : 32 et SEQ ID NO : 33)
   - (SEQ ID NO : 34 et SEQ ID NO : 35)
   - (SEQ ID NO : 36 et SEQ ID NO : 37)
   - (SEQ ID NO : 38 et SEQ ID NO : 39)
   - (SEQ ID NO : 40, SEQ ID NO : 41 ou SEQ ID NO : 42)
   - (SEQ ID NO : 43, SEQ ID NO : 44 ou SEQ ID NO : 45), ou
   - (SEQ ID NO : 46, SEQ ID NO : 47 ou SEQ ID NO : 48)

   x étant 2 ou 3,
   ou les séquences desdites sondes ayant au moins 92% d'identité avec les susdites séquences SEQ ID NO : 29, SEQ ID NO : 30, SEQ ID NO : 31, SEQ ID NO : 32, SEQ ID NO: 33, SEQ ID NO: 34, SEQ ID NO: 35, SEQ ID NO: 36, SEQ ID NO : 37, SEQ ID NO : 38, SEQ ID NO : 39, SEQ ID NO: 40, SEQ ID NO : 41, SEQ ID NO : 42, SEQ ID NO : 43, SEQ ID NO: 44, SEQ ID NO : 45, SEQ ID NO : 46, SEQ ID NO : 47 ou SEQ ID NO : 48,
   une sonde dudit couple étant une sonde capture liée à au moins une molécule de d'attache positionnée en 3' ou en 5' de sa séquence et l'autre sonde dudit couple étant une sonde signal liée à au moins une molécule signal positionnée en 3' ou en 5' de sa séquence,
   ladite sonde capture et ladite sonde signal étant capables de s'hybrider avec l'acide nucléique ribosomique d'une algue toxique du genre ***Dinophysis,***
   ladite molécule d'attache étant une molécule de biotine,
   ladite molécule signal étant la digoxigénine
c) une solution d'hybridation contenant 0,3 M de NaCl, 0,08M de Tris-HCl et 0,04% SDS et de pH 8,
d) une solution de lavage contenant 0,1 M de K₂PO₄, de 0,1 M de KH₂PO₄ et de 0,1 M de KCl et est de pH 7,6,
e) une solution de lyse étant une solution commerciale du kit Quick-RNA^{™} MiniPrep (Zymo Research^{®}, USA),
f) un support, ledit support étant une microplaque fonctionnalisée avec de la streptavidine ou de l'avidine
g) une solution contenant un anticorps anti-digoxigénine, ledit anticorps anti-digoxigénine étant lié à la péroxidase de raifort (HRP)
h) un substrat chromogène, ledit substrat chromogène étant le 3,3', 5, 5'-Tetramethylbenzidine (TMB),
i) un contrôle positif, ledit contrôle positif étant une molécule d'acide nucléique synthétique complémentaire de ladite sonde signal et de ladite sonde capture
j) un contrôle négatif, ledit contrôle négatif étant une molécule d'acide nucléique synthétique non complémentaire de ladite sonde signal et de ladite sonde capture lesdites sondes captures étant conservées lyophilisées sur ledit support, lesdites sondes signal étant conservées dans ladite solution d'hybridation.

Un autre aspect de ce cinquième ne faisant pas partie de l'invention décrit un kit pour la détection de cellules vivantes actives d'algues toxiques d'algues toxiques du genre ***Alexandrium,*** ledit kit contenant :
a) au moins un couple de sondes spécifiques d'algues toxiques du genre ***Alexandrium,*** les séquences desdites sondes étant choisies parmi x éléments de l'un des ensembles suivants :
   - (SEQ ID NO: 1, SEQ ID NO : 2 ou SEQ ID NO : 3)
   - (SEQ ID NO : 4 et SEQ ID NO : 5)
   - (SEQ ID NO : 6 et SEQ ID NO : 7)
   - (SEQ ID NO : 8, SEQ ID NO : 9 ou SEQ ID NO : 10)
   - (SEQ ID NO : 11, SEQ ID NO : 12 ou SEQ ID NO : 13)
   - (SEQ ID NO : 14, SEQ ID NO : 15 ou SEQ ID NO : 16)
   - (SEQ ID NO : 17, SEQ ID NO : 18 ou SEQ ID NO : 19)
   - (SEQ ID NO : 20, SEQ ID NO : 21 ou SEQ ID NO : 22)
   - (SEQ ID NO : 23, SEQ ID NO : 24 ou SEQ ID NO : 25)
   - (SEQ ID NO : 26, SEQ ID NO : 27 ou SEQ ID NO : 28)

   x étant 2 ou 3,
   ou les séquences desdites sondes ayant au moins 92% d'identité avec les susdites séquences SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO: 10, SEQ ID NO: 11, SEQ ID NO: 12, SEQ ID NO: 13, SEQ ID NO : 14, SEQ ID NO : 15, SEQ ID NO : 16, SEQ ID NO: 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 25, SEQ ID NO : 26, SEQ ID NO : 27, SEQ ID NO : 28,
   une sonde dudit couple étant une sonde capture liée à au moins une molécule de d'attache positionnée en 3' ou en 5' de sa séquence et l'autre sonde dudit couple étant une sonde signal liée à au moins une molécule signal positionnée en 3' ou en 5' de sa séquence,
   ladite sonde capture et ladite sonde signal étant capables de s'hybrider avec l'acide nucléique ribosomique d'une algue toxique du genre ***Alexandrium,***
   ladite molécule d'attache étant une molécule de biotine,
   ladite molécule signal étant la digoxigénine
b) au moins un couple de sondes spécifiques d'algues toxiques du genre ***Dinophysis,*** les séquences desdites sondes étant choisies parmi x éléments de l'un des ensembles suivants :
   - (SEQ ID NO : 29, SEQ ID NO : 30 ou SEQ ID NO : 31)
   - (SEQ ID NO : 32 et SEQ ID NO : 33)
   - (SEQ ID NO : 34 et SEQ ID NO : 35)
   - (SEQ ID NO : 36 et SEQ ID NO : 37)
   - (SEQ ID NO : 38 et SEQ ID NO : 39)
   - (SEQ ID NO : 40, SEQ ID NO : 41 ou SEQ ID NO : 42),
   - (SEQ ID NO : 43, SEQ ID NO : 44 ou SEQ ID NO : 45), ou
   - (SEQ ID NO : 46, SEQ ID NO : 47, SEQ ID NO : 48)

   x étant 2 ou 3,
   ou les séquences desdites sondes ayant au moins 92% d'identité avec les susdites séquences SEQ ID NO : 29, SEQ ID NO : 30, SEQ ID NO : 31, SEQ ID NO : 32, SEQ ID NO: 33, SEQ ID NO: 34, SEQ ID NO: 35, SEQ ID NO: 36, SEQ ID NO : 37, SEQ ID NO : 38, SEQ ID NO : 39, SEQ ID NO: 40, SEQ ID NO : 41, SEQ ID NO : 42, SEQ ID NO : 43, SEQ ID NO: 44, SEQ ID NO : 45, SEQ ID NO : 46, SEQ ID NO : 47 ou SEQ ID NO : 48,
   une sonde dudit couple étant une sonde capture liée à au moins une molécule de d'attache positionnée en 3' ou en 5' de sa séquence et l'autre sonde dudit couple étant une sonde signal liée à au moins une molécule signal positionnée en 3' ou en 5' de sa séquence,
   ladite sonde capture et ladite sonde signal étant capables de s'hybrider avec l'acide nucléique ribosomique d'une algue toxique du genre ***Dinophysis,***
   ladite molécule d'attache étant une molécule de biotine,
   ladite molécule signal étant la digoxigénine
c) au moins un couple de sondes spécifiques d'algues toxiques du genre ***Pseudo-nitzschia,*** les séquences desdites sondes étant choisies parmi x éléments de l'un des ensembles suivants :
   - (SEQ ID NO : 49 et SEQ ID NO : 50)
   - (SEQ ID NO : 51 et SEQ ID NO : 52)
   - (SEQ ID NO : 53, SEQ ID NO : 54 ou SEQ ID NO : 55)
   - (SEQ ID NO : 56, SEQ ID NO : 57, SEQ ID NO : 58)
   - (SEQ ID NO : 59, SEQ ID NO : 60, SEQ ID NO : 61)

   x étant 2 ou 3,
   ou les séquences desdites sondes ayant au moins 92% d'identité avec les susdites séquences SEQ ID NO : 49, SEQ ID NO : 50, SEQ ID NO : 51, SEQ ID NO : 52, SEQ ID NO: 53, SEQ ID NO: 54, SEQ ID NO: 55, SEQ ID NO: 56, SEQ ID NO : 57, SEQ ID NO : 58, SEQ ID NO : 59, SEQ ID NO: 60, SEQ ID NO : 61,
   une sonde dudit couple étant une sonde capture liée à au moins une molécule de d'attache positionnée en 3' ou en 5' de sa séquence et l'autre sonde dudit couple étant une sonde signal liée à au moins une molécule signal positionnée en 3' ou en 5' de sa séquence,
   ladite sonde capture et ladite sonde signal étant capables de s'hybrider avec l'acide nucléique ribosomique d'une algue toxique du genre ***Pseudo-nitzschia,***
   ladite molécule d'attache étant une molécule de biotine,
   ladite molécule signal étant la digoxigénine
d) une solution d'hybridation contenant 0,3 M de NaCl, 0,08 M de Tris-HCl et 0,04% SDS et de pH 8,
e) une solution de lavage contenant 0,1 M de K₂PO₄, de 0,1 M de KH₂PO₄ et de 0,1 M de KCl et est de pH 7,6.
f) une solution de lyse étant une solution commerciale du kit Quick-RNA^{™} MiniPrep (Zymo Research^{®}, USA),
g) un support, ledit support étant une microplaque fonctionnalisée avec de la streptavidine ou de l'avidine
h) une solution contenant un anticorps anti-digoxigénine, ledit anticorps anti-digoxigénine étant lié à la péroxidase de raifort (HRP)
i) un substrat chromogène, ledit substrat chromogène étant le 3,3', 5, 5'-Tetramethylbenzidine (TMB),
j) un contrôle positif, ledit contrôle positif étant une molécule d'acide nucléique synthétique complémentaire de ladite sonde signal et de ladite sonde capture
k) un contrôle négatif, ledit contrôle négatif étant une molécule d'acide nucléique synthétique non complémentaire de ladite sonde signal et de ladite sonde capture lesdites sondes captures étant conservées lyophilisées sur ledit support, lesdites sondes signal étant conservées dans ladite solution d'hybridation.

Un autre aspect de ce cinquième aspect décrit un kit pour la détection de cellules vivantes actives d'algues toxiques d'algues toxiques du genre ***Dinophysis,*** ledit kit contenant :
a) au moins un couple de sondes spécifiques d'algues toxiques du genre ***Dinophysis,*** les séquences desdites sondes étant choisies parmi x éléments de l'un des ensembles suivants :
   - (SEQ ID NO : 29, SEQ ID NO : 30 ou SEQ ID NO : 31)
   - (SEQ ID NO : 32 et SEQ ID NO : 33)
   - (SEQ ID NO : 34 et SEQ ID NO : 35)
   - (SEQ ID NO : 36 et SEQ ID NO : 37)
   - (SEQ ID NO : 38 et SEQ ID NO : 39)
   - (SEQ ID NO : 40, SEQ ID NO : 41 ou SEQ ID NO : 42)
   - (SEQ ID NO : 43, SEQ ID NO : 44 ou SEQ ID NO : 45), ou
   - (SEQ ID NO : 46, SEQ ID NO : 47 ou SEQ ID NO : 48)

   x étant 2 ou 3,
   ou les séquences desdites sondes ayant au moins 92% d'identité avec les susdites séquences SEQ ID NO : 29, SEQ ID NO : 30, SEQ ID NO : 31, SEQ ID NO : 32, SEQ ID NO: 33, SEQ ID NO: 34, SEQ ID NO: 35, SEQ ID NO: 36, SEQ ID NO : 37, SEQ ID NO : 38, SEQ ID NO : 39, SEQ ID NO: 40, SEQ ID NO : 41, SEQ ID NO : 42, SEQ ID NO : 43, SEQ ID NO: 44, SEQ ID NO : 45, SEQ ID NO : 46, SEQ ID NO : 47 ou SEQ ID NO : 48,
   une sonde dudit couple étant une sonde capture liée à au moins une molécule de d'attache positionnée en 3' ou en 5' de sa séquence et l'autre sonde dudit couple étant une sonde signal liée à au moins une molécule signal positionnée en 3' ou en 5' de sa séquence,
   ladite sonde capture et ladite sonde signal étant capables de s'hybrider avec l'acide nucléique ribosomique d'une algue toxique du genre ***Dinophysis,***
   ladite molécule d'attache étant une molécule de biotine,
   ladite molécule signal étant la digoxigénine
b) une solution d'hybridation contenant 0,3 M de NaCl, 0,08 M de Tris-HCl et 0,04% SDS et de pH 8,
c) une solution de lavage contenant 0,1 M de K₂PO₄, de 0,1 M de KH₂PO₄ et de 0,1 M de KCl et est de pH 7,6.
d) une solution de lyse étant une solution commerciale du kit Quick-RNA^{™} MiniPrep (Zymo Research^{®}, USA),
e) un support, ledit support étant une microplaque fonctionnalisée avec de la streptavidine ou de l'avidine
f) une solution contenant un anticorps anti-digoxigénine, ledit anticorps anti-digoxigénine étant lié à la péroxidase de raifort (HRP)
g) un substrat chromogène, ledit substrat chromogène étant le 3,3', 5, 5'-Tetramethylbenzidine (TMB),
h) un contrôle positif, ledit contrôle positif étant une molécule d'acide nucléique synthétique complémentaire de ladite sonde signal et de ladite sonde capture
i) un contrôle négatif, ledit contrôle négatif étant une molécule d'acide nucléique synthétique non complémentaire de ladite sonde signal et de ladite sonde capture lesdites sondes captures étant conservées lyophilisées sur ledit support, lesdites sondes signal étant conservées dans ladite solution d'hybridation.

Un autre aspect de ce cinquième aspect décrit un kit pour la détection de cellules vivantes actives d'algues toxiques d'algues toxiques du genre ***Pseudo-nitzschia,*** ledit kit contenant :
a) au moins un couple de sondes spécifiques d'algues toxiques du genre ***Pseudo-nitzschia,*** les séquences desdites sondes étant choisies parmi x éléments de l'un des ensembles suivants :
   - (SEQ ID NO : 49 et SEQ ID NO : 50)
   - (SEQ ID NO : 51 et SEQ ID NO : 52)
   - (SEQ ID NO : 53, SEQ ID NO : 54 ou SEQ ID NO : 55)
   - (SEQ ID NO : 56, SEQ ID NO : 57, SEQ ID NO : 58)
   - (SEQ ID NO : 59, SEQ ID NO : 60, SEQ ID NO : 61)

   x étant 2 ou 3,
   ou les séquences desdites sondes ayant au moins 92% d'identité avec les susdites séquences SEQ ID NO : 49, SEQ ID NO : 50, SEQ ID NO : 51, SEQ ID NO : 52, SEQ ID NO: 53, SEQ ID NO: 54, SEQ ID NO: 55, SEQ ID NO: 56, SEQ ID NO : 57, SEQ ID NO : 58, SEQ ID NO : 59, SEQ ID NO: 60, SEQ ID NO : 61,
   une sonde dudit couple étant une sonde capture liée à au moins une molécule de d'attache positionnée en 3' ou en 5' de sa séquence et l'autre sonde dudit couple étant une sonde signal liée à au moins une molécule signal positionnée en 3' ou en 5' de sa séquence,
   ladite sonde capture et ladite sonde signal étant capables de s'hybrider avec l'acide nucléique ribosomique d'une algue toxique du genre ***Pseudo-nitzschia,***
   ladite molécule d'attache étant une molécule de biotine,
   ladite molécule signal étant la digoxigénine
b) une solution d'hybridation contenant 0,3 M de NaCl, 0,08 M de Tris-HCl et 0,04% SDS et de pH 8,
c) une solution de lavage contenant 0,1 M de K₂PO₄, de 0,1 M de KH₂PO₄ et de 0,1 M de KCl et est de pH 7,6.
d) une solution de lyse étant une solution commerciale du kit Quick-RNA^{™} MiniPrep (Zymo Research^{®}, USA),
e) un support, ledit support étant une microplaque fonctionnalisée avec de la streptavidine ou de l'avidine
f) une solution contenant un anticorps anti-digoxigénine, ledit anticorps anti-digoxigénine étant lié à la péroxidase de raifort (HRP)
g) un substrat chromogène, ledit substrat chromogène étant le 3,3', 5, 5'-Tetramethylbenzidine (TMB),
h) un contrôle positif, ledit contrôle positif étant une molécule d'acide nucléique synthétique complémentaire de ladite sonde signal et de ladite sonde capture
i) un contrôle négatif, ledit contrôle négatif étant une molécule d'acide nucléique synthétique non complémentaire de ladite sonde signal et de ladite sonde capture
   lesdites sondes captures étant conservées lyophilisées sur ledit support,
   lesdites sondes signal étant conservées dans ladite solution d'hybridation.

Un sixième aspect concerne des dispositifs pour la détection de cellules vivantes actives d'algues toxiques.

### Mode de réalisation A

Ainsi, un sixième aspect ne faisant pas partie de l'invention concerne un dispositif constitué d'un support comprenant des sondes spécifiques d'algues toxiques du genre *Alexandrium* pour la mise en œuvre d'un procédé de détection de cellules vivantes actives d'algues toxiques dans un échantillon susceptible de contenir au moins une algue toxique du genre *Alexandrium,* lesdites sondes ayant une séquence choisie parmi les séquences :
SEQ ID NO : 1, SEQ ID NO : 2, SEQ ID NO : 3, SEQ ID NO : 4, SEQ ID NO : 7, SEQ ID NO: 8, SEQ ID NO: 10, SEQ ID NO: 11, SEQ ID NO: 13, SEQ ID NO : 14, SEQ ID NO : 16, SEQ ID NO : 17, SEQ ID NO: 19, SEQ ID NO : 20, SEQ ID NO : 22, SEQ ID NO : 23, SEQ ID NO: 25, SEQ ID NO : 26, SEQ ID NO : 28 ou les séquences ayant au moins 92% d'identité avec les susdites séquences SEQ ID NO : 1, SEQ ID NO: 2, SEQ ID NO: 3, SEQ ID NO : 4, SEQ ID NO : 7, SEQ ID NO : 8, SEQ ID NO : 10, SEQ ID NO : 11, SEQ ID NO : 13, SEQ ID NO : 14, SEQ ID NO : 16, SEQ ID NO: 17, SEQ ID NO : 19, SEQ ID NO : 20, SEQ ID NO : 22, SEQ ID NO: 23, SEQ ID NO : 25, SEQ ID NO : 26, SEQ ID NO : 28,
chaque sonde étant liée ou pouvant être liée à au moins une molécule d'attache positionnée en 3' ou en 5' de sa séquence,
lesdites sondes étant capables de s'hybrider avec l'acide nucléique ribosomique d'une algue toxique du genre *Alexandrium* éventuellement présent dans ledit échantillon afin de former un complexe.

### Mode de réalisation B

Un autre mode de réalisation ne faisant pas partie de l'invention concerne un dispositif tel que décrit précédemment selon le mode de réalisation A, comprenant **en plus** des sondes spécifiques d'algues toxiques du genre *Dinophysis* pour la mise en œuvre d'un procédé de détection de cellules vivantes actives d'algues toxiques dans un échantillon susceptible de contenir au moins une algue toxique du genre ***Alexandrium*** et/ou ***Dinophysis,*** lesdites sondes ayant une séquence choisie parmi les séquences :
SEQ ID NO : 30, SEQ ID NO : 31, SEQ ID NO : 33, SEQ ID NO: 35, SEQ ID NO : 37, SEQ ID NO : 38, SEQ ID NO : 39, SEQ ID NO: 41, SEQ ID NO : 42, SEQ ID NO : 44, SEQ ID NO : 45, SEQ ID NO: 47, SEQ ID NO : 48 ou la séquence de ladite sonde ayant au moins 92% d'identité avec les susdites séquences SEQ ID NO: 30, SEQ ID NO: 31, SEQ ID NO: 33, SEQ ID NO : 35, SEQ ID NO : 37, SEQ ID NO : 38, SEQ ID NO: 39, SEQ ID NO : 41, SEQ ID NO : 42, SEQ ID NO : 44, SEQ ID NO: 45, SEQ ID NO : 47, SEQ ID NO : 48
chaque sonde étant liée ou pouvant être liée à au moins une molécule d'attache positionnée en 3' ou en 5' de sa séquence,
lesdites sondes étant capables de s'hybrider avec l'acide nucléique ribosomique d'une algue toxique du genre ***Dinophysis*** éventuellement présent dans ledit échantillon afin de former un complexe.

### Mode de réalisation C

De la même façon, est décrit un des dispositifs tels que décrits précédemment selon le mode de réalisation A ou le mode de réalisation B, comprenant **en plus** des sondes spécifiques d'algues toxiques du genre ***Pseudo-nitzschia*** pour la mise en œuvre d'un procédé de détection de cellules vivantes actives d'algues toxiques dans un échantillon susceptible de contenir au moins une algue toxique du genre ***Alexandrium*** et/ou ***Pseudo-nitzschia,*** lesdites sondes ayant une séquence choisie parmi les séquences :
SEQ ID NO : 49, SEQ ID NO : 51, SEQ ID NO : 52, SEQ ID NO: 54, SEQ ID NO : 55, SEQ ID NO : 57, SEQ ID NO : 58, SEQ ID NO : 60, SEQ ID NO : 61 ou la séquence de ladite sonde ayant au moins 92% d'identité avec les susdites séquences SEQ ID NO: 49, SEQ ID NO: 51, SEQ ID NO: 52, SEQ ID NO : 54, SEQ ID NO : 55, SEQ ID NO : 57, SEQ ID NO: 58, SEQ ID NO : 60, SEQ ID NO : 61
chaque sonde étant liée ou pouvant être liée à au moins une molécule d'attache positionnée en 3' ou en 5' de sa séquence,
lesdites sondes étant capables de s'hybrider avec l'acide nucléique ribosomique d'une algue toxique du genre ***Pseudo-nitzschia*** éventuellement présent dans ledit échantillon afin de former un complexe.

### Mode de réalisation D

De la même façon, est décrit un des dispositifs tels que décrits précédemment selon le mode de réalisation A, B ou C, comprenant **en plus** des sondes spécifiques d'algues toxiques du genre ***Prorocentrum*** pour la mise en œuvre d'un procédé de détection de cellules vivantes actives d'algues toxiques dans un échantillon susceptible de contenir au moins une algue toxique du genre ***Alexandrium*** et/ou ***Prorocentrum,*** lesdites sondes ayant une séquence choisie parmi les séquences :
SEQ ID NO : 63, SEQ ID NO : 64, SEQ ID NO : 66, SEQ ID NO: 67, SEQ ID NO : 69, SEQ ID NO : 70, SEQ ID NO : 72, SEQ ID NO : 73 ou la séquence de ladite sonde ayant au moins 92% d'identité avec les susdites séquences SEQ ID NO : 63, SEQ ID NO : 64, SEQ ID NO : 66, SEQ ID NO: 67, SEQ ID NO : 69, SEQ ID NO : 70, SEQ ID NO : 72, SEQ ID NO : 73
chaque sonde étant liée ou pouvant être liée à au moins une molécule d'attache positionnée en 3' ou en 5' de sa séquence,
lesdites sondes étant capables de s'hybrider avec l'acide nucléique ribosomique d'une algue toxique du genre ***Prorocentrum*** éventuellement présent dans ledit échantillon afin de former un complexe.

### Mode de réalisation E

De la même façon, est décrit un des dispositifs tels que décrits précédemment selon le mode de réalisation A, B, C ou D, comprenant **en plus** des sondes spécifiques d'algues toxiques du genre ***Chattonella*** pour la mise en œuvre d'un procédé de détection de cellules vivantes actives d'algues toxiques dans un échantillon susceptible de contenir au moins une algue toxique du genre ***Alexandrium*** et/ou ***Chattonella,*** lesdites sondes ayant une séquence choisie parmi les séquences :
SEQ ID NO : 75, SEQ ID NO : 76, SEQ ID NO : 77, SEQ ID NO : 79, SEQ ID NO : 80, SEQ ID NO : 82 ou la séquence de ladite sonde ayant au moins 92% d'identité avec les susdites séquences SEQ ID NO: 75, SEQ ID NO: 76, SEQ ID NO : 77, SEQ ID NO : 79, SEQ ID NO : 80, SEQ ID NO : 82
chaque sonde étant liée ou pouvant être liée à au moins une molécule d'attache positionnée en 3' ou en 5' de sa séquence,
lesdites sondes étant capables de s'hybrider avec l'acide nucléique ribosomique d'une algue toxique du genre ***Chattonella*** éventuellement présent dans ledit échantillon afin de former un complexe

### Mode de réalisation F

De la même façon, est décrit un des dispositifs tels que décrits précédemment selon le mode de réalisation A, B, C, D ou E, comprenant **en plus** des sondes spécifiques d'algues toxiques du genre ***Gymnodinium*** pour la mise en œuvre d'un procédé de détection de cellules vivantes actives d'algues toxiques dans un échantillon susceptible de contenir au moins une algue toxique du genre ***Alexandrium*** et/ou ***Gymnodinium,*** lesdites sondes ayant une séquence choisie parmi les séquences :
SEQ ID NO : 84, SEQ ID NO : 85, SEQ ID NO : 87, SEQ ID NO: 88, SEQ ID NO : 90, SEQ ID NO : 91, SEQ ID NO : 93, SEQ ID NO : 94 ou la séquence de ladite sonde ayant au moins 92% d'identité avec les susdites séquences SEQ ID NO : 84, SEQ ID NO : 85, SEQ ID NO : 87, SEQ ID NO : 88, SEQ ID NO : 90, SEQ ID NO : 91, SEQ ID NO : 93, SEQ ID NO : 94
chaque sonde étant liée ou pouvant être liée à au moins une molécule d'attache positionnée en 3' ou en 5' de sa séquence,
lesdites sondes étant capables de s'hybrider avec l'acide nucléique ribosomique d'une algue toxique du genre ***Gymnodinium*** éventuellement présent dans ledit échantillon afin de former un complexe.

### Mode de réalisation G

De la même façon, est décrit un des dispositifs tels que décrits précédemment selon le mode de réalisation A, B, C, D, E ou F, comprenant **en plus** des sondes spécifiques d'algues toxiques du genre ***Karenia*** pour la mise en œuvre d'un procédé de détection de cellules vivantes actives d'algues toxiques dans un échantillon susceptible de contenir au moins une algue toxique du genre ***Alexandrium*** et/ou ***Karenia,*** lesdites sondes ayant une séquence choisie parmi les séquences :
SEQ ID NO : 96, SEQ ID NO : 97, SEQ ID NO : 99, SEQ ID NO : 100, SEQ ID NO : 102, SEQ ID NO : 103, SEQ ID NO : 105, SEQ ID NO : 106, SEQ ID NO : 108, SEQ ID NO : 109, SEQ ID NO : 111, SEQ ID NO : 112, SEQ ID NO : 114, SEQ ID NO : 115, SEQ ID NO : 117, SEQ ID NO : 118 ou la séquence de ladite sonde ayant au moins 92% d'identité avec les susdites séquences SEQ ID NO : 96, SEQ ID NO : 97, SEQ ID NO : 99, SEQ ID NO : 100, SEQ ID NO : 102, SEQ ID NO : 103, SEQ ID NO : 105, SEQ ID NO : 106, SEQ ID NO : 108, SEQ ID NO : 109, SEQ ID NO : 111, SEQ ID NO : 112, SEQ ID NO : 114, SEQ ID NO : 115, SEQ ID NO : 117, SEQ ID NO : 118
chaque sonde étant liée ou pouvant être liée à au moins une molécule d'attache positionnée en 3' ou en 5' de sa séquence,
lesdites sondes étant capables de s'hybrider avec l'acide nucléique ribosomique d'une algue toxique du genre ***Karenia*** éventuellement présent dans ledit échantillon afin de former un complexe.

### Mode de réalisation H

De la même façon, est décrit un des dispositifs tels que décrits précédemment selon le mode de réalisation A, B, C, D, E, F ou G, comprenant **en plus** des sondes spécifiques d'algues toxiques du genre ***Lingulodinium*** pour la mise en œuvre d'un procédé de détection de cellules vivantes actives d'algues toxiques dans un échantillon susceptible de contenir au moins une algue toxique du genre ***Alexandrium*** et/ou ***Lingulodinium,*** lesdites sondes ayant une séquence choisie parmi les séquences :
SEQ ID NO : 120, SEQ ID NO : 121, SEQ ID NO : 123, SEQ ID NO : 124, SEQ ID NO : 126, SEQ ID NO : 127 ou la séquence de ladite sonde ayant au moins 92% d'identité avec les susdites séquences SEQ ID NO : 120, SEQ ID NO : 121, SEQ ID NO : 123, SEQ ID NO : 124, SEQ ID NO : 126, SEQ ID NO : 127
chaque sonde étant liée ou pouvant être liée à au moins une molécule d'attache positionnée en 3' ou en 5' de sa séquence,
lesdites sondes étant capables de s'hybrider avec l'acide nucléique ribosomique d'une algue toxique du genre *Lingulodinium* éventuellement présent dans ledit échantillon afin de former un complexe.

### Mode de réalisation I

De la même façon, est décrit un des dispositifs tels que décrits précédemment selon le mode de réalisation A, B, C, D, E, F, G ou H, comprenant **en plus** des sondes spécifiques d'algues toxiques du genre ***Heterosigma*** pour la mise en œuvre d'un procédé de détection de cellules vivantes actives d'algues toxiques dans un échantillon susceptible de contenir au moins une algue toxique du genre ***Alexandrium*** et/ou ***Heterosigma,*** lesdites sondes ayant une séquence choisie parmi les séquences :
les séquences SEQ ID NO : 128 ou la séquence de ladite sonde ayant au moins 92%
d'identité avec les sus desdites séquences SEQ ID NO : 128

chaque sonde étant liée ou pouvant être liée à au moins une molécule d'attache positionnée en 3' ou en 5' de sa séquence,
lesdites sondes étant capables de s'hybrider avec l'acide nucléique ribosomique d'une algue toxique du genre ***Heterosigma*** éventuellement présent dans ledit échantillon afin de former un complexe.

Comme pour le premier aspect relatif à l'utilisation, toutes les combinaisons des modes de réalisation A, B, C, D, E, F, G, H et/ou 1 de ce sixième aspect peuvent être envisagées.

Dans l'ensemble des modes de réalisation de ce sixième aspect et selon un mode de réalisation particulier, ladite molécule d'attache est située en 5' de la séquence de ladite sonde.

Dans l'ensemble des modes de réalisation de ce sixième aspect et selon un autre mode de réalisation particulier, ladite molécule d'attache est située en 3' de la séquence de ladite sonde.

Dans l'ensemble des modes de réalisation de ce sixième aspect, ladite molécule d'attache peut être choisie parmi une molécule de biotine, avidine, streptavidine, un groupe thiol, un groupe amine et un carbone.

Dans l'ensemble des modes de réalisation de ce sixième aspect et selon un mode de réalisation particulièrement préféré, ladite molécule d'attache est une molécule de biotine.

Dans l'ensemble des modes de réalisation de ce sixième aspect et selon un mode de réalisation particulier, le dispositif peut être choisi parmi le groupe consistant en : une microplaque, une lame de verre, des billes magnétiques, des électrodes imprimées en différente matières comme le carbone ou l'or.

Dans l'ensemble des modes de réalisation de ce sixième aspect et selon un mode de réalisation particulier, ledit dispositif peut être :
- fonctionnalisé avec de la streptavidine ou de l'avidine et ladite molécule d'attache est une biotine
- fonctionnalisé avec un groupement aldéhyde, un groupement epoxy, un groupement carboxyl ou un groupement isothiocyanate et ladite molécule d'attache est un groupe amine ou carbone
- fonctionnalisé avec de l'or, du mercaptosilane ou un groupement maléimide et ladite molécule d'attache est un groupe thiol.

Dans l'ensemble des modes de réalisation de ce sixième aspect, l'échantillon peut être un échantillon d'eau de mer, d'eau saumâtre, de milieux de cultures ou de culture de microalgues produites à des fins commerciales.

Les figures et les exemples suivants illustreront mieux l'invention, sans pour autant en limiter sa portée, qui est définie par les revendications.

### FIGURES

**Figure 1** : Changements de la densité de cellules (cellules/mL) d'une culture *d'Alexandrium minutum* au cours du temps réalisée dans les conditions optimales de croissance.
**Figure 2** **:** Concentration moyenne du contenu en ARN totaux par cellule exprimé en ng d'ARN total par cellule obtenu à partir de conditions optimales de culture, c'est à dire correspondant à la phase exponentielle de croissance.
**Figure 3** **:** Test de spécificité des sondes ciblant *Lingulodinium polyedrum.* Hybridation de 200 ng d'ARN extrait à partir d'une culture non axénique de *Lingulodinium polyedrum* avec les couples de sondes suivants : SEQ ID NO : 119 et SEQ ID NO : 120 (sonde n.199/120), SEQ ID NO: 122 et SEQ ID NO: 123 (sonde n.122/123) ou SEQ ID NO: 125 et SEQ ID NO : 126 (sonde n. 125/126), de 0,1 µM de contrôle positif (PC, ADN synthétique complémentaire) ou de 0,1 µM de contrôle négatif (NC, ADN synthétique non complementaire).

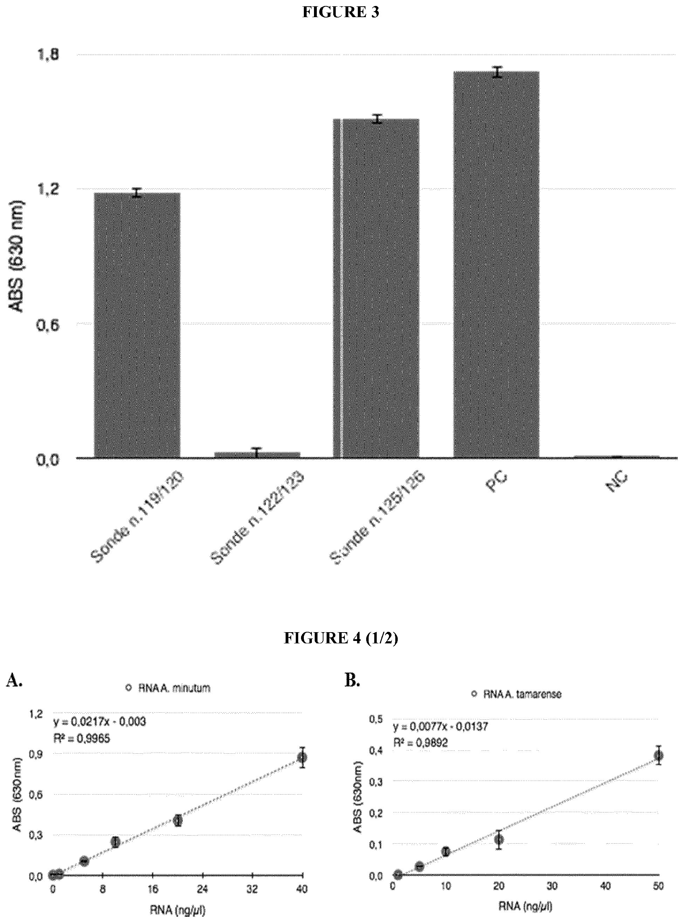

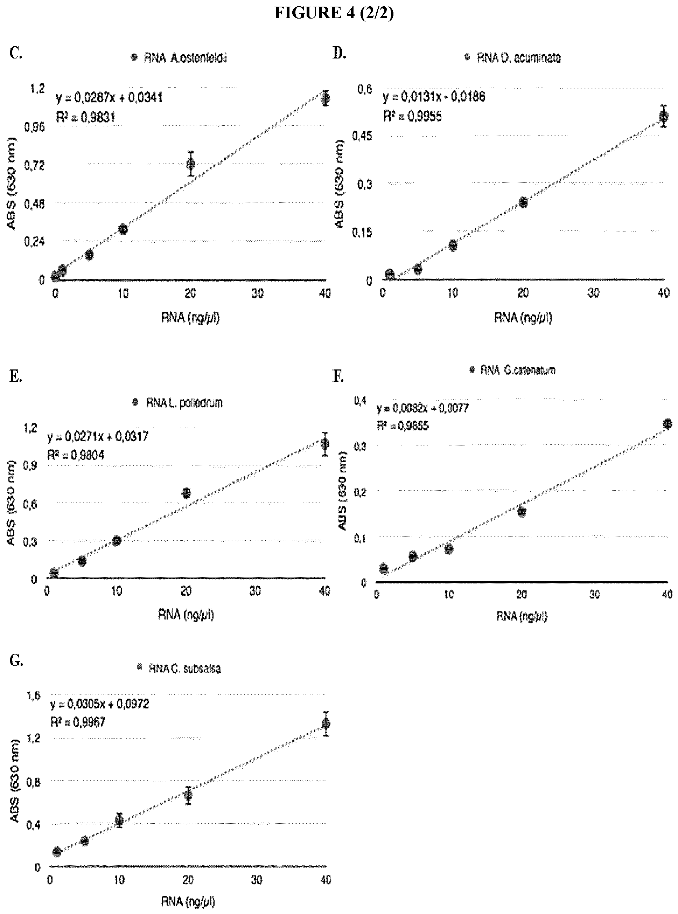

**Figure 4** : Correspondance entre la concentration en ARN (ng/µL) et l'absorbance à 630 nm : *Alexandrium minutum* et sondes de séquences SEQ ID NO : 7 et SEQ ID NO : 8 (A.) ; *Alexandrium tamarense* et sondes de séquences SEQ ID NO : 17 et SEQ ID NO : 18 (B.) ; *Alexandrium ostenfeldii* et sondes de séquences SEQ ID NO : 11 et SEQ ID NO : 12 (C.) ; *Dinophysis acuminata* et sondes de séquences SEQ ID NO : 40 et SEQ ID NO : 41 (D.) ; *Lingulodinium polyedrum* et sondes de séquences SEQ ID NO : 119 et SEQ ID NO : 120 (E.) ; *Gymnodinium catenatum* et sondes de séquences SEQ ID NO : 83 et SEQ ID NO : 84 (F.) ; *Chattonella subsalsa* et sondes de séquences SEQ ID NO : 74 et SEQ ID NO : 75 (G.).
**Figure 5** : Correspondance entre la concentration d'ARN et absorbance à 450 nm (protocole optimisé) *: Alexandrium minutum* et sondes de séquences SEQ ID NO : 7 et SEQ ID NO : 8 (A) *; Alexandrium ostenfeldii* et sondes de séquences SEQ ID NO : 11 et SEQ ID NO : 12 (B) ; *Pseudo-nitzschia* et sondes de séquences SEQ ID NO : 46 et SEQ ID NO : 47 (C, D et E).
**Figure 6** : Correspondance entre la concentration cellulaire estimée (cellules/L) et l'absorbance à 630 nm : *Alexandrium minutum* en phase stationnaire (AM - STAT) *et Alexandrium minutum* en phase exponentielle (AM - EXP).
**Figure** 7 : Correspondance entre la concentration cellulaire estimée (cellules/L) et l'absorbance à 450 nm : *Dinophysis sp* dans un échantillon environnemental contenant naturellement des *Dinophysis.*
**Figure 8** : Comparaison des absorbances lues à 450 nm et obtenues avec différents volumes de solution de révélation (TMB) réalisés avec différents tampons.
**Figure 9** : Comparaison des unités d'absorbance à 630 nm et 450 nm de tests réalisés avec des ARN obtenus à partir de nombres connus de cellules.
**Figure 10** : Comparaison des absorbances lues à 450 nm et obtenues par des tests d'hybridation réalisés avec différents tampons (protocole optimisé).
**Figure 11****.** Comparaison de détection de *Pseudo-nitzschia* par comptages cellulaires en microscopie et des signaux du test d'hybridation sandwich lus à 630 nm dans des échantillons environnementaux récoltés lors d'une prolifération naturelle. Les résultats sont rapportés à 1L d'échantillon d'eau de mer.
**Figure 12****:** Comparaison de détection *d'Alexandrium* par comptages cellulaires en microscopie et des signaux du test d'hybridation sandwich lus à 630 nm dans des échantillons environnementaux récoltés lors d'une prolifération naturelle. Les résultats sont rapportés à 1L d'échantillon d'eau de mer.

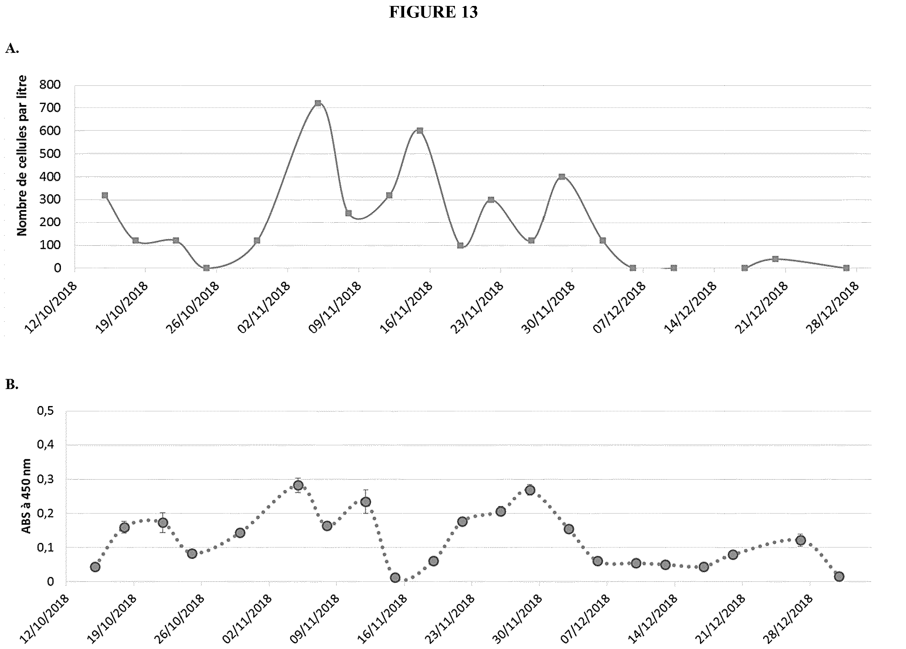

**Figure 13** : Comparaison de détection de *Dinophysis* par des signaux d'absorbance du test d'hybridation sandwich lus à 450 nm a) (protocole optimisé) et par comptages cellulaires en microscopie b). Les résultats sont rapportés à 1L d'eau de mer.

### EXEMPLES

Le développement de l'invention a utilisé différentes sortes d'échantillons et a requis différentes démarches expérimentales, lesquelles sont définies ci-après en incluant leur champ d'application. Les principaux résultats obtenus sont résumés dans le tableau 1.

### LES DIFFERENTS TYPES D'ECHANTILLONS

### Les échantillons issus de culture cellulaire

Les échantillons ont été obtenus à partir de cultures issues de collections internationales. Ces cultures ont été entretenues dans des conditions optimales de développement comme indiqué dans la partie EXEMPLE 1 : Validation des sondes, *a) Les conditions de culture.* Il s'agit de populations de micro-algues suffisamment homogènes pour présenter des phases de croissance synchrone et une condition physiologique optimale, i.e., un matériel biologique en condition et quantité maximales.

La sensibilité des essais a été optimale car le matériel obtenu est relativement pur puisqu'il contient une quantité très majoritaire des cellules cibles. Ces échantillons ont été utilisés dans une première phase de mise au point d'un procédé, plus particulièrement pour encadrer les limites d'action des expériences et déterminer les fenêtres d'étude les plus intéressantes.

### Les échantillons artificiels

Les échantillons artificiels ont été élaborés à partir d'eau environnementale qui ne présente pas au préalable les micro-algues d'intérêt, et ont été supplémentés par des cellules d'une culture homogène. Les cellules de la culture présentent un état physiologique choisi, par exemple, un état de phase exponentielle de croissance ou état de phase stationnaire, et sont majoritairement synchronisées, *i.e.,* elles se divisent ensemble au même moment. L'assemblage de l'eau environnementale avec un nombre déterminé de cellules de culture constitue un échantillon artificiel qui permet de prendre en considération l'impact des composants fréquemment trouvés dans l'eau environnementale.

La composition de l'eau environnementale dépend de facteurs biotiques et abiotiques (organismes présents, matières organiques diverses, pollution chimique ou biologique, *etc*.). La concentration de cette eau sur des filtres de faibles porosité, par exemple de 10 µM, va accumuler la matière de quelque origine qu'elle soit. L'extraction du matériel génétique et les co-purifications associées à cette extraction vont composer ce qu'on appelle la « matrice environnementale ». Cette matrice a un impact sur la sensibilité de détection de toute méthode moléculaire et entraine une diminution du signal recherché. Il a donc été primordial de réaliser une batterie d'expériences avec ces échantillons pour intégrer cette dimension non maitrisable qui est complètement absente des échantillons de culture cellulaire.

L'assemblage d'un échantillon artificiel, *i.e.* volume d'eau additionné d'un volume de culture cellulaire, ne peut pas être assimilé à l'assemblage *a posteriori* des matériels biologiques extraits indépendamment, *i.e.* matériel extrait du volume d'eau et matériel extrait de la culture cellulaire.

### Les échantillons naturels

Les échantillons naturels sont des échantillons issus d'environnements présentant des proliférations naturelles des micro-algues ciblées. L'hétérogénéité apparaît au niveau de l'échantillon lui-même et au niveau des micro-algues ciblées.

Dans l'environnement, les populations de micro-algues sont hétérogènes en termes de diversité et d'état physiologique. Une population d'un genre de micro-algues peut regrouper plusieurs espèces différentes et peut contenir tous les stades de vie d'une cellule. Par exemple, un grand nombre de cellules peut correspondre à une population en prolifération, donc active, ou à une population sénescente, donc peu active. Un faible nombre de cellules peut être très actif ou en dormance.

Les échantillons naturels *stricto sensus* sont hétérogènes en termes de richesse biologique, chimique et géographique.

La variabilité en contenu d'organismes et de matières organique et inorganique est très importante et aléatoire d'un échantillon à l'autre. Cette variabilité va directement impacter sur le degré de dilution de la micro-algue toxique ciblée : plus la densité en organismes vivants et matière est forte, plus la cible est diluée.

La localisation de l'échantillon dans la colonne d'eau va également influencer la répartition de la micro-algue ciblée. Certaines micro-algues se trouvent préférentiellement dans la zone euphotique ou eau de surface, d'autres préfèrent la profondeur et d'autres encore se déplacent activement dans toute la colonne d'eau. Elles sont toutes soumises à une horloge circadienne qui va déterminer leur localisation dans la colonne d'eau au cours d'une journée.

Toutes ces particularités rendent l'échantillon environnemental très hétérogène et très représentatif de la réalité.

### LES DIFFERENTS TYPES D'EXPERIENCES

### Courbes de calibration à partir d'ARN de culture

Le premier type d'expérience a été la réalisation de courbes de calibration à l'aide d'ARN totaux de cultures clonales mais non axéniques de micro-algues toxiques.

Ces expériences ont eu pour objectif de définir la fenêtre de détection et quantification des ARN ciblés à l'aide de micro-algues en phase de croissance active. Les valeurs obtenues ont servi à établir la sensibilité, la reproductibilité et la robustesse du test colorimétrique et font références pour les analyses d'échantillons environnementaux.

### Courbes de calibration à partir d'échantillons environnementaux artificiels

Le deuxième type d'expérience a été la réalisation de courbes de calibration à l'aide d'échantillons naturels artificiellement contaminés par des cellules issues de cultures clonales mais non axéniques de micro-algues toxiques.

Ces expériences ont permis d'intégrer la variabilité liée à la dimension environnementale et de calibrer l'impact de matrice d'origines différentes et variées sur le test colorimétrique. En conséquence, les limites obtenues sont par définition inférieures à celles obtenues avec des échantillons de culture.

### Suivi d'échantillons environnementaux naturels

Le troisième type d'expérience a été l'application dans un milieu naturel à partir d'échantillons naturels naturellement contaminés par des micro-algues toxiques.

Ces expériences ont permis de valider les développements et améliorations du test colorimétrique mais aussi de démontrer son applicabilité sur le terrain en termes de robustesse, reproductibilité, fiabilité et sensibilité. Il s'agit d'une application terrain en vue d'une industrialisation.

### LES PRINCIPAUX RESULTATS

**Tableau 1 : Comparaison des seuils de sensibilité entre le procédé de l'invention avant et après sont optimisation selon le type d'échantillons et d'expériences menés.**

| | Expériences menées avec des cultures | | Expériences menées avec des échantillons naturels supplémentés en cellules de culture | Suivis terrain manées avec des échantillons naturellement contaminés |
|---|---|---|---|---|
| **LOD** | **ng total** | **Nombre totale de cellules** | **Cellules/Litre** | **Cellules/Litres** |
| Procédé de l'invention avant optimisation | 1 | 50 | 100 - 500 | 160 (*Alexandrium*) et *500 (Pseudo-nitzschia)* |
| Procédé de l'invention après optimisation | 0,1 | 5 | < 200 voire < 5 | < *2 (Dynophysis)* |

### EXEMPLE 1 : Validation des sondes

### a) Les conditions de culture

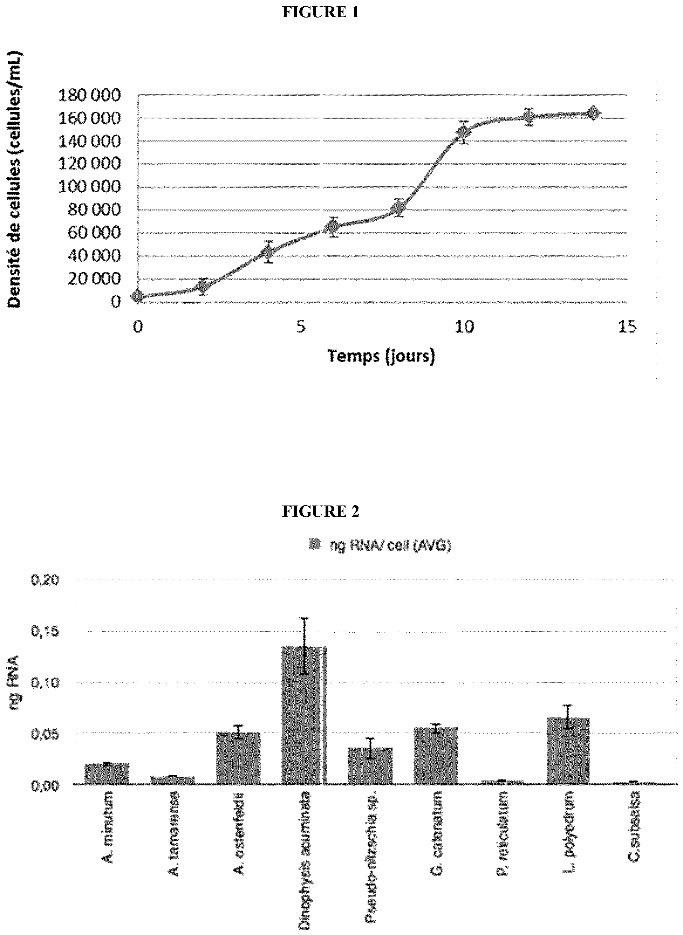

Les cultures d'algues utilisées pour tester les sondes décrites sont listées dans le tableau 2. Toutes les cultures sont actuellement maintenues à Microbia Environnement sur le site d'incubation d'entreprises de l'Observatoire Océanologique de Banyuls-sur-mer, France. Les cultures sont entretenues dans des milieux en eau de mer de type F/2 proposé par Guillard et Ryther 1962 et de type L1 proposé par Guillard et Hargraves (1993) (à différentes températures et sous une luminosité d'intensité de 100 µE.m⁻².s⁻¹ avec un cycle jour : nuit de 12 :12. Les milieux F/2 et L1 sont des milieux à base d'eau de mer couramment utilisés pour cultiver des algues marines et contiennent des éléments trace, des vitamines, et parfois de la silice. Les cellules sont comptées tous les 2 jours par cytométrie de flux à l'aide du FACSCanto^{®} II flow cytometer (BD Biosciences^{®}, USA). Les comptages mettent en évidence une phase exponentielle de croissance (entre le jour 2 et le jour 10) et une phase stationnaire à partir du jour 10 **(****Figure 1****).**

**Tableau 2 : Espèces pour tester les sondes décrites incluant la classe, le milieu de croissance et le numéro de souche.**

| Micro-algue | Classe | Milieu de Culture | Numéro d'identification de la souche |
|---|---|---|---|
| *Alexandrium minutum* | Dinophyceae | F/2 | AM 205 |
| *Alexandrium tamarense* | Dinophyceae | F/2 | VGO928 |
| *Alexandrium ostenfeldii* | Dinophyceae | F/2 | VGO956 |
| *Dinophysis acuminata* | Dinophyceae | F/2 | VGO1063 |
| *Dinophysis acuta* | Dinophyceae | F/2 | VGO1065 |
| *Pseudo-nitzschia artica* | Bacillariophyceae | L1 | |
| *Gymnodinium catenatum* | Dinophyceae | L1 | GC12V |
| *Lingulodinium polyedrum* | Dinophyceae | L1 | GG1AM |
| *Chattonella subsalsa* | Raphidophyceae | L1 | CS0704 |

### b) Préparation des ARN

Un nombre connu de cellules de culture est, soit filtré sur une membrane de polycarbonate d'une porosité de 3 µm (Whatman^{®} Nuclepore Track-Etched Membranes) à l'aide d'un système de filtration et d'une pompe à vide, soit placé dans un tube de 15 mL et centrifugé à 5 000 g pendant 8 minutes. Le surnageant est éliminé en laissant environ 2 mL d'échantillon avant d'être centrifugé à nouveau à 10 000 g pendant 1 minute pour éliminer complétement le restant de surnageant et préserver le culot de cellules. 1 mL de TRI- Reagent (Sigma^{®}, France) ou 1 mL de tampon de lyse du kit Quick-RNA^{™} MiniPrep (Zymo Research^{®}, USA) est immédiatement ajouté à chaque culot ou filtrat et homogénéisé. La lyse cellulaire est achevée en ajoutant des billes (0,5 mm, Zymo Research^{®}, USA) et en appliquant des vibrations grâce à un broyeur de type Tissue Lyser (Qiagen^{®}, USA) pendant 2 minutes à vitesse maximale.

Les ARN totaux sont isolés grâce au kit Quick-RNA^{™} MiniPrep kit ou grâce à une extraction au TriReagent. La concentration en ARN est mesurée à l'aide d'un spectrophotomètre de type Nanodrop (Peqlab^{®}, Erlangen, Germany). Les échantillons sont soit utilisés immédiatement soit stockés à -80 °C jusqu'à utilisation.

Les ARN totaux de 10 000 à 1 000 000 de cellules ont été extraits en 3 réplicas à partir de différentes cultures et de différentes souches d'algues toxiques. Les valeurs de concentration d'ARN obtenues ont été utilisées pour obtenir une valeur moyenne du contenu en ARN par cellule dans des conditions optimales de culture **(****Figure 2****).** Ayers *et al.* (2005) supposent que la croissance exponentielle obtenues dans des conditions optimales de croissance en culture correspond approximativement à ce qui se passe durant une efflorescence.

### c) Conception et synthèse des sondes nucléiques

Les sondes utilisées sont synthétisées selon les méthodes connues de l'homme de l'art. Elles sont réhydratées dans de l'eau ultrapure pour obtenir une solution mère d'une concentration à 100 µM. Trois sondes oligonucléotidiques ont été désignées et testées pour l'algue toxique *Lingulodinium polyedrum* (Tableau 3). Les sondes de séquences SEQ ID NO : 119 et SEQ ID NO : 120 ont été utilisées pour tester le contrôle position (PC) et le contrôle négatif (NC).

**Tableau 3 : Sondes oligonucléotidiques ciblant Lingulodinum polyedrum.**

| **Espèce** | **Couple de sondes testé (SEQ ID NO :)** | **Séquence (5'-3')** | **Tm** | **GC (%)** | **Séquence (5'-3')** | **Tm** | **GC (%)** |
|---|---|---|---|---|---|---|---|
| *PC (contrôle positif)* | *119*/*120* | GGC AAA CAG GAC TGT CAC CCT CAT T (SEQ ID NO: 119) | 59,3 | 52 | AGT GCT CTT TTC CAA GAG GCT TAC ATC TG (SEQ ID NO : 120) | 60,1 | 45 |
| *NC (contrôle négatif)* | *119*/*120* | GGC AAA CAG GAC TGT CAC CCT CAT T (SEQ ID NO: 119) | 59,3 | 52 | AGT GCT CTT TTC CAA GAG GCT TAC ATC TG (SEQ ID NO : 120) | 60,1 | 45 |
| *Lingulodinium polyedrum* | *119*/*120* | GGC AAA CAG GAC TGT CAC CCT CAT T (SEQ ID NO: 119) | 59,3 | 52 | AGT GCT CTT TTC CAA GAG GCT TAC ATC TG (SEQ ID NO : 120) | 60,1 | 45 |
| *Lingulodinium polyedrum* | *122*/*123* | GGA CTG TCA CCC TCA TTA GTG CTC T (SEQ ID NO : 122) | 59,3 | 52 | TTT CCA AGA GGC TTA CAT CTG CAC CC (SEQ ID NO : 123) | 59,5 | 50 |
| *Lingulodinium polyedrum* | *125*/*126* | CTG CAC CCC CAT TGG CAA CGC ATC T (SEQ ID NO : 125) | 62,6 | 60 | CCA GAC TAC AAC TCA AGG CTA CCA G (SEQ ID NO : 126) | 59,3 | 52 |

### d) Test d'hybridation Sandwich

Des tests de spécificité et de sensibilité des sondes sont réalisés par hybridation sandwich. La sonde capture biotinylée ou modifiée par un groupement amine (SEQ ID NO: 119 ; SEQ ID NO : 122 ; SEQ ID NO : 125) est couplée à un support solide fonctionnalisé soit par de la neutravidine soit par du N-hydroxysulfosuccinimide et la sonde signal est couplée à une molécule de digoxigenine (SEQ ID NO : 120 ; SEQ ID NO : 123 ; SEQ ID NO : 126). La sonde signal est placée en présence des molécules d'acides nucléiques qui peuvent contenir l'acide nucléique ribosomique cible, complémentaire des sondes capture et signal. Le mélange est placé en présence de la sonde capture qui va s'hybrider à ses cibles complémentaires formant ainsi un hybride de trois molécules : la sonde capture, la sonde signal et l'acide nucléique ribosomique cible. Les complexes hybrides sont révélés par la digoxigenine attaché à la sonde signal grâce à une réaction colorimétrique initiés par une enzyme de type peroxydase de raifort avec son substrat produisant une couleur bleue. La densité optique de la couleur obtenue est mesurée par un spectrophotomètre. L'intensité de la couleur est proportionnelle à la quantité de l'acide nucléique ribosomique cible présent dans l'extrait de l'échantillon analysé. Grâce à une courbe de calibration réalisées avec des ARN synthétiques, la quantité en acide nucléique cible est déterminée. Grâce à une courbe de calibration réalisée avec des ARN extraits de nombre connu de cellules cibles en phase exponentielle de croissance, la quantité d'ARN déterminée par la première calibration est associée à un nombre estimé de cellules d'algues toxiques vivantes actives présentes dans l'échantillon analysé.

Le test complet est réalisé en moins d'une heure.

Les échantillons pour le test d'hybridation sandwich sont préparés comme suit :
Les cellules issues des cultures sont collectées par filtration sur une membrane en polycarbonate (porosité de 3 µm ; Whatman^{®} Nuclepore Track-Etched Membranes). Les membranes sont transférées dans un tube (Eppendorf^{®}) contenant 1 mL de solution TriReagent^{®} (Sigma^{®}, France) et chauffées à 65°C pendant 10 minutes. Elles sont ensuite soumises au broyeur en présence de billes d'une taille de 0,5 mm (Bashing Beads^{®}, Zymoresearch^{®}) pendant 1 minute à vitesse maximale pour extraire le matériel génétique. Le surnageant est récolté et 200 µL de chloroforme sont ajoutés et mélangés. Les échantillons sont centrifugés pendant 15 minutes à 4°C et la phase aqueuse est transférée dans un tube propre. 0,5 volume d'isopropanol est ajouté et le mélange est incubé 1 heure à -20°C. Après une centrifugation de 20 minutes à 9000 g à 4°C, le surnageant est éliminé et le culot est lavé deux fois avec de l'éthanol 70%. Les culots sont séchés à l'air libre puis solubilisés dans 50 à 100 µL d'eau ultra pure. La quantité et la qualité des ARN obtenues sont mesurées par spectrophotométrie au NanoDrop (Thermo Scientific^{®}) ou au NanoVue (Biochrom^{®} Spectrophotometers). Les ARN totaux sont fragmentés à l'aide d'une solution comprenant 40 mM Trizma base, pH 8.0 / 100 mM KOAc / 30 mM MgOAc pendant 10 minutes à 65°C avant l'hybridation.

Les étapes d'hybridation sont réalisées dans une microplaque standard de 96 puits (Nunc^{®}, Danemark) fonctionnalisée par une solution de NeutrAvidine à 1 µg/mL, incubée pendant 24h et lavées avec une solution saline comme du PBS 1X (K₂PO₄, 0,1 M ; KH₂PO₄, 0,1 M ; KCl, 0,1 M ; pH 7,6) ou par du N-hydroxysulfosuccinimide (PolyAn, Allemagne). La première étape d'hybridation consiste à mélanger 200 ng d'ARN avec le tampon d'hybridation (0,3 M NaCl ; 0,08 M Tris-HCl ; 0,04% SDS ; pH 8) à un volume final de 100 µL contenant la sonde signal (1mM). Le mélange est chauffé à 60°C pendant 10 minutes. Les échantillons sont ensuite refroidis et une solution d'EDTA à 0,05 M final est ajoutée. Le mélange est ajouté dans les puits de la microplaque et incubé pendant 10 minutes à 60°C. La microplaque est lavée trois fois avec une solution saline comme du PBX 1X. 100 µL d'un anticorps anti-DIG-HRP à une concentration de 75 mU/mL sont ensuite ajoutés et incubés pendant 15 minutes à température ambiante. Puis 100 µL de TMB sont ajoutés et l'absorbance est mesuré après 15 minutes d'incubation à une longueur d'onde de 630 nm. La réaction peut aussi être stoppée à l'aide d'un acide comme l'acide sulfurique et l'absorbance est mesurée à une longueur d'onde de 450 nm. Chaque étape de la révélation est réalisée à température ambiante avec une agitation constante et à l'abri de la lumière.

Le criblage des couples de sondes suivants : SEQ ID NO : 119 et SEQ ID NO : 120, SEQ ID NO : 122 et SEQ ID NO : 123 ou SEQ ID NO : 125 et SEQ ID NO : 126 a été conduit à une même température d'hybridation. Les résultats ont été comparés à ceux obtenus sur les contrôles positifs (PC) et négatifs (NC) **(****Figure 3****).** Le contrôle positif est un fragment d'ADN synthétique de concentration 0,1 µM complémentaire aux deux sondes et le contrôle négatif est un fragment d'ADN synthétique de concentration de 0,1 µM non complémentaire aux deux sondes. Les Sondes SEQ ID NO : 119 et SEQ ID NO : 120 ; et SEQ ID NO : 125 et SEQ ID NO : 126 ont été choisis pour réaliser les courbes de calibration.

### e) Courbes de calibration pour quantifier les ARN cibles

Les meilleures combinaisons de sondes ont été utilisées pour établir les courbes de calibration. Ces courbes de calibration mesurées à 630 nm ont été élaborées à partir d'ARN extraits d'un nombre déterminé de cellules algales issues d'une culture non axénique, ce qui entend une contamination bactérienne de la culture, comme décrit dans les parties a) et b) de cet exemple. Une dilution des ARNs a été réalisée pour obtenir une gamme de concentration de 0,5 ng/µl à 40 ng/µl soit de 15 à 1200 ng d'ARN total dans un volume réactionnel de 30 µL. Les étapes d'hybridation sont réalisées comme dans la partie d) de cet exemple. La figure 4 montre les courbes de calibration obtenues avec la dilution en série à une absorbance de 630 nm pour les algues toxiques suivantes :
*A. Alexandrium minutum ;* couple de sondes testé : SEQ ID NO : 7 et SEQ ID NO : 8
B. *Alexandrium tamarense ;* couple de sondes testé : SEQ ID NO : 17 et SEQ ID NO : 18
C. *Alexandrium ostenfeldii ;* couple de sondes testé : SEQ ID NO : 11 et SEQ ID NO : 12
D. *Dinophysis acuminata ;* couple de sondes testé : SEQ ID NO : 40 et SEQ ID NO : 41
E. *Lingulodinium polyedrum* ; couple de sondes testé : SEQ ID NO : 119 et SEQ ID NO : 120
F. *Gymnodinium catenatum ;* couple de sondes testé : SEQ ID NO : 83 et SEQ ID NO : 84
G. *Chattonella subsalsa ;* couple de sondes testé : SEQ ID NO : 74 et SEQ ID NO : 75.

Dans cette expérience de courbe de calibration, le résultat montre un seuil de détection minimum entre 0,5 et 5 ng/µL d'un volume réactionnel de 30 µL. **La quantité totale d'ARN peut être rapportée à un litre d'échantillon de culture soit entre 25 et 250 ng/L.**

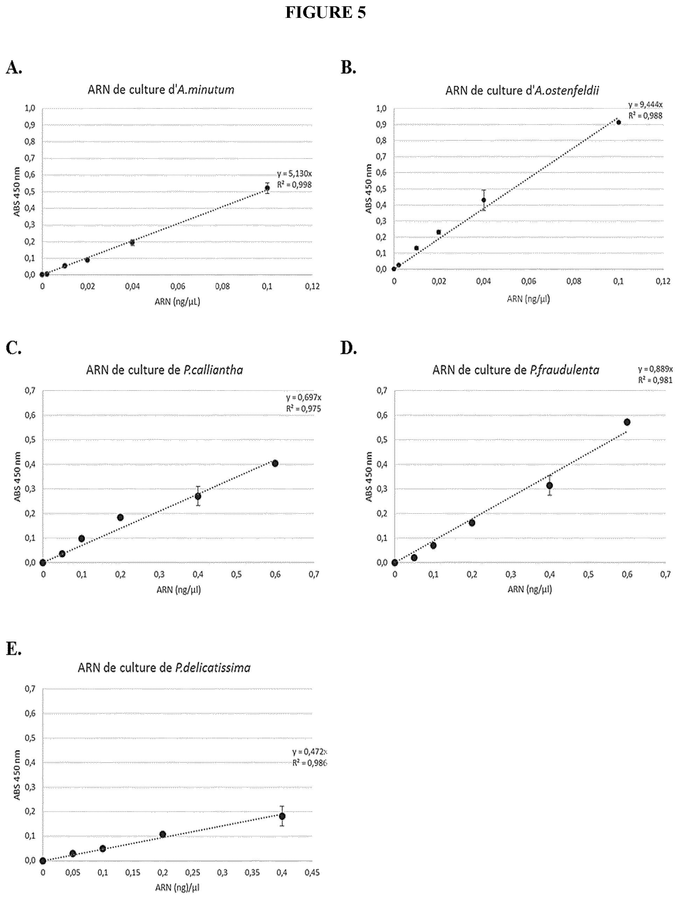

Avec la procédure optimisée (voir g)), les résultats ont montré **(****Figure 5****)** une limite de quantification de 0,01 ng/µL, *i.e.,* une quantité de 0,5 ng d'ARN totaux dans 50 µL de volume réactionnel. La limite de détection est de 0,002 ng/µL de volume réactionnel, *i.e.,* une quantité de 0,1 ng d'ARN totaux dans 50 µL de volume réactionnel. **Les résultats peuvent être rapportés à 1 litre d'échantillon soit 0,1 ng/L.**

### f) Courbes de calibration réalisées à partir d'échantillons environnementaux supplémentés en cellules ou naturellement contaminés et comptés par microscopie

Les courbes de calibration ont été réalisées en ajoutant un nombre connu de cellules issues de culture à un échantillon environnemental non contaminé par *Alexandrium.* Des cellules issues de culture non axénique *d'Alexandrium minutum* ont été récoltées soit en phase exponentielle de croissance soit en phase stationnaire comme décrit précédemment Figure 1. Entre 50 et 5 000 cellules ont été collectées en triplicas et filtrées sur une membrane de polycarbonate d'une porosité de 3 µm (Whatman^{®} Nuclepore Track-Etched Membranes).

L'extraction ARN est réalisée avec le kit QuickRNA^{®} (ZymoResearch^{®}, USA) avec un volume d'élution de 60 µL d'eau ultrapure. Les ARN totaux sont fragmentés à l'aide d'une solution comprenant 40 mM Trizma base, pH 8.0, 100 mM KOAc et 30 mM MgOAc) pendant 10 minutes à 65°C avant l'hybridation.

Les étapes d'hybridation sont réalisées dans une microplaque standard de 96 puits (Nunc^{®}, Denemark) fonctionnalisée par une solution de NeutrAvidine à 1 µg/mL, incubée pendant 24h avec la sonde de séquence SEQ ID NO : 1 à une concentration de 1 µM. Après 24h la microplaque est lavée avec une solution saline comme du PBS 1X (K₂PO₄, 0,1 M ; KH₂PO₄, 0,1 M ; KCl, 0,1 M ; pH 7,6). L'éluât avec les ARN est mélangé avec le tampon d'hybridation (0,3 M NaCl, 0,08 M Tris-HCl, 0,04% SDS, pH 8) à un volume final de 100 µL contenant la sonde de séquence SEQ ID NO : 2 à une concentration de 1 µM. Le mix d'hybridation est chauffé à 60°C pendant 10 minutes, puis une solution d'EDTA à 0,05 M final est ajoutée. 100 µl du mélange est reparti dans chaque puits de la microplaque et incubé pendant 10 minutes à 60°C. La microplaque est lavée trois fois avec une solution saline comme du PBX 1X. 100 µL d'un anticorps anti-DIG-HRP à une concentration de 75 mU/mL sont ensuite ajoutés et incubés pendant 15 minutes à température ambiante. 100 µL de substrat comme du TMB est ajouté et l'absorbance est mesuré après 15 minutes de réaction à une longueur d'onde de 630 nm.

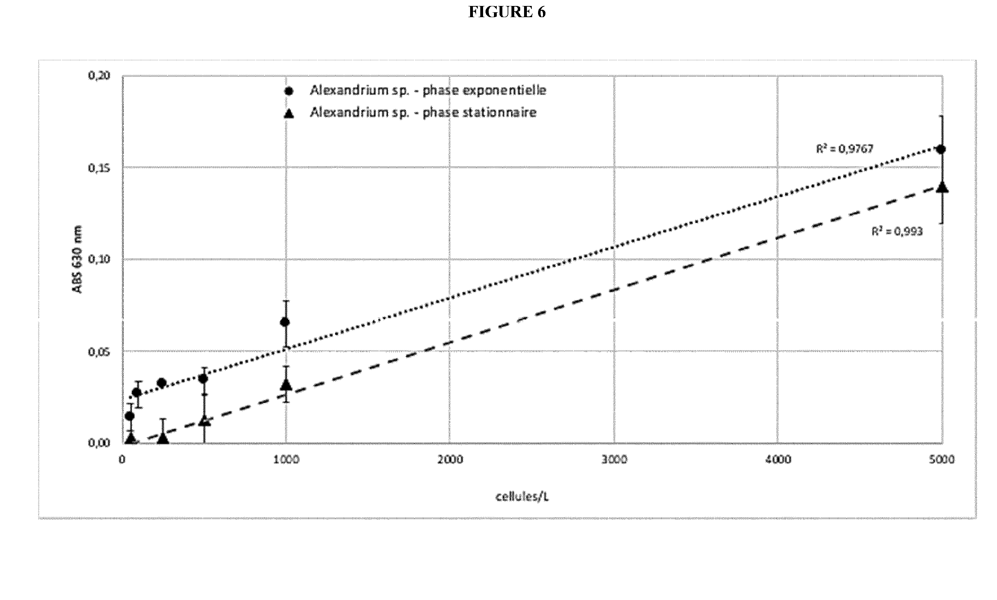

Les résultats **(****Figure 6****)** montrent un seuil de quantification minimale équivalent de 250 et 500 cellules vivantes actives par litre d'échantillon en phase stationnaire et de 100 à 250 cellules par litre d'échantillon en phase exponentielle, ce qui correspond respectivement de 5,05 à 10,1 ng d'ARN totaux par litre d'échantillon et de 2,02 à 5,05 ng d'ARN totaux par litre d'échantillon.

La micro-algue *Dinophysis* étant très difficilement cultivable, les courbes de calibration sont réalisées à l'aide d'échantillons environnementaux naturellement contaminés. Un échantillon naturellement contaminé par *Dinophysis* a été compté par microscopie et entre 0 et 150 cellules ont été collectées par filtration et utilisées pour réaliser la courbe de calibration. L'hybridation des ARN totaux de l'extrait naturel a été réalisée comme décrit dans le paragraphe d). L'absorbance a été lue à 450 nm après arrêt de la réaction colorimétrique par de l'acide sulfurique.

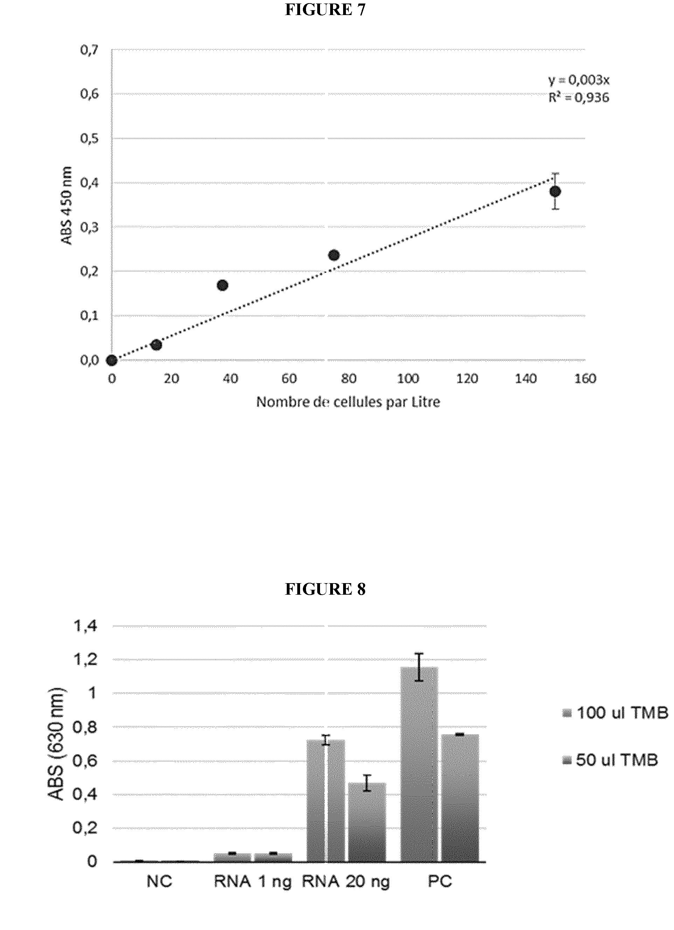

Les résultats **(****Figure 7****)** montrent avec la procédure optimisée, une limite de quantification de 5 cellules vivantes actives estimées par litre sur la base d'un signal dont l'intensité est égale à 10 fois celle de l'écart type du blanc soit 0,01 unité d'absorbance. La limite de détection est établie à un équivalent de 2 cellules vivantes actives par litre d'échantillon naturel sur la base d'un signal dont l'intensité est égale à 3 fois celle de l'écart type du blanc soit 0,003 unité d'absorbance. La correspondance en ARN n'est pas possible car la courbe est réalisée à partir d'échantillons environnementaux contaminés dont le contenu en ARN totaux n'est pas équivalent à celui obtenu à partir d'une culture cellulaire.

### g) Amélioration de la sensibilité du test colorimétrique

Les limites de détection et quantification ont été améliorées grâce à l'optimisation des conditions de réalisation de la procédure en testant différents tampons d'hybridation, différents volumes de solutions de révélation et différentes longueurs d'onde pour la lecture des absorbances.

Dans une première expérience, deux volumes de la solution de révélation sont testés. Les résultats indiquent une amélioration de la sensibilité de détection **(****Figure 8****).** Les résultats montrent que le volume de 100 µL de solution TMB permet d'augmenter le seuil de sensibilité de la procédure d'un facteur 1,7 en unité d'absorbance à 630 nm.

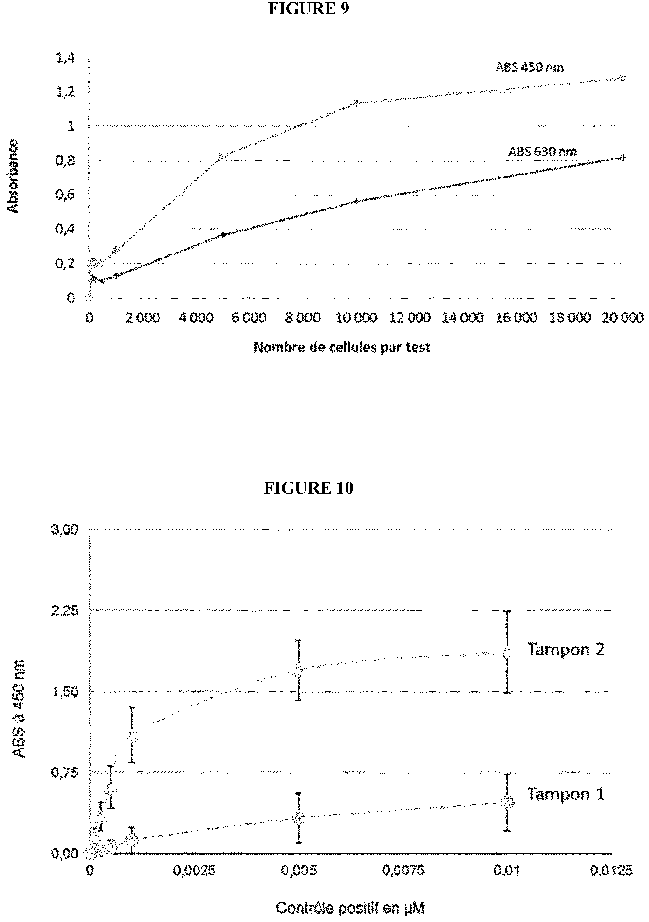

Dans une deuxième expérience, des tests d'hybridation sont réalisés en dupliquas et les résultats sont mesurés soit à 630 nm soit à 450 nm après arrêt de la révélation par de l'acide sulfurique. Les résultats indiquent que la sensibilité est augmentée d'un facteur allant de 2 à 4 d'unité d'absorbance en utilisant la longueur d'onde 450 nm **(****Figure 9****).**

La troisième expérience a consisté à réaliser les expériences d'hybridation soit avec le tampon d'hybridation 1 soit avec le tampon d'hybridation 2 réalisés selon les indications décrites. Les résultats sont rapportés su la **Figure 10** et montrent que le tampon 2 permet d'augmenter le seuil de sensibilité de la procédure d'un facteur 2,8 en unité d'absorbance à 450 nm (figure 10).

### EXEMPLE 2 : Détection d'algues à partir d'échantillons environnementaux naturels

Des comparaisons de mise en œuvre entre la présente méthode et la technique traditionnelle basée sur l'identification et le comptage des algues en microscopie avec la méthode Utermöhl (1958) ont été effectuées sur des échantillons naturels.

### a) Détection de Pseudo-nitzschia

Le suivi de *Pseudo-nitzschia* a été réalisé dans le bassin de Thau, Bouzigues, sud de la France du 30 mars 2017 au 30 juin 2017. Les échantillons d'eau ont été collectés deux fois par semaine à 50 cm de profondeur. En parallèle, un sub-échantillon de 50 mL a été collecté, fixé au Lugol, et sédimenté pendant 24h.

Le comptage au microscope selon la méthode Utermöhla nécessité une étape de sédimentation de 12 heures puis un comptage minutieux des cellules sous microscope. La totalité de la méthode Utermöhla été exécuté en 24 à 48 heures. Cette méthode permet de détecter la présence ou l'absence de cellules d'algue toxique du genre *Pseudo-nitzschia* et ainsi de déterminer le nombre de cellules d'algue toxique du genre *Pseudo-nitzschia.* Toutefois, cette méthode ne permet pas de déterminer l'activité des cellules d'algue toxique du genre *Pseudo-nitzschia.*

L'exécution de la présente méthode a été réalisée en moins d'une heure et a permis de déterminer l'activité des cellules d'algue toxique du genre *Pseudo-nitzschia* ainsi que le nombre de cellule.

Pour chaque test d'hybridation, 3 litres d'eau de mer sont immédiatement filtrés sur membranes en polycarbonate (porosité de 3 µm ; Whatman^{®} Nuclepore Track-Etched Membranes). Les membranes sont transférées dans un tube (Eppendorf^{®}) contenant 2 mL de solution de lyse ZR (ZymoResearch^{®}, USA) et chauffées à 65°C pendant 10 minutes. Elles sont ensuite soumises au broyeur en présence de billes d'une taille de 0,5 mm (Bashing Beads^{®}, ZymoResearch^{®}) pendant 1 minute à vitesse maximale. L'extraction ARN est realisée avec le kit QuickRNA^{®} (ZymoResearch^{®}, USA) avec un volume d'élution de 180 µL d'eau ultrapure. Les ARN totaux sont fragmentés à l'aide d'une solution comprenant 40 mM Trizma base, pH 8.0 / 100 mM KOAc / 30 mM MgOAc) pendant 10 minutes à 65°C avant l'hybridation.

Les étapes d'hybridation sont réalisées dans une microplaque standard de 96 puits (Nunc^{®}, Denemark) fonctionnalisée par une solution de NeutrAvidine à 1µg mL-1, incubée pendant 24h avec la sonde de séquence SEQ ID NO : 46 à une concentration de 1µM. Après 24h la microplaque est lavée avec une solution saline comme du PBS 1X (K₂PO₄, 0.1 M; KH₂PO₄, 0.1 M; KCl, 0.1 M, pH 7,6). L'éluât avec les ARN est melangé avec le tampon d'hybridation (0,3 M NaCl, 0.08 M Tris-HCl, 0.04% SDS, pH 8) à un volume final de 300 µl contenant la sonde signal de séquence SEQ ID NO : 47 (1 mM). Le mix d'hybridation est chauffé à 60°C pendant 10 minutes, puis une solution d'EDTA à 0,05 M final est ajoutée. 100 µl du mélange est reparti dans 3 puits de la microplaque et incubé pendant 10 minutes à 60°C. La microplaque est lavée trois fois avec une solution saline comme du PBX 1X. 100 µL d'un anticorps anti-DIG-HRP à une concentration de 75 mU/mL sont ensuite ajoutés et incubés pendant 15 minutes à température ambiante. 100 µL de substrat comme du TMB est ajouté et l'absorbance est mesuré après 15 minutes de réaction à une longueur d'onde de 630 nm. Chaque étape de la révélation est réalisée à température ambiante avec une agitation constante et à l'abri de la lumière.

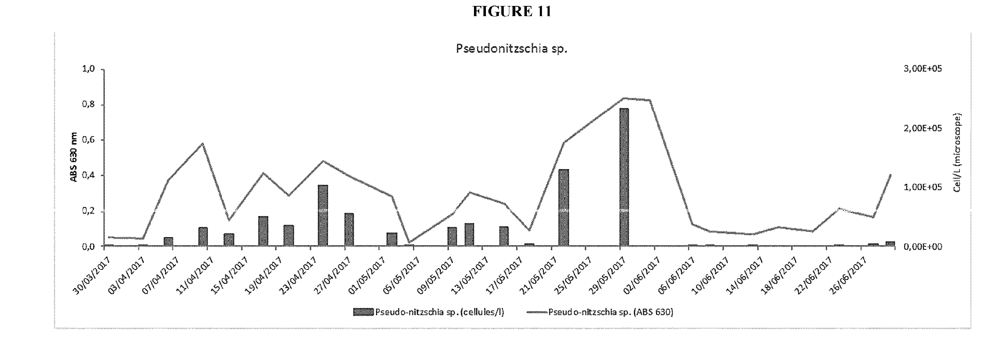

De manière générale, les résultats obtenus par les tests d'hybridation sandwich avec des échantillons environnementaux naturellement contaminés sont en accord avec les comptages obtenus par microscopie. Toutefois, la présente méthode permet d'obtenir les résultats en moins d'une heure, contre 24 à 48 heures pour la méthode de comptages. Le test d'hybridation a permis la détection d'approximativement 500 cellules vivantes actives de *Pseudo-nitzschia* par litre d'échantillon **(****Figure 11****).**

### b) Détection d'Alexandrium

Le suivi *d'Alexandrium* a été réalisé dans le bassin de Thau, Bouzigues, sud de la France du 05 octobre 2017 au 17 novembre 2017. Les échantillons d'eau ont été collectés deux fois par semaine à 50 cm de profondeur. En parallèle, un sub-échantillon de 50 mL a été collecté, fixé au Lugol, et sédimenté pendant 24h. Le comptage au microscope selon la méthode Utermöhl a nécessité une étape de sédimentation de 12 heures puis un comptage minutieux des cellules sous microscope. La totalité de la méthode Utermöhla été exécuté en 24 à 48 heures. Cette méthode permet de détecter la présence ou l'absence de cellules d'algue toxique du genre *Alexandrium* et ainsi de déterminer le nombre de cellules. Toutefois, cette méthode ne permet pas de déterminer l'activité des cellules d'algue toxique du genre *Alexandrium.*

L'exécution de la présente méthode a été réalisée en moins d'une heure et a permis de déterminer l'activité des cellules d'algue toxique du genre *Alexandrium* ainsi que le nombre des cellules.

Pour chaque test d'hybridation, 3 litres d'eau environnementale sont collectés et immédiatement filtrés sur membranes en polycarbonate (porosité de 5 µm ; Whatman^{®} Nuclepore Track-Etched Membranes). Les membranes sont transférées dans un tube (Eppendorf^{®}) contenant 2 mL de solution de lyse ZR (ZymoResearch^{®}, USA) et chauffées à 60°C pendant 10 minutes. Elles sont ensuite soumises au broyeur en présence de billes d'une taille de 0,5 mm (Bashing Beads^{®}, ZymoResearch^{®}) pendant 2 minutes à vitesse maximale. L'extraction ARN est realisée avec le kit QuickRNA^{®} (ZymoResearch^{®}, USA) avec un volume d'élution de 180 µl d'eau ultrapure soit 60 µL par réplicas. Les ARN totaux sont fragmentés à l'aide d'une solution comprenant 40 mM Trizma base, pH 8.0 / 100 mM KOAc / 30 mM MgOAc) pendant 10 minutes à 65°C avant l'hybridation et les échantillons sont stabilisés avec une solution de EDTA 0,05%.

Les étapes d'hybridation sont réalisées dans une microplaque standard de 96 puits (Nunc^{®}, Denemark) fonctionnalisée par une solution de NeutrAvidine à 1µg/mL, incubée pendant 24h avec la sonde de séquence SEQ ID NO : 1 à une concentration de 1 µM. Après 24h la microplaque est lavée avec une solution saline comme du PBS 1X (K₂PO₄, 0,1 M ; KH₂PO₄, 0,1 M ; KCl, 0,1 M ; pH 7,6). L'éluât avec les ARN est melangé avec le tampon d'hybridation (0,3 M NaCl, 0.08 M Tris-HCl, 0.04% SDS, pH 8) à un volume final de 300 µl contenant la sonde de séquence SEQ ID NO : 2 à une concentration de 1 µM. Le mix d'hybridation est chauffé à 60°C pendant 10 minutes, puis une solution d'EDTA à 0,05 M final est ajoutée. 100 µl du mélange est reparti dans 3 puits de la microplaque et incubé pendant 15 minutes à 60°C. La microplaque est lavée trois fois avec une solution saline comme du PBS 1X. 100 µL d'un anticorps anti-DIG-HRP à une concentration de 75 mU/mL sont ensuite ajoutés et incubés pendant 15 minutes à température ambiante. 100 µL de substrat comme du TMB est ajouté et l'absorbance est mesuré après 15 minutes de réaction à une longueur d'onde de 630 nm. Chaque étape de la révélation est réalisée à température ambiante avec une agitation constante et à l'abri de la lumière.

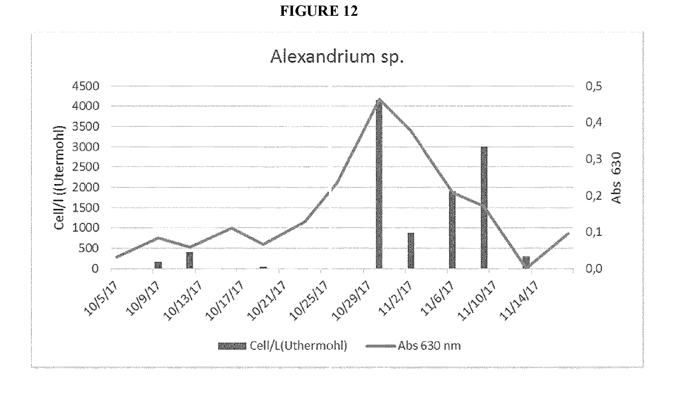

De manière générale, les résultats obtenus par les tests d'hybridation sandwich avec des échantillons environnementaux naturellement contaminés sont en accord avec les comptages obtenus par microscopie. Toutefois, la présente méthode permet d'obtenir les résultats en moins d'une heure, contre 24 à 48 heures pour la méthode de comptages. Le test d'hybridation a permis la détection d'approximativement 160 cellules vivantes actives *d'Alexandrium* par litre d'échantillon **(****Figure 12****).**

### c) Détection de Dinophysis sp.

Le suivi de *Dinophysis* a été réalisé dans la lagune de Leucate, sud de la France du 15 octobre 2018 au 30 mars 2019. Les échantillons d'eau ont été collectés deux fois par semaine à 50 cm de profondeur. En parallèle, un sub-échantillon de 50 mL a été collecté, fixé au Lugol, et sédimenté pendant 24h. Le comptage au microscope selon la méthode Utermöhl a nécessité une étape de sédimentation de 12 heures puis un comptage minutieux des cellules sous microscope. La totalité de la méthode Utermöhl a été exécuté en 24 à 48 heures. La méthode n'a pas permis de déterminer l'activité mais seulement la présence ou l'absence et le nombre de cellules. L'exécution de la présente méthode a été réalisée en moins d'une heure et a permis de déterminer l'activité et le nombre des cellules en comparaison des résultats obtenus *a posteriori* par la méthode Utermöhl.

Pour chaque test d'hybridation, 3 litres d'eau sont immédiatement filtrés sur membranes en polycarbonate (porosité de 5 µm ; Whatman^{®} Nuclepore Track-Etched Membranes). Les membranes sont transférées dans un tube (Eppendorf^{®}) contenant 2 mL de solution de lyse ZR (ZymoResearch^{®}, USA) et chauffées à 60°C pendant 10 minutes. Elles sont ensuite soumises au broyeur en présence de billes d'une taille de 0,5 mm (Bashing Beads^{®}, ZymoResearch^{®}) pendant 2 minutes à vitesse maximale. L'extraction ARN est realisée avec le kit QuickRNA^{®} (ZymoResearch^{®}, USA) avec un volume d'élution de 180 µl d'eau ultrapure. Les ARN totaux sont fragmentés à l'aide d'une solution comprenant 40 mM Trizma base, pH 8.0 / 100 mM KOAc / 30 mM MgOAc) pendant 10 minutes à 65°C avant l'hybridation et les échantillons sont stabilisés avec une solution de EDTA 0,05%.

Les étapes d'hybridation sont réalisées dans une microplaque standard de 96 puits (Nunc^{®}, Denemark) fonctionnalisée par une solution de NeutrAvidine à 1µg/mL, incubée pendant 24h avec la sonde n. 1 (SEQ ID NO : 1) à une concentration de 1 µM. Après 24h la microplaque est lavée avec une solution saline comme du PBS 1X (K₂PO₄, 0,1 M ; KH₂PO₄, 0,1 M ; KCl, 0,1 M ; pH 7,6). L'éluât avec les ARN est melangé avec le tampon d'hybridation (0,3 M NaCl, 0.08 M Tris-HCl, 0.04% SDS, pH 8) à un volume final de 300 µl contenant la sonde n. 2 'SEQ ID NO : 2) à une concentration de 1 µM. Le mix d'hybridation est chauffé à 60°C pendant 10 minutes, puis une solution d'EDTA à 0,05 M final est ajoutée. 100 µl du mélange est reparti dans 3 puits de la microplaque et incubé pendant 15 minutes à 60°C. La microplaque est lavée trois fois avec une solution saline comme du PBS 1X. 100 µL d'un anticorps anti-DIG-HRP à une concentration de 75 mU/mL sont ensuite ajoutés et incubés pendant 15 minutes à température ambiante. 100 µL de substrat comme du TMB est ajouté et l'absorbance est mesuré après 15 minutes de réaction et après arrêt de la réaction avec de l'acide sulfurique à une longueur d'onde de 450 nm. Chaque étape de la révélation est réalisée à température ambiante avec une agitation constante et à l'abri de la lumière.

De manière générale, les résultats d'absorbance obtenus par les tests d'hybridation sandwich avec des échantillons environnementaux naturellement contaminés sont en accord avec les comptages obtenus par microscopie. Le test d'hybridation a permis la détection de moins de 40 cellules vivantes actives de *Dinophysis sp.* par litre sur la base des comptages microscopiques

### (Figure 13).

### REFERENCES

Ayers, K., Rhodes, L.L., Tyrrell, J., Gladstone, M., Scholin, C., 2005. International accreditation of sandwich hybridisation assay format DNA probes for microalgae. N.Z. J. Mar. Freshwater Res. 39, 1225-1231.
Daniel. MacDougall, Warren B. Crummett. et al. ACS (1980) Guidelines for Data Acquisition and Data Quality Evaluation in Environmental Chemistry, Analytical chemistry, 52, 14, 2242-2249
Guillard, R.R.L., Hargraves, P.E., (1993). Stichochrysis immobilisis a diatom, not a chrysophyte. Phycologia 32, 234-236
Guillard, R.R.L. and Ryther, J.H. (1962). Studies of marine planktonic diatoms. I. Cyclotella nana Hustedt and Detonula confervacea Cleve. Can. J. Microbiol. 8 : 229-239
JV Tyrrell, PR Bergquist, PL Bergquist, CA Scholin. Compositions and methods for detecting raphidophytes, US Patent 6,787,648
Sonja Diercks, Katja Metfies, Linda K., Molecular probe sets for the detection of toxic algae for use in sandwich hybridization formats. Journal of Plankton Research, Vol. 30, Issue 4, 1 April 2008, Pages 439-448
Sonja Diercks, Linda K Medlin, Katja Metfies. Colorimetric detection of the toxic Dinoflagellate Alexandrium minutum using sandwich hybridization in a microtiter plate assay - Harmful Algae, Vol. 7, Issue 2, Feb 2008, Pages 137-145
Joe D. Taylor, Jessica U. Kegel, Jane M. Lewis, Linda K. Medlin. Validation of the detection of Alexandrium species using specific RNA probes tested in a microarray format: Calibration of signal using variability of RNA content with environmental conditions. Harmful Algae, Vol. 37, 2014, Pages 17-27
UtermöhlH., (1958), Zur Vervollkommnung der quantitativen Phytoplankton-Methodik : Mit 1 Tabelle und 15 abbildungen im Text und auf 1 Tafel, Internationale Vereinigung für Theoretische und Angewandte Limnologie : Mitteilungen, 9 (1), p 1-38
Yuji Tanaka and Makoto Tsuneoka (2018), Control of Ribosomal RNA Transcription by Nutrients. Chapitre 2, Pages 25 - 51 de "Gene expression and regulation in mammalian cells: Transcription towards the establishment of novel therapeutics."

## Revendications

1. Utilisation d'au moins un couple de sondes spécifiques d'algues toxiques du genre *Alexandrium* pour la mise en œuvre d'un procédé de détection de cellules vivantes actives d'algues toxiques dans un échantillon naturel susceptible de contenir au moins une algue toxique du genre *Alexandrium,* ledit procédé étant réalisé à partir des ARN extraits totaux dudit échantillon naturel susceptible de contenir au moins une algue toxique du genre *Alexandrium* et dans lequel les séquences des sondes desdits couples étant les suivantes :
- (SEQ ID NO : 1 et SEQ ID NO : 2),
- (SEQ ID NO : 4 et SEQ ID NO : 5),
- (SEQ ID NO : 11 et SEQ ID NO : 12), ou
- (SEQ ID NO : 17 et SEQ ID NO : 18),
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence, ladite sonde capture et ladite sonde signal étant capables de s'hybrider avec l'ARN ribosomique d'une algue toxique du genre *Alexandrium* éventuellement présent dans ledit échantillon afin de former un complexe.

2. Utilisation selon la revendication 1 comprenant en plus l'utilisation d'au moins un couple de sondes spécifiques d'algues toxiques du genre *Dinophysis* pour la mise en œuvre d'un procédé de détection de cellules vivantes actives d'algues toxiques dans un échantillon naturel susceptible de contenir au moins une algue toxique du genre *Alexandrium et*/*ou Dinophysis,* les séquences desdites sondes étant choisies parmi x éléments de l'un des ensembles suivants :
- (SEQ ID NO : 29, SEQ ID NO : 30 ou SEQ ID NO : 31)
- (SEQ ID NO : 32 et SEQ ID NO : 33)
- (SEQ ID NO : 34 et SEQ ID NO : 35)
- (SEQ ID NO : 36 et SEQ ID NO : 37)
- (SEQ ID NO : 38 et SEQ ID NO : 39)
- (SEQ ID NO : 40, SEQ ID NO : 41 ou SEQ ID NO : 42)
- (SEQ ID NO : 43, SEQ ID NO : 44 ou SEQ ID NO : 45), ou
- (SEQ ID NO : 46, SEQ ID NO : 47 ou SEQ ID NO : 48)
x étant 2 ou 3,
ou les séquences desdites sondes ayant au moins 92% d'identité avec les susdites séquences SEQ ID NO: 29, SEQ ID NO: 30, SEQ ID NO: 31, SEQ ID NO: 32, SEQ ID NO : 33, SEQ ID NO : 34, SEQ ID NO : 35, SEQ ID NO : 36, SEQ ID NO : 37, SEQ ID NO : 38, SEQ ID NO : 39, SEQ ID NO : 40, SEQ ID NO : 41, SEQ ID NO : 42, SEQ ID NO : 43, SEQ ID NO : 44, SEQ ID NO : 45, SEQ ID NO : 46, SEQ ID NO : 47 ou SEQ ID NO : 48,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence, ladite sonde capture et ladite sonde signal étant capables de s'hybrider avec l'ARN ribosomique d'une algue toxique du genre ***Dinophysis*** éventuellement présent dans ledit échantillon,
en particulier, les séquences des sondes desdits couples étant les suivantes :
- (SEQ ID NO : 29 et SEQ ID NO : 30), (SEQ ID NO : 29 et SEQ ID NO : 31), (SEQ ID NO : 30 et SEQ ID NO : 31)
- (SEQ ID NO : 32 et SEQ ID NO : 33)
- (SEQ ID NO : 34 et SEQ ID NO : 35)
- (SEQ ID NO : 36 et SEQ ID NO : 37)
- (SEQ ID NO : 38 et SEQ ID NO : 39)
- (SEQ ID NO : 40 et SEQ ID NO : 41), (SEQ ID NO : 40 et SEQ ID NO : 42), (SEQ ID NO : 41 et SEQ ID NO : 42)
- (SEQ ID NO : 43 et SEQ ID NO : 44), (SEQ ID NO : 43 et SEQ ID NO : 45), (SEQ ID NO : 44 et SEQ ID NO : 45)
- (SEQ ID NO : 46 et SEQ ID NO : 47), (SEQ ID NO : 46 et SEQ ID NO : 48), (SEQ ID NO : 47 et SEQ ID NO : 48.

3. Utilisation selon la revendication 1 ou 2, dans laquelle,
- ladite sonde capture est liée à au moins une molécule d'attache positionnée en 5' de sa séquence et ladite sonde signal est liée à au moins une molécule de marquage positionnée en 5' de sa séquence, ou
- ladite sonde capture est liée à au moins une molécule d'attache positionnée en 5' de sa séquence et ladite sonde signal est liée à au moins une molécule de marquage positionnée en 3' de sa séquence, ou
- ladite sonde capture est liée à au moins une molécule d'attache positionnée en 3' de sa séquence et ladite sonde signal est liée à au moins une molécule de marquage positionnée en 5' de sa séquence, ou
- ladite sonde capture est liée à au moins une molécule d'attache positionnée en 3' de sa séquence et ladite sonde signal est liée à au moins une molécule de marquage positionnée en 3' de sa séquence,
ladite « au moins une molécule d'attache » étant notamment choisie parmi une molécule de biotine, avidine, streptavidine, un groupe thiol, un groupe amine et un carbone, de préférence une molécule de biotine,
laquelle ladite « au moins une molécule de marquage » étant notamment choisie parmi :
- un fluorochrome,
- une biotine,
- une molécule liée à une biotine,
- la digoxigénine,
- une enzyme utilisant un substrat chimioluminescent,
- une enzyme utilisant un substrat chromogène telle que la phosphatase alcaline, ledit substrat chromogène étant alors le Nitrobleu de tetrazolium (NBT) ou le bromochlorylindolophosphate (BCIP), ou la péroxydase de raifort (HRP), ledit substrat chromogène étant alors choisi parmi le 3,3'-Diaminobenzidine (DAB), le 3,3',5,5'-Tetramethylbenzidine (TMB) ou le 2,2'-azino-bis(acide 3-ethylbenzothiazoline-6-sulphonique) (ABTS), et
- une enzyme utilisant un substrat à oxydation électrochimique, de préférence la digoxigénine.

4. Couple de sondes pour la détection de cellules vivantes actives d'algues toxiques du genre *Alexandrium* dont les séquences des sondes desdits couples sont les suivantes :
- (SEQ ID NO : 1 et SEQ ID NO : 2),
- (SEQ ID NO : 4 et SEQ ID NO : 5),
- (SEQ ID NO : 11 et SEQ ID NO : 12), ou
- (SEQ ID NO : 17 et SEQ ID NO : 18).

5. Procédé de détection de cellules vivantes actives d'algues toxiques dans un échantillon naturel susceptible de contenir au moins une algue toxique du genre *Alexandrium* comprenant les étapes suivantes :
a) hybridation éventuelle résultant de la mise en contact des ARN extraits totaux dudit échantillon avec une sonde capture et une sonde signal spécifiques d'algues toxique du genre *Alexandrium,* la sonde capture et la sonde signal formant un couple de sondes, les séquences des sondes desdits couples étant les suivantes :
- (SEQ ID NO : 1 et SEQ ID NO : 2),
- (SEQ ID NO : 4 et SEQ ID NO : 5),
- (SEQ ID NO : 11 et SEQ ID NO : 12), ou
- (SEQ ID NO : 17 et SEQ ID NO : 18),
ladite sonde capture et ladite sonde signal étant capables de s'hybrider avec l'ARN ribosomique d'une algue toxique du genre *Alexandrium* éventuellement présent dans ledit échantillon afin de former un complexe ;
b) détection dudit complexe éventuel
l'hybridation indiquant la présence d'algue toxique du genre *Alexandrium,*
la durée de la mise en œuvre dudit procédé de détection étant en particulier inférieure à une heure.

6. Procédé de détection de cellules vivantes actives d'algues toxiques selon la revendication 5 dans un échantillon naturel susceptible de contenir au moins une algue toxique du genre *Alexandrium,* ledit procédé comprenant en outre :
▪ avant l'étape a), une étape préliminaire d'extraction des ARN dudit échantillon à analyser,
▪ après l'étape b), une étape de quantification des algues toxiques du genre *Alexandrium,* dans le cas d'une hybridation dans l'étape a),
la durée de la mise en œuvre des étapes a) et b) étant inférieure à une heure.

7. Procédé de détection de cellules vivantes actives d'algues toxiques selon l'une quelconque des revendications 5 ou 6, dans un échantillon naturel susceptible de contenir en plus au moins une algue toxique du genre *Dinophysis,* comprenant en plus de l'étape d'hybridation éventuelle résultant de la mise en contact dudit échantillon avec une sonde capture et une sonde signal spécifiques d'algues toxiques du genre *Alexandrium,*
une étape d'hybridation éventuelle résultant de la mise en contact dudit échantillon avec une sonde capture et une sonde signal spécifiques d'algues toxique du genre *Dinophysis,* la sonde capture et la sonde signal formant un couple de sondes, les séquences dudit couple de sondes étant choisies parmi x éléments de l'un des ensembles suivants :
- (SEQ ID NO : 29, SEQ ID NO : 30 ou SEQ ID NO : 31)
- (SEQ ID NO : 32 et SEQ ID NO : 33)
- (SEQ ID NO : 34 et SEQ ID NO : 35)
- (SEQ ID NO : 36 et SEQ ID NO : 37)
- (SEQ ID NO : 38 et SEQ ID NO : 39)
- (SEQ ID NO : 40, SEQ ID NO : 41 ou SEQ ID NO : 42)
- (SEQ ID NO : 43, SEQ ID NO : 44 ou SEQ ID NO : 45), ou
- (SEQ ID NO : 46, SEQ ID NO : 47 ou SEQ ID NO : 48)
x étant 2 ou 3,
ou les séquences desdites sondes ayant au moins 92% d'identité avec les susdites séquences SEQ ID NO : 29 à SEQ ID NO : 48,
l'hybridation indiquant la présence d'algue toxique du genre *Dinophysis.*

8. Procédé de détection selon l'une quelconque des revendications 5 à 7 dans lequel :
- ladite sonde capture est liée à au moins une molécule d'attache positionnée en 5' de sa séquence et ladite sonde signal est liée à au moins une molécule de marquage positionnée en 5' de sa séquence, ou
- ladite sonde capture est liée à au moins une molécule d'attache positionnée en 5' de sa séquence et ladite sonde signal est liée à au moins une molécule de marquage positionnée en 3' de sa séquence, ou
- ladite sonde capture est liée à au moins une molécule d'attache positionnée en 3' de sa séquence et ladite sonde signal est liée à au moins une molécule de marquage positionnée en 5' de sa séquence, ou
- ladite sonde capture est liée à au moins une molécule d'attache positionnée en 3' de sa séquence et ladite sonde signal est liée à au moins une molécule de marquage positionnée en 3' de sa séquence,
ladite « au moins une molécule d'attache » étant notamment choisie parmi une molécule de biotine, avidine, streptavidine, un groupe thiol, un groupe amine et un groupe carbone, de préférence une molécule de biotine,
ladite « au moins une molécule de marquage » étant notamment choisie parmi :
- un fluorochrome,
- une biotine,
- une molécule liée à une biotine,
- la digoxigénine,
- une enzyme utilisant un substrat chimioluminescent,
- une enzyme utilisant un substrat chromogène telle que la phosphatase alcaline, ledit substrat chromogène étant alors le Nitrobleu de tetrazolium (NBT) ou le bromochlorylindolophosphate (BCIP), ou la péroxydase de raifort (HRP), ledit substrat chromogène étant alors choisi parmi le 3,3'-Diaminobenzidine (DAB), le 3,3',5,5'-Tetramethylbenzidine (TMB) ou le 2,2'-azino-bis(acide 3-ethylbenzothiazoline-6-sulphonique) (ABTS), ou
- une enzyme utilisant un substrat à oxydation électrochimique, de préférence la digoxigénine.

9. Kit pour la détection de cellules vivantes actives d'algues toxiques d'algues toxiques du genre *Alexandrium,* ledit kit contenant :
a) au moins un couple de sondes spécifiques d'algues toxiques du genre *Alexandrium,* les séquences des sondes desdits couples étant les suivantes :
- (SEQ ID NO : 1 et SEQ ID NO : 2),
- (SEQ ID NO : 4 et SEQ ID NO : 5),
- (SEQ ID NO : 11 et SEQ ID NO : 12), ou
- (SEQ ID NO : 17 et SEQ ID NO : 18),
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'ARN ribosomique d'une algue toxique du genre *Alexandrium,*
b) éventuellement une solution d'hybridation,
c) éventuellement une solution de lavage, et
d) éventuellement une solution de révélation.

10. Kit selon la revendication 9, ledit kit contenant **en plus :**
a) au moins un couple de sondes spécifiques d'algues toxiques du genre ***Dinophysis,*** les séquences desdites sondes étant choisies parmi x éléments de l'un des ensembles suivants :
- (SEQ ID NO : 29, SEQ ID NO : 30 ou SEQ ID NO : 31)
- (SEQ ID NO : 32 et SEQ ID NO : 33)
- (SEQ ID NO : 34 et SEQ ID NO : 35)
- (SEQ ID NO : 36 et SEQ ID NO : 37)
- (SEQ ID NO : 38 et SEQ ID NO : 39)
- (SEQ ID NO : 40, SEQ ID NO : 41 ou SEQ ID NO : 42)
- (SEQ ID NO : 43, SEQ ID NO : 44 ou SEQ ID NO : 45), ou
- (SEQ ID NO : 46, SEQ ID NO : 47 ou SEQ ID NO : 48)
x étant 2 ou 3,
ou les séquences desdites sondes ayant au moins 92% d'identité avec les susdites séquences SEQ ID NO : 29, SEQ ID NO : 30, SEQ ID NO : 31, SEQ ID NO : 32, SEQ ID NO : 33, SEQ ID NO : 34, SEQ ID NO: 35, SEQ ID NO: 36, SEQ ID NO : 37, SEQ ID NO : 38, SEQ ID NO: 39, SEQ ID NO: 40, SEQ ID NO : 41, SEQ ID NO : 42, SEQ ID NO : 43, SEQ ID NO : 44, SEQ ID NO : 45, SEQ ID NO : 46, SEQ ID NO : 47 ou SEQ ID NO : 48,
une sonde dudit couple étant une sonde capture liée à au moins une molécule d'attache positionnée en 3' ou en 5' de sa séquence et l'autre sonde dudit couple étant une sonde signal liée à au moins une molécule de marquage positionnée en 3' ou en 5' de sa séquence,
ladite sonde capture et ladite sonde signal étant capables de s'hybrider avec l'ARN ribosomique d'une algue toxique du genre ***Dinophysis.***

11. Kit la revendication 9 ou 10, ledit kit comprenant en plus un support,
ledit support étant notamment choisi parmi le groupe consistant en : une microplaque, une lame de verre, des billes magnétiques, des électrodes imprimées en différente matières comme le carbone ou l'or,
de préférence une microplaque ou des billes magnétiques.

12. Procédé de détection de cellules vivantes actives d'algues toxiques dans un échantillon naturel susceptible de contenir au moins une algue toxique du genre *Alexandrium* selon l'une quelconque des revendications 5 ou 6, dans lequel :
▪ l'étape a) d'hybridation éventuelle comprend l'addition d'un complexe éventuel formé entre l'ARN ribosomique d'une algue toxique du genre *Alexandrium,* et une sonde signal sur un support contenant une sonde capture, et
▪ l'étape b) de détection de l'hybridation éventuelle du susdit complexe avec ladite sonde capture détecte l'hybridation ayant lieu entre la sonde capture et l'ARN ribosomique du susdit complexe.

13. Procédé de détection de cellules vivantes actives d'algues toxiques selon la revendication 12, dans un échantillon naturel susceptible de contenir en plus au moins une algue toxique du genre ***Dinophysis,*** comprenant, en plus, l'addition d'un complexe éventuel formé entre l'ARN ribosomique d'une algue toxique du genre ***Dinophysis*** et une sonde signal sur un support contenant une sonde capture,
ladite sonde capture et ladite sonde signal formant un couple de sondes, les séquences dudit couple de sondes étant choisies parmi x éléments de l'un des ensembles suivants :
- (SEQ ID NO : 29, SEQ ID NO : 30 ou SEQ ID NO : 31)
- (SEQ ID NO : 32 et SEQ ID NO : 33)
- (SEQ ID NO : 34 et SEQ ID NO : 35)
- (SEQ ID NO : 36 et SEQ ID NO : 37)
- (SEQ ID NO : 38 et SEQ ID NO : 39)
- (SEQ ID NO : 40, SEQ ID NO : 41 ou SEQ ID NO : 42)
- (SEQ ID NO : 43, SEQ ID NO : 44 ou SEQ ID NO : 45), ou
- (SEQ ID NO : 46, SEQ ID NO : 47 ou SEQ ID NO : 48)
x étant 2 ou 3,
ou les séquences desdites sondes ayant au moins 92% d'identité avec les susdites séquences SEQ ID NO : 29 à SEQ ID NO : 48,
l'hybridation indiquant la présence d'algue toxique du genre ***Dinophysis.***

## Patentansprüche

1. Verwendung mindestens eines Paares von Sonden, die für toxische Algen der Gattung *Alexandrium* spezifisch sind, zur Durchführung eines Verfahrens zum Nachweis lebender aktiver Zellen toxischer Algen in einer natürlichen Probe, die mindestens eine toxische Alge der Gattung *Alexandrium* enthalten kann, wobei dieses Verfahren ausgehend von dieser aus der natürlichen Probe extrahierten Gesamt-RNA durchgeführt wird, die mindestens eine toxische Alge der Gattung *Alexandrium* enthalten kann, und wobei die Sequenzen der Sonden dieser Paare Folgende sind:
- (SEQ ID NO: 1 und SEQ ID NO: 2),
- (SEQ ID NO: 4 und SEQ ID NO: 5),
- (SEQ ID NO: 11 und SEQ ID NO: 12), oder
- (SEQ ID NO: 17 und SEQ ID NO: 18),
wobei eine Sonde dieses Paares eine Fängersonde ist, die an mindestens ein Anheftungsmolekül gebunden ist, das 3' oder 5' ihrer Sequenz positioniert ist, und die andere Sonde dieses Paares eine Signalsonde ist, die an mindestens ein Markierungsmolekül gebunden ist, das 3' oder 5' ihrer Sequenz positioniert ist, wobei diese Fängersonde und diese Signalsonde in der Lage sind, mit der ribosomalen RNA einer toxischen Alge der Gattung *Alexandrium,* die möglicherweise in dieser Probe vorhanden ist, zu hybridisieren, um einen Komplex zu bilden.

2. Verwendung nach Anspruch 1, zusätzlich umfassend die Verwendung mindestens eines Paares von Sonden, die für toxische Algen der Gattung *Dinophysis* spezifisch sind, zur Durchführung eines Verfahrens zum Nachweis lebender aktiver Zellen toxischer Algen in einer natürlichen Probe, die mindestens eine toxische Alge der Gattung *Alexandrium* und/oder *Dinophysis* enthalten kann, wobei die Sequenzen dieser Sonden ausgewählt sind aus x Elementen einer der folgenden Gruppen:
- (SEQ ID NO: 29, SEQ ID NO: 30 oder SEQ ID NO: 31)
- (SEQ ID NO: 32 und SEQ ID NO: 33),
- (SEQ ID NO: 34 und SEQ ID NO: 35),
- (SEQ ID NO: 36 und SEQ ID NO: 37),
- (SEQ ID NO: 38 und SEQ ID NO: 39),
- (SEQ ID NO: 40, SEQ ID NO: 41 oder SEQ ID NO: 42),
- (SEQ ID NO: 43, SEQ ID NO: 44 oder SEQ ID NO: 45) oder
- (SEQ ID NO: 46, SEQ ID NO: 47 oder SEQ ID NO: 48),
wobei x für 2 oder 3 steht,
oder die Sequenzen dieser Sonden, die mindestens 92 % Identität mit den oben genannten Sequenzen SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 oder SEQ ID NO: 48 aufweisen,
wobei eine Sonde dieses Paares eine Fängersonde ist, die an mindestens ein Anheftungsmolekül gebunden ist, das 3' oder 5' ihrer Sequenz positioniert ist, und die andere Sonde dieses Paares eine Signalsonde ist, die an mindestens ein Markierungsmolekül gebunden ist, das 3' oder 5' ihrer Sequenz positioniert ist, wobei diese Fängersonde und diese Signalsonde in der Lage sind, mit der ribosomalen RNA einer toxischen Alge der Gattung ***Dinophysis,*** die möglicherweise in dieser Probe vorhanden ist, zu hybridisieren,
wobei die Sequenzen der Sonden dieser Paare insbesondere die Folgenden sind:
- (SEQ ID NO: 29 et SEQ ID NO: 30), (SEQ ID NO: 29 und SEQ ID NO: 31), (SEQ ID NO: 30 und SEQ ID NO: 31)
- (SEQ ID NO: 32 und SEQ ID NO: 33),
- (SEQ ID NO: 34 und SEQ ID NO: 35),
- (SEQ ID NO: 36 und SEQ ID NO: 37),
- (SEQ ID NO: 38 und SEQ ID NO: 39),
- (SEQ ID NO: 40 und SEQ ID NO: 41), (SEQ ID NO: 40 und SEQ ID NO: 42), (SEQ ID NO: 41 und SEQ ID NO: 42)
- (SEQ ID NO: 43 und SEQ ID NO: 44), (SEQ ID NO: 43 und SEQ ID NO: 45), (SEQ ID NO: 44 und SEQ ID NO: 45)
- (SEQ ID NO: 46 und SEQ ID NO: 47), (SEQ ID NO: 46 und SEQ ID NO: 48), (SEQ ID NO: 47 und SEQ ID NO: 48).

3. Verwendung nach Anspruch 1 oder 2, wobei
- diese Fängersonde an mindestens ein Anheftungsmolekül gebunden ist, das 5' ihrer Sequenz positioniert ist, und diese Signalsonde an mindestens ein Markierungsmolekül gebunden ist, das 5' ihrer Sequenz positioniert ist, oder
- diese Fängersonde an mindestens ein Anheftungsmolekül gebunden ist, das 5' ihrer Sequenz positioniert ist, und diese Signalsonde an mindestens ein Markierungsmolekül gebunden ist, das 3' ihrer Sequenz positioniert ist, oder
- diese Fängersonde an mindestens ein Anheftungsmolekül gebunden ist, das 3' ihrer Sequenz positioniert ist, und diese Signalsonde an mindestens ein Markierungsmolekül gebunden ist, das 5' ihrer Sequenz positioniert ist, oder
- diese Fängersonde an mindestens ein Anheftungsmolekül gebunden ist, das 3' ihrer Sequenz positioniert ist, und diese Signalsonde an mindestens ein Markierungsmolekül gebunden ist, das 3' ihrer Sequenz positioniert ist,
wobei dieses "mindestens eine Anheftungsmolekül" insbesondere ausgewählt ist aus einem Biotin-, Avidin-, Streptavidinmolekül, einer Thiolgruppe, einer Amingruppe oder einer Kohlenstoffgruppe, vorzugsweise einem Biotinmolekül,
wobei dieses "mindestens eine Markierungsmolekül" insbesondere ausgewählt ist aus:
- einem Fluorochrom,
- einem Biotin,
- einem Molekül, das an ein Biotin gebunden ist,
- dem Digoxigenin,
- einem Enzym, das ein chemilumineszierendes Substrat verwendet,
- einem Enzym, das ein chromogenes Substrat wie alkalische Phosphatase verwendet, wobei dieses chromogene Substrat Nitroblau-Tetrazolium (NBT) oder Bromchlorindolophosphat (BCIP) oder Meerrettichperoxidase (HRP) ist, wobei dieses chromogene Substrat ausgewählt ist aus 3,3'-Diaminobenzidin (DAB), 3,3',5,5'-Tetramethylbenzidin (TMB) oder 2,2'-Azino-bis(3-ethylbenzothiazolin-6-sulfonsäure) (ABTS), und
- einem Enzym, das ein elektrochemisches Oxidationssubstrat verwendet, vorzugsweise Digoxigenin.

4. Paar von Sonden zum Nachweis lebender aktiver Zellen toxischer Algen der Gattung *Alexandrium,* wobei die Sequenzen der Sonden dieser Paare Folgende sind:
- (SEQ ID NO: 1 und SEQ ID NO: 2),
- (SEQ ID NO: 4 und SEQ ID NO: 5),
- (SEQ ID NO: 11 und SEQ ID NO: 12), oder
- (SEQ ID NO: 17 und SEQ ID NO: 18).

5. Verfahren zum Nachweis lebender aktiver Zellen toxischer Algen in einer natürlichen Probe, die mindestens eine toxische Alge der Gattung *Alexandrium* enthalten kann, umfassend die folgenden Schritte:
a) mögliche Hybridisierung, die sich durch Inkontaktbringen der aus dieser Probe extrahierten Gesamt-RNA mit einer Fängersonde und einer Signalsonde ergibt, die für toxische Algen der Gattung *Alexandrium* spezifisch sind, wobei die Fängersonde und die Signalsonde ein Sondenpaar bilden und die Sequenzen der Sonden dieser Paare Folgende sind:
- (SEQ ID NO: 1 und SEQ ID NO: 2),
- (SEQ ID NO: 4 und SEQ ID NO: 5),
- (SEQ ID NO: 11 und SEQ ID NO: 12), oder
- (SEQ ID NO: 17 und SEQ ID NO: 18),
wobei diese Fängersonde und diese Signalsonde in der Lage sind, mit der ribosomalen RNA einer toxischen Alge der Gattung *Alexandrium,* die möglicherweise in dieser Probe vorhanden ist, zu hybridisieren, um einen Komplex zu bilden;
b) Nachweisen dieses möglichen Komplexes, der auf das Vorhandensein toxischer Algen der Gattung *Alexandrium* hinweist,
wobei die Dauer der Durchführung dieses Nachweisverfahrens insbesondere weniger als eine Stunde beträgt.

6. Verfahren zum Nachweis lebender aktiver Zellen toxischer Algen nach Anspruch 5 in einer natürlichen Probe, die mindestens eine toxische Alge der Gattung *Alexandrium* enthalten kann, wobei dieses Verfahren ferner umfasst:
• vor Schritt a) einen vorbereitenden Schritt der Extraktion der RNAs aus dieser zu analysierenden Probe,
• nach Schritt b) einen Schritt der Quantifizierung der toxischen Algen der Gattung *Alexandrium,* im Falle einer Hybridisierung in Schritt a),
wobei die Dauer der Durchführung der Schritte a) und b) weniger als eine Stunde beträgt.

7. Verfahren zum Nachweis lebender aktiver Zellen toxischer Algen nach einem der Ansprüche 5 oder 6 in einer natürlichen Probe, die zusätzlich mindestens eine toxische Alge der Gattung *Dinophysis* enthalten kann, umfassend zusätzlich zu dem Schritt der möglichen Hybridisierung, die sich aus dem Inkontaktbringen dieser Probe mit einer Fängersonde und einer Signalsonde ergibt, die für toxische Algen der Gattung *Alexandrium* spezifisch sind,
einen Schritt der möglichen Hybridisierung, die sich aus dem Inkontaktbringen dieser Probe mit einer Fängersonde und einer Signalsonde ergibt, die für toxische Algen der Gattung *Dinophysis* spezifisch sind, wobei die Fängersonde und die Signalsonde ein Sondenpaar bilden und die Sequenzen dieses Sondenpaares ausgewählt sind aus x Elementen einer der folgenden Gruppen:
- (SEQ ID NO: 29, SEQ ID NO: 30 oder SEQ ID NO: 31)
- (SEQ ID NO: 32 und SEQ ID NO: 33),
- (SEQ ID NO: 34 und SEQ ID NO: 35),
- (SEQ ID NO: 36 und SEQ ID NO: 37),
- (SEQ ID NO: 38 und SEQ ID NO: 39),
- (SEQ ID NO: 40, SEQ ID NO: 41 oder SEQ ID NO: 42)
- (SEQ ID NO: 43, SEQ ID NO: 44 oder SEQ ID NO: 45) oder
- (SEQ ID NO: 46, SEQ ID NO: 47 oder SEQ ID NO: 48)
wobei x für 2 oder 3 steht,
oder die Sequenzen dieser Sonden mindestens 92 % Identität mit den oben genannten Sequenzen SEQ ID NO: 29 bis SEQ ID NO: 48 aufweisen,
wobei die Hybridisierung auf das Vorhandensein von toxischen Algen der Gattung *Dinophysis* hinweist.

8. Nachweisverfahren nach einem der Ansprüche 5 bis 7, wobei:
- diese Fängersonde an mindestens ein Anheftungsmolekül gebunden ist, das 5' ihrer Sequenz positioniert ist, und diese Signalsonde an mindestens ein Markierungsmolekül gebunden ist, das 5' ihrer Sequenz positioniert ist, oder
- diese Fängersonde an mindestens ein Anheftungsmolekül gebunden ist, das 5' ihrer Sequenz positioniert ist, und diese Signalsonde an mindestens ein Markierungsmolekül gebunden ist, das 3' ihrer Sequenz positioniert ist, oder
- diese Fängersonde an mindestens ein Anheftungsmolekül gebunden ist, das 3' ihrer Sequenz positioniert ist, und diese Signalsonde an mindestens ein Markierungsmolekül gebunden ist, das 5' ihrer Sequenz positioniert ist, oder
- diese Fängersonde an mindestens ein Anheftungsmolekül gebunden ist, das 3' ihrer Sequenz positioniert ist, und diese Signal-Sonde an mindestens ein Markierungsmolekül gebunden ist, das 3' ihrer Sequenz positioniert ist,
wobei dieses "mindestens eine Anheftungsmolekül" insbesondere aus ausgewählt ist aus einem Biotin-, Avidin-, Streptavidinmolekül, einer Thiolgruppe, Amingruppe oder Kohlenstoffgruppe, vorzugsweise einem Biotinmolekül,
wobei dieses "mindestens eine Markierungsmolekül" insbesondere ausgewählt ist aus:
- einem Fluorochrom,
- einem Biotin,
- einem Molekül, das an ein Biotin gebunden ist,
- dem Digoxigenin,
- einem Enzym, das ein chemilumineszierendes Substrat verwendet,
- einem Enzym, das ein chromogenes Substrat wie alkalische Phosphatase verwendet, wobei dieses chromogene Substrat Nitroblau-Tetrazolium (NBT) oder Bromchlorindolophosphat (BCIP) oder Meerrettichperoxidase (HRP) ist, wobei dieses chromogene Substrat ausgewählt ist aus 3,3'-Diaminobenzidin (DAB), 3,3',5,5'-Tetramethylbenzidin (TMB) oder 2,2'-Azino-bis(3-ethylbenzothiazolin-6-sulfonsäure) (ABTS), oder
- einem Enzym, das ein elektrochemisches Oxidationssubstrat verwendet, vorzugsweise Digoxigenin.

9. Kit zum Nachweis lebender aktiver Zellen toxischer Algen der Gattung *Alexandrium,* wobei der Kit enthält:
a) mindestens ein Paar von Sonden, die für toxische Algen der Gattung *Alexandrium* spezifisch sind, wobei die Sequenzen der Sonden dieser Paare Folgende sind:
- (SEQ ID NO: 1 und SEQ ID NO: 2),
- (SEQ ID NO: 4 und SEQ ID NO: 5),
- (SEQ ID NO: 11 und SEQ ID NO: 12), oder
- (SEQ ID NO: 17 und SEQ ID NO: 18),
wobei eine Sonde dieses Paares eine Fängersonde ist, die an mindestens ein Anheftungsmolekül gebunden ist, das 3' oder 5' ihrer Sequenz positioniert ist, und die andere Sonde dieses Paares eine Signalsonde ist, die an mindestens ein Markierungsmolekül gebunden ist, das 3' oder 5' ihrer Sequenz positioniert ist,
wobei diese Fängersonde und diese Signalsonde in der Lage sind, mit der ribosomalen RNA einer toxischen Alge der Gattung *Alexandrium* zu hybridisieren,
b) gegebenenfalls eine Hybridisierungslösung,
c) gegebenenfalls eine Waschlösung und
d) gegebenenfalls eine Entwicklungslösung.

10. Kit nach Anspruch 9, wobei dieser Kit zusätzlich enthält:
a) mindestens ein Paar von Sonden, die für toxische Algen der Gattung *Dinophysis* spezifisch sind, wobei die Sequenzen dieser Sonden ausgewählt sind aus x Elementen einer der folgenden Gruppen:
- (SEQ ID NO: 29, SEQ ID NO: 30 oder SEQ ID NO: 31)
- (SEQ ID NO: 32 und SEQ ID NO: 33),
- (SEQ ID NO: 34 und SEQ ID NO: 35),
- (SEQ ID NO: 36 und SEQ ID NO: 37),
- (SEQ ID NO: 38 und SEQ ID NO: 39),
- (SEQ ID NO: 40, SEQ ID NO: 41 oder SEQ ID NO: 42)
- (SEQ ID NO: 43, SEQ ID NO: 44 oder SEQ ID NO: 45) oder
- (SEQ ID NO: 46, SEQ ID NO: 47 oder SEQ ID NO: 48)
wobei x für 2 oder 3 steht,
oder die Sequenzen dieser Sonden, die mindestens 92 % Identität mit den oben genannten Sequenzen SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 oder SEQ ID NO: 48 aufweisen,
wobei eine Sonde dieses Paares eine Fängersonde ist, die an mindestens ein Anheftungsmolekül gebunden ist, das 3' oder 5' ihrer Sequenz positioniert ist, und die andere Sonde dieses Paares eine Signalsonde ist, die an mindestens ein Markierungsmolekül gebunden ist, das 3' oder 5' ihrer Sequenz positioniert ist,
wobei diese Fängersonde und diese Signalsonde in der Lage sind, mit der ribosomalen RNA einer toxischen Alge der Gattung ***Dinophysis*** zu hybridisieren.

11. Kit nach Anspruch 9 oder 10, wobei dieser Kit zusätzlich einen Träger umfasst,
wobei dieser Träger insbesondere ausgewählt ist aus der Gruppe bestehend aus: einer Mikroplatte, einem Glasobjektträger, Magnetkügelchen, gedruckten Elektroden aus verschiedenen Materialien wie Kohlenstoff oder Gold,
vorzugsweise einer Mikroplatte oder Magnetkügelchen.

12. Verfahren zum Nachweis lebender aktiver Zellen toxischer Algen in einer natürlichen Probe, die mindestens eine toxische Alge der Gattung *Alexandrium* enthalten kann, nach einem der Ansprüche 5 oder 6, wobei:
• der Schritt a) zur möglichen Hybridisierung das Zugeben eines möglichen Komplexes umfasst, der zwischen der ribosomalen RNA einer toxischen Alge der Gattung *Alexandrium* und einer Signalsonde auf einem Träger, der eine Fängersonde enthält, gebildet wird, und
• der Schritt b) zum Nachweis der möglichen Hybridisierung des oben genannten Komplexes mit dieser Fängersonde die Hybridisierung zwischen der Fängersonde und der ribosomalen RNA des oben genannten Komplexes nachweist.

13. Verfahren zum Nachweis lebender aktiver Zellen toxischer Algen nach Anspruch 12 in einer natürlichen Probe, die zusätzlich mindestens eine toxische Alge der Gattung *Dinophysis* enthalten kann, umfassend zusätzlich das Zugeben eines möglichen Komplexes, der zwischen der ribosomalen RNA einer toxischen Alge der Gattung *Dinophysis* und einer Signalsonde auf einem Träger, der eine Fängersonde enthält, gebildet wird, wobei,
wobei diese Fängersonde und diese Signalsonde ein Sondenpaar bilden und die Sequenzen dieses Sondenpaares ausgewählt sind aus x Elementen einer der folgenden Gruppen:
- (SEQ ID NO: 29, SEQ ID NO: 30 oder SEQ ID NO: 31)
- (SEQ ID NO: 32 und SEQ ID NO: 33),
- (SEQ ID NO: 34 und SEQ ID NO: 35),
- (SEQ ID NO: 36 und SEQ ID NO: 37),
- (SEQ ID NO: 38 und SEQ ID NO: 39),
- (SEQ ID NO: 40, SEQ ID NO: 41 oder SEQ ID NO: 42)
- (SEQ ID NO: 43, SEQ ID NO: 44 oder SEQ ID NO: 45) oder
- (SEQ ID NO: 46, SEQ ID NO: 47 oder SEQ ID NO: 48)
wobei x für 2 oder 3 steht,
oder die Sequenzen dieser Sonden mindestens 92 % Identität mit den oben genannten Sequenzen SEQ ID NO: 29 bis SEQ ID NO: 48 aufweisen,
wobei die Hybridisierung auf das Vorhandensein von toxischen Algen der Gattung ***Dinophysis*** hinweist.

## Claims

1. Use of at least one pair of probes specific to toxic alga of the genus *Alexandrium* for the implementation of a method for the detection of active living cells of toxic alga in a natural sample likely to contain at least one toxic alga of the genus *Alexandrium,* said process being carried out using total RNA extracted from said natural sample likely to contain at least one toxic alga of the genus *Alexandrium,* and wherein the sequences of the probes of said pairs are as follows:
- (SEQ ID NO: 1 and SEQ ID NO: 2),
- (SEQ ID NO: 4 and SEQ ID NO: 5),
- (SEQ ID NO: 11 and SEQ ID NO: 12), or
- (SEQ ID NO: 17 and SEQ ID NO: 18),
one probe of said pair being a capture probe linked to at least one attachment molecule positioned at 3' or 5' of its sequence and the other probe of said pair being a signal probe linked to at least one marking molecule positioned at 3' or 5' of its sequence,
said capture probe and said signal probe being capable of hybridizing with the ribosomal nucleic acid of a toxic alga of the genus *Alexandrium* optionally present in said sample to form a complex.

2. Use according to claim 1 comprising in addition the use of at least one pair of probes specific to toxic alga of the genus *Dinophysis* for the implementation of a method for the detection of active living cells of toxic alga in a natural sample likely to contain at least one toxic alga of the genus *Alexandrium* and/or *Dinophysis,* the sequences of said probes being chosen from x elements of one of the following sets:
- (SEQ ID NO: 29, SEQ ID NO: 30 or SEQ ID NO: 31)
- (SEQ ID NO: 32 and SEQ ID NO: 33)
- (SEQ ID NO: 34 and SEQ ID NO: 35)
- (SEQ ID NO: 36 and SEQ ID NO: 37)
- (SEQ ID NO: 38 and SEQ ID NO: 39)
- (SEQ ID NO: 40, SEQ ID NO: 41 or SEQ ID NO: 42)
- (SEQ ID NO: 43, SEQ ID NO: 44 or SEQ ID NO: 45), or
- (SEQ ID NO: 46, SEQ ID NO: 47 or SEQ ID NO: 48)
x being 2 or 3,
or the sequences of said probes having at least 92% identity with the abovementioned sequences SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 or SEQ ID NO: 48,
one probe of said pair being a capture probe linked to at least one attachment molecule positioned at 3' or 5' of its sequence and the other probe of said pair being a signal probe linked to at least one marking molecule positioned at 3' or 5' of its sequence,
said capture probe and said signal probe being capable of hybridizing with the ribosomal nucleic acid of a toxic alga of the genus ***Dinophysis*** optionally present in said sample to form a complex,
in particular, the sequences of the probes of said pairs are as follows:
- (SEQ ID NO: 29 and SEQ ID NO: 30), (SEQ ID NO: 29 and SEQ ID NO: 31), (SEQ ID NO: 30 and SEQ ID NO: 31)
- (SEQ ID NO: 32 and SEQ ID NO: 33)
- (SEQ ID NO: 34 and SEQ ID NO: 35)
- (SEQ ID NO: 36 and SEQ ID NO: 37)
- (SEQ ID NO: 38 and SEQ ID NO: 39)
- (SEQ ID NO: 40 and SEQ ID NO: 41), (SEQ ID NO: 40 and SEQ ID NO: 42), (SEQ ID NO: 41 and SEQ ID NO: 42)
- (SEQ ID NO: 43 and SEQ ID NO: 44), (SEQ ID NO: 43 and SEQ ID NO: 45), (SEQ ID NO: 44 and SEQ ID NO: 45)
- (SEQ ID NO: 46 and SEQ ID NO: 47), (SEQ ID NO: 46 and SEQ ID NO: 48), (SEQ ID NO: 47 and SEQ ID NO: 48.

3. Use according to claim 1 or 2, wherein,
- said capture probe is linked to at least one attachment molecule positioned at 5' of its sequence and said signal probe is linked to at least one marker molecule positioned at 5' of its sequence, or
- said capture probe is linked to at least one attachment molecule positioned at 5' of its sequence and said signal probe is linked to at least one marker molecule positioned at 3' of its sequence, or
- said capture probe is linked to at least one attachment molecule positioned at 3' of its sequence and said signal probe is linked to at least one marker molecule positioned at 5' of its sequence, or
- said capture probe is linked to at least one attachment molecule positioned at 3' of its sequence and said signal probe is linked to at least one labelling molecule positioned at 3' of its sequence,
said "at least one attachment molecule" being in particular selected from a biotin, avidin, streptavidin molecule, a thiol group, an amine group and a carbon, preferably a biotin molecule,
said "at least one marking molecule" being in particular selected from:
- a fluorochrome,
- a biotin,
- a biotin-bound molecule,
- digoxigenin,
- an enzyme using a chemiluminescent substrate,
- an enzyme using a chromogenic substrate such as alkaline phosphatase, said chromogenic substrate can be Tetrazolium Nitroblue (NBT) or Bromochlorylindolophosphate (BCIP), said enzyme using a chromogenic substrate may be horseradish peroxidase (HRP) and said chromogenic substrate may be selected from 3,3'-Diaminobenzidine (DAB), 3,3',5,5'-Tetramethylbenzidine (TMB), or 2,2'-azino-bis(3-ethylbenzothiazoline-6-sulphonic acid) (ABTS), and
- an enzyme using an electrochemically oxidised substrate, preferably digoxigenin.

4. Pair of probes for the detection of active living cells of toxic alga of the genus *Alexandrium,* the sequences of which are as follows:
- (SEQ ID NO: 1 and SEQ ID NO: 2),
- (SEQ ID NO: 4 and SEQ ID NO: 5),
- (SEQ ID NO: 11 and SEQ ID NO: 12), or
- (SEQ ID NO: 17 and SEQ ID NO: 18).

5. Method for detecting active living cells of toxic alga in a natural sample likely to contain at least one toxic alga of the genus *Alexandrium* comprising the following steps:
a) optional hybridization resulting from the contact of the said sample with a capture probe and a signal probe specific to toxic alga of the genus ***Alexandrium,*** the capture probe and the signal probe forming a pair of probes, the sequences of the said pair of probes being as follows:
- (SEQ ID NO: 1 and SEQ ID NO: 2),
- (SEQ ID NO: 4 and SEQ ID NO: 5),
- (SEQ ID NO: 11 and SEQ ID NO: 12), or
- (SEQ ID NO: 17 and SEQ ID NO: 18),
said capture probe and said signal probe being capable of hybridizing with the ribosomal nucleic acid of a toxic alga of the genus *Alexandrium* optionally present in said sample to form a complex;
b) detection of said optional complex,
hybridization indicating the presence of toxic alga of the genus *Alexandrium,*
the duration of the implementation of said detection method being in particular less than one hour.

6. Method for detecting active living cells of toxic alga according to claim 5 in a natural sample likely to contain at least one toxic alga of the genus *Alexandrium,* said method further comprising:
▪ before the step a), a step of preparation of the said sample to be analysed in order to obtain a prepared sample,
▪ after step b), a step for the quantification of toxic alga of the genus *Alexandrium* in the case of hybridization in step a),
the duration of the implementation of steps (a) and (b) being less than one hour.

7. Method for detecting active living cells of toxic alga according to any of claims 5 to 6, in a natural sample likely to contain in addition at least one toxic alga of the genus *Dinophysis,* comprising in addition to the optional hybridization step resulting from bringing said sample into contact with a capture probe and a signal probe specific to toxic alga of the genus *Alexandrium,*
an optional hybridization step resulting from bringing said sample into contact with a capture probe and a signal probe specific to toxic alga of the genus *Dinophysis,* the capture probe and the signal probe forming a pair of probes, the sequences of said pair of probes being chosen from x elements of one of the following sets:
- (SEQ ID NO: 29, SEQ ID NO: 30 or SEQ ID NO: 31)
- (SEQ ID NO: 32 and SEQ ID NO: 33)
- (SEQ ID NO: 34 and SEQ ID NO: 35)
- (SEQ ID NO: 36 and SEQ ID NO: 37)
- (SEQ ID NO: 38 and SEQ ID NO: 39)
- (SEQ ID NO: 40, SEQ ID NO: 41 or SEQ ID NO: 42)
- (SEQ ID NO: 43, SEQ ID NO: 44 or SEQ ID NO: 45), or
- (SEQ ID NO: 46, SEQ ID NO: 47 or SEQ ID NO: 48)
x being 2 or 3,
or the sequences of said probes having at least 92% identity with said sequences SEQ ID NO: 29 to SEQ ID NO: 48.
Hybridization indicating the presence of toxic alga of the genus *Dinophysis.*

8. Method for detecting according to any of claims 5 to 7 wherein:
- said capture probe is linked to at least one attachment molecule positioned at 5' of its sequence and said signal probe is linked to at least one marker molecule positioned at 5' of its sequence, or
- said capture probe is linked to at least one attachment molecule positioned at 5' of its sequence and said signal probe is linked to at least one marker molecule positioned at 3' of its sequence, or
- said capture probe is linked to at least one attachment molecule positioned at 3' of its sequence and said signal probe is linked to at least one marker molecule positioned at 5' of its sequence, or
- said capture probe is linked to at least one attachment molecule positioned at 3' of its sequence and said signal probe is linked to at least one labelling molecule positioned at 3' of its sequence,
said "at least one attachment molecule" being in particular selected from a biotin, avidin, streptavidin molecule, a thiol group, an amine group and a carbon, preferably a biotin molecule,
said "at least one marking molecule" being in particular selected from:
- a fluorochrome,
- a biotin,
- a biotin-bound molecule,
- digoxigenin,
- an enzyme using a chemiluminescent substrate,
- an enzyme using a chromogenic substrate such as alkaline phosphatase, said chromogenic substrate can be Tetrazolium Nitroblue (NBT) or Bromochlorylindolophosphate (BCIP), said enzyme using a chromogenic substrate may be horseradish peroxidase (HRP) and said chromogenic substrate may be selected from 3,3'-Diaminobenzidine (DAB), 3,3',5,5'-Tetramethylbenzidine (TMB), or 2,2'-azino-bis(3-ethylbenzothiazoline-6-sulphonic acid) (ABTS), and
- an enzyme using an electrochemically oxidised substrate, preferably digoxigenin.

9. Kit for the detection of active living cells of toxic alga of toxic alga of the genus *Alexandrium,* said kit containing:
a) at least one pair of probes specific to toxic alga of the genus *Alexandrium,* the sequences of the probes of said pairs are as follows:
- (SEQ ID NO: 1 and SEQ ID NO: 2),
- (SEQ ID NO: 4 and SEQ ID NO: 5),
- (SEQ ID NO: 11 and SEQ ID NO: 12), or
- (SEQ ID NO: 17 and SEQ ID NO: 18),
one probe of said pair being a capture probe linked to at least one attachment molecule positioned at 3' or 5' of its sequence and the other probe of said pair being a signal probe linked to at least one marker molecule positioned at 3' or 5' of its sequence
said capture probe and said signal probe being capable of hybridizing with the ribosomal nucleic acid of a toxic alga of the genus *Alexandrium,*
b) optionally a hybridization solution,
c) optionally a washing solution, and
d) optionally a revelation solution.

10. Kit according to claim 9, said kit containing in **addition:**
a) at least one pair of probes specific to toxic alga of the genus ***Dinophysis,*** the sequences of said probes being selected from x elements of one of the following sets:
- (SEQ ID NO: 29, SEQ ID NO: 30 or SEQ ID NO: 31)
- (SEQ ID NO: 32 and SEQ ID NO: 33)
- (SEQ ID NO: 34 and SEQ ID NO: 35)
- (SEQ ID NO: 36 and SEQ ID NO: 37)
- (SEQ ID NO: 38 and SEQ ID NO: 39)
- (SEQ ID NO: 40, SEQ ID NO: 41 or SEQ ID NO: 42)
- (SEQ ID NO: 43, SEQ ID NO: 44 or SEQ ID NO: 45), or
- (SEQ ID NO: 46, SEQ ID NO: 47 or SEQ ID NO: 48)
x being 2 or 3,
or the sequences of said probes having at least 92% identity with the abovementioned sequences SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47 or SEQ ID NO: 48,
one probe of said pair being a capture probe linked to at least one attachment molecule positioned at 3' or 5' of its sequence and the other probe of said pair being a signal probe linked to at least one marker molecule positioned at 3' or 5' of its sequence
said capture probe and said signal probe being capable of hybridizing with the ribosomal nucleic acid of a toxic alga of the genus ***Dinophysis.***

11. Kit according to claim 9 or 10, said kit comprising in addition a support,
said support can be chosen from the group consisting of: a microplate, a glass slide, magnetic beads, electrodes printed in different materials such as carbon or gold,
preferably a microplate or magnetic balls.

12. Method for detecting active living cells of toxic alga in a natural sample likely to contain at least one toxic alga of the genus Alexandrium according to claim 5 or 6, wherein:
▪ said step a) of optional hybridization comprises the addition of a optional complex formed between ribosomal RNA from a toxic alga of the genus *Alexandrium* and a signal probe on a support containing a capture probe, and
▪ said step b) of detection of the optional hybridization of the aforementioned complex with the said capture probe detects the hybridization taking place between the capture probe and the ribosomal nucleic acid of the aforementioned complex.

13. Method for detecting active living cells of toxic alga according to claim 12, in a natural sample which may additionally contain at least one toxic alga of the genus ***Dinophysis,*** comprising, furthermore, the addition of an optional complex formed between the ribosomal nucleic acid of a toxic alga of the genus ***Dinophysis*** and a signal probe on a support containing a capture probe,
said capture probe and said signal probe forming a probe pair, the sequences of said probe pair being selected from x elements of one of the following sets:
- (SEQ ID NO: 29, SEQ ID NO: 30 or SEQ ID NO: 31)
- (SEQ ID NO: 32 and SEQ ID NO: 33)
- (SEQ ID NO: 34 and SEQ ID NO: 35)
- (SEQ ID NO: 36 and SEQ ID NO: 37)
- (SEQ ID NO: 38 and SEQ ID NO: 39)
- (SEQ ID NO: 40, SEQ ID NO: 41 or SEQ ID NO: 42)
- (SEQ ID NO: 43, SEQ ID NO: 44 or SEQ ID NO: 45), or
- (SEQ ID NO: 46, SEQ ID NO: 47 or SEQ ID NO: 48)
x being 2 or 3,
or the sequences of said probes having at least 92% identity with the abovementioned sequences SEQ ID NO: 29 to SEQ ID NO: 48,
hybridization indicating the presence of toxic alga of the genus ***Dinophysis.***
